# EUROPEAN PATENT APPLICATION

(11) **EP 4 349 815 A1**
(43) Date of publication of application: **10.04.2024**
(21) Application number: 22815198.1
(22) Date of filing: 30.05.2022
(51) Int. Cl.: C07D 215/28, C07D 401/04, A61K 31/47, A61K 31/4709, A61K 31/55, A61K 31/496, A61K 31/5377, A61P 35/00, A61P 31/00, A61P 13/00

(54) **7-NITRO-8-HYDROXYQUINOLINE DERIVATIVE, PREPARATION METHOD THEREFOR AND MEDICAL USE THEREOF**

(30) Priority: 31.05.2021 CN 202110602821
(71) Applicant: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: DENG, Yijun, Taizhou, Jiangsu 225316 (CN); WU, Liang, Taizhou, Jiangsu 225316 (CN); XIAO, Lu, Shanghai 201203 (CN)
(74) Representative: Regimbeau
(86) International application number: PCT/CN2022/095835
(87) International publication number: WO 2022/253152

(57) **Abstract**

A 7-nitro-8-hydroxyquinoline derivative, a preparation method therefor and the medical use thereof. Specifically, disclosed are a compound as shown in general formula (I), a preparation method therefor, a pharmaceutical composition containing same, and the use thereof in the treatment of infectious diseases or cancers. The compound has excellent antibacterial and antitumor activities and can be developed into a drug for treating infectious diseases or tumors.

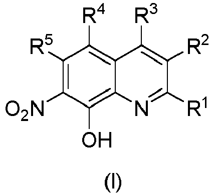

## Description

### FIELD OF THE INVENTION

The present invention relates to 7-nitro-8-hydroxyquinoline derivative, a method for preparing the same, and a medical use thereof.

### BACKGROUND OF THE INVENTION

Nitroxoline, the chemical name of which is 5-nitro-8-hydroxyquinoline, was developed as an oral antibiotic drug in the 1960s. It was mainly used for urinary system infections, and had a relatively safe history of use.

Moreover, in recent years, new studies have found that nitroxoline can simultaneously inhibit the methionine aminopeptidase MetAP2 and the silence information regulator 2-related enzyme SIRT1 in vascular endothelial cells, exerting a synergistic inhibitory effect on tumor angiogenesis, as well as an inhibitory effect on the proliferation of tumor cells. Therefore, nitroxoline has been re-developed to treat tumors including bladder cancer.

### SUMMARY OF THE INVENTION

The inventor has designed and synthesized a series of 7-nitro-8-hydroxyquinoline derivatives through intensive research. They show excellent antibacterial activity or antitumor activity, and can be developed as a drug for treating infectious disease or tumor.

Therefore, the present invention provides a compound of formula (I), or a tautomer, a stereoisomer, a mesomer, a racemate, a enantiomer, a diastereoisomer thereof, or a mixture thereof, or a pharmaceutically acceptable salt thereof: 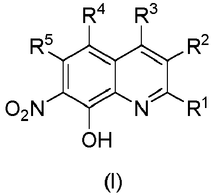 wherein:
R¹ is selected from the group consisting of H atom, D atom, alkyl, halogen, haloalkyl, hydroxyalkyl, alkoxy, haloalkoxy, hydroxy, cyano, amino, nitro, alkenyl, alkynyl, cycloalkyl, heterocyclyl, aryl and heteroaryl;
R² is selected from the group consisting of H atom, alkyl, cycloalkyl, aryl, -C(O)-OR⁶, -(CH₂)ₙ-NR⁷R⁸, -(CH₂)ₙ-NR⁹R¹⁰, D atom, haloalkyl, hydroxyalkyl, alkoxy, haloalkoxy, hydroxy, halogen, cyano, nitro, alkenyl, alkynyl, heterocyclyl, aryl and heteroaryl;
R³ is selected from the group consisting of H atom, D atom, alkyl, halogen, alkoxy, hydroxy, haloalkyl, haloalkoxy, cyano, cycloalkyl, -(CH₂)ₙ-heterocyclyl, aryl, heteroaryl, -NR⁷R⁸, -O-(CH₂)ₙ-NR⁷R⁸, -O-(CH₂)ₙ-R¹¹, -(CH=CH)ₘC(O)-NR⁷R⁸, -(CH=CH)ₘC(O)-NR⁹R¹⁰, nitro, alkenyl, alkynyl and hydroxyalkyl, wherein the cycloalkyl, heterocyclyl, aryl and heteroaryl are each optionally further substituted by one or more substituent(s) selected from the group consisting of alkyl, alkoxy, halogen, haloalkyl, oxo, -C(O)-NR⁷R⁸, -C(O)-OR⁶, -C(O)-R⁶, -S(O)ₓR₆, -O-(CH₂)ₙ-R¹¹, aryl, cycloalkyl, heteroaryl, hydroxyalkyl, hydroxy, cyano, amino, nitro, alkenyl and alkynyl;
R⁴ is selected from the group consisting of H atom, halogen, cyano, alkyl, cycloalkyl, haloalkyl, hydroxyalkyl, -(CH₂)ₙ-NR⁷R⁸, -(CH₂)ₙ-NR⁹R¹⁰, D atom, alkoxy, haloalkoxy, hydroxy, nitro, alkenyl, alkynyl, heterocyclyl, aryl and heteroaryl;
R⁵ is selected from the group consisting of H atom, D atom, halogen, alkyl, -NR⁷R⁸, haloalkyl, hydroxyalkyl, alkoxy, haloalkoxy, hydroxy, cyano, nitro, alkenyl, alkynyl, cycloalkyl, heterocyclyl, aryl and heteroaryl;
R⁶ is selected from the group consisting of alkyl, H atom, D atom, haloalkyl, hydroxyalkyl, alkoxy, haloalkoxy, hydroxy, halogen, cyano, amino, nitro, alkenyl, alkynyl, cycloalkyl, heterocyclyl, aryl and heteroaryl;
R⁷ and R⁸ are each independently selected from the group consisting of H atom, alkyl, heterocyclyl, D atom, haloalkyl, hydroxyalkyl, alkoxy, haloalkoxy, hydroxy, halogen, cyano, amino, nitro, alkenyl, alkynyl, cycloalkyl, aryl and heteroaryl;
R⁹ and R¹⁰ together with the nitrogen atom to which they are attached form a nitrogen-containing heterocyclyl, the nitrogen-containing heterocyclyl optionally comprises one or more heteroatom(s) selected from the group consisting of N, O and S in addition to N, the nitrogen-containing heterocyclyl is optionally further substituted by one or more substituent(s) selected from the group consisting of alkyl, haloalkyl, hydroxyalkyl, alkoxy, haloalkoxy, hydroxy, halogen, oxo, cyano, amino, nitro, alkenyl, alkynyl, cycloalkyl, heterocyclyl, aryl and heteroaryl;
R¹¹ is selected from the group consisting of halogen, cycloalkyl, aryl, haloalkyl, alkyl, alkoxy, hydroxyalkyl, haloalkoxy, hydroxy, cyano, amino, nitro, alkenyl, alkynyl, heterocyclyl and heteroaryl;
m is 0, 1, 2 or 3;
n is 0, 1, 2, 3, 4 or 5; and
x is 0, 1 or 2;
provided that the compound of formula (I) is not

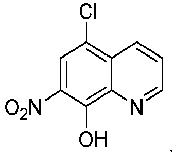

In some preferred embodiments, in the compound of formula (I), or or the tautomer, the stereoisomer, the mesomer, the racemate, the enantiomer, the diastereoisomer thereof, or the mixture thereof, or the pharmaceutically acceptable salt thereof,
R³ is selected from the group consisting of H atom, D atom, C₁₋₆ alkyl, halogen, C₁₋₆ alkoxy, hydroxy, haloC₁₋₆ alkyl, haloC₁₋₆ alkoxy, cyano, C₃₋₈ cycloalkyl, -(CH₂)ₙ-3 to 8 membered heterocyclyl, C₆₋₁₄ aryl, 5 to 14 membered heteroaryl, -NR⁷R⁸, -O-(CH₂)ₙ-NR⁷R⁸, -O-(CH₂)ₙ-R¹¹, -(CH=CH)ₘC(O)-NR⁷R⁸ and -(CH=CH)ₘC(O)-NR⁹R¹⁰, wherein the 3 to 8 membered heterocyclyl is optionally further substituted by one or more substituent(s) selected from the group consisting of C₁₋₆ alkyl, C₁₋₆ alkoxy, halogen, haloC₁₋₆ alkyl, oxo, -C(O)-NR⁷R⁸, -C(O)-OR⁶, -C(O)-R⁶, -S(O)ₓR₆, -O-(CH₂)ₙ-R¹¹ and C₆₋₁₄ aryl;
R⁶ is C₁₋₆ alkyl;
R⁷ and R⁸ are each independently selected from the group consisting of H atom, C₁₋₆ alkyl and 3 to 8 membered heterocyclyl;
R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N;
R¹¹ is selected from the group consisting of halogen, C₃₋₈ cycloalkyl, C₆₋₁₄ aryl, haloC₁₋₆ alkyl, C₁₋₆ alkyl and C₁₋₆ alkoxy;
m is 1;
n is 0, 1, 2 or 3; and
x is 2;
particularly, the 3 to 8 membered heterocyclyl and 3 to 8 membered nitrogen-containing heterocyclyl are selected from the group consisting of pyrrolidinyl, morpholinyl, piperazinyl, oxetanyl, piperidinyl, 3-pyrrolinyl and 6-azaspiro[2.5]octyl; the 5 to 14 membered heteroaryl is imidazolyl;
preferably, R³ is selected from the group consisting of H atom, -CH₃, 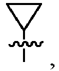 Cl atom, 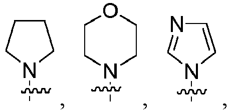 Br atom, 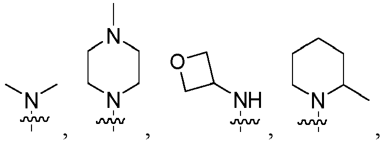 -OCH₃, 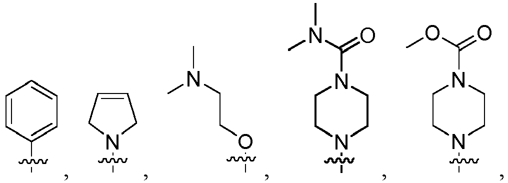 F atom, 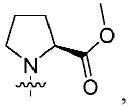 -OH, 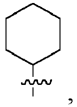 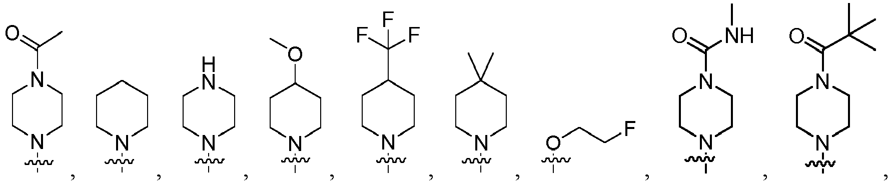 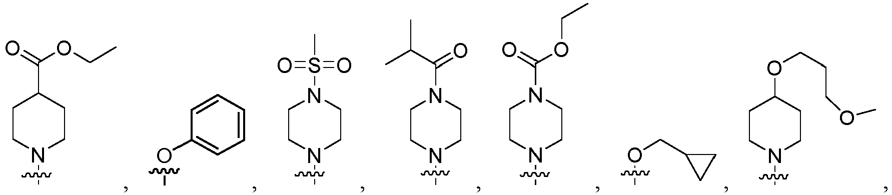 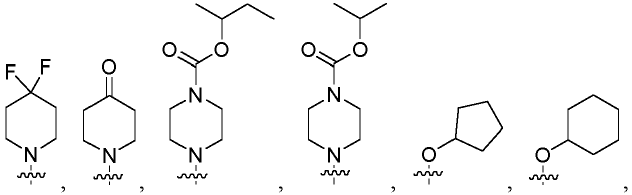 -OCH₂CH₃, -CF₃, 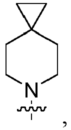 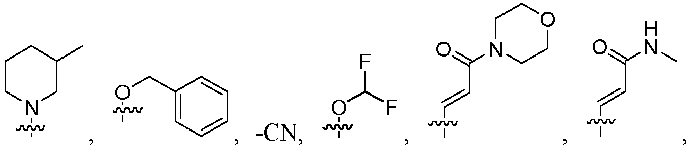 -CHF₂, 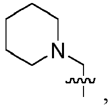 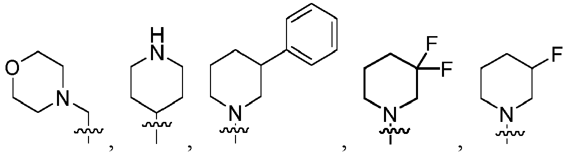 and D atom.

In some preferred embodiments, in the compound of formula (I), or or the tautomer, the stereoisomer, the mesomer, the racemate, the enantiomer, the diastereoisomer thereof, or the mixture thereof, or the pharmaceutically acceptable salt thereof,
R⁴ is selected from the group consisting of H atom, halogen, cyano, C₁₋₆ alkyl, C₃₋₈ cycloalkyl, haloC₁₋₆ alkyl, hydroxyC₁₋₆ alkyl, -(CH₂)ₙ-NR⁷R⁸ and -(CH₂)ₙ-NR⁹R¹⁰;
R⁷ and R⁸ are each independently H atom or C₁₋₆ alkyl;
R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N; particularly, R⁹ and R¹⁰ together with the nitrogen atom to which they are attached form a morpholinyl, piperidinyl, pyrrolidinyl or piperazinyl;
preferably, R⁴ is selected from the group consisting of H atom, I atom, -CH₃, Cl atom, -CN, F atom, 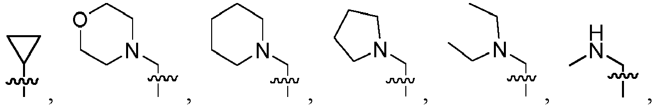 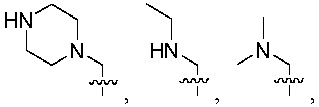 -CHF₂, -CH₂OH and Br atom.

In some preferred embodiments, in the compound of formula (I), or or the tautomer, the stereoisomer, the mesomer, the racemate, the enantiomer, the diastereoisomer thereof, or the mixture thereof, or the pharmaceutically acceptable salt thereof,
R² is selected from the group consisting of H atom, C₁₋₆ alkyl, C₃₋₈ cycloalkyl, C₆₋₁₄ aryl, -C(O)-OR⁶, -(CH₂)ₙ-NR⁷R⁸ and -(CH₂)ₙ-NR⁹R¹⁰;
R⁶ is C₁₋₆ alkyl;
R⁷ and R⁸ are each independently H atom or C₁₋₆ alkyl;
R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N, the 3 to 8 membered nitrogen-containing heterocyclyl is optionally further substituted by one or more oxo;
particularly, the 3 to 8 membered nitrogen-containing heterocyclyl is selected from the group consisting of piperidinyl, morpholinyl and piperazinyl optionally substituted by oxo;
preferably, R² is selected from the group consisting of H atom, -CH₃, 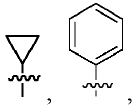 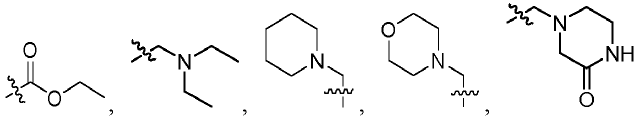 and

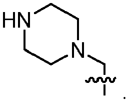

In some preferred embodiments, in the compound of formula (I), or or the tautomer, the stereoisomer, the mesomer, the racemate, the enantiomer, the diastereoisomer thereof, or the mixture thereof, or the pharmaceutically acceptable salt thereof,
R⁵ is selected from the group consisting of H atom, D atom, halogen, C₁₋₆ alkyl and -NR⁷R⁸; R⁷ is H atom, and R⁸ is C₁₋₆ alkyl;
preferably, R⁵ is selected from the group consisting of H atom, -CH₃, F atom, Br atom, Cl atom, D atom and -NHCH₃.

In some preferred embodiments, in the compound of formula (I), or or the tautomer, the stereoisomer, the mesomer, the racemate, the enantiomer, the diastereoisomer thereof, or the mixture thereof, or the pharmaceutically acceptable salt thereof,
R¹ is selected from the group consisting of H atom, halogen, D atom and C₁₋₆ alkyl;
preferably, R¹ is selected from the group consisting of H atom, F atom, -CH₃ and D atom.

In some preferred embodiments, the compound of formula (I), or or the tautomer, the stereoisomer, the mesomer, the racemate, the enantiomer, the diastereoisomer thereof, or the mixture thereof, or the pharmaceutically acceptable salt thereof is a compound of formula (II), or a tautomer, a stereoisomer, a mesomer, a racemate, a enantiomer, a diastereoisomer thereof, or a mixture thereof, or a pharmaceutically acceptable salt thereof: 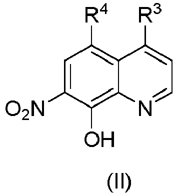
R³ is selected from the group consisting of haloC₁₋₆ alkyl, C₃₋₈ cycloalkyl, 3 to 8 membered heterocyclyl, C₆₋₁₄ aryl and -O-(CH₂)ₙ-R¹¹, wherein the 3 to 8 membered heterocyclyl is optionally further substituted by one or more substituent(s) selected from the group consisting of C₁₋₆ alkyl, C₁₋₆ alkoxy, halogen, haloC₁₋₆ alkyl, -C(O)-OR⁶ and -O-(CH₂)ₙ-R¹¹; R⁶ is C₁₋₆ alkyl; R¹¹ is selected from the group consisting of C₃₋₈ cycloalkyl and C₁₋₆ alkoxy; and n is 0, 1, 2, 3 or 4; particularly, the 3 to 8 membered heterocyclyl is piperazinyl or piperidinyl; preferably, R³ is selected from the group consisting of 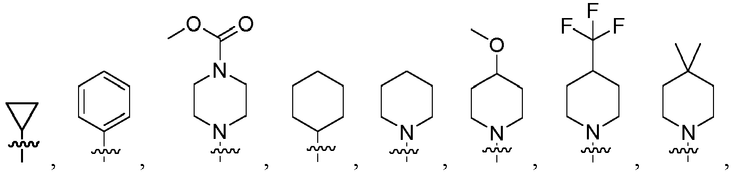 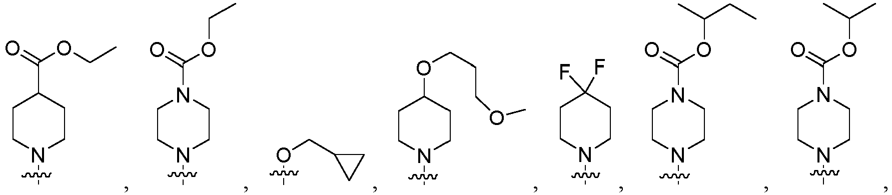 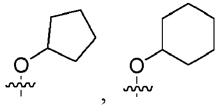 and -CF₃; and
R⁴ is H atom or halogen; preferably, R⁴ is H atom, Cl atom or F atom.

In some preferred embodiments, in the compound of formula (I), or or the tautomer, the stereoisomer, the mesomer, the racemate, the enantiomer, the diastereoisomer thereof, or the mixture thereof, or the pharmaceutically acceptable salt thereof,
R¹ is selected from the group consisting of H atom, halogen, D atom and C₁₋₆ alkyl; preferably, R¹ is selected from the group consisting of H atom, F atom, -CH₃ and D atom;
R² is selected from the group consisting of H atom, C₁₋₆ alkyl, C₃₋₈ cycloalkyl, -CH₂-NR⁷R⁸ and -CH₂-NR⁹R¹⁰; R⁷ and R⁸ are each independently H atom or C₁₋₆ alkyl; R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N; particularly, R⁹ and R¹⁰ together with the nitrogen atom to which they are attached form a piperidinyl; preferably, R² is selected from the group consisting of H atom, -CH₃, 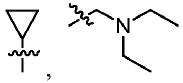 and 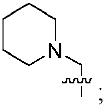
R³ is selected from the group consisting of H atom, D atom, C₁₋₆ alkyl, halogen, C₁₋₆ alkoxy, haloC₁₋₆ alkyl, haloC₁₋₆ alkoxy, cyano, C₃₋₈ cycloalkyl, -(CH₂)ₙ-3 to 8 membered heterocyclyl and -NR⁷R⁸, wherein the 3 to 8 membered heterocyclyl is optionally further substituted by one or more substituent(s) selected from the group consisting of C₁₋₆ alkyl, -C(O)-NR⁷R⁸ and -O-(CH₂)ₙ-R¹¹; R⁷ and R⁸ are each independently C₁₋₆ alkyl; R¹¹ is C₁₋₆ alkoxy; n is 0, 1, 2, 3 or 4; particularly, the 3 to 8 membered heterocyclyl is piperazinyl or piperidinyl; preferably, R³ is selected from the group consisting of H atom, -CH₃, 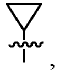 Cl atom, Br atom, 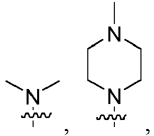 -OCH₃, 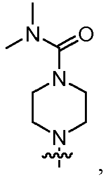 F atom, 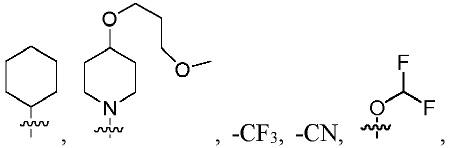 -CHF₂, 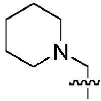 and D atom;
R⁴ is selected from the group consisting of H atom, halogen, C₃₋₈ cycloalkyl, hydroxyC₁₋₆ alkyl and -CH₂-NR⁷R⁸; R⁷ and R⁸ are each independently C₁₋₆ alkyl; preferably, R⁴ is selected from the group consisting of H atom, Cl atom, F atom, 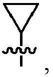 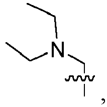 -CH₂OH and Br atom; and
R⁵ is selected from the group consisting of H atom, D atom, halogen, C₁₋₆ alkyl and -NR⁷R⁸; R⁷ is H atom, and R⁸ is C₁₋₆ alkyl; preferably, R⁵ is selected from the group consisting of H atom, -CH₃, F atom, Br atom, Cl atom, D atom and -NHCH₃;
particularly,
R¹ is H atom or D atom;
R² is selected from the group consisting of H atom, C₁₋₆ alkyl, C₃₋₈ cycloalkyl, -CH₂-NR⁷R⁸ and -CH₂-NR⁹R¹⁰; R⁷ and R⁸ are each independently H atom or C₁₋₆ alkyl; R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N; particularly, R⁹ and R¹⁰ together with the nitrogen atom to which they are attached form a piperidinyl; preferably, R² is selected from the group consisting of H atom, -CH₃, 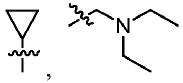 and 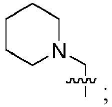
R³ is selected from the group consisting of H atom, D atom, halogen, C₁₋₆ alkoxy, haloC₁₋₆ alkyl, haloC₁₋₆ alkoxy, cyano, C₃₋₈ cycloalkyl and -(CH₂)ₙ-3 to 8 membered heterocyclyl, wherein the 3 to 8 membered heterocyclyl is optionally further substituted by one or more substituent(s) selected from the group consisting of C₁₋₆ alkyl, -C(O)-NR⁷R⁸ and -O-(CH₂)ₙ-R¹¹; R⁷ and R⁸ are each independently C₁₋₆ alkyl; R¹¹ is C₁₋₆ alkoxy; n is 0, 1, 2, 3 or 4; particularly, the 3 to 8 membered heterocyclyl is piperazinyl or piperidinyl; preferably, R³ is selected from the group consisting of H atom, Cl atom, Br atom, 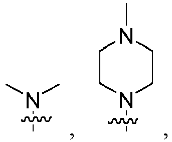 -OCH₃, 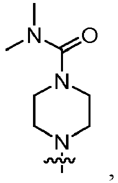 F atom, 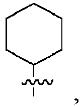 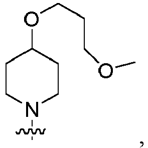 -CF₃, -CN, 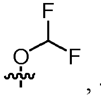 -CHF₂, 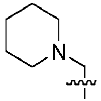 and D atom;
R⁴ is selected from the group consisting of H atom, halogen, C₃₋₈ cycloalkyl, hydroxyC₁₋₆ alkyl and -CH₂-NR⁷R⁸; R⁷ and R⁸ are each independently C₁₋₆ alkyl; preferably, R⁴ is selected from the group consisting of H atom, Cl atom, F atom, 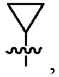 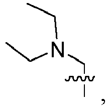 -CH₂OH and Br atom; and
R⁵ is selected from the group consisting of H atom, D atom, halogen, C₁₋₆ alkyl and -NR⁷R⁸; R⁷ is H atom, and R⁸ is C₁₋₆ alkyl; preferably, R⁵ is selected from the group consisting of H atom, -CH₃, F atom, Br atom, Cl atom, D atom and -NHCH₃;
more particularly,
R¹ is H atom;
R² is H atom;
R³ is halogen or haloC₁₋₆ alkyl; preferably, R³ is Cl atom or -CF₃;
R⁴ is H atom or halogen; preferably, R⁴ is H atom or Cl atom; and
R⁵ is H atom.

In some preferred embodiments, in the compound of formula (I), or or the tautomer, the stereoisomer, the mesomer, the racemate, the enantiomer, the diastereoisomer thereof, or the mixture thereof, or the pharmaceutically acceptable salt thereof,
R¹ is H atom or D atom;
R² is selected from the group consisting of H atom, C₁₋₆ alkyl, C₃₋₈ cycloalkyl, -CH₂-NR⁷R⁸ and -CH₂-NR⁹R¹⁰; R⁷ and R⁸ are each independently C₁₋₆ alkyl; R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N; particularly, the 3 to 8 membered nitrogen-containing heterocyclyl is piperidinyl; preferably, R² is selected from the group consisting of H atom, -CH₃, 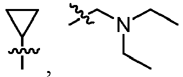 and 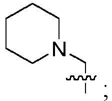
R³ is selected from the group consisting of H atom, D atom, C₁₋₆ alkyl, halogen, C₁₋₆ alkoxy, haloC₁₋₆ alkyl, haloC₁₋₆ alkoxy, cyano, C₃₋₈ cycloalkyl, -(CH₂)ₙ-3 to 8 membered heterocyclyl and -O-(CH₂)ₙ-R¹¹, wherein the 3 to 8 membered heterocyclyl is optionally further substituted by one or more -C(O)-OR⁶ or -O-(CH₂)ₙ-R¹¹; R⁶ is C₁₋₆ alkyl; R¹¹ is C₃₋₈ cycloalkyl or C₁₋₆ alkoxy; n is 0, 1, 2, 3 or 4; particularly, the 3 to 8 membered heterocyclyl is selected from the group consisting of pyrrolidinyl, piperazinyl and piperidinyl; preferably, R³ is selected from the group consisting of H atom, -CH₃, 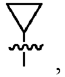 Cl atom, Br atom, -OCH₃, 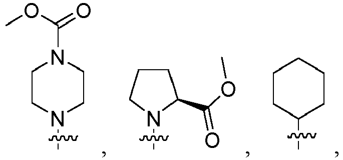 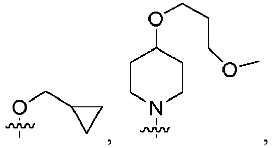 -CF₃, -CN, 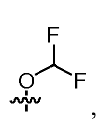 -CHF₂, 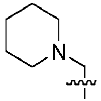 and D atom;
R⁴ is selected from the group consisting of H atom, halogen, C₁₋₆ alkyl, C₃₋₈ cycloalkyl and -CH₂-NR⁷R⁸; R⁷ and R⁸ are each independently C₁₋₆ alkyl; preferably, R⁴ is selected from the group consisting of H atom, -CH₃, Cl atom, F atom, 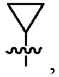 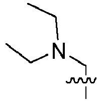 and Br atom; and
R⁵ is selected from the group consisting of H atom, D atom, halogen, C₁₋₆ alkyl and -NR⁷R⁸; R⁷ is H atom, and R⁸ is C₁₋₆ alkyl; preferably, R⁵ is selected from the group consisting of H atom, -CH₃, F atom, Br atom, Cl atom, D atom and -NHCH₃;
particularly,
R¹ is H atom or D atom;
R² is selected from the group consisting of H atom, C₁₋₆ alkyl, C₃₋₈ cycloalkyl, -CH₂-NR⁷R⁸ and -CH₂-NR⁹R¹⁰; R⁷ and R⁸ are each independently C₁₋₆ alkyl; R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N; particularly, the 3 to 8 membered nitrogen-containing heterocyclyl is piperidinyl; preferably, R² is selected from the group consisting of H atom, -CH₃, 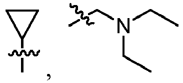 and 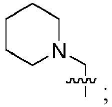
R³ is selected from the group consisting of H atom, D atom, halogen, C₁₋₆ alkoxy, haloC₁₋₆ alkyl, haloC₁₋₆ alkoxy, cyano, C₃₋₈ cycloalkyl, -(CH₂)ₙ-3 to 8 membered heterocyclyl and -O-(CH₂)ₙ-R¹¹, wherein the 3 to 8 membered heterocyclyl is optionally further substituted by one or more -O-(CH₂)ₙ-R¹¹; R¹¹ is C₃₋₈ cycloalkyl or C₁₋₆ alkoxy; n is 0, 1, 2, 3 or 4; particularly, the 3 to 8 membered heterocyclyl is piperidinyl; preferably, R³ is selected from the group consisting of H atom, Cl atom, Br atom, -OCH₃, 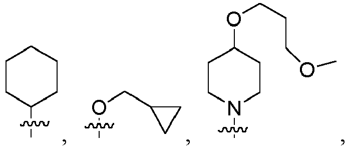 -CF₃, -CN, 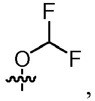 -CHF₂, 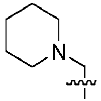 and D atom;
R⁴ is selected from the group consisting of H atom, halogen and C₃₋₈ cycloalkyl; preferably, R⁴ is selected from the group consisting of H atom, Cl atom, F atom, 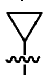 and Br atom; and
R⁵ is selected from the group consisting of H atom, D atom, halogen and -NR⁷R⁸; R⁷ is H atom, and R⁸ is C₁₋₆ alkyl; preferably, R⁵ is selected from the group consisting of H atom, F atom, Br atom, Cl atom, D atom and -NHCH₃;
more particularly,
R¹ is H atom;
R² is H atom;
R³ is halogen or haloC₁₋₆ alkyl; preferably, R³ is Cl atom or -CF₃;
R⁴ is halogen; preferably, R⁴ is Cl atom or F atom; and
R⁵ is H atom.

In some preferred embodiments, in the compound of formula (I), or or the tautomer, the stereoisomer, the mesomer, the racemate, the enantiomer, the diastereoisomer thereof, or the mixture thereof, or the pharmaceutically acceptable salt thereof,
R¹ is H atom;
R² is H atom or C₁₋₆ alkyl; preferably, R² is H atom or -CH₃;
R³ is H atom or C₃₋₈ cycloalkyl; preferably, R³ is selected from the group consisting of H atom and 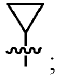
R⁴ is H atom or cyano; and
R⁵ is H atom or C₁₋₆ alkyl; preferably, R⁵ is H atom or -CH₃.

In some preferred embodiments, in the compound of formula (I), or or the tautomer, the stereoisomer, the mesomer, the racemate, the enantiomer, the diastereoisomer thereof, or the mixture thereof, or the pharmaceutically acceptable salt thereof,
R¹ is H atom or D atom;
R² is selected from the group consisting of H atom, C₁₋₆ alkyl, C₃₋₈ cycloalkyl, C₆₋₁₄ aryl and -C(O)-OR⁶; R⁶ is C₁₋₆ alkyl; preferably, R² is selected from the group consisting of H atom, -CH₃, 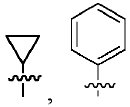 and 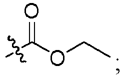
R³ is selected from the group consisting of H atom, D atom, halogen, C₁₋₆ alkoxy, haloC₁₋₆ alkyl, C₃₋₈ cycloalkyl, 3 to 8 membered heterocyclyl, C₆₋₁₄ aryl and -O-CH₂-R¹¹, wherein the 3 to 8 membered heterocyclyl is optionally further substituted by one or more substituent(s) selected from the group consisting of C₁₋₆ alkyl, halogen, haloC₁₋₆ alkyl and -C(O)-OR⁶; R⁶ is C₁₋₆ alkyl; R¹¹ is C₃₋₈ cycloalkyl or C₆₋₁₄ aryl; particularly, the 3 to 8 membered heterocyclyl is piperidinyl; preferably, R³ is selected from the group consisting of H atom, 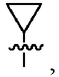 Cl atom, -OCH₃, 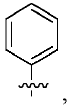 F atom, 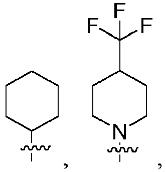 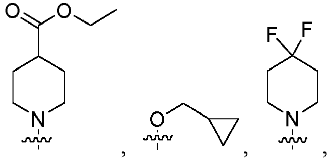 -CF₃, 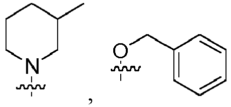 and D atom;
R⁴ is selected from the group consisting of H atom, halogen and C₃₋₈ cycloalkyl; preferably, R⁴ is selected from the group consisting of H atom, Cl atom, F atom, 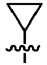 and Br atom;
R⁵ is selected from the group consisting of H atom, D atom, halogen and C₁₋₆ alkyl; preferably, R⁵ is selected from the group consisting of H atom, -CH₃, F atom, Br atom, Cl atom and D atom;
particularly,
R¹ is H atom or D atom;
R² is selected from the group consisting of H atom, C₁₋₆ alkyl, C₃₋₈ cycloalkyl and C₆₋₁₄ aryl; preferably, R² is selected from the group consisting of H atom, -CH₃, 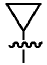 and 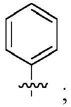
R³ is selected from the group consisting of H atom, halogen, C₁₋₆ alkoxy, haloC₁₋₆ alkyl, C₃₋₈ cycloalkyl, 3 to 8 membered heterocyclyl, C₆₋₁₄ aryl and -O-CH₂-R¹¹, wherein the 3 to 8 membered heterocyclyl is optionally further substituted by one or more substituent(s) selected from the group consisting of C₁₋₆ alkyl, halogen and haloC₁₋₆ alkyl; R¹¹ is C₃₋₈ cycloalkyl or C₆₋₁₄ aryl; particularly, the 3 to 8 membered heterocyclyl is piperidinyl; preferably, R³ is selected from the group consisting of H atom, 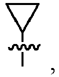 Cl atom, -OCH₃, 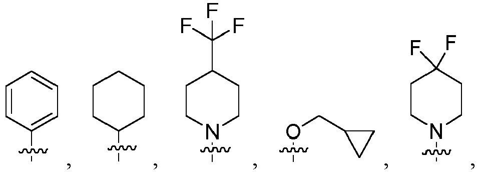 -CF₃, 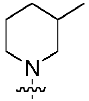 and 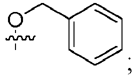
R⁴ is selected from the group consisting of H atom, halogen and C₃₋₈ cycloalkyl; preferably, R⁴ is selected from the group consisting of H atom, Cl atom, F atom and 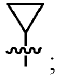
R⁵ is selected from the group consisting of H atom, D atom and halogen; preferably, R⁵ is selected from the group consisting of H atom, F atom, Br atom, Cl atom and D atom;
more particularly,
R¹ is H atom;
R² is selected from the group consisting of H atom, C₃₋₈ cycloalkyl and C₆₋₁₄ aryl; preferably, R² is selected from the group consisting of H atom, 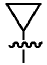 and 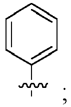
R³ is selected from the group consisting of H atom, halogen, haloC₁₋₆ alkyl, C₃₋₈ cycloalkyl, 3 to 8 membered heterocyclyl, C₆₋₁₄ aryl and -O-CH₂-R¹¹, wherein the 3 to 8 membered heterocyclyl is optionally further substituted by one or more C₁₋₆ alkyl; R¹¹ is C₃₋₈ cycloalkyl or C₆₋₁₄ aryl; particularly, the 3 to 8 membered heterocyclyl is piperidinyl; preferably, R³ is selected from the group consisting of H atom, Cl atom, 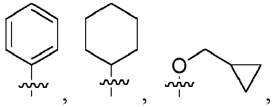 -CF₃, 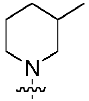 and 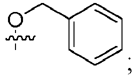
R⁴ is H atom or halogen; preferably, R⁴ is H atom, Cl atom or F atom;
R⁵ is H atom or halogen; preferably, R⁵ is H atom, Br atom or Cl atom.

In some preferred embodiments, the compound of formula (I), or or the tautomer, the stereoisomer, the mesomer, the racemate, the enantiomer, the diastereoisomer thereof, or the mixture thereof, or the pharmaceutically acceptable salt thereof is a compound of formula (III), or a tautomer, a stereoisomer, a mesomer, a racemate, a enantiomer, a diastereoisomer thereof, or a mixture thereof, or a pharmaceutically acceptable salt thereof: 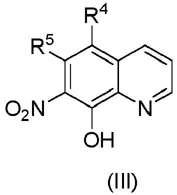
preferably, R⁴ is halogen, preferably Cl atom or F atom; and
R⁵ is halogen, preferably F atom, Cl atom or Br atom.

Typical compounds of formula (I) of the present invention include, but are not limited to the following compounds:

| Compound No. | Structure and name of compound |
|---|---|
| 1 | 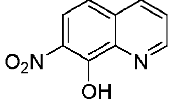 |
| | 7-Nitroquinolin-8-ol |
| 2 | 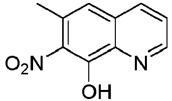 |
| | 6-Methyl-7-nitroquinolin-8-ol |
| 3 | 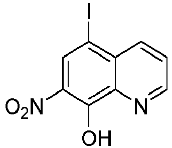 |
| | 5-Iodo-7-nitroquinolin-8-ol |
| 4 | 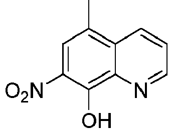 |
| | 5-Methyl-7-nitroquinolin-8-ol |
| 5 | 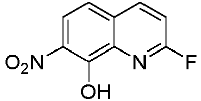 |
| | 2-Fluoro-7-nitroquinolin-8 -ol |
| 6 | 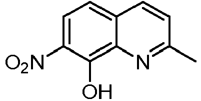 |
| | 2-Methyl-7-nitroquinolin-8-ol |
| 7 | 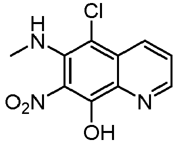 |
| | 5-Chloro-6-(methylamino)-7-nitroquinolin-8-ol |
| 8 | 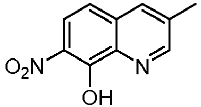 |
| | 3 -Methyl -7-nitroquinolin-8 -ol |
| 9 | 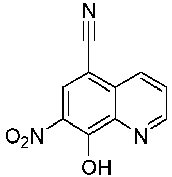 |
| | 8-Hydroxy-7-nitroquinoline-5-carbonitrile |
| 10 | 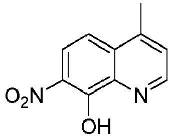 |
| | 4-Methyl-7-nitroquinolin-8-ol |
| 11 | 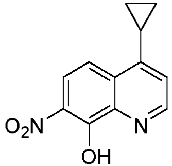 |
| | 4-Cyclopropyl-7-nitroquinolin-8-ol |
| 12 | 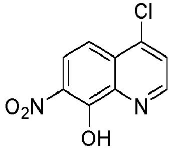 |
| | 4-Chloro-7-nitroquinolin-8-ol |
| 13 | 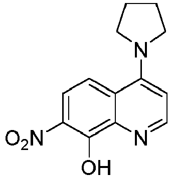 |
| | 7-Nitro-4-(pyrrolidin-1-yl)quinolin-8-ol |
| 14 | 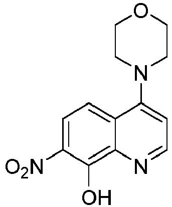 |
| | 4-Morpholino-7-nitroquinolin-8-ol |
| 15 | 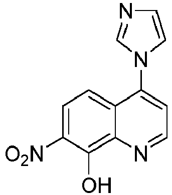 |
| | 4-(*1H*-Imidazol-1-yl)-7-nitroquinolin-8-ol |
| 16 | 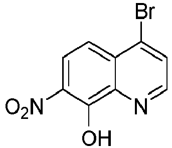 |
| | 4-Bromo-7-nitroquinolin-8-ol |
| 17 | 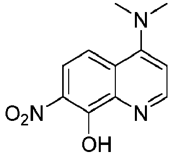 |
| | 4-(Dimethylamino)-7-nitroquinolin-8-ol |
| 18 | 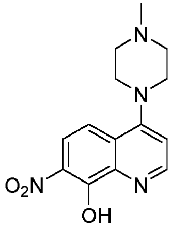 |
| | 4-(4-Methylpiperazinyl)-7-nitroquinolin-8-ol |
| 19 | 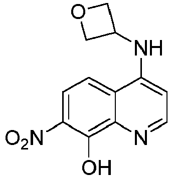 |
| | 7-Nitro-4-(3-oxetanylamino)quinolin-8-ol |
| 20 | 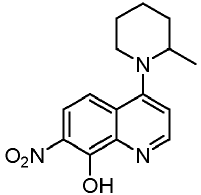 |
| | 4-(2-Methylpiperidinyl)-7-nitroquinolin-8-ol |
| 21 | 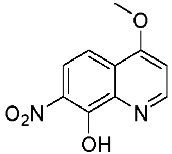 |
| | 4-Methoxy-7-nitroquinolin-8-ol |
| 22 | 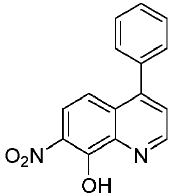 |
| | 4-Phenyl-7-nitroquinolin-8-ol |
| 23 | 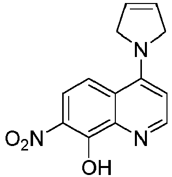 |
| | 4-(2,5-Dihydro-1*H*-pyrrolyl)-7-nitroquinolin-8-ol |
| 24 | 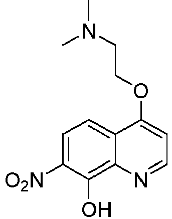 |
| | 4-(2-(Dimethylamino)ethoxy)-7-nitroquinolin-8-ol |
| 25 | 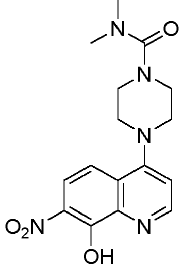 |
| | 4-(8-Hydroxy-7-nitroquinolin-4-yl)-N,N-dimethylpiperazine-1-carboxamide |
| 26 | 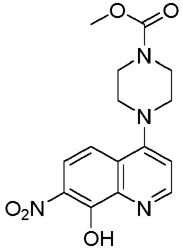 |
| | Methyl 4-(8-hydroxy-7-nitroquinolin-4-yl)-N,N-dimethylpiperazine-1 -carboxylate |
| 27 | 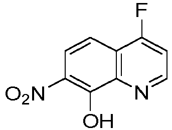 |
| | 4-Fluoro-7-nitroquinolin-8 -ol |
| 28 | 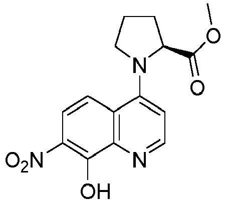 |
| | Methyl (8-hydroxy-7-nitroquinolin-4-yl)-L-prolinate |
| 29 | 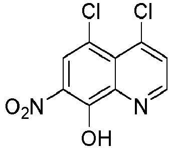 |
| | 4,5-Dichloro-7-nitroquinolin-8-ol |
| 30 | 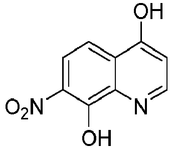 |
| | 7-Nitroquinoline-4, 8-diol |
| 31 | 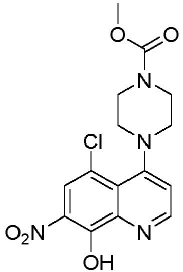 |
| | Methyl 4-(5-chloro-8-hydroxy-7-nitroquinolin-4-yl)-piperazine-1-car boxylate |
| 32 | 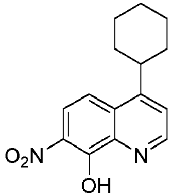 |
| | 4-Cyclohexyl-7-nitroquinolin-8-ol |
| 33 | 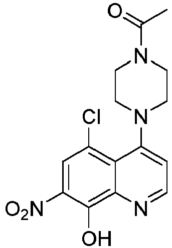 |
| | 4-(5-Chloro-8-hydroxy-7-nitroquinolin-4-yl)-1-acetylpiperazi ne |
| 34 | 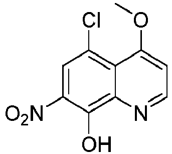 |
| | 5-Chloro-4-Methoxy-7-nitroquinolin-8-ol |
| 35 | 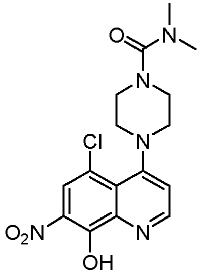 |
| | 4-(5-Chloro-8-hydroxy-7-nitroquinolin-4-yl)-N,N-dimethylpi perazine-1 -carboxamide |
| 36 | 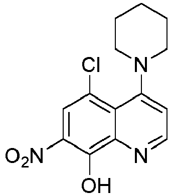 |
| | 5-Chloro-7-nitro-4-piperidinylquinolin-8-ol |
| 37 | 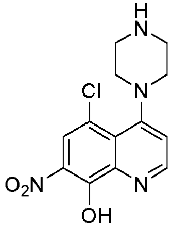 |
| | 5-Chloro-7-nitro-4-(piperazin-1-yl)quinolin-8-ol |
| 38 | 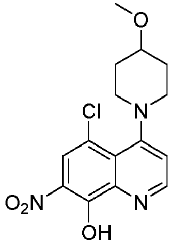 |
| | 5-Chloro-4-(4-methoxypiperidin-1-yl)-7-nitroquinolin-8-ol |
| 39 | 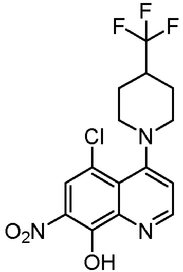 |
| | 5-Chloro-7-nitro-4-(4-(trifluoromethyl)piperidin-1-yl)quinolin -8-ol |
| 40 | 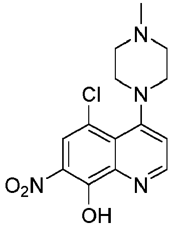 |
| | 5-Chloro-4-(4-methylpiperazin-1-yl)-7-nitroquinolin-8-ol |
| 41 | 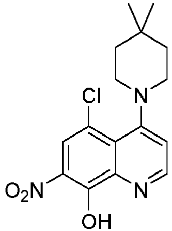 |
| | 5-Chloro-4-(4,4-dimethylpiperidin-1-yl)-7-nitroquinolin-8-ol |
| 42 | 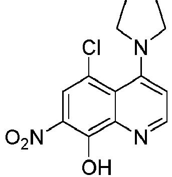 |
| | 5-Chloro-7-nitro-4-(pyrrolidin-1-yl)quinolin-8-ol |
| 43 | 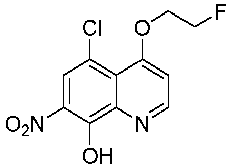 |
| | 5-Chloro-4-(2-fluoroethoxy)-7-nitroquinolin-8-ol |
| 44 | 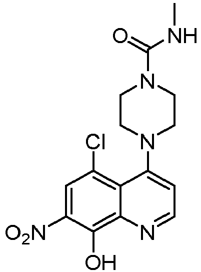 |
| | 4-(5-Chloro-8-hydroxy-7-nitroquinolin-4-yl)-N-methylpiperaz ine-1-carboxamide |
| 45 | 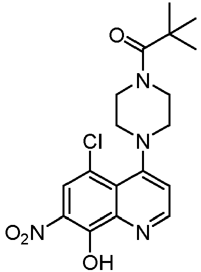 |
| | 4-(5-Chloro-8-hydroxy-7-nitroquinolin-4-yl)-1-(2,2,2-trimeth ylacetyl)piperazine |
| 46 | 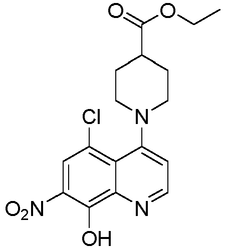 |
| | Ethyl 1-(5-chloro-8-hydroxy-7-nitroquinolin-4-yl)piperidine-4-carb oxylate |
| 47 | 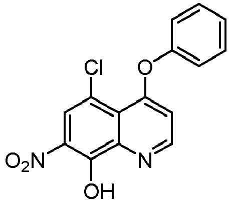 |
| | 5-Chloro-7-nitro-4-phenoxyquinolin-8-ol |
| 48 | 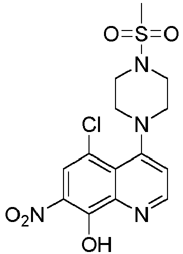 |
| | 5-Chloro-4-(4-(methylsulfonyl)piperazin-1-yl)-7-nitroquinolin -8-ol |
| 49 | 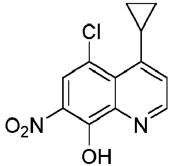 |
| | 5-Chloro-4-cyclopropyl-7-nitroquinolin-8-ol |
| 50 | 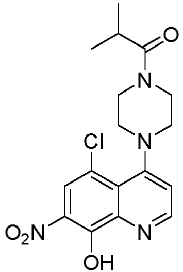 |
| | 4-(5-Chloro-8-hydroxy-7-nitroquinolin-4-yl)-1-(2,2-dimethylacetyl)piperazine |
| 51-A | 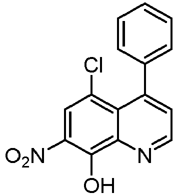 |
| | 5-Chloro-7-nitro-4-phenylquinolin-8-ol |
| 52 | 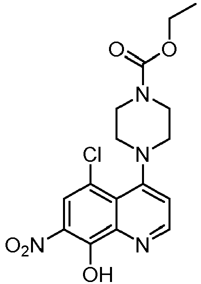 |
| | Ethyl 4-(5-chloro-8-hydroxy-7-nitroquinolin-4-yl)piperazine-1-carb oxylate |
| 53 | 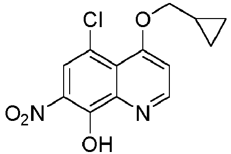 |
| | 5-Chloro-4-(cyclopropylmethoxy)-7-nitroquinolin-8-ol |
| 54-A | 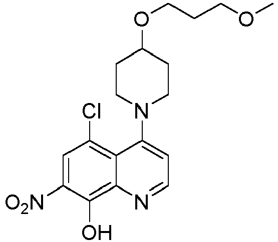 |
| | 5-Chloro-4-(4-(3-methoxypropoxy)piperidin-1-yl)-7-nitroquin olin-8-ol |
| 55 | 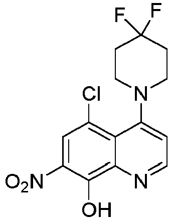 |
| | 5-Chloro-4-(4,4-difluoropiperidin-1-yl)-7-nitroquinolin-8-ol |
| 56 | 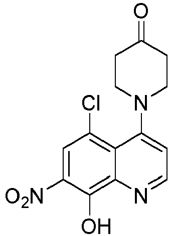 |
| | 1-(5-Chloro-8-hydroxy-7-nitroquinolin-4-yl)piperidin-4-one |
| 57 | 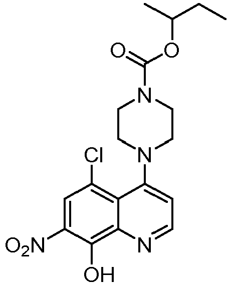 |
| | (1-Methyl)propyl 4-(5-chloro-8-hydroxy-7-nitroquinolin-4-yl)piperazine-1-carb oxylate |
| 58 | 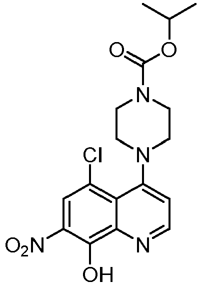 |
| | Isopropyl 4-(5-chloro-8-hydroxy-7-nitroquinolin-4-yl)piperazine-1-carb oxylate |
| 59 | 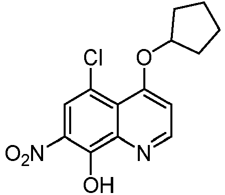 |
| | 5-Chloro-4-(cyclopentyloxy)-7-nitroquinolin-8-ol |
| 60 | 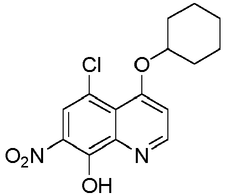 |
| | 5-Chloro-4-(cyclohexyloxy)-7-nitroquinolin-8-ol |
| 61 | 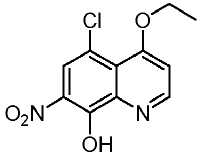 |
| | 5-Chloro-4-ethoxy-7-nitroquinolin-8-ol |
| 62 | 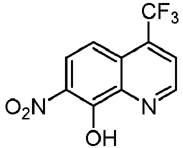 |
| | 7-Nitro-4-trifluoromethylquinolin-8-ol |
| 63 | 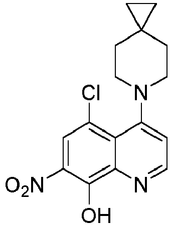 |
| | 5-Chloro-7-nitro-4-(6-azaspiro[2.5]octan-6-yl)quinolin-8-ol |
| 64 | 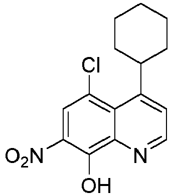 |
| | 5-Chloro-4-cyclohexyl-7-nitroquinolin-8-ol |
| 65 | 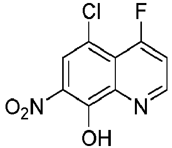 |
| | 5-Chloro-4-fluoro-7-nitroquinolin-8-ol |
| 66 | 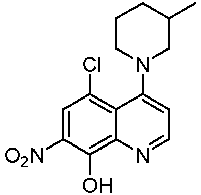 |
| | 5-Chloro-4-(3-methylpiperidin-1-yl)-7-nitroquinolin-8-ol |
| 67-A | 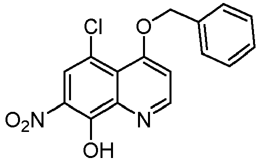 |
| | 4-(Benzyloxy)-5 -chloro-7-nitroquinolin-8-ol |
| 68 | 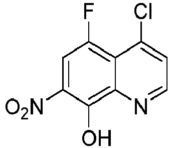 |
| | 4-Chloro-5-fluoro-7-nitroquinolin-8-ol |
| 69 | 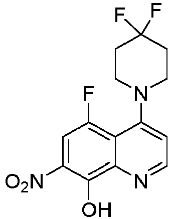 |
| | 4-(4,4-Difluoropiperidin-1-yl)-5-fluoro-7-nitroquinolin-8-ol |
| 70 | 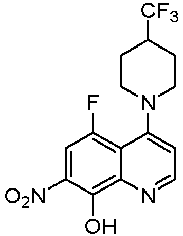 |
| | 5-Fluoro-7-nitro-4-(4-(trifluoromethyl)piperidin-1-yl)quinolin -8-ol |
| 71 | 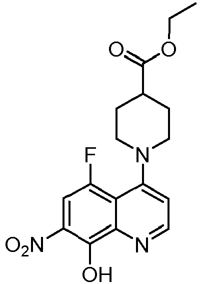 |
| | Ethyl 1-(5-fluoro-8-hydroxy-7-nitroquinolin-4-yl)piperidine-4-carbo xylate |
| 72 | 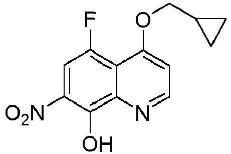 |
| | 4-(Cyclopropylmethoxy)-5-fluoro-7-nitroquinolin-8-ol |
| 73-A | 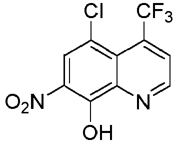 |
| | 5-Chloro-7-nitro-4-(trifluoromethyl)-quinolin-8-ol |
| 74 | 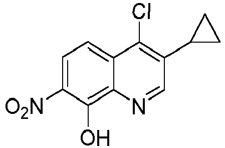 |
| | 4-Chloro-3-cyclopropyl-7-nitroquinolin-8-ol |
| 75 | 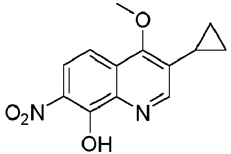 |
| | 3-Cyclopropyl-4-methoxy-7-nitroquinolin-8-ol |
| 76 | 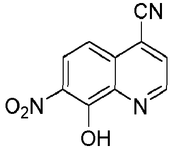 |
| | 8-Hydroxy-7-nitroquinoline-4-carbonitrile |
| 77 | 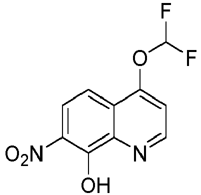 |
| | 4-(Difluoromethoxy)-7-nitroquinolin-8-ol |
| 78 | 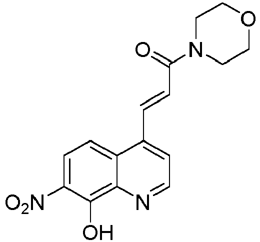 |
| | 3-(8-Hydroxy-7-nitroquinolin-4-yl)-N-acryloylmorpholine |
| 79 | 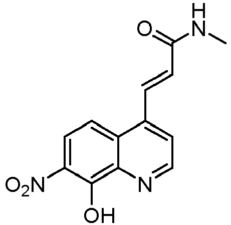 |
| | 3-(8-Hydroxy-7-nitroquinolin-4-yl)-N-methacrylamide |
| 80 | 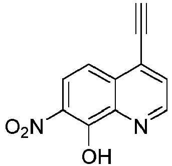 |
| | 4-Ethynyl-7-nitroquinolin-8-ol |
| 81 | 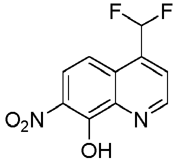 |
| | 4-(Difluoromethyl)-7-nitroquinolin-8-ol |
| 82 | 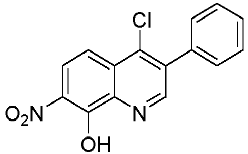 |
| | 4-Chloro-7-nitro-3-phenylquinolin-8-ol |
| 83 | 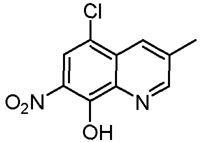 |
| | 5-Chloro-3 -methyl-7-nitroquinolin-8-ol |
| 84 | 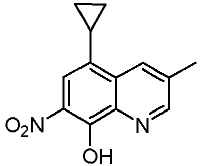 |
| | 5-Cyclopropyl-3-methyl-7-nitroquinolin-8-ol |
| 85 | 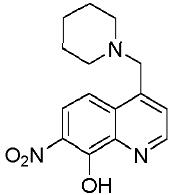 |
| | 7-Nitro-4-(piperidin-1-yl-methyl)quinolin-8-ol |
| 86 | 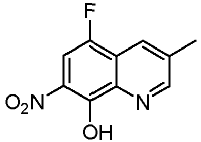 |
| | 5-Fluoro-3-methyl-7-nitroquinolin-8-ol |
| 87-A | 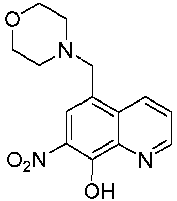 |
| | 5-(Morpholinomethyl)-7-nitroquinolin-8-ol |
| 88 | 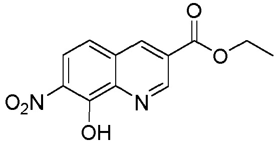 |
| | Ethyl 8-hydroxy-7-nitroquinoline-3-carboxylate |
| 89 | 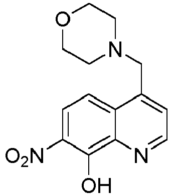 |
| | 4-(Morpholinomethyl)-7-nitroquinolin-8-ol |
| 90-A | 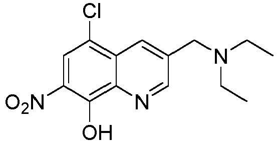 |
| | 5-Chloro-3 -((diethylamino)methyl)-7-nitroquinolin-8-ol |
| 91-A | 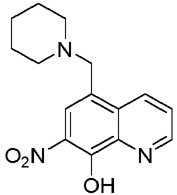 |
| | 7-Nitro-5-(piperidin-1-ylmethyl)quinolin-8-ol |
| 92 | 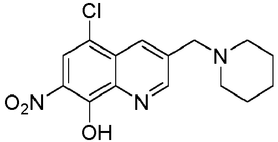 |
| | 5-Chloro-7-nitro-3-(piperidin-1-ylmethyl)quinolin-8-ol |
| 93-A | 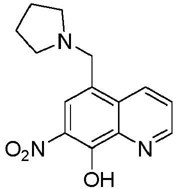 |
| | 7-Nitro-5-(pyrrolidin-1-ylmethyl)quinolin-8-ol |
| 94 | 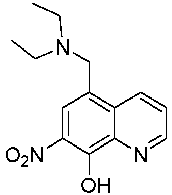 |
| | 5-((Diethylamino)methyl)-7-nitroquinolin-8-ol |
| 95 | 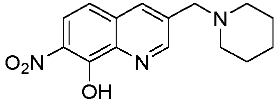 |
| | 7-Nitro-3-(piperidin-1-ylmethyl)quinolin-8-ol |
| 96 | 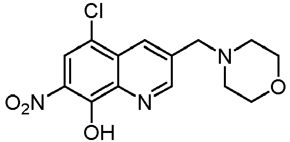 |
| | 5-Chloro-3-(morpholinomethyl)-7-nitroquinolin-8-ol |
| 97 | 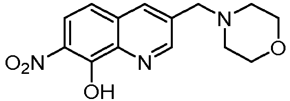 |
| | 3-(Morpholinomethyl)-7-nitroquinolin-8-ol |
| 98 | 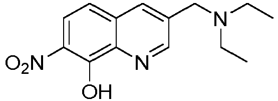 |
| | 3-((Diethylamino)methyl)-7-nitroquinolin-8-ol |
| 99-A | 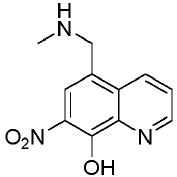 |
| | 5-((Methylamino)methyl)-7-nitroquinolin-8-ol |
| 100 | 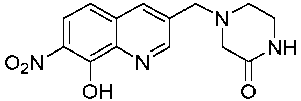 |
| | 4-((8-Hydroxy-7-nitroquinolin-3-yl)methyl)piperazin-2-one |
| 101-A | 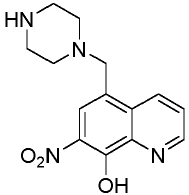 |
| | 7-Nitro-5-(piperazin-1-ylmethyl)quinolin-8-ol |
| 102-A | 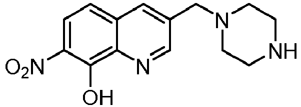 |
| | 7-Nitro-3-(piperazin-1-ylmethyl)quinolin-8-ol |
| 103-A | 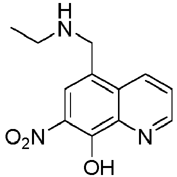 |
| | 5-((Ethylamino)methyl)-7-nitroquinolin-8-ol |
| 104 | 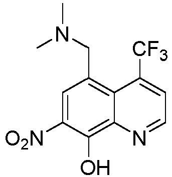 |
| | 5-((Dimethylamino)methyl)-7-nitro-4-(trifluoromethyl)quinoli n-8-ol |
| 105 | 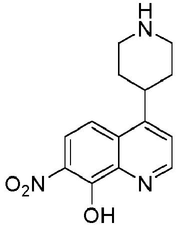 |
| | 7-Nitro-4-(piperidin-4-yl)quinolin-8-ol |
| 106 | 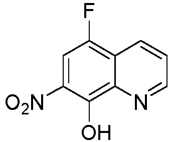 |
| | 5 -Fluoro-7-nitroquinolin-8 -ol |
| 107 | 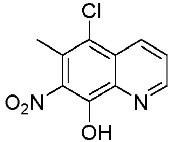 |
| | 5-Chloro-6-methyl-7-nitroquinolin-8-ol |
| 108-A | 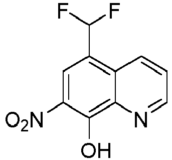 |
| | 5-(Difluoromethyl)-7-nitroquinolin-8-ol |
| 109 | 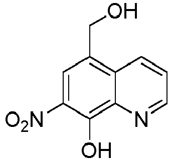 |
| | 5-(Hydroxymethyl)-7-nitroquinolin-8-ol |
| 110 | 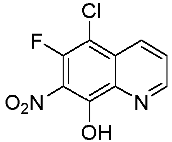 |
| | 5-Chloro-6-fluoro-7-nitroquinolin-8-ol |
| 111 | 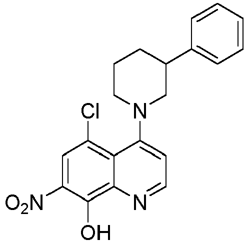 |
| | 5-Chloro-7-nitro-4-(3-phenylpiperidin-1-yl)quinolin-8-ol |
| 112 | 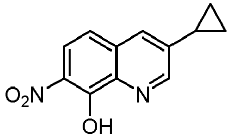 |
| | 3 -Cyclopropyl-7-nitroquinolin-8-ol |
| 113 | 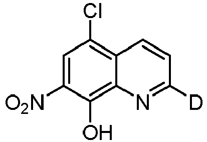 |
| | 5-Chloro-7-nitroquinolin-2-d-8-ol |
| 114 | 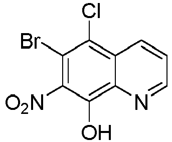 |
| | 6-Bromo-5 -chl oro-7 -nitroquinolin-8-ol |
| 115 | 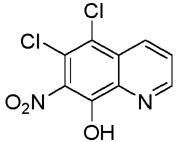 |
| | 5,6-Dichloro-7-nitroquinolin-8-ol |
| 116 | 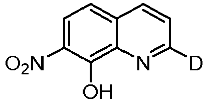 |
| | 7-Nitroquinolin-2-*d*-8-ol |
| 117 | 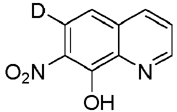 |
| | 7-Nitroquinolin-6-*d*-8-ol |
| 118 | 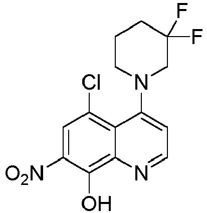 |
| | 5-Chloro-4-(3,3-difluoropiperidin-1-yl)-7-nitroquinolin-8-ol |
| 119 | 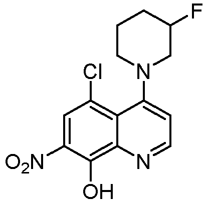 |
| | 5-Chloro-4-(3-fluoropiperidin-1-yl)-7-nitroquinolin-8-ol |
| 120 | 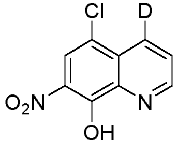 |
| | 5-Chloro-7-nitroquinolin-4-d-8-ol |
| 121 | 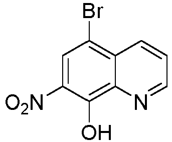 |
| | 5-Bromo-7-nitroquinolin-8-ol |
| 122 | 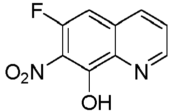 |
| | 6-Fluoro-7-nitroquinolin-8-ol |
| 123 | 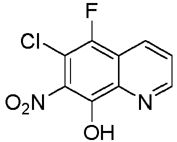 |
| | 6-Chloro-5-fluoro-7-nitroquinolin-8-ol |
| 124 | 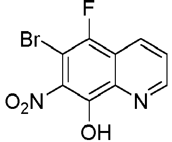 |
| | 6-Bromo-5-fluoro-7-nitroquinolin-8-ol |
| 125 | 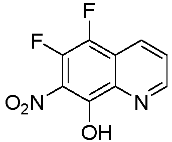 |
| | 5,6-Difluoro-7-nitroquinolin-8-ol |

In some preferred embodiments, the pharmaceutically acceptable salt of the present invention is the hydrochloride, acetate or trifluoroacetate of each specific compound above;
preferably, the pharmaceutically acceptable salt is selected from the group consisting of 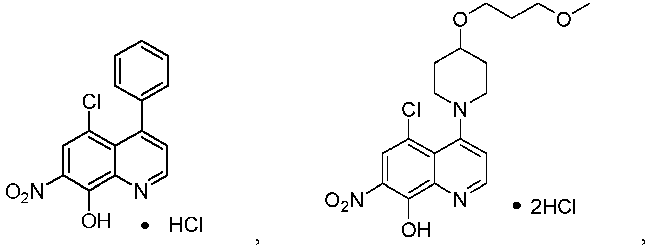 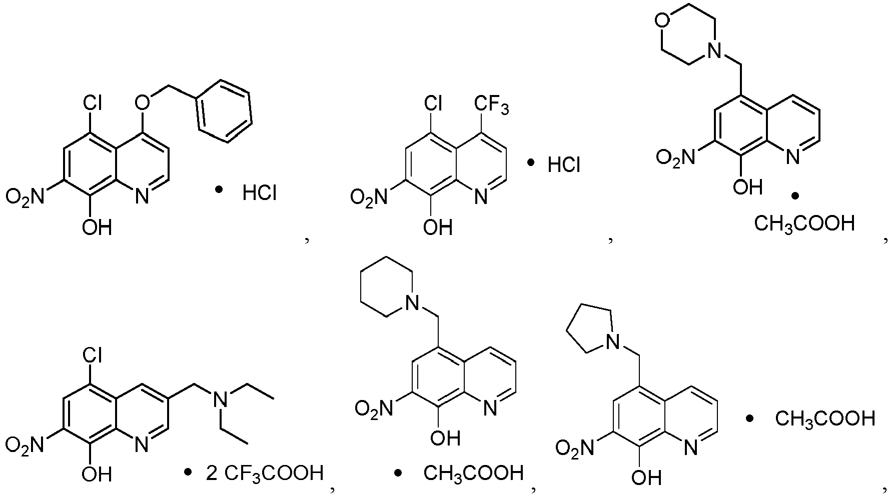 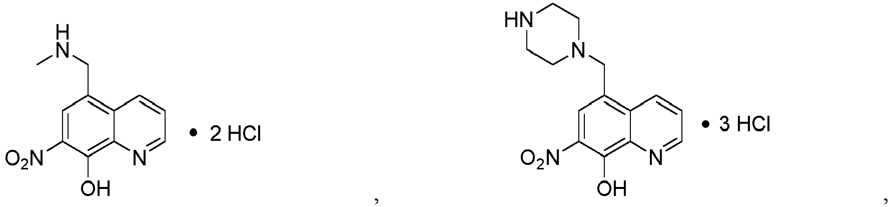 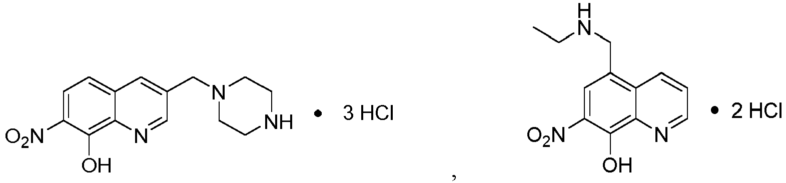 and

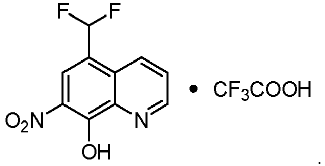

In another aspect, the present invention also provides an intermediate for preparing the compound of formula (I), which is particularly selected from the group consisting of the following compounds or pharmaceutically acceptable salts thereof: 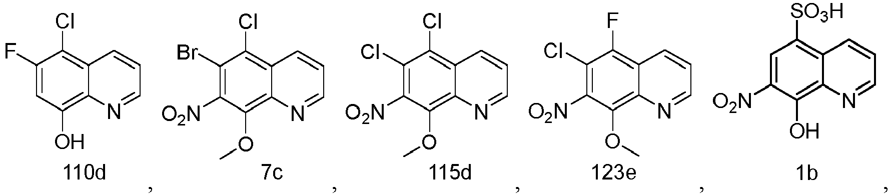 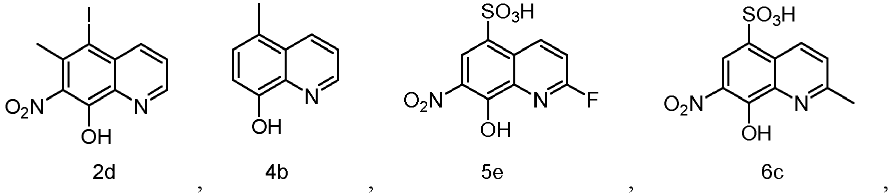 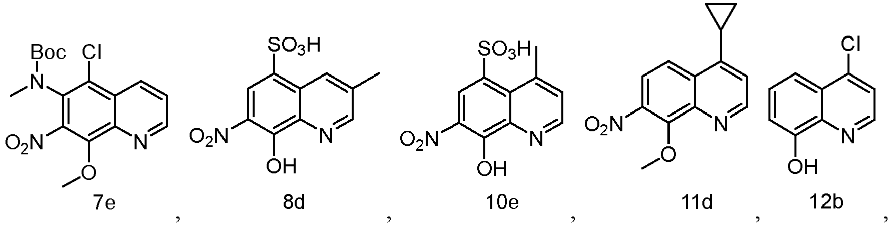 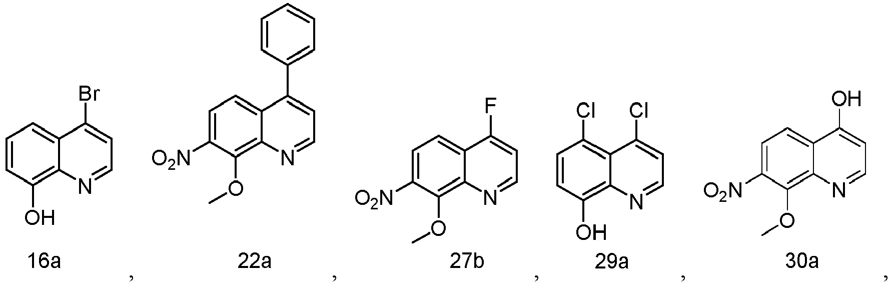 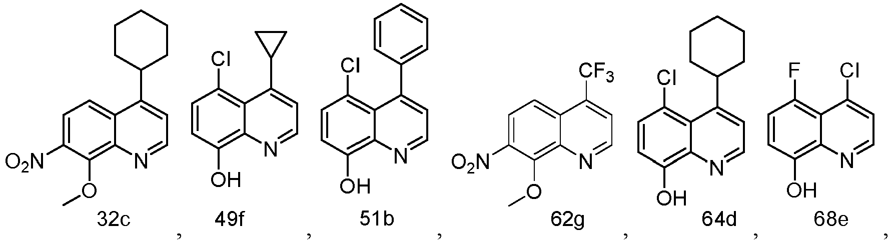 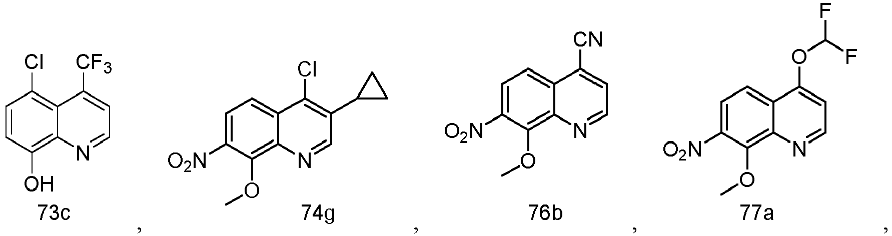 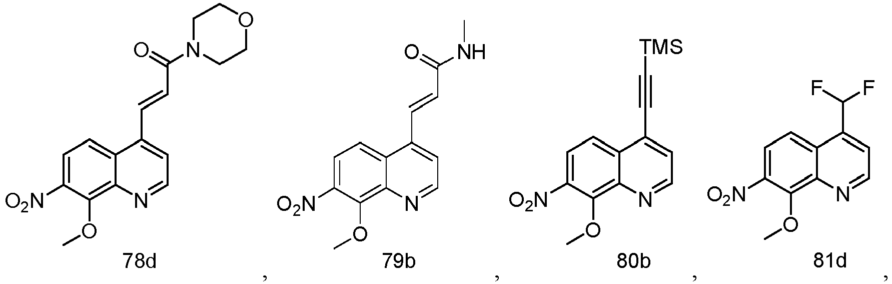 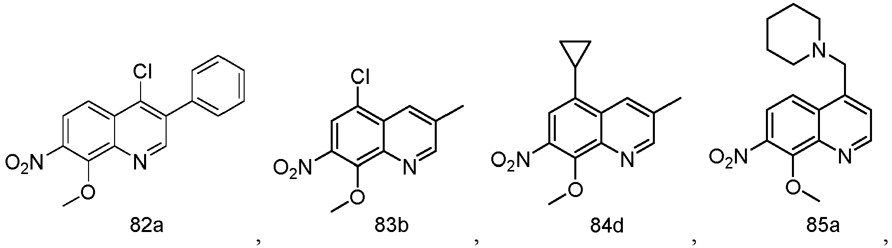 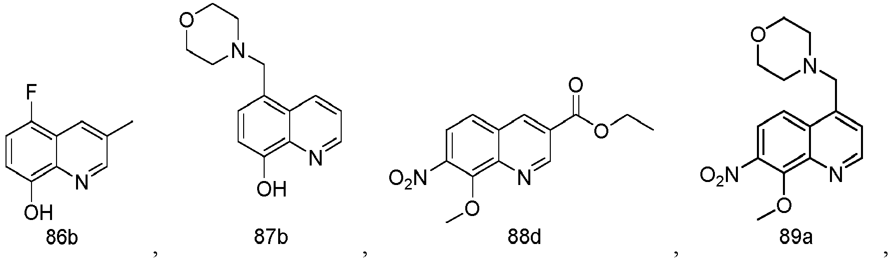 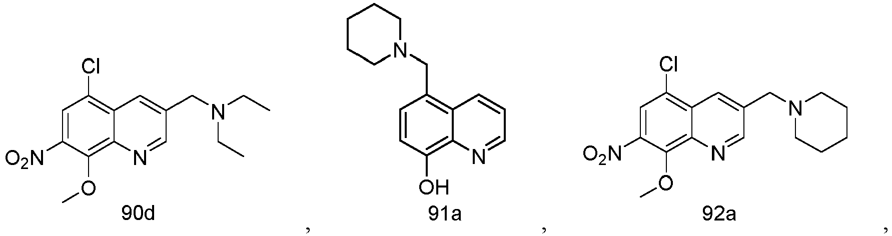 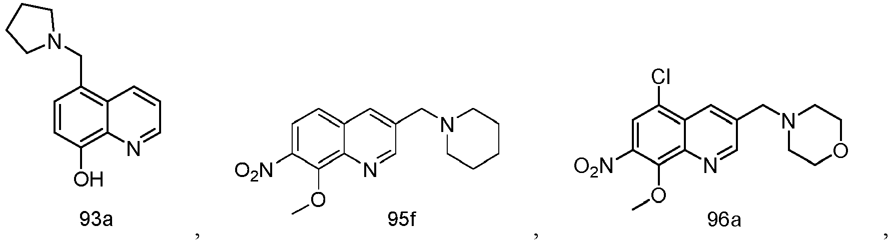 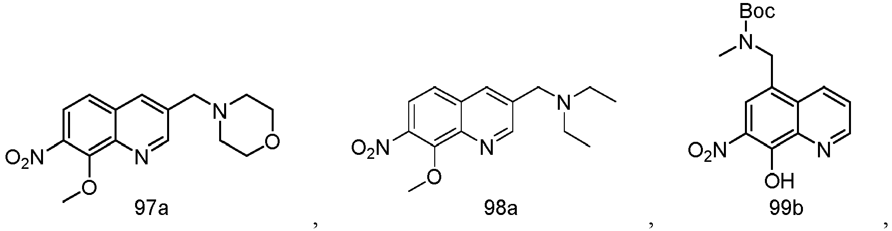 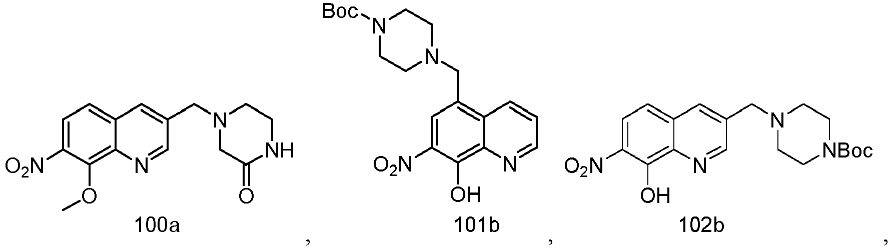 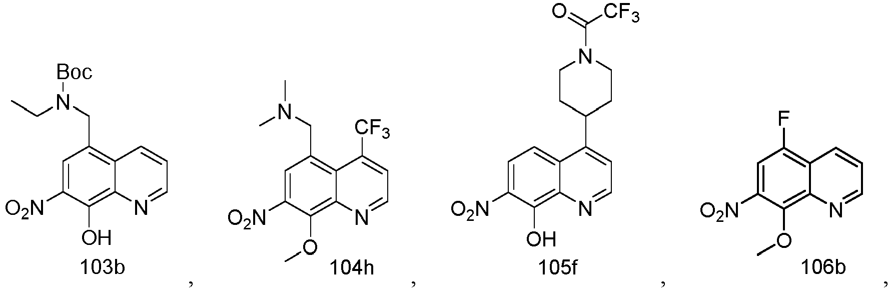 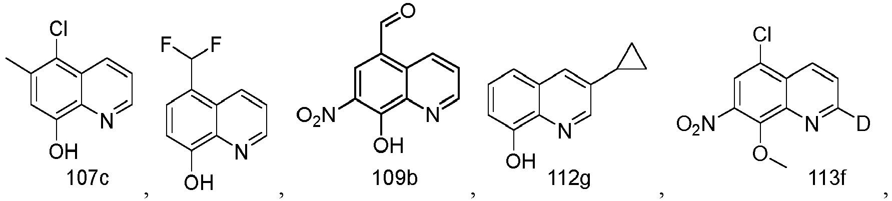 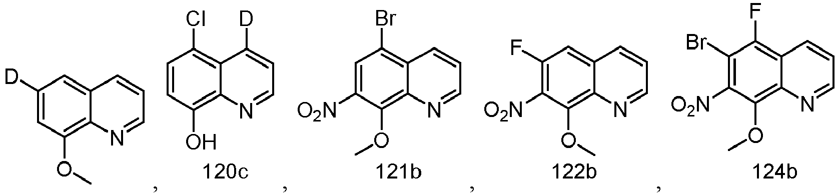 and

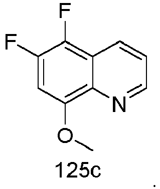

The present invention also provides a method for preparing the aforementioned compound of formula (III), comprising the following step of:
method I: 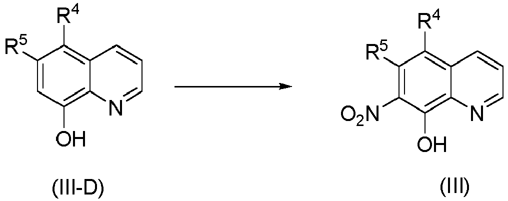 reacting a compound of formula (III-D) in the presence of nitrite and acid to obtain the compound of formula (III); wherein, the nitrite is preferably one or more of sodium nitrite, potassium nitrite and calcium nitrite, and more preferably sodium nitrite; the acid is preferably one or more of hydrochloric acid, acetic acid and trifluoroacetic acid, and more preferably hydrochloric acid.

In method I, preferably, 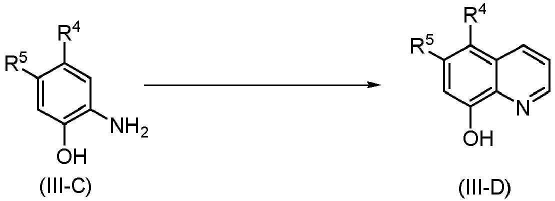 reacting a compound of formula (III-C) in the presence of glycerol or acrolein diethyl acetal and acid and oxidizing agent to obtain the compound of formula (III-D); wherein, the oxidizing agent is preferably sodium 3-nitrobenzene sulfonate. In general, those skilled in the art know that for glycerol, sulfuric acid is used; for acrolein diethyl acetal, hydrochloric acid and/or sulfuric acid is used.

In method I, further preferably, 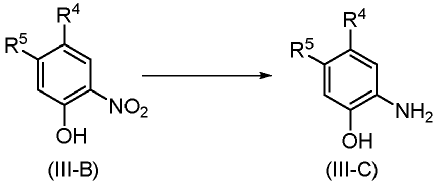 reacting a compound of formula (III-B) in the presence of solvent and reducing agent to obtain the compound of formula (III-C); wherein, the reducing agent is preferably iron or Na₂S₂O₄.

In method I, more further preferably, 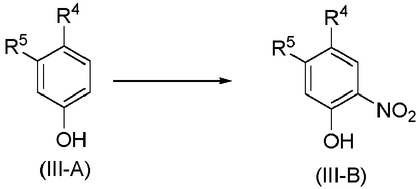 reacting a compound of formula (III-A) in the presence of solvent and HNO₃ to obtain the compound of formula (III-B); wherein, the solvent is preferably 1,2-dichloroethane; the reaction is preferably carried out in a phase transfer catalyst, and the phase transfer catalyst is preferably tetrabutylammonium bromide.

The present invention also provides a method for preparing the aforementioned compound of formula (III), comprising the following step of:
method II: 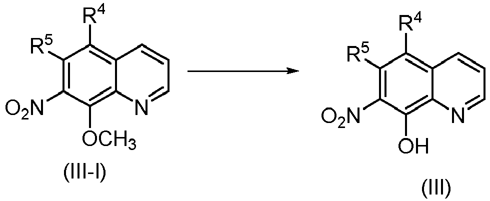 reacting a compound of formula (III-I) in the presence of solvent and LiCl at 80 to 130°C to obtain the compound of formula (III); wherein, the solvent is preferably N,N-dimethylformamide.

In method II, preferably, 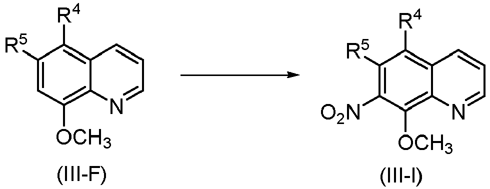 reacting a compound of formula (III-F) in the presence of solvent, HNO₃ and sulfuric acid to obtain the compound of formula (III-I); wherein, the solvent is preferably acetic anhydride.

In method II, further preferably, 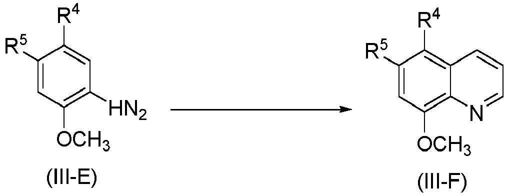 reacting a compound of formula (III-E) in the presence of glycerol or acrolein diethyl acetal and acid and oxidizing agent to obtain the compound of formula (III-F); wherein, the oxidizing agent is preferably sodium 3-nitrobenzene sulfonate. In general, those skilled in the art know that for glycerol, sulfuric acid is used; for acrolein diethyl acetal, hydrochloric acid and/or sulfuric acid is used.

The present invention also provides a method for preparing the aforementioned compound of formula (III), comprising the following step of:
method III: 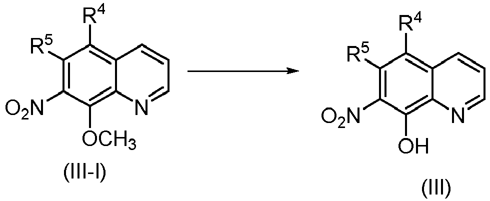 reacting a compound of formula (III-I) in the presence of solvent and LiCl at 80 to 130°C to obtain the compound of formula (III); wherein, the solvent is preferably N,N-dimethylformamide.

In method III, preferably, 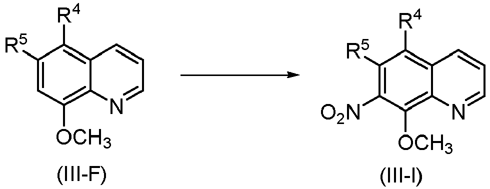 reacting a compound of formula (III-F) in the presence of solvent, HNO₃ and sulfuric acid to obtain the compound of formula (III-I); wherein, the solvent is preferably acetic anhydride.

In method III, further preferably, 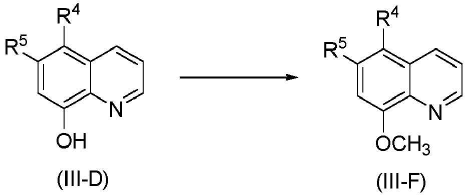 reacting a compound of formula (III-D) in the presence of solvent, methylating reagent and base to obtain the compound of formula (III-F); wherein, the methylating reagent is preferably CH₃I or dimethyl sulfate; the base is preferably an inorganic base, and more preferably potassium carbonate, sodium carbonate.

In method III, more further preferably, 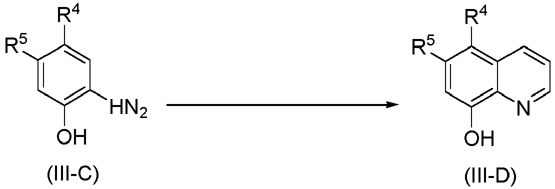 reacting a compound of formula (III-C) in the presence of glycerol or acrolein diethyl acetal and acid and oxidizing agent to obtain the compound of formula (III-D); wherein, the oxidizing agent is preferably sodium 3-nitrobenzene sulfonate. In general, those skilled in the art know that for glycerol, sulfuric acid is used; for acrolein diethyl acetal, hydrochloric acid and/or sulfuric acid is used.

In method III, still more further preferably, 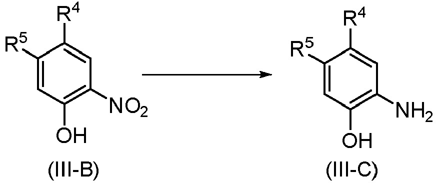 reacting a compound of formula (III-B) in the presence of solvent and reducing agent to obtain the compound of formula (III-C); wherein, the reducing agent is preferably iron or Na₂S₂O₄.

In method I, method II and method III, preferably, R⁴ is halogen, preferably Cl atom or F atom; and, R⁵ is halogen, preferably F atom, Cl atom or Br atom.

The present invention also provides a pharmaceutical composition comprising the aforementioned compound of formula (I), or or the tautomer, the stereoisomer, the mesomer, the racemate, the enantiomer, the diastereoisomer thereof, or the mixture thereof, or the pharmaceutically acceptable salt thereof, and one or more pharmaceutically acceptable excipients.

The present invention also relates to a use of the aforementioned compound of formula (I), or or the tautomer, the stereoisomer, the mesomer, the racemate, the enantiomer, the diastereoisomer thereof, or the mixture thereof, or the pharmaceutically acceptable salt thereof in the preparation of a medicament for treating infectious disease or cancer;
wherein, the infectious disease is preferably a systemic infection, reproductive system infection or urinary system infection; particularly, the infectious disease is induced by Gram-negative bacteria or Gram-positive bacteria; more particularly, the Gram-negative bacteria include *Escherichia coli, Acinetobacter baumannii* and *Klebsiella pneumoniae;* the Gram-positive bacteria include *Staphylococcus aureus,* the *Staphylococcus aureus* is preferably methicillin-resistant *Staphylococcus aureus* and/or methicillin-sensitive *Staphylococcus aureus;*
wherein, the cancer is preferably bladder cancer or prostate cancer.

The present invention also relates to the aforementioned compound of formula (I), or or the tautomer, the stereoisomer, the mesomer, the racemate, the enantiomer, the diastereoisomer thereof, or the mixture thereof, or the pharmaceutically acceptable salt thereof, or the aforementioned pharmaceutical composition, for use as a medicament.

The present invention also relates to the aforementioned compound of formula (I), or or the tautomer, the stereoisomer, the mesomer, the racemate, the enantiomer, the diastereoisomer thereof, or the mixture thereof, or the pharmaceutically acceptable salt thereof, or the aforementioned pharmaceutical composition, for use in treating infectious disease or cancer,
wherein, the infectious disease is preferably a systemic infection, reproductive system infection or urinary system infection; particularly, the infectious disease is induced by Gram-negative bacteria or Gram-positive bacteria; more particularly, the Gram-negative bacteria include *Escherichia coli*, *Acinetobacter baumannii* and *Klebsiella pneumoniae*, the *Escherichia coli* is preferably carbapenem-resistant *Escherichia coli,* the *Acinetobacter baumannii* is preferably carbapenem-resistant *Acinetobacter baumannii*; the Gram-positive bacteria include *Staphylococcus aureus,* the *Staphylococcus aureus* is preferably methicillin-resistant *Staphylococcus aureus* and/or methicillin-sensitive *Staphylococcus aureus*;
the cancer is preferably bladder cancer or prostate cancer.

The present invention also relates to a method for treating infectious disease or cancer comprising a step of administering the aforementioned compound of formula (I), or or the tautomer, the stereoisomer, the mesomer, the racemate, the enantiomer, the diastereoisomer thereof, or the mixture thereof, or the pharmaceutically acceptable salt thereof, or the aforementioned pharmaceutical composition to a patient in need thereof,
wherein, the infectious disease is preferably a systemic infection, reproductive system infection or urinary system infection; particularly, the infectious disease is induced by Gram-negative bacteria or Gram-positive bacteria; more particularly, the Gram-negative bacteria include *Escherichia coli*, *Acinetobacter baumannii* and *Klebsiella pneumoniae*, the *Escherichia coli* is preferably carbapenem-resistant *Escherichia coli,* the *Acinetobacter baumannii* is preferably carbapenem-resistant *Acinetobacter baumannii*; the Gram-positive bacteria include *Staphylococcus aureus,* the *Staphylococcus aureus* is preferably methicillin-resistant *Staphylococcus aureus* and/or methicillin-sensitive *Staphylococcus aureus*;
the cancer is preferably bladder cancer or prostate cancer.

The pharmaceutical composition of the present invention can be various conventional dosage forms, for example, tablet, aqueous suspension, oily suspension, dispersible powder, dispersible granule, emulsion, hard capsule, soft capsule, sterile aqueous solution for injection, sterile oil-in-water microemulsion for injection, or suppository. Each of the above dosage forms can be prepared by conventional preparation methods.

It is well known to those skilled in the art that the dosage of a drug depends on a variety of factors including, but not limited to the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound or the type of pharmaceutically acceptable salt thereof can be verified according to the traditional therapeutic regimens.

### Definition of terms

Regarding the terms not defined herein, they have the meanings commonly understood by those skilled in the art. Regarding the terms defined herein, they have the meanings defined in the description.

The term "substitution" or "substituent" refers to one or more hydrogen atoms being replaced by a group indicated. When the substitution position is not indicated, the substitution can occur at any position, provided that the formation of a stable or chemically feasible chemical is allowed.

The term "optional" or "optionally" means that the event or circumstance described subsequently can, but need not, occur, and such a description includes the situation in which the event or circumstance occurs or the situation in which the event or circumstance does not occur.

Where any variable (such as R) appears more than once in the structure of a compound, its definition in each case is independent. For example, if a group is substituted by 0 to 2 R, the group can optionally be substituted by up to two R, and R in each case has independent options.

The term "alkyl" refers to a saturated linear or branched monovalent hydrocarbon group having 1 to 20 (for example, 1, 2, 3, 4, 5, 6, 7, 8, 9, 10, 11, 12, 13, 14, 15, 16, 17, 18, 19 and 20) carbon atoms. The alkyl is preferably a C₁₋₁₀ alkyl, and more preferably C₁₋₆ alkyl. Examples of alkyl include, but are not limited to, methyl, ethyl, *n*-propyl, isopropyl, *n*-butyl, isobutyl, *tert*-butyl, *sec*-butyl, *n*-pentyl, 2,2-dimethylpropyl, 2-methylbutyl, *n*-hexyl, 2,2-dimethylbutyl, 2-methylpentyl, 3-methylpentyl, 2,3-dimethylbutyl, *n*-heptyl, 2-methylhexyl, 3-methylhexyl, 2,3-dimethylpentyl, 2,4-dimethylpentyl, 2,2-dimethylpentyl, 3,3-dimethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 3-ethylhexyl, 4-ethylhexyl, 2-methyl-3-ethylpentyl, n-nonyl, 2-methyl-3-ethylhexyl, *n*-decyl and 3,3-diethylhexyl.

The term "alkenyl" refers to a linear or branched monovalent hydrocarbon group having 2 to 6 (for example 2, 3, 4, 5 and 6) carbon atoms and at least one carbon-carbon double bond, wherein the carbon-carbon double bond may be located anywhere within the alkenyl. The alkenyl is preferably a C₂₋₅ alkenyl. Examples of alkenyl include, but are not limited to, -CH=CH₂, -CH=CH-CH₃, -CH₂-CH=CH₂, -CH=CH-CH₂-CH₃, -CH₂-CH=CH-CH₃, -CH=CH-CH=CH₂, -CH=C(CH₃)-CH₃ and -CH₂-C(CH₃)=CH₂.

The term "alkynyl" refers to a linear or branched monovalent hydrocarbon group having 2 to 6 (for example 2, 3, 4, 5 and 6) carbon atoms and at least one carbon-carbon triple bond, wherein the carbon-carbon triple bond may be located anywhere within the alkynyl. The alkynyl is preferably a C₂₋₅ alkynyl. Examples of alkynyl include, but are not limited to, -C≡CH, -C≡C-CH₃, -CH₂-C≡CH, -C≡C-CH₂-CH₃, -CH₂-CH₂-C≡CH, -CH(CH₃)C≡CH and -CH₂-C≡C-CH₃.

The term "cycloalkyl" includes two categories, one is conventional cycloalkyl, and the other is heterostructured cycloalkyl.

The conventional cycloalkyl refers to an aliphatic saturated or partially unsaturated monovalent cyclic hydrocarbon group having 3 to 20 (for example, 3, 4, 5, 6, 7, 8, 9, 10, 11, 12, 13, 14, 15, 16, 17, 18, 19 and 20) carbon atoms. The conventional cycloalkyl is preferably a C₃₋₁₂ conventional cycloalkyl, more preferably a C₃₋₁₀ conventional cycloalkyl, further preferably a C₃₋₈ conventional cycloalkyl, and most preferably a C₃₋₆ conventional cycloalkyl. The conventional cycloalkyl optionally comprises one or more double or triple bonds.

The conventional cycloalkyl can be a monocyclic cycloalkyl. Examples of monocyclic cycloalkyl include, but are not limited to, cyclopropyl, cyclobutyl, cyclopentyl, cyclopentenyl, cyclohexyl, cyclohexenyl, cyclohexadienyl, cycloheptyl, cycloheptatrienyl and cyclooctyl. The conventional cycloalkyl can also be a polycyclic cycloalkyl (for example, bicycloalkyl and tricycloalkyl). Polycyclic cycloalkyl includes spiro cycloalkyl, fused cycloalkyl and bridged cycloalkyl.

The term "spiro cycloalkyl" refers to a 5 to 20 membered (for example, 5, 6, 7, 8, 9, 10, 11, 12, 13, 14, 15, 16, 17, 18, 19 and 20 membered) spiro cycloalkyl. The spiro cycloalkyl is preferably a 6 to 14 membered spiro cycloalkyl, and more preferably a 7 to 10 membered spiro cycloalkyl. The spiro cycloalkyl can be a mono-spiro cycloalkyl, a di-spiro cycloalkyl, or a poly-spiro cycloalkyl, and the spiro cycloalkyl is preferably a mono-spiro cycloalkyl, and more preferably a 4-membered/4-membered, 4-membered/5-membered, 4-membered/6-membered, 5-membered/5-membered, or 5-membered/6-membered mono-spiro cycloalkyl. Examples of spiro cycloalkyl include, but are not limited to:

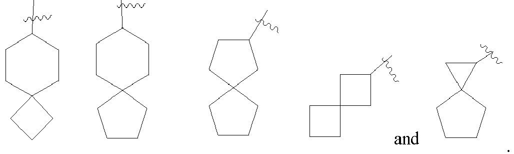

The term "fused cycloalkyl" refers to a 5 to 20 membered (for example, 5, 6, 7, 8, 9, 10, 11, 12, 13, 14, 15, 16, 17, 18, 19 and 20 membered) fused cycloalkyl. The fused cycloalkyl is preferably a 6 to 14 membered fused cycloalkyl, and more preferably a 7 to 10 membered fused cycloalkyl. The fused cycloalkyl can be a bicyclic, tricyclic, tetracyclic, pentacyclic or more fused cycloalkyl, and the fused cycloalkyl is preferably a bicyclic or tricyclic fused cycloalkyl, and more preferably a 5-membered/5-membered, or 5-membered/6-membered fused cycloalkyl. Examples of fused cycloalkyl include, but are not limited to:

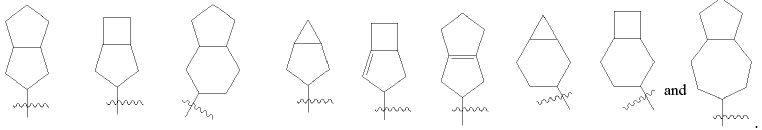

The term "bridged cycloalkyl" refers to a 5 to 20 membered (for example, 5, 6, 7, 8, 9, 10, 11, 12, 13, 14, 15, 16, 17, 18, 19 and 20 membered) bridged cycloalkyl. The bridged cycloalkyl is preferably a 6 to 14 membered bridged cycloalkyl, and more preferably a 7 to 10 membered bridged cycloalkyl. The bridged cycloalkyl can be a bicyclic, tricyclic, tetracyclic, pentacyclic or more bridged cycloalkyl, and the bridged cycloalkyl is preferably a bicyclic, tricyclic or tetracyclic bridged cycloalkyl, and more preferably a bicyclic or tricyclic bridged cycloalkyl. Examples of bridged cycloalkyl include, but are not limited to:

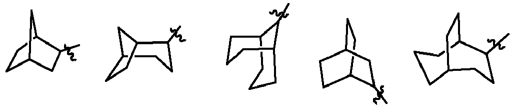

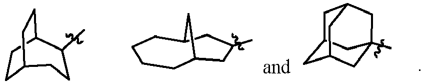

The term "heterostructured cycloalkyl" includes monocyclic cycloalkyl, spiro cycloalkyl, fused cycloalkyl and bridged cycloalkyl fused with any one selected from the group consisting of conventional aryl, conventional heteroaryl and conventional heterocyclyl, and the attachment point is located on the corresponding conventional cycloalkyl (referring to monocyclic cycloalkyl, spiro cycloalkyl, fused cycloalkyl or bridged cycloalkyl). Examples of heterostructured cycloalkyl include, but are not limited to:

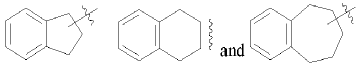

The term "heterocyclyl" includes two categories, one is conventional heterocyclyl, and the other is heterostructured heterocyclyl.

The conventional heterocyclyl refers to an aliphatic saturated or partially unsaturated monovalent cyclic hydrocarbon group having 3 to 20 (for example, 3, 4, 5, 6, 7, 8, 9, 10, 11, 12, 13, 14, 15, 16, 17, 18, 19 and 20) ring atoms, wherein one or more ring atoms are replaced by one or more elements selected from the group consisting of N, O, S, S(O) and S(O)₂, and the replacement does not form -O-O-, -O-S- or -S-S-. The conventional heterocyclyl is preferably a C₃₋₁₂ conventional heterocyclyl, wherein 1 to 4 (for example 1, 2, 3 and 4) atoms are heteroatoms; more preferably a C₃₋₈ conventional heterocyclyl, wherein 1 to 3 (for example 1, 2 and 3) atoms are heteroatoms; and most preferably a C₅₋₇ conventional heterocyclyl, wherein 1 to 2 or 1 to 3 atoms are heteroatoms.

The conventional heterocyclyl can be a monocyclic heterocyclyl. Examples of monocyclic heterocyclyl include, but are not limited to oxetanyl, 3-pyrrolinyl, pyrrolidinyl, imidazolidinyl, tetrahydrofuranyl, tetrahydrothienyl, dihydroimidazolyl, dihydrofuryl, dihydropyrazolyl, dihydropyrrolyl, piperidinyl, piperazinyl, morpholinyl, thiomorpholinyl, homopiperazinyl and pyranyl, and preferably 1,2,5-oxadiazolyl, pyranyl or morpholinyl. The conventional heterocyclyl can also be a polycyclic heterocyclyl. Polycyclic heterocyclyl includes spiro heterocyclyl, fused heterocyclyl and bridged heterocyclyl.

The term "spiro heterocyclyl" refers to a 5 to 20 membered (for example, 5, 6, 7, 8, 9, 10, 11, 12, 13, 14, 15, 16, 17, 18, 19 and 20 membered) spiro heterocyclyl. The spiro heterocyclyl is preferably a 6 to 14 membered spiro heterocyclyl, and more preferably a 7 to 10 membered spiro heterocyclyl. The spiro heterocyclyl can be a mono-spiro heterocyclyl, a di-spiro heterocyclyl, or a poly-spiro heterocyclyl, and the spiro heterocyclyl is preferably a mono-spiro heterocyclyl or a di-spiro heterocyclyl, and more preferably a 3-membered/6-membered, 4-membered/4-membered, 4-membered/5-membered, 4-membered/6-membered, 5-membered/5-membered, or 5-membered/6-membered mono-spiro heterocyclyl. Examples of spiro heterocyclyl include, but are not limited to:

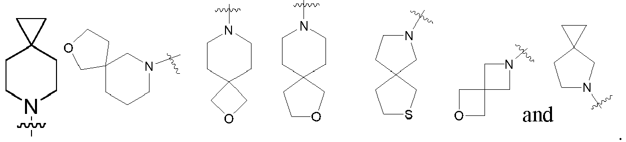

The term "fused heterocyclyl" refers to a 5 to 20 membered (for example, 5, 6, 7, 8, 9, 10, 11, 12, 13, 14, 15, 16, 17, 18, 19 and 20 membered) fused heterocyclyl. The fused heterocyclyl is preferably a 6 to 14 membered fused heterocyclyl, and more preferably a 7 to 10 membered fused heterocyclyl. The fused heterocyclyl can be a bicyclic, tricyclic, tetracyclic, pentacyclic or more fused heterocyclyl, and the fused heterocyclyl is preferably a bicyclic or tricyclic fused heterocyclyl, and more preferably a 5-membered/5-membered, or 5-membered/6-membered bicyclic fused heterocyclyl. Examples of fused heterocyclyl include, but are not limited to:

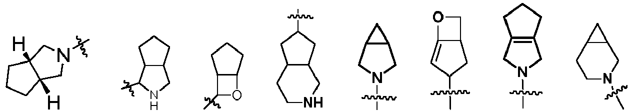

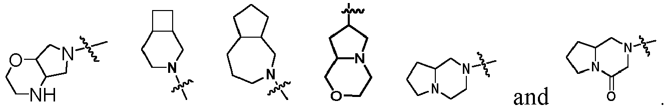

The term "bridged heterocyclyl" refers to a 5 to 14 membered (for example, 5, 6, 7, 8, 9, 10, 11, 12, 13 and 14 membered) bridged heterocyclyl. The bridged heterocyclyl is preferably a 6 to 14 membered bridged heterocyclyl, and more preferably a 7 to 10 membered bridged heterocyclyl. The bridged heterocyclyl can be a bicyclic, tricyclic, tetracyclic, pentacyclic or more bridged heterocyclyl, and the bridged heterocyclyl is preferably a bicyclic, tricyclic or tetracyclic bridged heterocyclyl, and more preferably a bicyclic or tricyclic bridged heterocyclyl. Examples of bridged heterocyclyl include, but are not limited to:

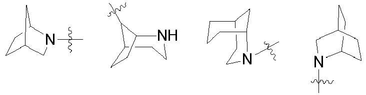

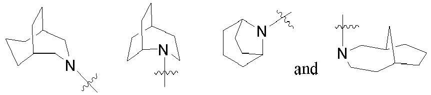

The term "heterostructured heterocyclyl" includes monocyclic heterocyclyl, spiro heterocyclyl, fused heterocyclyl and bridged heterocyclyl fused with any one selected from the group consisting of conventional aryl, conventional heteroaryl and conventional cycloalkyl, and the attachment point is located on the corresponding conventional heterocyclyl (referring to monocyclic heterocyclyl, spiro heterocyclyl, fused heterocyclyl or bridged heterocyclyl). Examples of heterostructured heterocyclyl include, but are not limited to:

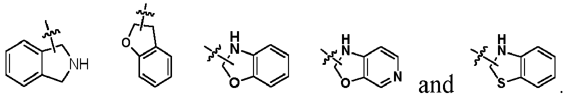

The term "aryl" includes two categories, one is conventional aryl, and the other is heterostructured aryl.

The conventional aryl refers to a 6 to 14 membered (for example, 6, 7, 8, 9, 10, 11, 12, 13 and 14 membered) aromatic hydrocarbon group. The conventional aryl is preferably a C₆₋₁₀ conventional aryl, and more preferably phenyl, naphthyl, phenanthryl or anthracenyl.

The term "heterostructured aryl" includes conventional aryl fused with any one selected from the group consisting of conventional heteroaryl, conventional heterocyclyl and conventional cycloalkyl, and the attachment point is located on the conventional aryl. Examples of heterostructured aryl include, but are not limited to: 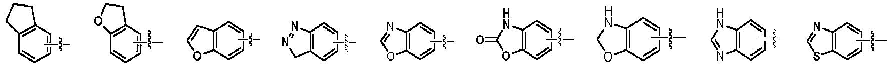 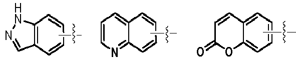 and

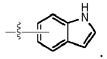

The term "heteroaryl" includes two categories, one is conventional heteroaryl, and the other is heterostructured heteroaryl.

The conventional heteroaryl refers to a 5 to 14 membered (for example, 5, 6, 7, 8, 9, 10, 11, 12, 13 and 14 membered) aromatic hydrocarbon group, wherein 1 to 4 (for example, 1, 2, 3 and 4) carbon atoms are replaced by heteroatoms, the heteroatom is selected from the group consisting of O, S and N. Preferably, the number of ring atoms is 5 to 10, including 1 to 3 (for example, 1, 2 and 3) heteroatoms. More preferably, the number of ring atoms is 5 or 6, including 1 to 2 heteroatoms. Examples of conventional heteroaryl include, but are not limited to imidazolyl, furyl, thienyl, thiazolyl, pyrazolyl, oxazolyl, pyrrolyl, tetrazolyl, pyridyl, pyrimidinyl, thiadiazolyl and pyrazinyl, preferably imidazolyl, thiazolyl, pyrazolyl, pyrimidinyl or thiazolyl, and more preferably pyrazolyl or thiazolyl.

The term "heterostructured heteroaryl" includes conventional heteroaryl fused with any one selected from the group consisting of conventional aryl, conventional cycloalkyl and conventional heterocyclyl, and the attachment point is located on the conventional heteroaryl. Examples of heterostructured heteroaryl include, but are not limited to: 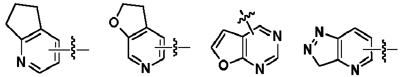 and

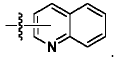

The term "alkoxy" includes -O-alkyl and -O-cycloalkyl, wherein the "alkyl" and "cycloalkyl" are as defined above. Examples of alkoxy include, but are not limited to: methoxy, ethoxy, propoxy, butoxy, cyclopropyloxy, cyclobutyloxy, cyclopentyloxy and cyclohexyloxy.

The term "haloalkyl" refers to an alkyl substituted by one or more halogen(s), wherein the alkyl is as defined above.

The term "haloalkoxy" refers to an alkoxy substituted by one or more halogen(s), wherein the alkoxy is as defined above.

The term "hydroxy" refers to an -OH group.

The term "halogen" refers to -F, -Cl, -Br or -I group.

The term "amino" refers to a -NH₂ group.

The term "cyano" refers to a -CN group.

The term "nitro" refers to a -NO₂ group.

The term "oxo" refers to a =O group.

The term "carboxy" refers to a -C(=O)OH group.

The term "thiol" refers to a -SH group.

The term "alkoxycarbonyl" refers to a -C(=O)O-alkyl or a -C(=O)O-cycloalkyl, wherein the alkyl and cycloalkyl are as defined above.

The term "acyl" refers to a -C(=O)R group, where R is selected from the group consisting of alkyl, cycloalkyl, heterocyclyl, aryl and heteroaryl.

The symbol " 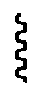 " refers to the attachment point.

The term "pharmaceutically acceptable" refers to the use in the preparation of a pharmaceutical composition that is generally safe, non-toxic, biologically satisfactory and can be accepted as a medicament to a mammal, such as a human.

The term "pharmaceutically acceptable salt" should be understood as referring to the following salts, which are pharmaceutically acceptable salts and which possess the expected pharmacological activity of the parent compound (referring to the compound of the formula). Such salts include:
(1) Acid addition salts formed with inorganic acids, or acid addition salts formed with organic acids; wherein, the inorganic acids can be one or more of hydrochloric acid, hydrobromic acid, sulfuric acid, nitric acid and phosphoric acid; the organic acids can be one or more of formic acid, oxalic acid, succinic acid, acetic acid, benzenesulfonic acid, benzoic acid, camphorsulfonic acid, citric acid, ethanesulfonic acid, fumaric acid, glucoheptonic acid, gluconic acid, glutamic acid, glycollic acid, hydroxynaphthoic acid, 2-hydroxyethanesulfonic acid, lactic acid, maleic acid, malic acid, mandelic acid, methanesulfonic acid, muconic acid, 2-naphthalenesulfonic acid, propionic acid, salicylic acid, succinic acid, dibenzoyl-L-tartaric acid, tartaric acid, p-toluenesulfonic acid, trimethylacetic acid and trifluoroacetic acid; and
(2) Salts formed when acid protons present in the parent compound are substituted by metal ions, for example, alkali metal ions (e.g., Na⁺, K⁺ or Li⁺), alkaline earth metal ions (such as Ca²⁺ or Mg²⁺) or aluminum ions; or, when coordinated with organic or inorganic bases; wherein, the organic bases can be one or more of pyridines, imidazoles, pyrazines, indoles, purines, tertiary amines and anilines, preferably one or more of pyridine, methylpyridine, 4-dimethylaminopyridine, 2-methyl-5-ethylpyridine, triethylamine, N,N-diisopropylethanamine, N,N-dimethylaniline, diethanolamine, ethanolamine, N-methylglucosamine, triethanolamine and tromethamine; the inorganic bases can be one or more of aluminum hydroxide, calcium hydroxide, potassium hydroxide, sodium carbonate and sodium hydroxide.

The term "isotopic derivative" refers to a compound that differs from the parent compound described herein solely by the presence of one or more isotopically enriched atoms. For example, a isotopic derivative has the structure shown by the formula, wherein hydrogen is replaced by "deuterium" or "tritium", and/or fluorine is replaced by ¹⁸F, and/or carbon is replaced by ¹¹C, ¹³C or ¹⁴C, while leaving the rest unchanged. Isotopic derivatives may be used as analytical tools or probes in biological assays, or as tracers for *in vivo* diagnostic imaging of disease, or as tracers for pharmacodynamic, pharmacokinetic, or receptor studies. Deuterated compounds often retain activity comparable to that of non-deuterated compounds, and when deuterated at specific sites they can achieve better metabolic stability, resulting in certain therapeutic advantages (e.g. increased half-life *in vivo* or decreased dosage requirements). Therefore, the isotope derivative is preferably a deuterated compound.

The term "solvate" refers to a substance formed from the parent compound described herein and a suitable solvent. The solvent is preferably water or an organic solvent.

The term "pharmaceutical composition" refers to a mixture consisting of a pharmaceutical compound (referring to one or more of the compound of the formula, a pharmaceutically acceptable salt thereof, a tautomer thereof, a stereoisomer thereof, a enantiomer thereof, a diastereoisomer thereof, a isotope derivative thereof, a crystal form thereof, a solvate thereof, a metabolite thereof and a racemate containing the same described herein) and pharmaceutically acceptable excipients.

The term "pharmaceutically acceptable excipient" refers to a pharmaceutically acceptable excipient used to deliver a pharmaceutical compound described herein to a subject. Depending on the administration method, the pharmaceutical composition may comprises 0.1% to 99% by weight of the pharmaceutical compound.

The term "subject" refers to a mammal, including, for example, camel, donkey, zebra, cattle, pig, horse, goat, sheep, cat, dog, rat, rabbit, guinea pig, mouse, primate. In some particular embodiments, the subject is a human. In some particular embodiments, the subject is a human who is susceptible to, suspected of having, or has suffered from a cancer or bacterial infection.

The term "treatment" refers to eliminating the disease, preventing disease progression, slowing disease progression, reducing the duration of one or more symptoms associated with the disease, improving or reversing at least one measurable parameter associated with the disease, or increasing the survival of subjects with the disease.

The term "effective amount" refers to an amount of the active ingredient of the drug (referring to the pharmaceutical compound) that elicits the desired effect in a subject. In particular embodiments, those skilled in the art can determine the selection of an effective amount based on consideration of a variety of factors (e.g., through clinical trials), the factors include the disease to be treated, the symptoms involved, the route of administration, the severity of the disease, the patient's body weight, the patient's immune status, and other factors known to those skilled in the art. The effective amount can be obtained from the dose-response curve derived from an animal model testing system, and allows to be determined according to the opinion of a physician and the situation of each patient. The correlation between animal and human doses is described in Freireich et al., 1966, Cancer Chemother Rep 50:219, and the body surface area of human can be approximately determined by the height and body weight of the patient. The effective amount of the pharmaceutical compounds of the present invention can be 0.5 mg/kg to 500 mg/kg, preferably 1 mg/kg to 200 mg/kg, more preferably 10 mg/kg to 100 mg/kg.

Herein, the same pharmaceutical active ingredient (which refers to a single pharmaceutical compound) or different pharmaceutical active ingredients (which refers to two or more pharmaceutical compounds) can be administered at one time, or can be divided into multiple smaller doses, and administered at a certain time interval. It should be understood that the exact dose, duration, and interval of treatment is a function of the disease being treated, and can be determined by inference using animal or clinical trial data. The administration can include a single administration, or two or more administrations at an appropriate time interval. Wherein, the time interval between two adjacent administrations can be 30 minutes, 40 minutes, 50 minutes, 60 minutes, 2 hours, 3 hours, 4 hours, 5 hours, 6 hours, 7 hours, 8 hours, 9 hours, 10 hours, 12 hours, 14 hours, 16 hours, 18 hours, 20 hours, 22 hours, 24 hours, one and a half days, 2 days, 3 days, 4 days, 5 days, 6 days, 7 days, 8 days, 9 days, 10 days, 1 week, 2 weeks, 3 weeks, 4 weeks, 5 weeks, 6 weeks, 1 month, 2 months, 3 months , 4 months, 5 months, 6 months, 7 months, 8 months, 9 months, 10 months, 11 months or 12 months.

Each pharmaceutical active ingredient (each pharmaceutical compound) mentioned herein can be used as the only active compound, or can be administered in combination with other active compounds (which refers to compounds other than the pharmaceutical compounds described herein), as long as they do not produce other adverse effects, such as allergic responses. Combined administration includes simultaneous or sequential administration of each active compound.

The term "combined administration" refers to a method of providing two or more active compounds simultaneously or sequentially to a subject for therapeutic purposes. When "combined administration" is involved, the time interval between each administration is sufficient to achieve synergy between each active compound administered.

When the term "about" is applied to a parameter such as weight, volume, pH, concentration, temperature, etc., it is intended that the parameter may vary by ±10%, and is sometimes more preferably within ±5%. As those skilled in the art will appreciate, when a parameter is not critical, numbers are generally given for purposes of illustration only and not limitation.

### DETAILED DESCRIPTION OF THE INVENTION

By reading the following examples, those skilled in the art will better understand the present invention. These examples are only used to explain the present invention, and do not limit the scope of the present invention.

The compound of the present invention is prepared by using convenient starting materials and general preparation procedures. The present invention provides typical or preferential reaction conditions, such as reaction temperature, time, solvent, pressure and molar ratio of reactants. However, unless otherwise specified, other reaction conditions can also be adopted. Optimal conditions may vary with the use of specific reactants or solvents, but under normal circumstances, reaction optimization steps and conditions can be determined.

In addition, certain protecting groups may be used in the present invention to protect certain functional groups from unnecessary reactions. The protecting groups suitable for various functional groups and their protection or deprotection conditions are well known to those skilled in the art. For example, "Protective Groups in Organic Synthesis" by T. W. Greene and G. M. Wuts (3rd edition, Wiley, New York, 1999 and citations in the book) describes the protection or deprotection of a large number of protecting groups in detail.

The isolation and purification of compounds and intermediates are carried out by appropriate methods and steps according to specific needs, such as filtration, extraction, distillation, crystallization, column chromatography, preparative thin-layer chromatography, preparative high performance liquid chromatography or a combination of the above methods. The specific method used may be referred to the examples described in the present invention. Of course, other similar isolation and purification methods can also be used. They can be characterized using conventional methods (including physical constants and spectral data).

The purity analysis method is as follows: a Kinetex EVO C18 (50×4.6 mm, 5 µm, 100Å) chromatographic column is used, acetonitrile-water is used as the mobile phase for gradient elution, the flow rate is 1.5 mL/min, and the detection wavelength is 220 nm.

MS is determined by a LC (Agilent 1260 Infinity II)/MS (G6125B single quadrupole) mass spectrograph (manufacturer: Agilent) (Photodiode Array Detector).

The structures of the compounds are identified by hydrogen nuclear magnetic resonance, and the equipment model is WNMR-I-400MHz.

Preparative liquid chromatography is conducted on an Agilent 1260 Infinity II high performance liquid chromatograph (manufacturer: Agilent). The chromatographic column is Daisogel C18 10 µm 100A (30 mm×250 mm), and the mobile phase is acetonitrile/water.

GF254 silica gel plate of Qingdao Haiyang Chemical is used for the thin-layer silica gel chromatography (TLC). The dimension of the silica gel plate used in TLC is 0.20 mm to 0.25 mm, and the dimension of the silica gel plate used in product purification is 0.5 mm.

Silica gel of 100 to 200 mesh, 200 to 300 mesh or 300 to 400 mesh of Qingdao Haiyang Chemical is used as a carrier for gel column chromatography.

The known starting materials of the present invention can be prepared by the known methods in the art, or can be purchased from Wanghua Mall, Beijing Ouhe Technology, Sigma, J&K Scientific, Yishiming, Shanghai Shuya Chemical, Shanghai Innochem Science & Technology, Energy Chemical, Shanghai Bide Pharmatech and the like.

Unless otherwise stated, the reactions are carried out under a nitrogen atmosphere.

Nitrogen atmosphere means that a reaction flask is equipped with a nitrogen balloon (about 1 L).

The reaction solvent, organic solvent or inert solvent are each expressed as the solvent used that does not participate in the reaction under the described reaction conditions, which includes for example, benzene, toluene, acetonitrile, tetrahydrofuran (THF), dimethylformamide (DMF), chloroform, dichloromethane, ether, methanol, N-methylpyrrolidone (NMP).

Unless otherwise specified in the examples, a solution means an aqueous solution.

Unless otherwise defined, all professional and scientific terms used herein have the same meaning as those familiar to those skilled in the art. In addition, any methods and materials similar or equivalent to the content described herein can be applied to the method of the present invention.

Unless otherwise specified, the mixing ratios of different solvents are volume ratios.

### Example 1

### Synthesis of 7-nitroquinolin-8-ol (1)

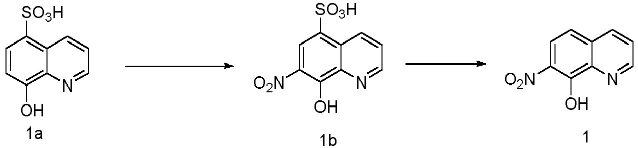

At 0°C, nitric acid (65 weight%) (8.0 mL, 116.4 mmol) was slowly added dropwise to a suspension of 8-hydroxyquinoline-5-sulfonic acid (1a) (10.0 g, 44.4 mmol) in sulfuric acid (98 weight%) (40.0 mL). The resulting mixture was stirred at 0°C for 10 minutes, poured into ice water (50.0 mL) and continued to stir for 15 minutes. The pH of the aqueous phase was adjusted to about 5 to 6 with saturated aqueous potassium carbonate solution. The resulting mixture was filtered under reduced pressure, and the filter cake was washed with water (10.0 mL × 3), and dried under vacuum to obtain 8-hydroxy-7-nitroquinoline-5-sulfonic acid (1b) (9.0 g, yield 76%).

At room temperature, sulfuric acid (98 weight%) (2.8 mL) was slowly added to a suspension of 8-hydroxy-7-nitroquinoline-5-sulfonic acid (1b) (9.0 g, 33.3 mmol) in acetic acid (28.0 mL). The resulting mixture was stirred at 120°C for 6 hours, cooled to room temperature, poured into ice water (100.0 mL) and continued to stir for 15 minutes. The pH of the aqueous phase was adjusted to about 6 to 7 with ammonia (25 to 28 weight%, NH₃ content). The resulting mixture was filtered under reduced pressure, and the filter cake was washed successively with water (10.0 mL × 2) and methanol (5.0 mL × 2), and dried under vacuum to obtain 7-nitroquinolin-8-ol (1) (5.0 g, yield 78%).

¹H NMR (400 MHz, DMSO-*d₆*) δ: 9.02 (dd, *J* = 4.0, 1.6 Hz, 1H), 8.52 (dd, *J* = 8.4, 1.6 Hz, 1H), 8.04 (d, *J* = 9.2 Hz, 1H), 7.81 (dd, *J* = 8.4, 4.4Hz, 1H), 7.48 (d, *J* = 9.2 Hz, 1H).

MS calculated: 190.04, MS found: 191.2 [M+H]⁺.

### Example 2

### Synthesis of 6-methyl-7-nitroquinolin-8-ol (2)

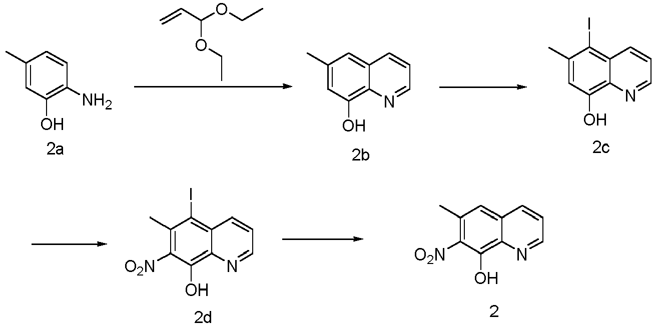

At room temperature, acrolein diethyl acetate (1.5 mL, 20.25 mmol) was added to a solution of 2-amino-5-methylphenol (2a) (1.0 g, 8.1 mmol) in hydrochloric acid (1.0 M) (40.0 mL). The reaction mixture was stirred at 110°C for 24 hours, and cooled to room temperature. The pH of the aqueous phase was adjusted to about 7 with solid sodium carbonate. The resulting mixture was extracted with dichloromethane (30.0 mL × 3), the combined organic phase was dried over anhydrous sodium sulfate and filtered, and the filtrate was concentrated under reduced pressure. The resulting residue was purified by silica gel column chromatography (eluent: dichloromethane/methanol = 10/1) to obtain 6-methylquinolin-8-ol (2b) (0.81 g, yield 63%).

At room temperature, 6-methylquinolin-8-ol (2b) (500.0 mg, 3.12 mmol) and N-iodosuccinimide (858.0 mg, 3.0 mmol) were added to chloroform (40.0 mL). The resulting mixture was stirred at 40°C for 15 minutes, cooled to room temperature, and diluted with aqueous sodium thiosulfate solution (10 weight%) (30.0 mL). The organic phase was separated, and the aqueous phase was extracted with dichloromethane (20.0 mL × 2). The combined organic phase was washed with saturated brine (20.0 mL × 2), dried over anhydrous sodium sulfate and filtered. The filtrate was concentrated to dryness under reduced pressure to obtain 5-iodo-6-methylquinolin-8-ol (2c) (232.0 mg, yield 92%).

At 0°C, a solution of sodium nitrate (59.4 mg, 0.70 mol) and lanthanum nitrate hexahydrate (30.2 mg, 0.070 mmol) in hydrochloric acid (6.0 M) (14.4 mL) was added dropwise to a solution of 5-iodo-6-methylquinolin-8-ol (2c) (200.0 mg, 0.70 mmol) in diethyl ether (20.0 mL). The reaction mixture was warmed up to room temperature, stirred for 1 hour, and extracted with ethyl acetate (50.0 mL × 3). The combined organic phase was washed with saturated brine (20.0 mL), dried over anhydrous sodium sulfate and filtered, and the filtrate was concentrated under reduced pressure. The resulting residue was purified by silica gel column chromatography (eluent: petroleum ether/ethyl acetate = 3/1) to obtain 5-iodo-6-methyl-7-nitroquinolin-8-ol (2d) (50.0 mg, yield 22%).

At room temperature, 5-iodo-6-methyl-7-nitroquinolin-8-ol (2d) (50.0 mg, 0.15 mmol) and tetrakis(triphenylphosphine)palladium (7.27 mg, 0.015 mmol) were added to N,N-dimethylformamide (3.0 mL). The reaction solution was stirred in a microwave reactor at 140 °C for 30 minutes, cooled to room temperature, and diluted with ammonia (2.0 M) (20.0 mL). The resulting mixture was extracted with ethyl acetate (20.0 mL × 2). The combined organic phase was washed with saturated brine (20.0 mL), dried over anhydrous sodium sulfate and filtered, and the filtrate was concentrated under reduced pressure. The resulting residue was purified by silica gel column chromatography (eluent: dichloromethane/methanol = 10/1) to obtain 6-methyl-7-nitroquinolin-8-ol (2) (16.0 mg, yield 52%).

¹H NMR (400 MHz, DMSO-*d*₆) δ: 8.95-8.93 (m, 1H), 8.29-8.27 (m, 1H), 7.93-7.90 (m, 1H), 7.10 (s, 1H), 2.51 (s, 3H).

MS calculated: 204.05; MS found: 205.0 [M+H]⁺.

### Example 3

### Synthesis of 5-iodo-7-nitroquinolin-8-ol (3)

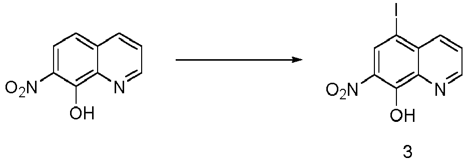

At room temperature, 7-nitroquinolin-8-ol (250.0 mg, 1.31 mmol) and N-iodosuccinimide (429.0 mg, 1.5 mmol) were added to chloroform (15.0 mL). The resulting mixture was stirred at 40°C for 15 minutes, cooled to room temperature, and diluted with aqueous sodium thiosulfate solution (10 weight%) (20.0 mL). The organic phase was separated, and the aqueous phase was extracted with dichloromethane (20.0 mL × 2). The combined organic phase was washed with saturated brine (20.0 mL × 2), dried over anhydrous sodium sulfate and filtered. The filtrate was concentrated to dryness under reduced pressure to obtain 5-iodo-7-nitroquinolin-8-ol (3) (367.0 mg, yield 88%).

¹H NMR (400 MHz, DMSO-*d*₆) δ: 9.02 (d, *J* = 2.8 Hz, 1H), 8.55 (d, *J* = 3.6 Hz, 1H), 8.45 - 8.42 (m, 1H), 7.95 - 7.92 (m, 1H).

MS calculated: 315.93; MS found: 316.9 [M+H]⁺.

### Example 4

### Synthesis of 5-methyl-7-nitroquinolin-8-ol (4)

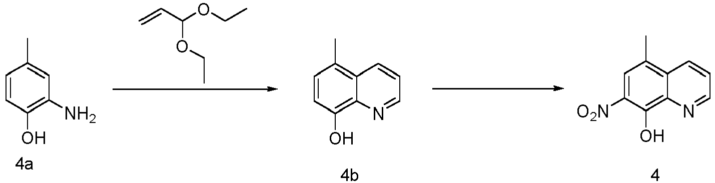

At room temperature, acrolein diethyl acetate (1.5 mL, 20.25 mmol) was added to a solution of 2-amino-4-methylphenol (4a) (1.0 g, 8.1 mmol) in hydrochloric acid (1.0 M) (40.0 mL). The reaction mixture was stirred at 110°C for 24 hours, and cooled to room temperature. The pH of the aqueous phase was adjusted to about 7 to 8 with solid sodium carbonate. The resulting mixture was extracted with dichloromethane (30.0 mL × 3), the combined organic phase was dried over anhydrous sodium sulfate and filtered, and the filtrate was concentrated under reduced pressure. The resulting residue was purified by silica gel column chromatography (eluent: petroleum ether/ethyl acetate = 1/1) to obtain 5-methylquinolin-8-ol (4b) (0.50 g, yield 46%).

At 0°C, nitric acid (65 weight%) (0.8 mL, 11.6 mmol) was slowly added dropwise to a suspension of 5-methylquinolin-8-ol (200.0 mg, 1.26 mmol) in sulfuric acid (98 weight%) (4.0 mL). The resulting mixture was stirred at 0°C for 10 minutes, poured into ice water (15.0 mL) and continued to stir for 15 minutes. The pH of the aqueous phase was adjusted to about 6 to 7 with saturated aqueous potassium carbonate solution. The resulting mixture was filtered under reduced pressure, the filter cake was washed with water (2.5 mL × 3), and dried under vacuum to obtain 5-methyl-7-nitroquinolin-8-ol (4) (180.0 mg, yield 70%).

¹H NMR (400 MHz, DMSO-*d*₆) δ: 9.04 - 9.03 (m, 1H), 8.55 - 8.52 (m, 1H), 7.89 (s, 1H), 7.85 - 7.82 (m, 1H), 2.59 (s, 3H).

MS calculated:204.05; MS found: 205.1 [M+H]⁺.

### Example 5

### Synthesis of 2-fluoro-7-nitroquinolin-8-ol (5)

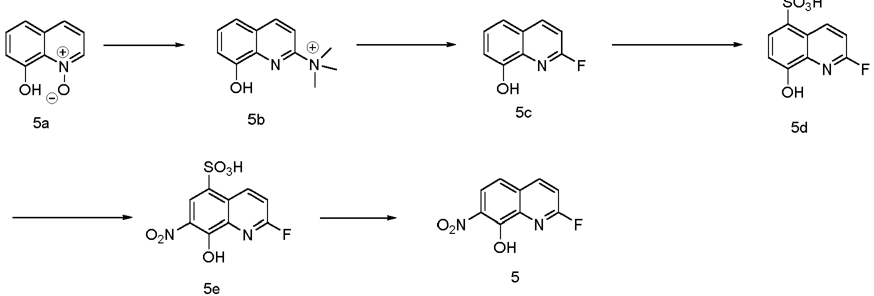

At 0°C, trifluoroacetic anhydride (2.5 mL, 37.0 mmol) was added to a solution of 8-hydroxyquinoline-N-oxide (5a) (2.0 g, 12.5 mmol) and trimethylamine (6.0 mL, 125.0 mmol) in dichloromethane (15.0 mL). The resulting mixture was warmed up to room temperature, stirred for 2 hours, and concentrated under reduced pressure to remove the organic solvent. Diethyl ether (10.0 mL) was added to the resulting residue, stirred for 10 minutes, and filtered under reduced pressure. The filter cake was washed with diethyl ether (10.0 mL), and then purified by silica gel column chromatography (eluent: dichloromethane/methanol = 10/1) to obtain 8-hydroxy-N,N,N-trimethylquinolin-2-aminium (5b) (1.7 g, yield 73%).

At room temperature, a solution (1.0 M) (3.0 mL) of tetra-n-butylammonium fluoride in tetrahydrofuran was added to a solution of 8-hydroxy-N,N,N-trimethylquinolin-2-aminium (5b) (203.0 mg, 1.0 mmol) in N,N-dimethylformamide (5.0 mL). The reaction solution was stirred at 90°C for 1 hour, cooled to room temperature, diluted with ethyl acetate (20.0 mL), and washed with water (20.0 mL) and saturated brine (20.0 mL) successively. The organic phase was separated, dried over anhydrous sodium sulfate and filtered, and the filtrate was concentrated under reduced pressure. The resulting residue was purified by silica gel column chromatography (eluent: petroleum ether/ethyl acetate = 5/1) to obtain 2-fluoroquinolin-8-ol (5c) (106.0 mg, yield 65%).

At room temperature, fuming sulfuric acid (18 to 24 weight% SO₃ content) (0.2 mL) was added to a solution of 2-fluoroquinolin-8-ol (5c) (100.0 mg, 0.61 mmol) in sulfuric acid (98 weight%) (0.4 mL). The reaction solution was stirred at 65°C for 2 hours, cooled to room temperature, poured into ice water (5.0 mL), and filtered under reduced pressure. The filter cake was washed with acetone (2.0 mL × 3), and dried under vacuum to obtain 2-fluoro-8-hydroxyquinoline-5-sulfonic acid (5d) (130.0 mg, yield 94%).

At 0°C, nitric acid (65 weight%) (0.8 mL, 11.6 mmol) was added dropwise to a solution of 2-fluoro-8-hydroxyquinoline-5-sulfonic acid (5d) (100.0 mg, 0.41 mmol) in sulfuric acid (98 weight%) (4.0 mL). The resulting mixture was stirred at 0°C for 10 minutes, poured into ice water (15.0 mL) and continued to stir for 15 minutes. The pH of the aqueous phase was adjusted to about 5 to 6 with saturated aqueous potassium carbonate solution. The resulting mixture was filtered under reduced pressure, the filter cake was washed with water (1.0 mL × 3), and dried under vacuum to obtain 2-fluoro-8-hydroxy-7-nitroquinoline-5-sulfonic acid (5e) (90.0 mg, yield 76%).

At room temperature, sulfuric acid (98 weight%) (0.8 mL) was slowly added to a suspension of 2-fluoro-8-hydroxy-7-nitroquinoline-5-sulfonic acid (5e) (90.0 mg, 0.32 mmol) in acetic acid (2.8 mL). The resulting mixture was stirred at 120°C for 6 hours, cooled to room temperature, poured into ice water (15.0 mL) and continued to stir for 15 minutes. The pH of the aqueous phase was adjusted to about 6 to 7 with ammonia (25 to 28 weight%, NH₃ content). The resulting mixture was filtered under reduced pressure, the filter cake was washed with water (1.0 mL × 2), and dried under vacuum to obtain 2-fluoro-7-nitroquinolin-8-ol (5) (52.0 mg, yield 78%).

¹H NMR (400 MHz, CDCl₃) δ: 9.50-9.46 (m, 1H), 8.56 (d, *J* = 8.8 Hz, 1H), 8.22 (s, 1H), 7.42-7.39 (m, 1H), 7.28 (s, 1H).

MS calculated:208.03; MS found: 207.1 [M-H]⁻.

### Example 6

### Synthesis of 2-methyl-7-nitroquinolin-8-ol (6)

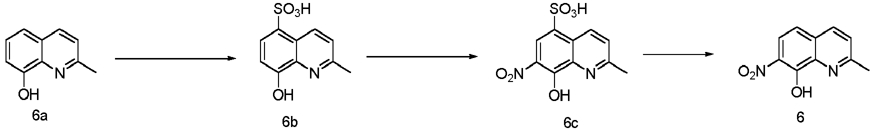

At room temperature, fuming sulfuric acid (18 to 24 weight% SO₃ content) (2.0 mL) was added to a solution of 2-methyl-8-hydroxyquinoline (6a) (1.0 g, 6.28 mmol) in sulfuric acid (98 weight%) (4.0 mL). The reaction solution was stirred at 65°C for 2 hours, cooled to room temperature, and poured into ice water (20.0 mL). The resulting suspension was diluted with acetone (60.0 mL), stirred for 10 minutes, and filtered under reduced pressure. The filter cake was washed with acetone (15.0 mL × 2), and dried under vacuum to obtain 8-hydroxy-2-methylquinoline-5-sulfonic acid (6b) (1.4 g, yield 94%).

At 0°C, nitric acid (65 weight%) (0.8 mL, 11.6 mmol) was added dropwise to a solution of 8-hydroxy-2-methylquinoline-5-sulfonic acid (6b) (100.0 mg, 0.42 mmol) in sulfuric acid (98 weight%) (4.0 mL). The resulting mixture was stirred at 0°C for 10 minutes, poured into ice water (15.0 mL) and continued to stir for 15 minutes. The pH of the aqueous phase was adjusted to about 5 to 6 with saturated aqueous potassium carbonate solution. The resulting mixture was filtered under reduced pressure, the filter cake was washed with water (1.0 mL × 3), and dried under vacuum to obtain 8-hydroxy-2-methyl-7-nitroquinoline-5-sulfonic acid (6c) (94.3 mg, yield 79%).

At room temperature, sulfuric acid (98 weight%) (0.8 mL) was slowly added to a suspension of 8-hydroxy-2-methyl-7-nitroquinoline-5-sulfonic acid (6c) (90.0 mg, 0.32 mmol) in acetic acid (2.8 mL). The resulting mixture was stirred at 120°C for 6 hours, cooled to room temperature, poured into ice water (15.0 mL) and continued to stir for 15 minutes. The pH of the aqueous phase was adjusted to about 6 to 7 with ammonia (25 to 28 weight%, NH₃ content). The resulting mixture was filtered under reduced pressure, the filter cake was washed with water (1.0 mL × 2), and dried under vacuum to obtain 2-methyl-7-nitroquinolin-8-ol (6) (51.0 mg, yield 78%).

¹H NMR (400 MHz, DMSO-*d*₆) δ: 8.38 (d, *J* = 8.4 Hz, 1H), 7.97 (d, *J =* 9.2 Hz, 1H), 7.68 (d, *J* = 8.4 Hz, 1H), 7.41 (d, *J* = 9.2 Hz, 1H), 2.77 (s, 3H).

MS calculated: 204.05; MS found: 205.1 [M+H]⁺.

### Example 7

### Synthesis of 5-chloro-6-(methylamino)-7-nitroquinolin-8-ol (7)

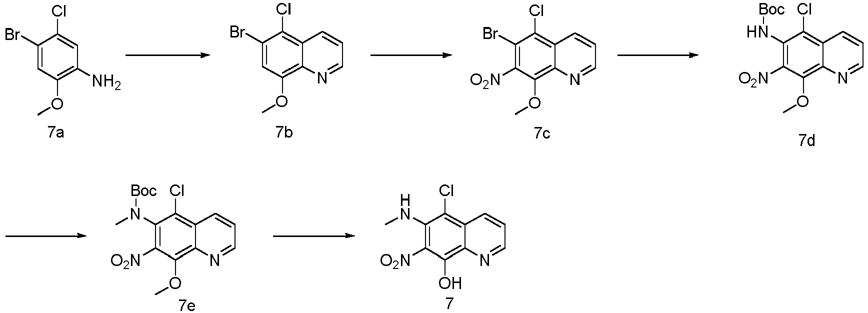

At room temperature, 4-bromo-5-chloro-2-methoxyaniline (7a) (3.80 g, 16.1 mmol), sodium 3-nitrobenzene sulfonate (4.34 g, 19.3 mmol) and glycerol (2.96 mL, 40.2 mmol) were successively added to sulfuric acid (70 wt.%) (40.4 mL). The resulting mixture was stirred at 140°C for 4 hours, and cooled to room temperature. The pH of the aqueous phase was adjusted to about 8 to 9 with aqueous sodium hydroxide solution (1.0 M). The resulting mixture was extracted with dichloromethane (50.0 mL × 3). The organic phases were combined, dried over anhydrous sodium sulfate, filtered, and concentrated to dryness under reduced pressure to obtain crude 6-bromo-5-chloro-8-methoxyquinoline (7b) (3.80 g, crude yield 87%).

At 0°C, nitric acid (65 wt.%) (8.4 mL, 198.0 mmol) was slowly added dropwise to a solution of crude 6-bromo-5-chloro-8-methoxyquinoline (7b) (1.80 g, 6.6 mmol) in acetic anhydride (30.0 mL), and stirred for 30 minutes. Sulfuric acid (98 wt.%) (1.20 mL, 22.6 mmol) was slowly added dropwise. The reaction mixture was stirred at 0°C for 30 minutes, then warmed up to room temperature and stirred for 48 hours, and then cooled to 0°C. The pH of the aqueous phase was adjusted to about 8 to 9 with aqueous sodium hydroxide solution (1.0 M). The resulting mixture was extracted with ethyl acetate (50.0 mL × 3). The organic phases were combined, washed with saturated brine (50.0 mL), dried over anhydrous sodium sulfate, filtered, and concentrated under reduced pressure. The resulting residue was purified by silica gel column chromatography (gradient elution, eluent: petroleum ether/ethyl acetate = 9/1 -2/1) to obtain 6-bromo-5-chloro-8-methoxy-7-nitroquinoline (7c) (0.551 g, yield 24%).

At room temperature, 6-bromo-5-chloro-8-methoxy-7-nitroquinoline (7c) (300.0 mg, 0.94 mmol), tert-butyl carbamate (165.0 mg, 1.41 mmol), palladium acetate (21.0 mg, 0.094 mmol), 4,5-bis(diphenylphosphino)-9,9-dimethylxanthene (108.0 mg, 0.187 mmol) and cesium carbonate (606.0 mg, 1.86 mmol) were added successively to 1,4-dioxane (6.0 mL). The resulting mixture was stirred at 95°C for 4 hours, cooled to room temperature, and filtered through diatomite, and the filter cake was washed with ethyl acetate (10.0 mL × 3). The filtrate was combined and concentrated to dryness under reduced pressure. The resulting residue was purified by silica gel column chromatography (gradient elution, eluent: petroleum ether/ethyl acetate = 9/1 -2/1) to obtain *tert*-butyl (5-chloro-8-methoxy-7-nitroquinolin-6-yl)carbamate (7d) (130.0 mg, yield 56%).

At room temperature, *tert*-butyl (5-chloro-8-methoxy-7-nitroquinolin-6-yl)carbamate (7d) (120.0 mg, 0.34 mmol), cesium carbonate (332.0 mg, 1.02 mmol), and methyl iodide (62.8 mg, 0.44 mmol) were added successively to N,N-dimethylformamide (3.0 mL). The resulting mixture was stirred at room temperature for 2 hours, and concentrated to dryness under reduced pressure. The resulting residue was purified by silica gel column chromatography (eluent: petroleum ether/ethyl acetate = 1.5/1) to obtain *tert*-butyl (5-chloro-8-methoxy-7-nitroquinolin-6-yl)(methyl)carbamate (7e) (122.0 mg, yield 96%).

At room temperature, *tert*-butyl (5-chloro-8-methoxy-7-nitroquinolin-6-yl)(methyl)carbamate (7e) (122.0 mg, 0.33 mmol) and lithium chloride (278.6 mg, 6.6 mmol) were added to N,N-dimethylformamide (4.0 mL). The resulting mixture was stirred at 120°C for 1.5 hours, cooled to room temperature, and concentrated to dryness under reduced pressure. A solution of hydrogen chloride in 1,4-dioxane (4.0 M) (1.0 mL) was added to the resulting residue, stirred at room temperature for 2 hours, and concentrated to dryness under reduced pressure. The pH of the aqueous phase was adjusted to about 9 to 10 by the addition of ammonia (25 to 28 wt.%, NH₃ content) to the resulting residue. The resulting mixture was filtered under reduced pressure, the filter cake was washed with water (0.5 mL × 2), and dried under vacuum to obtain 5-chloro-6-(methylamino)-7-nitroquinolin-8-ol (7) (31.0 mg, yield 37%).

¹H NMR (400 MHz, DMSO-*d6*) δ 8.39 (s, 1H), 8.12 (d, *J* = 8.4 Hz, 1H), 7.50 (dd, *J=* 8.4, 4.0 Hz, 1H), 5.62 (br s, 1H), 2.80 (s, 3H).

MS calculated: 253.03; MS found: 254.1, 256.1 [M+H]⁺.

### Example 8

### Synthesis of 3-methyl-7-nitroquinolin-8-ol (8)

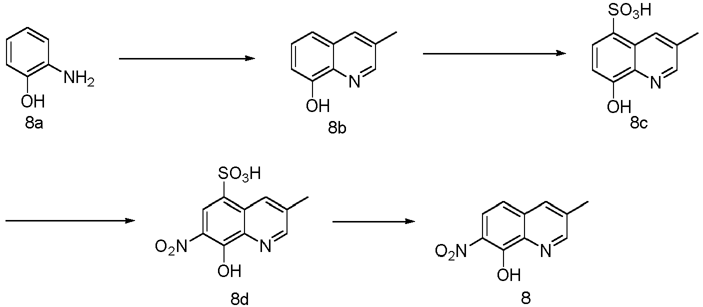

At 110°C, 2-methacrolein (1.0 mL, 4.0 mmol) was slowly added dropwise to a solution of 2-aminophenol (8a) (200.0 mg, 1.83 mmol) in hydrochloric acid (6.0 M) (10.0 mL). The reaction mixture was stirred at 110°C for 2 hours, and cooled to room temperature. The pH of the aqueous phase was adjusted to about 7 to 8 with aqueous sodium hydroxide solution (6.0 M). The resulting mixture was extracted with ethyl acetate (15.0 mL × 3). The combined organic phase was washed with saturated brine (20.0 mL), dried over anhydrous magnesium sulfate and filtered, and the filtrate was concentrated under reduced pressure. The resulting residue was purified by silica gel column chromatography (gradient elution, eluent: petroleum ether/ethyl acetate=5/1-3/1) to obtain 3-methylquinolin-8-ol (8b) (172.0 mg, yield 52%).

At room temperature, fuming sulfuric acid (18 to 24 weight% SO₃ content) (2.0 mL) was added to a solution of 3-methylquinolin-8-ol (8b) (100.0 mg, 0.63 mmol) in sulfuric acid (98 weight%) (4.0 mL). The reaction solution was stirred at 65°C for 2 hours, cooled to room temperature, and poured into ice water (20.0 mL). The resulting suspension was diluted with acetone (60.0 mL), stirred for 10 minutes, and filtered under reduced pressure. The filter cake was washed with acetone (15.0 mL × 2), and dried under vacuum to obtain 8-hydroxy-3-methylquinoline-5-sulfonic acid (8c) (139.0 mg, yield 77%).

At 0°C, nitric acid (65 weight%) (0.8 mL, 11.6 mmol) was added dropwise to a solution of 8-hydroxy-3-methylquinoline-5-sulfonic acid (8c) (139.0 mg, 0.58 mmol) in sulfuric acid (98 weight%) (4.0 mL). The resulting mixture was stirred at 0°C for 10 minutes, poured into ice water (15.0 mL) and continued to stir for 15 minutes. The pH of the aqueous phase was adjusted to about 5 to 6 with saturated aqueous potassium carbonate solution. The resulting mixture was filtered under reduced pressure, the filter cake was washed with water (1.0 mL × 3), and dried under vacuum to obtain 8-hydroxy-3-methyl-7-nitroquinoline-5-sulfonic acid (8d) (128.6 mg, yield 78%).

At room temperature, sulfuric acid (98 weight%) (0.8 mL) was slowly added to a suspension of 8-hydroxy-3-methyl-7-nitroquinoline-5-sulfonic acid (8d) (100.0 mg, 0.35 mmol) in acetic acid (2.8 mL). The resulting mixture was stirred at 120°C for 6 hours, cooled to room temperature, poured into ice water (15.0 mL) and continued to stir for 15 minutes. The pH of the aqueous phase was adjusted to about 6 to 7 with ammonia (25 to 28 weight%, NH₃ content). The resulting mixture was filtered under reduced pressure, the filter cake was washed with water (1.0 mL × 2), and dried under vacuum to obtain 3-methyl-7-nitroquinolin-8-ol (8) (49.3 mg, yield 69%).

¹H NMR (400 MHz, DMSO-*d*₆) δ: 8.87 (s, 1H), 8.25 (s, 1H), 8.01 (d, *J* = 9.2 Hz, 1H), 7.32 (d, *J* = 8.0 Hz, 1H), 2.54(s, 3H).

MS calculated: 204.05; MS found: 205.0 [M+H]⁺.

### Example 9

### Synthesis of 8-hydroxy-7-nitroquinoline-5-carbonitrile (9)

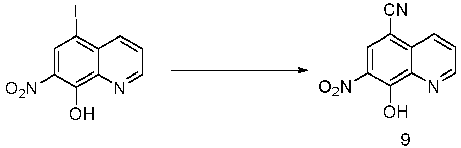

At room temperature, 5-iodo-7-nitroquinolin-8-ol (200.0 mg, 0.63 mmol), zinc cyanide (147.0 mg, 1.26 mmol) and tetrakis(triphenylphosphine)palladium (72.7 mg, 0.063 mmol) were added successively to N,N-dimethylformamide (3.0 mL). The reaction mixture was stirred in a microwave reactor at 140°C for 30 minutes, cooled to room temperature, and diluted with ammonia (2.0 M) (20.0 mL). The resulting mixture was extracted with ethyl acetate (20.0 mL × 2). The combined organic phase was washed with saturated brine (20.0 mL × 2), dried over anhydrous sodium sulfate and filtered, and the filtrate was concentrated under reduced pressure. The resulting residue was purified by silica gel column chromatography (eluent: dichloromethane/methanol = 10/1) to obtain 8-hydroxy-7-nitroquinoline-5-carbonitrile (9) (36.0 mg, yield 27%).

¹H NMR (400 MHz, DMSO-*d*₆) δ: 8.64 (s, 1H), 8.58 (s, 1H), 8.46 (s, *J* = 8.0 Hz, 1H), 7.93 - 7.90 (m, 1H), 7.06 (s, 1H).

MS calculated: 215.03; MS found: 216.0 [M+H]⁺.

### Example 10

### Synthesis of 4-methyl-7-nitroquinolin-8-ol (10)

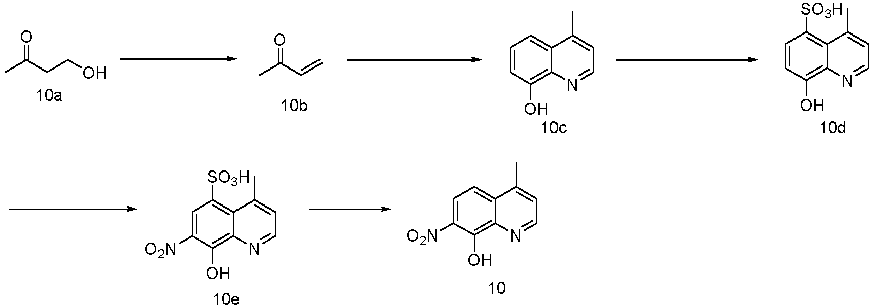

At room temperature, 4-hydroxy-2-butanone (10a) (12.3 g, 139.6 mmol) was added to a mixture of methanol (10.0 mL) and water (2.0 mL), followed by the addition of phosphoric acid (85 weight%) (0.4 mL). The resulting mixture was stirred at room temperature for 30 minutes, and distilled under reduced pressure (pressure range: 150.0 to 200.0 mmHg), and the fraction with a boiling point of 80°C was collected. Saturated brine (10.0 mL) was added to the collected fraction, and stirred at 4°C for 1 hour. The organic phase was separated, dried over anhydrous sodium sulfate, and filtered to obtain but-3-en-2-one (10b) (0.86 g, yield 9%).

At 110°C, but-3-en-2-one (10b) (0.86 g, 12.3 mmol) was slowly added dropwise to a solution of 2-aminophenol (200.0 mg, 1.83 mmol) in hydrochloric acid (6.0 M) (10.0 mL). The reaction mixture was stirred at 110°C for 2 hours, and cooled to room temperature. The pH of the aqueous phase was adjusted to about 7 to 8 with aqueous sodium hydroxide solution (6.0 M). The resulting mixture was extracted with ethyl acetate (20.0 mL × 3). The combined organic phase was washed with saturated brine (20.0 mL), dried over anhydrous magnesium sulfate and filtered, and the filtrate was concentrated under reduced pressure. The resulting residue was purified by silica gel column chromatography (gradient elution, eluent: petroleum ether/ethyl acetate=5/1-3/1) to obtain 4-methylquinolin-8-ol (10c) (171.9 mg, yield 63%).

At room temperature, fuming sulfuric acid (18 to 24 weight% SO₃ content) (2.0 mL) was added to a solution of 4-methylquinolin-8-ol (10c) (171.9 mg, 1.08 mmol) in sulfuric acid (98 weight%) (4.0 mL). The reaction solution was stirred at 65°C for 2 hours, cooled to room temperature, and poured into ice water (20.0 mL). The resulting suspension was diluted with acetone (60.0 mL), stirred for 10 minutes, and filtered under reduced pressure. The filter cake was washed with acetone (15.0 mL × 2), and dried under vacuum to obtain 8-hydroxy-4-methylquinoline-5-sulfonic acid (10d) (193.8 mg, yield 75%).

At 0°C, nitric acid (65 weight%) (0.8 mL, 11.6 mmol) was added dropwise to a solution of 8-hydroxy-4-methylquinoline-5-sulfonic acid (10d) (193.8 mg, 0.81 mmol) in sulfuric acid (98 weight%) (4.0 mL). The resulting mixture was stirred at 0°C for 10 minutes, poured into ice water (15.0 mL) and continued to stir for 15 minutes. The pH of the aqueous phase was adjusted to about 5 to 6 with saturated aqueous potassium carbonate solution. The resulting mixture was filtered under reduced pressure, the filter cake was washed with water (1.0 mL × 3), and dried under vacuum to obtain 8-hydroxy-4-methyl-7-nitroquinoline-5-sulfonic acid (10e) (179.6 mg, yield 78%).

At room temperature, sulfuric acid (98 weight%) (0.8 mL) was slowly added to a suspension of 8-hydroxy-4-methyl-7-nitroquinoline-5-sulfonic acid (10e) (90.0 mg, 0.32 mmol) in acetic acid (2.8 mL). The resulting mixture was stirred at 120°C for 6 hours, cooled to room temperature, poured into ice water (15.0 mL) and continued to stir for 15 minutes. The pH of the aqueous phase was adjusted to about 6 to 7 with ammonia (25 to 28 weight%, NH₃ content). The resulting mixture was filtered under reduced pressure, the filter cake was washed with water (1.0 mL × 2), and dried under vacuum to obtain 4-methyl-7-nitroquinolin-8-ol (10) (51.0 mg, yield 78%).

¹H NMR (400 MHz, DMSO-*d*₆) δ: 8.87 (s, 1H), 8.25 (s, 1H), 8.01 (d, *J* = 9.2 Hz, 1H), 7.32 (d, *J* = 8.0 Hz, 1H), 2.54(s, 3H).

MS calculated: 204.05; MS found: 205.0 [M+H]⁺.

### Example 11

### Synthesis of 4-cyclopropyl-7-nitroquinolin-8-ol (11)

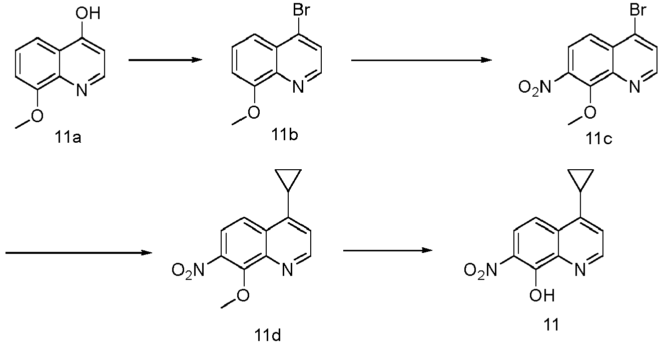

At room temperature, 4-hydroxy-8-methoxyquinoline (11a) (3.0 g, 17.1 mmol) and phosphorus tribromide (9.3 g, 34.2 mmol) were added successively to N,N-dimethylformamide (25.0 mL). The reaction mixture was stirred at 65°C for 6 hours, cooled to 0°C, and diluted with water (50.0 mL). The pH of the aqueous phase was adjusted to about 8 to 9 with ammonia (25 to 28 weight%, NH₃ content). The resulting mixture was extracted with ethyl acetate (100.0 mL × 3), the combined organic phase was dried over anhydrous sodium sulfate and filtered, and the filtrate was concentrated under reduced pressure. The resulting residue was purified by silica gel column chromatography (eluent: petroleum ether/ethyl acetate = 7/3) to obtain 4-bromo-8-methoxyquinoline (11b) (3.7 g, yield 91%).

At 0°C, 4-bromo-8-methoxyquinoline (11b) (3.0 g, 12.7 mmol), concentrated nitric acid (65 weight%) (10.0 mL) and concentrated sulfuric acid (98 weight%) (7.0 mL) were added successively to acetic anhydride (25.0 mL). The reaction mixture was warmed up to room temperature, stirred for 2 hours, and diluted with ice water (100.0 mL). The pH of the aqueous phase was adjusted to about 8 to 9 with ammonia (25 to 28 weight%, NH₃ content). The resulting mixture was extracted with dichloromethane (100.0 mL × 3), the combined organic phase was dried over anhydrous sodium sulfate and filtered, and the filtrate was concentrated under reduced pressure. The resulting residue was purified by silica gel column chromatography (eluent: petroleum ether/ethyl acetate = 4/1) to obtain 4-bromo-7-nitro-8-methoxyquinoline (11c) (0.30 g, yield 8%).

At room temperature, 4-bromo-7-nitro-8-methoxyquinoline (11c) (100.0 mg, 0.35 mmol), cyclopropylboronic acid (61.6 mg, 0.72 mmol), tetrakis(triphenylphosphine)palladium (82.0 mg, 0.07 mmol) and potassium carbonate (147.0 mg, 1.07 mmol) were added successively to a mixture of toluene (5.0 mL) and water (0.5 mL). The reaction solution was stirred at 100°C for 2 hours, cooled to room temperature, and diluted with water (20.0 mL). The resulting mixture was extracted with ethyl acetate (50.0 mL × 3), the combined organic phase was dried over anhydrous sodium sulfate and filtered, and the filtrate was concentrated under reduced pressure. The resulting residue was purified by silica gel column chromatography (eluent: petroleum ether/ethyl acetate = 3/1) to obtain 4-cyclopropyl-7-nitro-8-methoxyquinoline (11d) (60.0 mg, yield 70%).

At room temperature, 4-cyclopropyl-7-nitro-8-methoxyquinoline (11d) (60.0 mg, 0.25 mmol) and lithium chloride (105.0 mg, 2.5 mmol) were added to N,N-dimethylformamide (2.0 mL). The reaction solution was stirred at 180°C for 1 hour, and cooled to room temperature. The reaction solution was concentrated under reduced pressure to remove the organic solvent. Water (3.0 mL) was added to the resulting residue, stirred for 3 minutes, and filtered under reduced pressure. The filter cake was washed successively with water (0.5 mL × 2) and ethanol (0.5 mL × 2), and dried under vacuum to obtain 4-cyclopropyl-7-nitroquinolin-8-ol (11) (16.0 mg, yield 28%).

¹H-NMR (400 Hz, DMSO-*d*₆) δ: 8.50 - 8.40 (m, 1H), 8.00 - 7.88 (m, 1H), 7.15 - 7.06 (m, 1H), 6.95 - 6.86 (m, 1H), 2.30 - 2.45 (m, 1H), 1.20 - 1.05 (m, 2H), 0.90 - 0.70 (m, 2H).

MS calculated: 230.07; MS found: 231.1 [M+H]⁺.

### Example 12

### Synthesis of 4-chloro-7-nitroquinolin-8-ol (12)

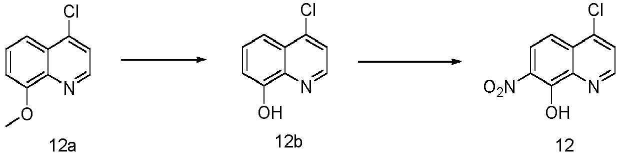

At 0°C, a solution of boron tribromide in dichloromethane (1.0 M) (50.0 mL, 50.0 mmol) was slowly added dropwise to a solution of 4-chloro-8-methoxyquinoline (12a) (2.0 g, 10.0 mmol) in dichloromethane (20.0 ml). The reaction mixture was warmed up to room temperature and stirred for 30 minutes. The pH of the aqueous phase was adjusted to about 8 to 9 with saturated aqueous sodium bicarbonate solution. The organic phase was separated, and the aqueous phase was extracted with dichloromethane (20.0 mL × 2). The combined organic phase was dried over anhydrous sodium sulfate, filtered, and concentrated under reduced pressure. The resulting residue was dried under vacuum to obtain crude 4-chloro-8-hydroxyquinoline (12b) (1.6 g, crude yield 89%).

At 0°C, sodium nitrite (2.0 g, 30.0 mmol) was added in batches to a suspension of 4-chloro-8-hydroxyquinoline (1.08 g, 6.0 mmol) (12b) in hydrochloric acid (2.0 M) (36.0 mL). The reaction mixture was stirred at 0°C for 1 hour, and then at room temperature for 4 hours, and filtered under reduced pressure. The filter cake was added to methanol (30.0 mL), stirred for 15 minutes, and filtered under reduced pressure. The resulting filter cake was washed with methanol (5.0 mL × 2), and dried under vacuum to obtain 4-chloro-7-nitroquinolin-8-ol (12) (0.80 g, yield 59%).

¹H-NMR (400 MHz, DMSO-d6): δ 7.68 (d, *J* = 9.6Hz, 1H), 8.04 (d, *J* = 4.8Hz, 1H), 8.18 (d, *J* = 9.6Hz,1H), 8.95 (d, *J* = 4.8Hz,1H).

MS calculated: 224.60; MS found: 225.0 [M+H]⁺.

### Example 13

### Synthesis of 7-nitro-4-(pyrrolidin-1-yl)quinolin-8-ol

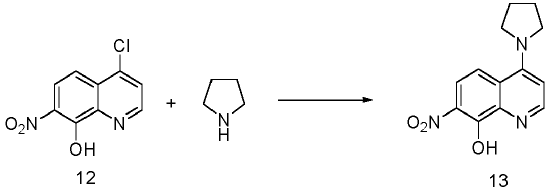

At room temperature, tetrahydropyrrole (142.0 mg, 4.45 mmol) was added to a solution of 4-chloro-7-nitroquinolin-8-ol (12) (100.0 mg, 0.45 mmol) in N,N-dimethylformamide (5.0 mL). The resulting mixture was stirred at 120°C for 2 hours, cooled to room temperature, and concentrated under reduced pressure to remove the organic solvent. Water (10.0 mL) was added to the resulting residue, stirred for 3 minutes, and filtered under reduced pressure. The filter cake was washed with water (1.0 mL × 3), and dried under vacuum to obtain 7-nitro-4-(pyrrolidin-1-yl)quinolin-8-ol (13) (92.0 mg, yield 79%).

¹H-NMR (400 MHz, DMSO-*d*₆) δ: 13.10 (br s, 1H), 8.08 (d, *J* = 5.6 Hz, 1H), 7.80 (d, *J* = 9.6 Hz, 1H), 6.92 (d, *J* = 10.0 Hz, 1H), 6.73 (d, *J* = 4.4 Hz, 1H), 3.84 (br s, 4H), 2.01 (br s, 4H).

MS calculated: 259.10; MS found: 260.1 [M+H]⁺.

### Example 14

### Synthesis of 4-morpholino-7-nitroquinolin-8-ol (14)

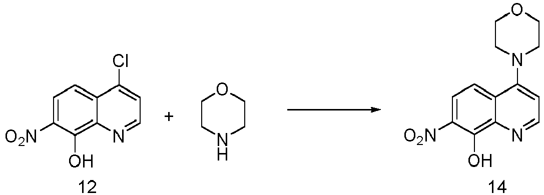

In accordance with the same synthesis method of Example 13, 4-morpholino-7-nitroquinolin-8-ol (14) (110.0 mg, yield 91%) was obtained from 4-chloro-7-nitroquinolin-8-ol (12) (100.0 mg, 0.44 mmol) and morpholine (192.0 mg, 2.2 mmol).

¹H NMR (400 MHz, DMSO-*d*₆) δ: 8.44 (d, *J* = 5.6 Hz, 1H), 7.94 (d, *J* = 9.6 Hz, 1H), 7.22 (d, *J* = 6.0 Hz, 1H), 6.66 (d, *J* = 9.6 Hz, 1H), 3.85 (br s, 4H), 3.54 (br s, 4H).

MS calculated: 275.09; MS found: 276.1 [M+H]⁺.

### Example 15

### Synthesis of 4-(1H-imidazol-1-yl)-7-nitroquinolin-8-ol (15)

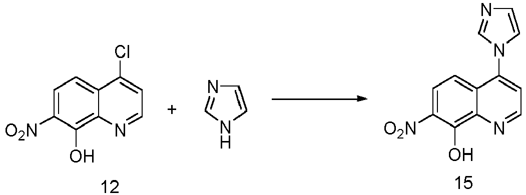

In accordance with the same synthesis method of Example 13, 4-(*1H*-imidazol-1-yl)-7-nitroquinolin-8-ol (15) (50.0 mg, yield 85%) was obtained from 4-chloro-7-nitroquinolin-8-ol (12) (50.0 mg, 0.23 mmol) and imidazole (61.5 mg, 0.9 mmol).

¹H-NMR (400 MHz, DMSO-*d₆*) δ: 8.15 (s, 1H), 8.05 - 8.08 (m, 2H), 7.78 (s,1H), 7.71 (m, 1H), 7.26(s, 1H),7.21(s, 1H).

MS calculated: 256.06; MS found: 257.1[M+H]⁺.

### Example 16

### Synthesis of 4-bromo-7-nitrouinolin-8-ol (16)

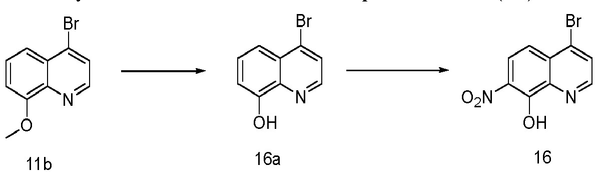

At 0°C, a solution of boron tribromide in dichloromethane (1.0 M) (36.4 mL, 36.4 mmol) was slowly added dropwise to a solution of 4-bromo-8-methoxyquinoline (11b) (1.9 g, 8.1 mmol) in dichloromethane (25.0 ml). The reaction mixture was warmed up to room temperature and stirred for 24 hours. The pH of the aqueous phase was adjusted to about 8 to 9 with saturated aqueous sodium bicarbonate solution. The organic phase was separated, and the aqueous phase was extracted with dichloromethane (50.0 mL × 3). The combined organic phase was dried over anhydrous sodium sulfate and filtered, and the filtrate was concentrated under reduced pressure. The resulting residue was purified by silica gel column chromatography (eluent: dichloromethane/methanol = 19/1) to obtain 4-bromoquinolin-8-ol (16a) (1.0 g, yield 55%).

At 0°C, sodium nitrite (1.4 g, 20.3 mmol) was added in batches to a suspension of 4-bromoquinolin-8-ol (16a) (460 mg, 2.05 mmol) in hydrochloric acid (2.0 M) (30.0 mL). The reaction mixture was warmed up to room temperature and stirred for 24 hours. The pH of the aqueous phase was adjusted to about 8 to 9 with ammonia (25 to 28 weight%, NH₃ content). The resulting mixture was filtered under reduced pressure, the filter cake was washed with ethanol (4.0 mL × 3), and dried under vacuum to obtain 4-bromo-7-nitroquinolin-8-ol (16) (500.0 mg, yield 91%).

¹H-NMR (400 Hz, DMSO-*d*₆) δ: 8.84 - 7.77 (m, 1 H), 8.20 - 8.12 (m, 2 H), 7.60 - 7.55 (m, 1 H).

MS calculated: 267.95; MS found: 269.0 [M+H]⁺.

### Example 17

### Synthesis of 4-(dimethylamino)-7-nitroquinolin-8-ol (17)

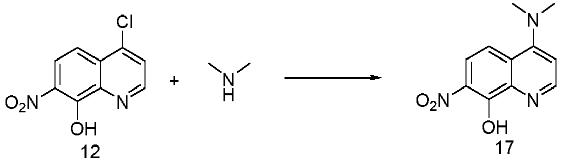

At room temperature, 4-chloro-7-nitroquinolin-8-ol (12) (100.0 mg, 0.44 mmol) was added to aqueous dimethylamine solution (40 weight%) (10.0 mL). The reaction mixture was stirred at 110°C for 2 hours, cooled to room temperature, and concentrated under reduced pressure to remove the solvent. Ethanol (5.0 mL) was added to the resulting residue, stirred for 3 minutes, and filtered under reduced pressure. The filter cake was washed with ethanol (1.0 mL × 2), and dried under vacuum to obtain 4-(dimethylamino)-7-nitroquinolin-8-ol (17) (36.0 mg, yield 35%).

¹H NMR (400 MHz, DMSO-*d₆*) δ: 8.18 (d, *J* = 6.8 Hz, 1H), 7.84 (d, *J* = 9.8 Hz, 1H), 6.95 (d, *J* = 6.8 Hz, 1H), 6.70 (d, *J* = 9.8 Hz, 1H), 3.32 (s, 6H).

MS calculated: 233.08; MS found: 234.1 [M+H]⁺.

### Example 18

### Synthesis of 4-(4-methylpiperazinyl)-7-nitroquinolin-8-ol (18)

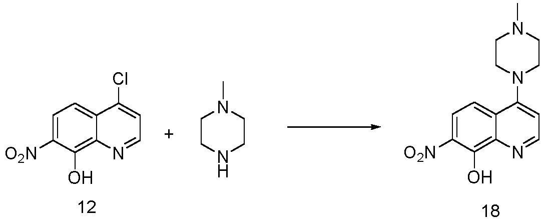

In accordance with the same synthesis method of Example 13, 4-(4-methylpiperazinyl)-7-nitroquinolin-8-ol (18) (40.0 mg, yield 63%) was obtained from 4-chloro-7-nitroquinolin-8-ol (12) (50.0 mg, 0.22 mmol) and 1-methylpiperazine (116.0 mg, 1.1 mmol).

¹H NMR (400 MHz, DMSO-*d*₆) δ: 8.57 (d, *J* = 6.0 Hz, 1H), 7.98 (d, *J* = 9.6 Hz, 1H), 7.34 (d, *J* = 6.0 Hz, 1H), 6.80 (d, *J* = 9.6 Hz, 1H), 3.55 - 3.21 (br s, 8H), 2.86 (s, 3H).

MS calculated: 288.12; MS found: 289.1 [M+H]⁺.

### Example 19

### Synthesis of 7-nitro-4-(3-oxetanylamino)quinolin-8-ol (19)

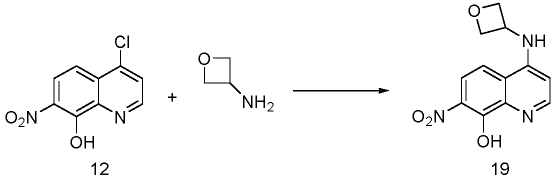

At room temperature, 3-oxetamine (78.9 mg, 1.08 mmol) was added to a solution of 4-chloro-7-nitroquinolin-8-ol (12) (80.0 mg, 0.36 mmol) in dimethyl sulfoxide (5.0 mL). The resulting mixture was stirred at 80°C for 4 hours, cooled to room temperature, and concentrated under reduced pressure to remove the organic solvent. Water (5.0 mL) was added to the resulting residue, stirred for 3 minutes, and filtered under reduced pressure. The resulting filter cake was mixed with methanol (10.0 mL), stirred for 3 minutes, and filtered under reduced pressure. The filter cake was washed with methanol (1.0 mL × 2), and dried under vacuum to obtain 7-nitro-4-(3-oxetanylamino)quinolin-8-ol (19) (65.0 mg, yield 69%).

¹H NMR (400 MHz, DMSO-*d*₆) δ: 8.89 (d, *J* = 4.4 Hz, 1H), 8.16 (d, *J* = 6.8 Hz, 1H), 7.96 (d, *J* = 9.6 Hz, 1H), 6.93 (d, *J* = 9.6 Hz, 1H), 6.57 (d, *J* = 6.8 Hz, 1H), 5.01 - 4.96 (m, 1H), 4.93 (t, *J* = 6.0 Hz, 2H), 4.74 (t, *J* = 6.0 Hz, 2H).

MS calculated: 261.07; MS found: 262.1 [M+H]⁺.

### Example 20

### Synthesis of 4-(2-methylpiperidinyl)-7-nitroquinolin-8-ol (20)

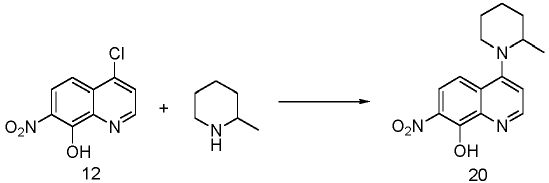

In accordance with the same synthesis method of Example 13, 4-(2-methylpiperidinyl)-7-nitroquinolin-8-ol (20) (19.0 mg, yield 15%) was obtained from 4-chloro-7-nitroquinolin-8-ol (12) (100.0 mg, 0.44 mmol) and 2-methylpiperidine (220.0 mg, 2.2 mmol).

¹H NMR (400 MHz, DMSO-*d*₆) δ: 8.02 - 7.86 (m, 2H), 7.20 - 7.10 (m, 1H), 6.76 - 6.62 (m, 1H), 3.14 - 2.83 (m, 3H), 1.85 - 1.55 (m, 6H), 1.10 - 0.95 (m, 3H).

MS calculated: 287.13; MS found: 288.1 [M+H]⁺.

### Example 21

### Synthesis of 4-methoxy-7-nitroquinolin-8-ol (21)

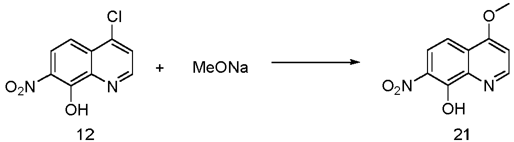

In accordance with the same synthesis method of Example 13, 4-methoxy-7-nitroquinolin-8-ol (21) (26.0 mg, yield 54%) was obtained from 4-chloro-7-nitroquinolin-8-ol (12) (50.0 mg, 0.22 mmol) and sodium methoxide (120.0 mg, 2.2 mmol).

¹H NMR (400 MHz, DMSO-*d*₆) δ: 8.56 - 8.42 (m, 1H), 7.91 - 7.84 (m, 1H), 7.10 - 7.03 (m, 1H), 6.65 - 6.56 (m, 1H), 4.01 (s, 3H).

MS calculated: 220.05; MS found: 221.0 [M+H]⁺.

### Example 22

### Synthesis of 4-phenyl-7-nitroquinolin-8-ol (22)

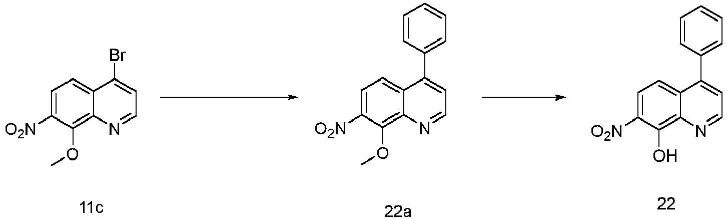

At room temperature, 4-bromo-7-nitro-8-methoxyquinoline (11c) (60.0 mg, 0.25 mmol), phenylboronic acid (62.0 mg, 0.50 mmol), tetrakis(triphenylphosphine)palladium (58.0 mg, 0.05 mmol) and potassium carbonate (100.0 mg, 0.75 mmol) were added successively to a mixed solvent of toluene (2.0 mL) and water (0.2 mL). The reaction mixture was stirred at 110°C for 2 hours, cooled to room temperature, and diluted with water (10.0 mL). The resulting mixture was extracted with ethyl acetate (10.0 mL × 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 7/3) to obtain 4-phenyl-7-nitro-8-methoxyquinoline (22a) (60.0 mg, yield 86%).

At room temperature, 4-phenyl-7-nitro-8-methoxyquinoline (22a) (60.0 mg, 0.21 mmol) and lithium chloride (90.0 mg, 2.1 mmol) were added to N,N-dimethylformamide (2.0 mL). The reaction mixture was stirred at 180°C for 1 hour, cooled to room temperature, and concentrated under reduced pressure to remove the organic solvent. Water (3.0 mL) was added to the resulting residue, stirred for 3 minutes, and filtered under reduced pressure. The filter cake was washed successively with water (0.5 mL × 2) and ethanol (0.5 mL × 2), and dried under vacuum to obtain 4-phenyl-7-nitroquinolin-8-ol (22) (25.0 mg, yield 45%).

¹H-NMR (400 MHz, DMSO-*d*₆) δ: 8.70 - 8.60 (m, 1H), 7.93 - 7.84 (m, 1H), 7.61 - 7.48 (m, 5H), 7.46 - 7.41 (m, 1H), 6.42 - 6.33 (m, 1H).

MS calculated: 266.07; MS found: 267.1 [M+H]⁺.

### Example 23

### Synthesis of 4-(2,5-dihydro-1H-pyrrolyl)-7-nitroquinolin-8-ol (23)

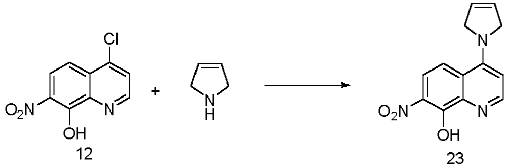

In accordance with the same synthesis method of Example 19, 4-chloro-7-nitroquinolin-8-ol (12) (60.0 mg, 0.27 mmol) and 3-pyrroline (91.0 mg, 1.32 mmol) were reacted at 100°C to obtain 4-(2,5-dihydro-1*H*-pyrrolyl)-7-nitroquinolin-8-ol (23) (34.6 mg, yield 50%).

¹H-NMR (400 MHz, DMSO-*d*₆) δ: 8.20 - 8.12 (m, 1 H), 7.87 - 7.80 (m, 1 H), 7.14 - 7.07 (m, 1 H), 6.75 - 6.70 (m, 1 H), 6.11 (s, 2 H), 4.75 (s, 4 H).

MS calculated: 257.08; MS found: 258.1 [M+H]⁺.

### Example 24

### Synthesis of 4-(2-(dimethylamino)ethoxy)-7-nitroquinolin-8-ol (24)

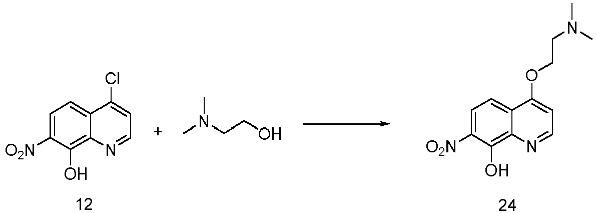

At room temperature, sodium hydride (60 weight%, a mixture in mineral oil) (130.0 mg, 3.24 mmol) was added to a solution of N,N-dimethylethanolamine (238.0 mg, 2.7 mmol) in dimethyl sulfoxide (2.0 mL). The resulting mixture was stirred at room temperature for 1 hour, followed by the addition of 4-chloro-7-nitroquinolin-8-ol (12) (60.0 mg, 0.27 mmol). The resulting mixture was warmed up to 95°C, stirred for 2 hours, cooled to room temperature, and concentrated under reduced pressure to remove the organic solvent. Water (3.0 mL) was added to the resulting residue, stirred for 3 minutes, and filtered under reduced pressure. The filter cake was washed successively with water (0.5 mL × 2) and ethanol (0.5 mL × 2), and dried under vacuum to obtain 4-(2-(dimethylamino)ethoxy)-7-nitroquinolin-8-ol (24) (25.7 mg, yield 34%).

¹H NMR (400 MHz, DMSO-*d*₆) δ: 8.90 - 8.70 (m, 1H), 8.20 - 8.10 (m, 1H), 7.60 - 7.35 (m, 2H), 4.71 - 4.50 (m, 2H), 3.80 - 3.60 (m, 2H), 3.10 - 2.80 (m, 4H).

MS calculated: 277.11; MS found: 278.1 [M+H]⁺.

### Example 25

### Synthesis of 4-(8-hydroxy-7-nitroquinolin-4-yl)-N,N-dimethylpiperazine-1-carboxamide (25)

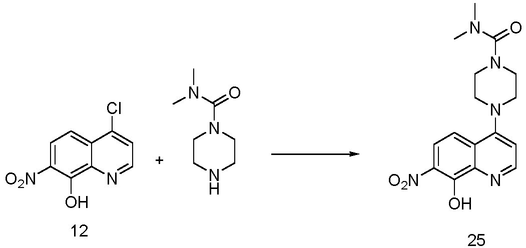

In accordance with the same synthesis method of Example 19, 4-chloro-7-nitroquinolin-8-ol (12) (60.0 mg, 0.27 mmol) and N,N-dimethylpiperazine-1-carboxamide (126.0 mg, 0.81 mmol) were reacted at 100°C to obtain 4-(8-hydroxy-7-nitroquinolin-4-yl)-N,N-dimethylpiperazine-1-carboxamide (25) (20.0 mg, yield 21%).

¹H NMR (400 MHz, DMSO-*d*₆) δ: 8.46 - 8.42 (m, 1H), 7.97 - 7.91 (m, 1H), 7.23 - 7.18 (m, 1H), 6.73 - 6.67 (m, 1H), 3.69 - 3.63 (m, 7H), 3.60 - 3.53 (m, 4H). MS calculated: 345.14; MS found: 346.1 [M+H]⁺.

### Example 26

### Synthesis of methyl 4-(8-hydroxy-7-nitroquinolin-4-yl)-N,N-dimethylpiperazine-1-carboxylate (26)

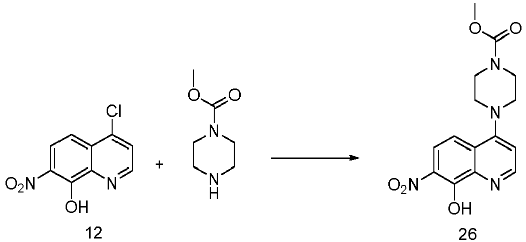

In accordance with the same synthesis method of Example 19, 4-chloro-7-nitroquinolin-8-ol (12) (60.0 mg, 0.27 mmol) and methyl piperazine-1-carboxylate (130.0 mg, 0.90 mmol) were reacted at 100°C to obtain methyl 4-(8-hydroxy-7-nitroquinolin-4-yl)-N,N-dimethylpiperazine-1-carboxylate (26) (21.7 mg, yield 24%).

¹H NMR (400 MHz, DMSO-*d*₆) δ: 8.46 - 8.42 (m, 1H), 7.97 - 7.91 (m, 1H), 7.23 - 7.18 (m, 1H), 6.73 - 6.67 (m, 1H), 3.69 - 3.63 (m, 7H), 3.60 - 3.53 (m, 4H).

MS calculated: 332.11; MS found: 333.1 [M+H]⁺.

### Example 27

### Synthesis of 4-fluoro-7-nitroquinolin-8-ol (27)

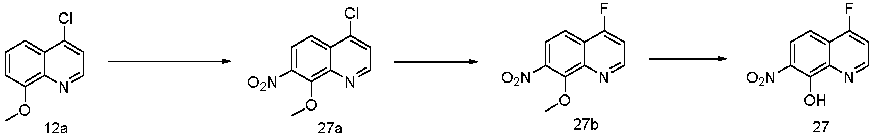

At 0°C, nitric acid (65 weight%) (3.0 mL, 43.6 mmol) was slowly added to a solution of 4-chloro-8-methoxyquinoline (12a) (5.0 g, 25.1 mmol) in acetic anhydride (40.0 mL), and stirred for 10 minutes. Sulfuric acid (98 weight%) (3.0 mL, 55.2 mmol) was slowly added dropwise. The reaction mixture was warmed up to room temperature, stirred for 1 hour, and diluted with ice water (50.0 mL). The pH of the aqueous phase was adjusted to about 8 to 9 with ammonia (25 to 28 weight%, NH₃ content). The resulting mixture was extracted with dichloromethane (10.0 mL × 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 2/1) to obtain 4-chloro-7-nitro-8-methoxyquinoline (27a) (1.19 g, yield 20%).

At room temperature, 4-chloro-7-nitro-8-methoxyquinoline (27a) (150.0 mg, 0.63 mmol) and cesium fluoride (480.0 mg, 3.1 mmol) were added to dimethyl sulfoxide (2.0 mL). The reaction mixture was stirred at 160°C for 30 minutes, cooled to room temperature, diluted with dichloromethane (10.0 mL), and washed with water (10.0 mL × 2). The organic phase was dried over anhydrous sodium sulfate and filtered, and the filtrate was concentrated under reduced pressure. The resulting residue was purified by silica gel column chromatography (eluent: petroleum ether/ethyl acetate = 4/1) to obtain 4-fluoro-7-nitro-8-methoxyquinoline (27b) (50.0 mg, yield 36%).

At room temperature, 4-fluoro-7-nitro-8-methoxyquinoline (27b) (50.0 mg, 0.22 mmol) and lithium chloride (100.0 mg, 2.2 mmol) were added to N,N-dimethylformamide (2.0 mL). The reaction mixture was stirred at 120°C for 1 hour, cooled to room temperature, and concentrated under reduced pressure to remove the organic solvent. Water (5.0 mL) was added to the resulting residue, stirred for 3 minutes, and filtered under reduced pressure. The filter cake was washed successively with water (0.5 mL × 2) and ethanol (0.5 mL × 2), and dried under vacuum to obtain 4-fluoro-7-nitroquinolin-8-ol (27) (46.0 mg, yield 98%).

¹H NMR (400 MHz, DMSO-*d*₆) δ 8.84 - 8.37 (m, 1H), 7.95 (d, *J* = 8.0 Hz, 1H), 7.50 - 7.22 (m, 1H), 6.44 (d, *J* = 8.0 Hz, 1H).

MS calculated: 208.03; MS found: 209.0 [M+H]⁺.

### Example 28

### Synthesis of methyl (8-hydroxy-7-nitroquinolin-4-yl)-L-prolinate (28)

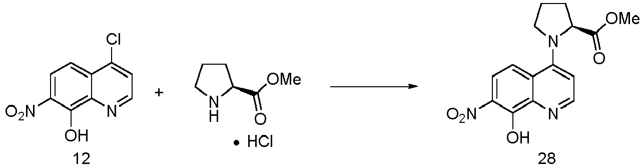

At room temperature, methyl *L*-prolinate hydrochloride (178.0 mg, 1.07 mmol) and N,N-diisopropylethylamine (139.0 mg, 1.08 mmol) were added to dimethyl sulfoxide (1.5 mL). The reaction solution was stirred at room temperature for 1 hour, followed by the addition of 4-chloro-7-nitroquinolin-8-ol (12) (80.0 mg, 0.36 mmol). The resulting mixture was stirred at 100°C for 2 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile=100%/0 - 70%/30%, gradient elution, 0.1% formic acid was added to the mobile phase by volume percentage, and the flow rate was 20.0 mL/min) to obtain the product methyl (8-hydroxy-7-nitroquinolin-4-yl)-*L*-prolinate (28) (115.0 mg, yield 99%).

¹H NMR (400 MHz, DMSO-*d*₆) δ: 8.22 - 8.11 (m, 1H), 7.89 - 7.72 (m, 1H), 6.85 - 6.77 (m, 1H), 6.74 - 6.66 (m, 1H), 5.16 - 5.06 (m, 1H), 4.10 - 3.95 (m, 2H), 3.67 (s, 3H), 2.16 - 1.89 (m, 4H).

MS calculated: 317.10; MS found: 318.1 [M+H]⁺.

### Example 29

### Synthesis of 4,5-dichloro-7-nitroquinolin-8-ol (29)

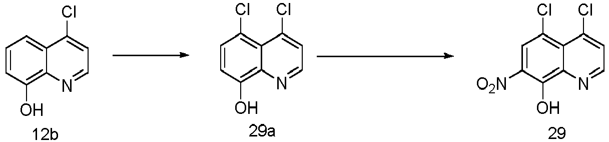

At 0°C, N-chlorosuccinimide (588 mg, 4.4 mmol) was added in three batches to a solution of 4-chloroquinolin-8-ol (12b) (790.0 mg, 4.4 mmol) in sulfuric acid (98 weight%) (5.0 mL). The reaction mixture was stirred at 0°C for 0.5 hours, and then at room temperature for 2 hours. The pH of the aqueous phase was adjusted to about 8 to 9 with ammonia (25 to 28 weight%, NH₃ content). The resulting mixture was extracted with dichloromethane (20.0 mL × 3). The combined organic phase was washed with saturated brine (20.0 mL), dried over anhydrous sodium sulfate, filtered, and concentrated under reduced pressure. The resulting residue was purified by silica gel column chromatography (eluent: petroleum ether/ethyl acetate = 1/1) to obtain 4,5-dichloroquinolin-8-ol (29a) (703.0 mg, yield 75%).

At 0°C, sodium nitrite (2.28 g, 33.0 mmol) was added in batches to a suspension of 4,5-dichloroquinolin-8-ol (29a) (0.703 g, 3.3 mmol) in hydrochloric acid (3.0 M) (20.0 mL). The reaction mixture was stirred at 0°C for 0.5 hours, and then at room temperature for 12 hours. The pH of the aqueous phase was adjusted to about 8 to 9 with ammonia (25 to 28 weight%, NH₃ content). The resulting mixture was filtered under reduced pressure, the filter cake was washed with water (3.0 mL × 2), and dried under vacuum to obtain 4,5-dichloro-7-nitroquinolin-8-ol (29) (0.641 g, yield 75%).

¹H NMR (400 MHz, DMSO-*d*₆) δ 8.93 (d, *J* = 4.8 Hz, 1H), 8.22 (s, 1H), 8.05 (d, *J* = 4.8 Hz, 1H).

MS calculated: 257.96; MS found: 259.0 [M+H]⁺.

### Example 30

### Synthesis of 7-nitroquinoline-4,8-diol (30)

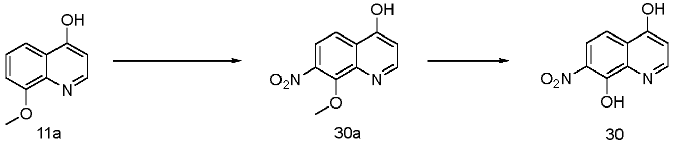

At 0°C, nitric acid (65 weight%) (10.0 mL, 145.5 mmol) was slowly added to a solution of 4-hydroxy-8-methoxyquinoline (11a) (3.0 g, 17.14 mmol) in acetic anhydride (25.0 mL), and stirred for 10 minutes. Sulfuric acid (98 weight%) (7.0 mL, 128.7 mmol) was slowly added dropwise. The reaction mixture was warmed up to room temperature, stirred for 2 hours, and diluted with ice water (100.0 mL). The pH of the aqueous phase was adjusted to about 8 to 9 with ammonia (25 to 28 weight%, NH₃ content). The resulting mixture was extracted with dichloromethane (100.0 mL × 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/4) to obtain 8-methoxy-7-nitroquinolin-4-ol (30a) (0.30 g, yield 8%).

At room temperature, 4-cyclopropyl-7-nitro-8-methoxyquinoline (70.0 mg, 0.32 mmol) and lithium chloride (200.0 mg, 4.78 mmol) were added to N,N-dimethylformamide (2.0 mL). The reaction mixture was stirred at 180°C for 1 hour, cooled to room temperature, and concentrated under reduced pressure to remove the organic solvent. Water (3.0 mL) was added to the resulting residue, stirred for 3 minutes, and filtered under reduced pressure. The filter cake was washed successively with water (0.5 mL × 2) and ethanol (0.5 mL × 2), and dried under vacuum to obtain 7-nitroquinoline-4,8-diol (30) (37.0 mg, yield 56%).

¹H NMR (400 MHz, DMSO-*d*₆) δ: 11.31 (br s, 1H), 7.71 - 6.65 (m, 1H), 7.62 - 7.56 (m, 1H), 6.65 - 6.55 (m, 1H), 6.10 - 6.00 (m, 1H).

MS calculated: 206.03; MS found: 207.1 [M+H]⁺.

### Example 31

### Synthesis of methyl 4-(5-chloro-8-hydroxy-7-nitroquinolin-4-yl)-piperazine-1-carboxylate (31)

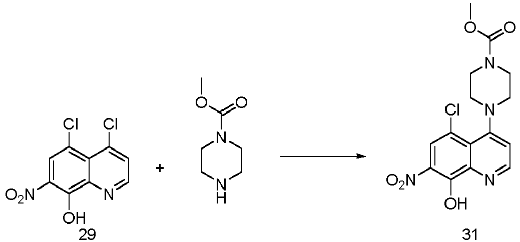

At room temperature, 4,5-dichloro-7-nitroquinolin-8-ol (29) (50.0 mg, 0.19 mmol) and methyl piperazine-1-carboxylate (82.2 mg, 0.57 mmol) were added to N,N-dimethylformamide (2.0 mL). The reaction solution was stirred at 100°C for 1 hour, cooled to room temperature, and concentrated under reduced pressure to remove the organic solvent. Water (3.0 mL) was added to the resulting residue, stirred for 3 minutes, and filtered under reduced pressure. The filter cake was washed successively with water (0.5 mL × 2) and ethanol (0.5 mL × 2), and dried under vacuum to obtain methyl 4-(5-chloro-8-hydroxy-7-nitroquinolin-4-yl)-piperazine-1-carboxylate (30.0 mg, yield 43%).

¹H NMR (400 MHz, DMSO-*d*₆) δ 8.49 - 8.29 (m, 1H), 8.00 - 7.85 (m, 1H), 7.46 - 7.28 (m, 1H), 3.63 (s, 3H), 3.37 (br s, 8H).

MS calculated: 366.07; MS found: 367.1 [M+H]⁺.

### Example 32

### Synthesis of 4-cyclohexyl-7-nitroquinolin-8-ol (32)

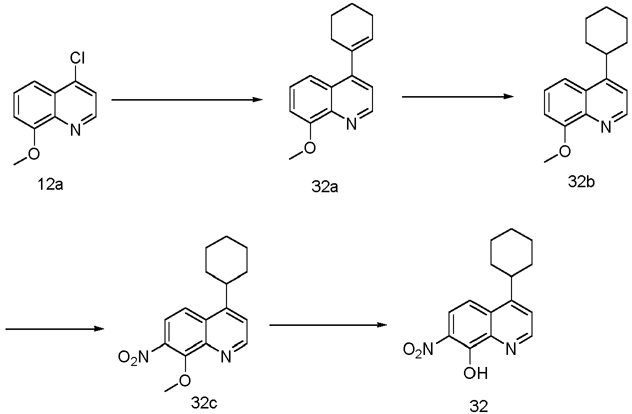

At room temperature, 4-chloro-8-methoxyquinoline (12a) (1.0 g, 5.2 mmol), cyclohexenylboronic acid (0.975 g, 7.7 mmol), tetrakis(triphenylphosphine)palladium (0.600 g, 0.50 mmol) and potassium carbonate (1.5 g, 11.0 mmol) were added successively to a mixed solvent of toluene (10.0 mL), ethanol (1.0 mL) and water (2.0 mL). The reaction mixture was stirred at 100°C for 12 hours, cooled to room temperature, and diluted with water (20.0 mL). The resulting mixture was extracted with ethyl acetate (50.0 mL × 2). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/1) to obtain 4-cyclohexenyl-8-methoxyquinoline (32a) (1.1 g, yield 89%).

At room temperature, 4-cyclohexenyl-8-methoxyquinoline (32a) (850.0 mg, 3.5 mmol) and palladium/carbon (palladium loading: 10 weight%) (85.0 mg, 0.080 mmol) were successively added to methanol (20.0 mL). The reaction mixture was stirred under a hydrogen atmosphere for 3 hours, and filtered under reduced pressure. The filter cake was washed with ethyl acetate (10.0 mL × 3), and the resulting filtrate was concentrated to dryness under reduced pressure to obtain 4-cyclohexyl-8-methoxyquinoline (32b) (850.0 mg, yield 99%).

At 0°C, nitric acid (65 weight%) (1.0 mL, 14.5 mmol) was slowly added to a solution of 4-cyclohexyl-8-methoxyquinoline (32b) (800.0 mg, 3.3 mmol) in acetic anhydride (5.0 mL), and stirred for 10 minutes. Concentrated sulfuric acid (0.5 mL, 9.2 mmol) was slowly added dropwise. The reaction mixture was stirred at 0°C for 1 hour, and diluted with water (20.0 mL). The pH of the aqueous phase was adjusted to about 9 to 10 with ammonia (25 to 28 weight%, NH₃ content). The resulting mixture was extracted with ethyl acetate (30.0 mL × 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/1) to obtain 4-cyclohexyl-7-nitro-8-methoxyquinoline (32c) (220.0 mg, yield 23%).

At room temperature, 4-cyclohexyl-7-nitro-8-methoxyquinoline (32c) (150.0 mg, 0.53 mmol) and lithium chloride (230.0 mg, 5.3 mmol) were added to N,N-dimethylformamide (5.0 mL). The reaction mixture was stirred at 160°C for 1 hour, cooled to room temperature, and concentrated under reduced pressure to remove the organic solvent. Water (2.0 mL) was added to the resulting residue, stirred for 3 minutes, and filtered under reduced pressure. The filter cake was washed with water (1.0 mL × 2), and dried under vacuum to obtain 4-cyclohexyl-7-nitroquinolin-8-ol (32) (124.9 mg, yield 87%).

¹H NMR (400 MHz, DMSO-*d*₆) δ 8.52 (d, *J* = 4.0 Hz, 1H), 7.93 (d, *J* = 8.0 Hz, 1H), 7.40 (d, *J* = 4.0 Hz, 1H), 6.71 (d, *J* = 8.0 Hz, 1H), 3.25 - 3.09 (m, 1H), 1.94 - 1.78 (m, 4H), 1.58 - 1.38 (m, 4H), 1.38 - 1.20 (m, 2H).

MS calculated: 272.12; MS found: 273.1 [M+H]⁺.

### Example 33

### Synthesis of 4-(5-chloro-8-hydroxy-7-nitroquinolin-4-yl)-1-acetylpiperazine (33)

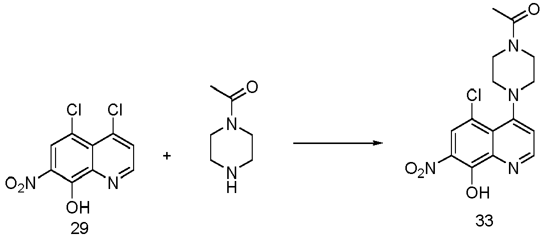

In accordance with the same synthesis method of Example 31, 4-(5-chloro-8-hydroxy-7-nitroquinolin-4-yl)-1-acetylpiperazine (33) (38.0 mg, yield 35%) was obtained from 4,5-dichloro-7-nitroquinolin-8-ol (29) (80.0 mg, 0.31 mmol) and 1-acetylpiperazine (119.0 mg, 0.93 mmol).

¹H NMR (400 MHz, DMSO-*d*₆) δ: 8.40 - 8.33 (m, 1H), 7.93 (s, 1H), 7.43 - 7.35 (m, 1H), 3.80 - 3.40 (m, 8H), 2.04 (s, 3H).

MS calculated: 350.08; MS found: 351.1 [M+H]⁺.

### Example 34

### Synthesis of 5-chloro-4-methoxy-7-nitroquinolin-8-ol (34)

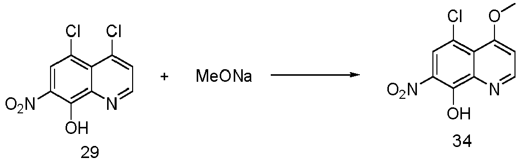

At room temperature, 4,5-dichloro-7-nitroquinolin-8-ol (29) (60.0 mg, 0.23 mmol) and sodium methoxide (62.1 mg, 1.15 mmol) were added to dimethyl sulfoxide (2.0 mL). The reaction mixture was stirred at 120°C for 2 hours, cooled to room temperature, and concentrated under reduced pressure to remove the organic solvent. Water (2.0 mL) was added to the resulting residue, stirred for 3 minutes, and filtered under reduced pressure. The filter cake was washed successively with water (0.5 mL × 2) and ethanol (0.5 mL × 2), and dried under vacuum to obtain 5-chloro-4-methoxy-7-nitroquinolin-8-ol (34) (51.0 mg, yield 87%).

¹H NMR (400 MHz, DMSO-*d*₆) δ: 8.50 - 8.44 (m, 1H), 7.83 (s, 1H), 7.15 - 7.09 (m, 1H), 3.93 (s, 3H).

MS calculated: 254.01; MS found: 255.0, 257.0 [M+H]⁺.

### Example 35

### Synthesis of 4-(5-chloro-8-hydroxy-7-nitroquinolin-4-yl)-N,N-dimethylpiperazine-1-carboxamide (35)

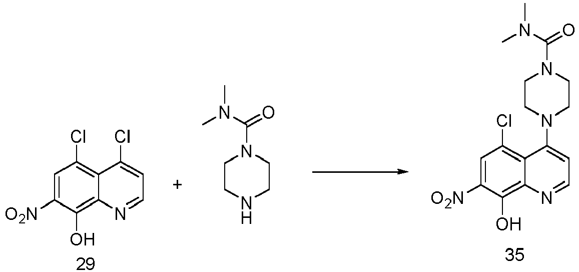

In accordance with the same synthesis method of Example 31, 4-(5-chloro-8-hydroxy-7-nitroquinolin-4-yl)-N,N-dimethylpiperazine-1-carboxamide (35) (30.0 mg, yield 42%) was obtained from 4,5-dichloro-7-nitroquinolin-8-ol (29) (50.0 mg, 0.19 mmol) and N,N-dimethylpiperazine-1-carboxamide (300.0 mg, 1.9 mmol).

¹H NMR (400 MHz, DMSO-*d*₆) δ 8.36 (d, *J* = 8.0 Hz, 1H), 7.92 (s, 1H), 7.39 (d, *J* = 8.0 Hz, 1H), 3.78 - 3.48 (m, 8H), 2.78 (s, 6H).

MS calculated: 379.10; MS found: 380.1 [M+H]⁺.

### Example 36

### Synthesis of 5-chloro-7-nitro-4-piperidinylquinolin-8-ol (36)

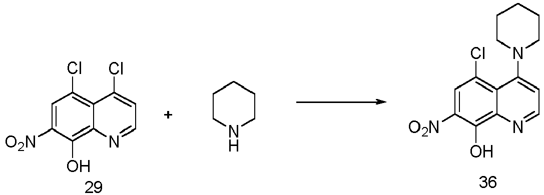

In accordance with the same synthesis method of Example 31, 5-chloro-7-nitro-4-piperidinylquinolin-8-ol (36) (50.0 mg, yield 71%) was obtained from 4,5-dichloro-7-nitroquinolin-8-ol (29) (60.0 mg, 0.23 mmol) and piperidine (170.0 mg, 2.3 mmol).

¹H NMR (400 MHz, DMSO-*d*₆) δ 8.32 (d, *J* = 8.0 Hz, 1H), 7.82 (s, 1H), 7.19 (d, *J* = 4.0 Hz, 1H), 3.12 - 3.01 (m, 4H), 1.79 - 1.61 (m, 6H).

MS calculated: 307.07; MS found: 308.1 [M+H]⁺.

### Example 37

### Synthesis of 5-chloro-7-nitro-4-(piperazin-1-yl)quinolin-8-ol (37)

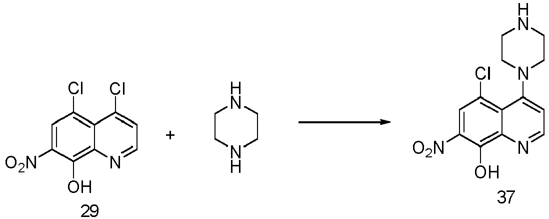

In accordance with the same synthesis method of Example 31, 5-chloro-7-nitro-4-(piperazin-1-yl)quinolin-8-ol (37) (200.0 mg, yield 65%) was obtained from 4,5-dichloro-7-nitroquinolin-8-ol (29) (259.0 mg, 1.0 mmol) and piperazine (258.6 mg, 3.0 mmol).

¹H NMR (400 MHz, CDCl₃) δ 8.89 - 8.70 (m, 1H), 8.25 - 8.06 (m, 1H), 7.88 - 7.73 (m, 1H), 3.05 - 2.97 (m, 4H), 2.29 - 1.98 (m, 4H).

MS calculated: 308.07; MS found: 309.1 [M+H]⁺.

### Example 38

### Synthesis of 5-chloro-4-(4-methoxypiperidin-1-yl)-7-nitroquinolin-8-ol (38)

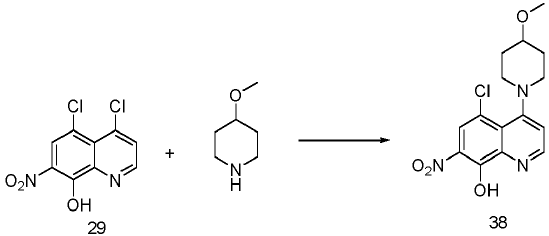

In accordance with the same synthesis method of Example 31, 5-chloro-4-(4-methoxypiperidin-1-yl)-7-nitroquinolin-8-ol (38) (40.0 mg, yield 51%) was obtained from 4,5-dichloro-7-nitroquinolin-8-ol (29) (60.0 mg, 0.23 mmol) and 4-methoxypiperidine (177.0 mg, 1.54 mmol).

¹H NMR (400 MHz, DMSO-*d*₆) δ 8.33 - 8.28 (m, 1H), 7.92 (s, 1H), 7.43 - 7.39 (m, 1H), 3.82 - 3.73 (m, 2H), 3.62 - 3.52 (m, 2H), 3.43 - 3.34 (m, 1H), 3.24 (s, 3H), 2.06 - 1.96 (m, 1H), 1.94 - 1.83 (m, 1H), 1.83 - 1.73 (m, 1H), 1.53 - 1.42 (m, 1H).

MS calculated: 337.08; MS found: 338.1 [M+H]⁺.

### Example 39

### Synthesis of 5-chloro-7-nitro-4-(4-(trifluoromethyl)piperidin-1-yl)quinolin-8-ol (39)

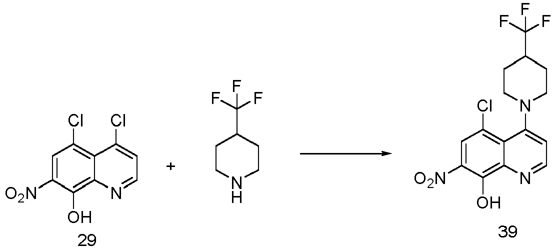

In accordance with the same synthesis method of Example 34, 5-chloro-7-nitro-4-(4-(trifluoromethyl)piperidin-1-yl)quinolin-8-ol (39) (80.0 mg, yield 93%) was obtained from 4,5-dichloro-7-nitroquinolin-8-ol (29) (60.0 mg, 0.23 mmol) and 4-(trifluoromethyl)piperidine (142.0 mg, 0.93 mmol).

¹H NMR (400 MHz, DMSO-*d*₆) δ: 8.50 - 8.40 (m, 1H), 7.87 (s, 1H), 7.25 - 7.14 (m, 1H), 3.70 - 3.50 (m, 1H), 3.00 - 2.60 (m, 4H), 2.00 - 1.55(m, 4H).

MS calculated: 375.06; MS found: 376.0 [M+H]⁺.

### Example 40

### Synthesis of 5-chloro-4-(4-methylpiperazin-1-yl)-7-nitroquinolin-8-ol (40)

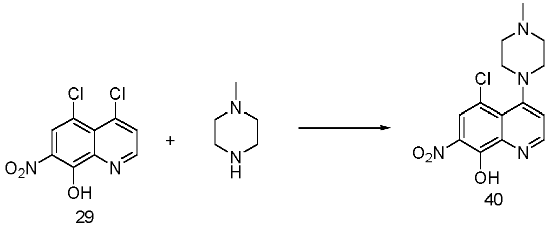

In accordance with the same synthesis method of Example 34, 5-chloro-4-(4-methylpiperazin-1-yl)-7-nitroquinolin-8-ol (40) (23.0 mg, yield 31%) was obtained from 4,5-dichloro-7-nitroquinolin-8-ol (29) (60.0 mg, 0.23 mmol) and 1-methylpiperazine (116.0 mg, 1.16 mmol).

¹H NMR (400 MHz, DMSO-*d*₆) δ: 8.50 - 8.35 (m, 1H), 8.14 (s, 1H), 7.94 (s, 1H), 7.46 - 7.38 (m, 1H), 3.85 - 3.50 (m, 4H), 3.15 - 2.90 (m, 4H), 2.65 - 2.55 (m, 3H).

MS calculated: 322.08; MS found: 323.1 [M+H]⁺.

### Example 41

### Synthesis of 5-chloro-4-(4,4-dimethylpiperidin-1-yl)-7-nitroquinolin-8-ol (41)

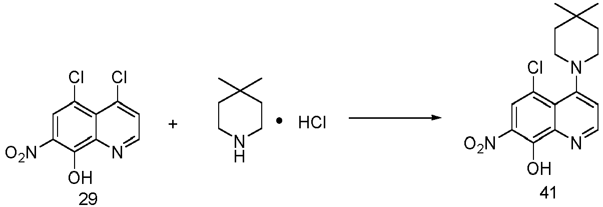

At room temperature, 4,5-dichloro-7-nitroquinolin-8-ol (29) (50.0 mg, 0.19 mmol), 4,4-dimethylpiperidine hydrochloride (87.0 mg, 0.58 mmol) and triethylamine (39.0 mg, 0.38 mmol) were added successively to acetonitrile (1.0 mL). The reaction mixture was stirred at 80°C for 1 hour, cooled to room temperature, and concentrated under reduced pressure to remove the organic solvent. Water (5.0 mL) was added to the resulting residue, stirred for 3 minutes, and filtered under reduced pressure. The filter cake was washed successively with water (0.5 mL × 2) and ethanol (0.5 mL × 2), and dried under vacuum to obtain 5-chloro-4-(4,4-dimethylpiperidin-1-yl)-7-nitroquinolin-8-ol (41) (52.0 mg, yield 80%).

¹H NMR (400 MHz, DMSO-*d*₆) δ 8.23 (d, *J* = 6.6 Hz, 1H), 7.86 (s, 1H), 7.35 (d, *J* = 6.8 Hz, 1H), 3.76 - 3.54 (m, 4H), 1.59 - 1.50 (m, 2H), 1.48- 1.40 (m, 2H) 1.06 (s, 3H), 0.91 (s, 3H).

MS calculated: 335.1; MS found: 336.1[M+H]⁺.

### Example 42

### Synthesis of 5-chloro-7-nitro-4-(pyrrolidin-1-yl)quinolin-8-ol (42)

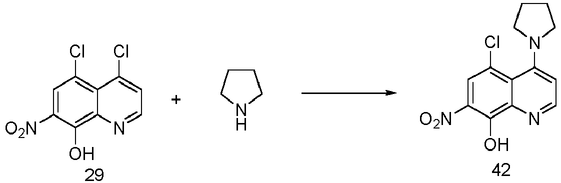

At room temperature, 4,5-dichloro-7-nitroquinolin-8-ol (29) (60.0 mg, 0.23 mmol) and tetrahydropyrrole (66.0 mg, 0.93 mmol) were added to acetonitrile (6.0 mL). The reaction mixture was stirred at 100°C for 1 hour, cooled to room temperature, and concentrated under reduced pressure to remove the organic solvent. Water (3.0 mL) was added to the resulting residue, stirred for 3 minutes, and filtered under reduced pressure. The filter cake was washed successively with water (0.5 mL × 2) and ethanol (0.5 mL × 2), and dried under vacuum to obtain 5-chloro-7-nitro-4-(pyrrolidin-1-yl)quinolin-8-ol (42) (58.0 mg, yield 86%).

¹H NMR (400 MHz, DMSO-*d*₆) δ: 8.30 - 8.10 (m, 1H), 7.74 (s, 1H), 7.00 - 6.85 (m, 1H), 7.46 - 7.38 (m, 1 H), 2.00 - 1.75 (m, 8 H).

MS calculated: 293.06; MS found: 294.0 [M+H]⁺.

### Example 43

### Preparation of 5-chloro-4-(2-fluoroethoxy)-7-nitroquinolin-8-ol (43)

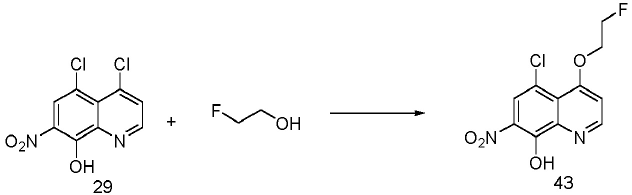

At room temperature, sodium hydride (60 weight%, a mixture in mineral oil) (46.0 mg, 1.16 mmol) was added to a solution of 2-fluoroethanol (147.0 mg, 2.3 mmol) in dimethyl sulfoxide (4.0 mL). The resulting mixture was stirred at room temperature for 1 hour, followed by the addition of 4,5-dichloro-7-nitroquinolin-8-ol (29) (60.0 mg, 0.23 mmol). The resulting mixture was stirred at 100°C for 2 hours, cooled to room temperature, and concentrated under reduced pressure to remove the organic solvent. Water (3.0 mL) was added to the resulting residue, stirred for 3 minutes, and filtered under reduced pressure. The filter cake was washed successively with water (0.5 mL × 2) and ethanol (0.5 mL × 2), and dried under vacuum to obtain 5-chloro-4-(2-fluoroethoxy)-7-nitroquinolin-8-ol (43) (40.0 mg, yield 61%).

¹H NMR (400 MHz, DMSO-*d*₆) δ: 8.46 (d, *J* = 4.4Hz, 1H), 7.85 (s, 1H), 7.13 (d, *J* = 5.2Hz, 1H), 4.91 - 4.88 (m, 1H), 4.79 - 4.76 (m, 1H), 4.46 - 4.43 (m, 1H), 4.39 - 4.36 (m, 1H).

MS calculated: 286.02; MS found: 287.0[M+H]⁺.

### Example 44

### Synthesis of 4-(5-chloro-8-hydroxy-7-nitroquinolin-4-yl)-N-methylpiperazine-1-carboxamide (44)

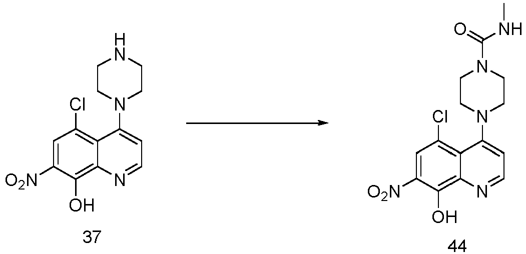

At 0°C, triethylamine (60.0 mg, 0.58 mmol), methylaminoformyl chloride (55.0 mg, 0.58 mmol) and N,N-dimethylformamide (0.1 mL) were added successively to a solution of 5-chloro-7-nitro-4-(piperazin-1-yl)quinolin-8-ol (37) (60.0 mg, 0.19 mmol) in dichloromethane (5.0 mL). The reaction mixture was warmed up to room temperature, stirred for 1 hour, and diluted with water (10.0 mL). The resulting mixture was extracted with dichloromethane (10.0 mL × 3). The organic phases were combined, dried over anhydrous sodium sulfate and filtered, and the filtrate was concentrated under reduced pressure. The resulting residue was purified by reversed-phase high performance liquid chromatography (the chromatographic column was Eclipse XDB-C18 (21.2 mm×250 mm, 7µm), the mobile phase was water/acetonitrile=100%/0 - 70%/30%, gradient elution, 0.1% formic acid was added to the mobile phase by volume percentage, and the flow rate was 20.0 mL/min) to obtain 4-(5-chloro-8-hydroxy-7-nitroquinolin-4-yl)-N-methylpiperazine-1-carboxamide (44) (46.0 mg, yield 66%).

¹H NMR (400 MHz, DMSO-*d*₆) δ 8.34 (d, *J* = 8.0 Hz, 1H), 7.91 (s, 1H), 7.39 (d, *J* = 8.0 Hz, 1H), 6.63 - 6.55 (m, 1H), 3.68 - 3.60 (m, 4H), 3.56 - 3.51 (m, 4H), 2.58 (d, *J* = 4.0 Hz, 3H).

MS calculated: 365.09; MS found: 366.1 [M+H]⁺.

### Example 45

### Synthesis of 4-(5-chloro-8-hydroxy-7-nitroquinolin-4-yl)-1-(2,2,2-trimethylacetyl)piperazine (45)

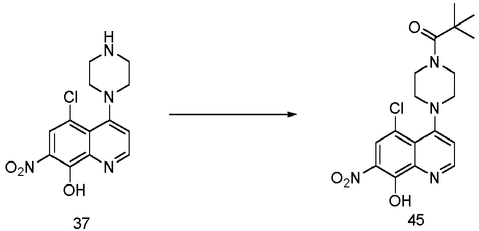

In accordance with the same synthesis method of Example 44, 4-(5-chloro-8-hydroxy-7-nitroquinolin-4-yl)-1-(2,2,2-trimethylacetyl)piperazine (45) (56.0 mg, yield 75%) was obtained from 5-chloro-7-nitro-4-(piperazin-1-yl)quinolin-8-ol (37) (60.0 mg, 0.19 mmol), trimethylacetyl chloride (73.0 mg, 0.60 mmol) and triethylamine (70.0 mg, 0.60 mmol).

¹H NMR (400 MHz, DMSO-*d*₆) δ 8.38 (d, *J* = 8.0 Hz, 1H), 7.93 (s, 1H), 7.37 (d, *J* = 8.0 Hz, 1H), 4.02 - 3.84 (m, 4H), 3.72 - 3.62 (m, 4H), 1.21 (s, 9H).

MS calculated: 392.13; MS found: 393.1 [M+H]⁺.

### Example 46

### Synthesis of ethyl 1-(5-chloro-8-hydroxy-7-nitroquinolin-4-yl)piperidine-4-carboxylate (46)

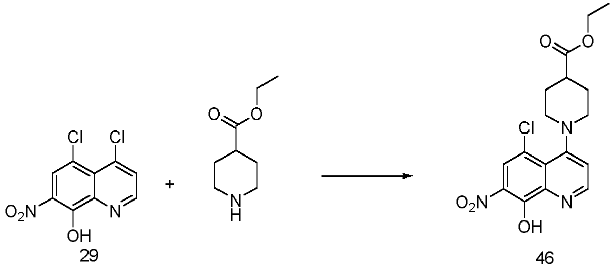

At room temperature, 4,5-dichloro-7-nitroquinolin-8-ol (29) (50.0 mg, 0.19 mmol) and ethyl 4-piperidinecarboxylate (91.0 mg, 0.58 mmol) were added to acetonitrile (1.0 mL). The reaction mixture was stirred at 80°C for 1 hour, cooled to room temperature, and concentrated under reduced pressure to remove the organic solvent. Water (5.0 mL) was added to the resulting residue, stirred for 30 minutes, and filtered under reduced pressure. The filter cake was washed with water (0.5 mL × 2), and dried under vacuum to obtain ethyl 1-(5-chloro-8-hydroxy-7-nitroquinolin-4-yl)piperidine-4-carboxylate (46) (47.0 mg, yield 64%).

¹H NMR (400 MHz, DMSO-*d*₆) δ: 8.37 (d, *J* = 6.0 Hz, 1H), 8.20 (d, *J* = 6.8 Hz, 1H), 7.92 (s, 1H), 4.12 (q, *J* = 6.8 Hz, 2H), 3.92 (d, *J* = 13.6 Hz, 1H), 3.73 (d, *J* = 11.4 Hz, 1H), 3.62 - 3.57 (m, 1H), 3.06 - 2.98 (m, 1H), 2.81 (m, 1H), 1.95 (m, 3H), 1.57 (m, 1H), 1.15 (t, *J* = 6.8 Hz, 3H).

MS calculated: 379.1; MS found: 380.1[M+H]⁺.

### Example 47

### Synthesis of 5-chloro-7-nitro-4-phenoxyquinolin-8-ol (47)

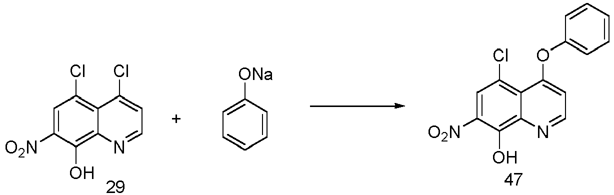

In accordance with the same synthesis method of Example 31, 5-chloro-7-nitro-4-phenoxyquinolin-8-ol (47) (71.9 mg, yield 99%) was obtained from 4,5-dichloro-7-nitroquinolin-8-ol (29) (60.0 mg, 0.23 mmol) and sodium phenolate (107.5 mg, 0.93 mmol).

¹H NMR (400 MHz, DMSO-*d*₆) δ: 8.53 - 8.48 (m, 2H), 7.94 (s, 1H), 7.52 - 7.45 (m, 2H), 7.30 - 7.22 (m, 1H), 7.16 - 7.10 (m, 2H), 6.92 - 6.88 (m, 1H).

MS calculated: 316.03; MS found: 317.1 [M+H]⁺.

### Example 48

### Synthesis of 5-chloro-4-(4-(methylsulfonyl)piperazin-1-yl)-7-nitroquinolin-8-ol (48)

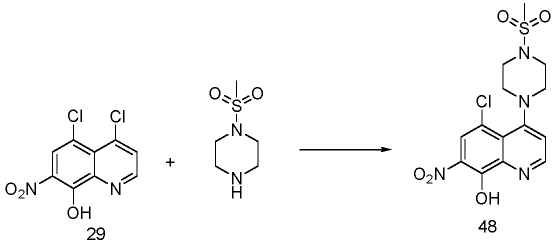

In accordance with the same synthesis method of Example 31, 5-chloro-4-(4-(methylsulfonyl)piperazin-1-yl)-7-nitroquinolin-8-ol (48) (62.0 mg, yield 70%) was obtained from 4,5-dichloro-7-nitroquinolin-8-ol (29) (60.0 mg, 0.23 mmol) and 1-(methylsulfonyl)piperazine (113.0 mg, 0.69 mmol).

¹H NMR (400 MHz, DMSO-*d*₆) δ 8.44 (d, *J* = 5.6 Hz, 1H), 7.88 (s, 1H), 7.25 (d, *J* = 5.8 Hz, 1H), 3.20 - 3.17 (m, 4H), 3.02 - 2.99 (m, 4H), 2.91 (s, 3H).

MS calculated: 386.05; MS found: 387.1 [M+H]⁺.

### Example 49

### Synthesis of 5-chloro-4-cyclopropyl-7-nitroquinolin-8-ol (49)

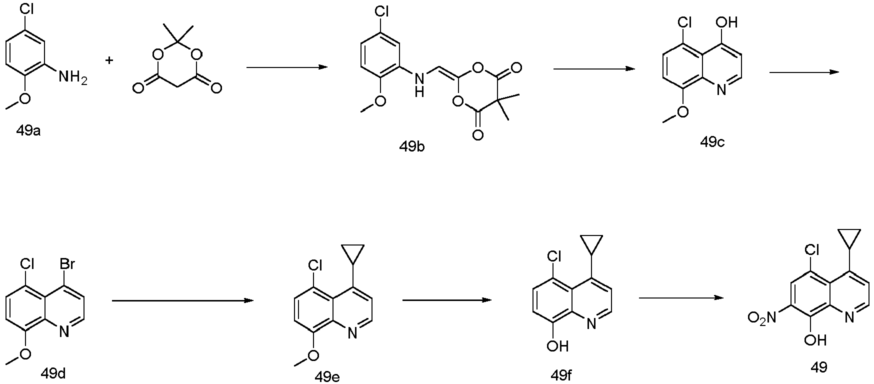

At room temperature, 2,2-dimethyl-1,3-dioxane-4,6-dione (12.0 g, 83.3 mmol) and triethyl orthoformate (13.7 g, 95.1 mmol) were added to ethanol (83.0 mL). The reaction mixture was stirred at 90°C for 4 hours, followed by the addition of 5-chloro-2-methoxyaniline (49a) (12.0 g, 76.4 mmol), stirred for 1 hour, and cooled to room temperature. The resulting mixture was filtered under reduced pressure, the filter cake was washed with ethanol (15.0 mL × 2), and dried under vacuum to obtain 5-(((5-chloro-2-methoxyphenyl)amino)methylene)-2,2-dimethyl-1,3-dioxane-4,6-dione (49b) (23.0 g, yield 97%).

At room temperature, 5-(((5-chloro-2-methoxyphenyl)amino)methylene)-2,2-dimethyl-1,3-dioxane-4,6-dione (49b) (23.0 g, 74.0 mmol) was added to diphenyl ether (125.0 mL). The reaction mixture was warmed up to 240°C, stirred for 3.5 hours, cooled to room temperature, diluted with petroleum ether (boiling range: 60 to 90°C) (600.0 mL), and stirred for 2 hours. The resulting mixture was filtered under reduced pressure, the filter cake was washed with petroleum ether (boiling range: 60 to 90°C) (50.0 mL × 3), and dried under vacuum to obtain 5-chloro-8-methoxyquinolin-4-ol (49c) (12.5 g, yield 81%).

At room temperature, 5-chloro-8-methoxyquinolin-4-ol (49c) (2.0 g, 7.35 mmol) was added to a mixed solvent of chloroform (20.0 mL) and N,N-dimethylformamide (2.0 mL), followed by the slowly dropwise addition of phosphorus oxybromide (4.2 g, 14.7 mmol). The reaction mixture was warmed up to 60°C, stirred for 2 hours, and cooled to room temperature, followed by the addition of ice water (30.0 mL). The pH of the aqueous phase was adjusted to about 9 to 10 with ammonia (25 to 28 weight%, NH₃ content). The resulting mixture was extracted with dichloromethane (100.0 mL × 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/1) to obtain 4-bromo-5-chloro-8-methoxyquinoline (49d) (1.4 g, yield 70%).

At room temperature, 4-bromo-5-chloro-8-methoxyquinoline (49d) (225.0 mg, 0.83 mmol), cyclopropylboronic acid (107.4 mg, 1.25 mmol), tetrakis(triphenylphosphine)palladium (129.1 mg, 0.083 mmol) and potassium carbonate (229.1 mg, 1.66 mmol) were added successively to a mixed solvent of toluene (10.0 mL), ethanol (1.0 mL) and water (2.0 mL). The reaction mixture was stirred at 90°C for 12 hours, cooled to room temperature, and diluted with water (20.0 mL). The resulting mixture was extracted with ethyl acetate (20.0 mL × 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/1) to obtain 5-chloro-4-cyclopropyl-8-methoxyquinoline (49e) (100.0 mg, yield 52%).

At room temperature, 5-chloro-4-cyclopropyl-8-methoxyquinoline (49e) (100.0 mg, 0.42 mmol) was added to a solution of boron tribromide in dichloromethane (1.0 M) (1.3 mL, 1.3 mmol). The reaction mixture was stirred at 50°C for 5 hours, and cooled to room temperature. The pH of the aqueous phase was adjusted to about 8 to 9 with saturated aqueous sodium bicarbonate solution. The resulting mixture was extracted with dichloromethane (10.0 mL × 3). The organic phases were combined, washed with saturated brine (10.0 mL), dried over anhydrous sodium sulfate, filtered, and concentrated under reduced pressure. The resulting residue was purified by silica gel column chromatography (eluent: dichloromethane/methanol = 80/1) to obtain 5-chloro-4-cyclopropylquinolin-8-ol (49f) (60.0 mg, yield 65%).

At room temperature, sodium nitrite (186.3 mg, 2.7 mmol) was added in batches to a suspension of 5-chloro-4-cyclopropylquinolin-8-ol (49f) (60 mg, 0.27 mmol) in hydrochloric acid (2.0 M) (5.0 mL). The reaction mixture was stirred at room temperature for 2 hours, and concentrated under reduced pressure to remove the solvent. The resulting residue was purified by reversed-phase high performance liquid chromatography (the chromatographic column was Eclipse XDB-C18 (21.2 mm×250 mm, 7µm), the mobile phase was water/acetonitrile=100% - 70%/30%, gradient elution, 0.1% formic acid was added to the mobile phase by volume percentage, and the flow rate was 20.0 mL/min) to obtain the product 5-chloro-4-cyclopropyl-7-nitroquinolin-8-ol (49) (8.5 mg, yield 12%).

¹H NMR (400 MHz, DMSO-*d*₆) δ: 9.04 - 8.79 (m, 1H), 8.22 - 7.93 (m, 1H), 7.75 - 7.41 (m, 1H), 3.00 - 2.92 (m, 1H), 1.24 - 1.17 (m, 2H), 1.03 - 0.95 (m, 2H).

MS calculated: 264.03; MS found: 265.0 [M+H]⁺.

### Example 50

### Synthesis of 4-(5-chloro-8-hydroxy-7-nitroquinolin-4-yl)-1-(2,2-dimethylacetyl)piperazine (50)

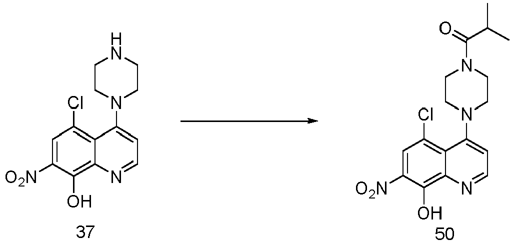

In accordance with the same synthesis method of Example 44, 4-(5-chloro-8-hydroxy-7-nitroquinolin-4-yl)-1-(2,2-dimethylacetyl)piperazine (50) (15.0 mg, yield 22%) was obtained from 5-chloro-7-nitro-4-(piperazin-1-yl)quinolin-8-ol (37) (56.0 mg, 0.18 mmol), isobutyryl chloride (38.4 mg, 0.36 mmol) and triethylamine (36.4 mg, 0.36 mmol).

¹H NMR (400 MHz, DMSO-*d₆*) δ: 8.15 (s, 1H), 7.91 (s, 1H), 7.25 (s, 1H), 4.35 - 4.21 (br s, 2H), 4.01 - 3.90 (br s, 2H), 2.95 - 2.88 (br s, 2H), 2.81 - 2.69 (br s, 2H), 2.05 - 1.94 (m, 1H), 1.01 (overlapping s, 6H).

MS calculated: 394.10; MS found: 395.1, 396.1 [M+H]⁺.

### Example 51

### Synthesis of 5-chloro-7-nitro-4-phenylquinolin-8-ol hydrochloride (51)

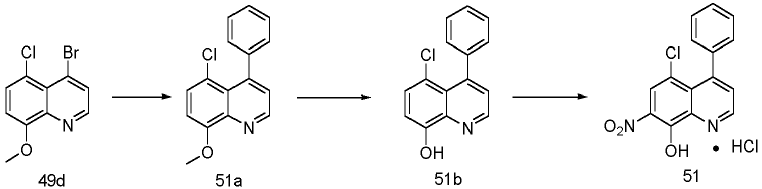

At room temperature, 4-bromo-5-chloro-8-methoxyquinoline (49d) (200.0 mg, 0.73 mmol), phenylboronic acid (134.0 mg, 1.10 mmol), tetrakis(triphenylphosphine)palladium (156.0 mg, 0.10 mmol) and potassium carbonate (207.0 mg, 1.50 mmol) were added successively to 1,4-dioxane (10.0 mL). The reaction mixture was stirred at 90°C for 12 hours, cooled to room temperature, and diluted with water (20.0 mL). The resulting mixture was extracted with dichloromethane (20.0 mL × 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 7/3) to obtain 5-chloro-8-methoxy-4-phenylquinoline (51a) (150.0 mg, yield 76%).

At 0°C, a solution of boron tribromide in dichloromethane (1.0 M) (1.5 mL, 1.5 mmol) was slowly added to a solution of 5-chloro-8-methoxy-4-phenylquinoline (51a) (150.0 mg, 0.56 mmol) in dichloromethane (2.0 mL). The reaction solution was warmed up to room temperature, stirred for 1 hour, stirred at 50°C for 3 hours, and cooled to room temperature. The pH of the aqueous phase was adjusted to about 8 to 9 with saturated aqueous sodium bicarbonate solution. The resulting mixture was extracted with dichloromethane (20.0 mL × 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/1) to obtain 5-chloro-4-phenylquinolin-8-ol (51b) (80.0 mg, yield 56%).

At 0°C, sodium nitrite (162.0 mg, 2.3 mmol) was added in batches to a suspension of 5-chloro-4-phenylquinolin-8-ol (60.0 mg, 0.23 mmol) in hydrochloric acid (3.0 M) (5.0 mL). The reaction mixture was warmed up to room temperature, stirred for 12 hours, and filtered under reduced pressure. The filter cake was washed with ethanol (0.5 mL × 2), and dried under vacuum to obtain 5-chloro-7-nitro-4-phenylquinolin-8-ol hydrochloride (51) (8.5 mg, yield 12%).

¹H NMR (400 MHz, DMSO-*d*₆) δ: 9.05 (d, *J* = 4.0 Hz, 1H), 8.09 (s, 1H), 7.70 (d, *J* = 4.0 Hz, 1H), 7.51 - 7.43 (m, 3H), 7.40 - 7.35 (m, 2H).

MS calculated: 300.03; MS found: 301.0 [M+H]⁺.

### Example 52

### Synthesis of ethyl 4-(5-chloro-8-hydroxy-7-nitroquinolin-4-yl)piperazine-1-carboxylate (52)

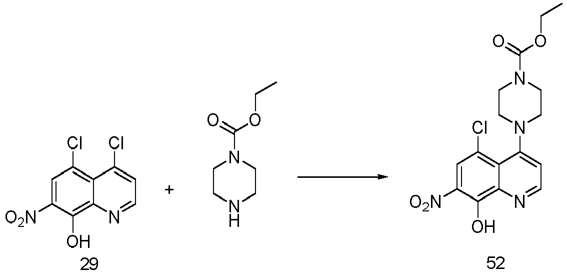

In accordance with the same synthesis method of Example 31, ethyl 4-(5-chloro-8-hydroxy-7-nitroquinolin-4-yl)piperazine-1-carboxylate (52) (73.0 mg, yield 83%) was obtained from 4,5-dichloro-7-nitroquinolin-8-ol (29) (60.0 mg, 0.23 mmol) and ethyl piperazine-1-carboxylate (110.0 mg, 0.70 mmol).

¹H NMR (400 MHz, DMSO-*d*₆) δ 8.38 (d, *J* = 4.0 Hz, 1H), 7.92 (s, 1H), 7.38 (d, *J* = 8.0 Hz, 1H), 4.13 - 4.05 (m, 2H), 3.74 - 3.62 (m, 8H), 1.20 (t, *J=* 7.0 Hz, 3H).

MS calculated: 380.09; MS found: 381.1 [M+H]⁺.

### Example 53

### Synthesis of 5-chloro-4-(cyclopropylmethoxy)-7-nitroquinolin-8-ol (53)

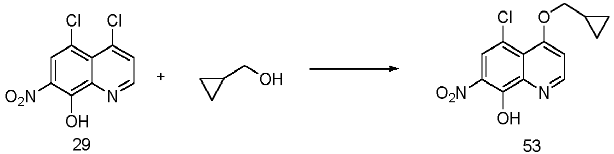

In accordance with the same synthesis method of Example 43, 5-chloro-4-(cyclopropylmethoxy)-7-nitroquinolin-8-ol (53) (14.0 mg, yield 21%) was obtained from 4,5-dichloro-7-nitroquinolin-8-ol (29) (60.0 mg, 0.23 mmol), cyclopropylmethanol (250.0 mg, 1.16 mmol) and sodium hydride (60 weight%, a mixture in mineral oil) (46.0 mg, 1.16 mmol).

¹H NMR (400 MHz, DMSO-*d*₆) δ: 8.45 - 8.35 (m, 1H), 7.83 (s, 1H), 7.10 - 7.00 (m, 1H), 4.10 - 3.95 (m, 2H), 1.40 - 1.25 (m, 1H), 0.65 - 0.55 (m, 2H), 0.46 - 0.35 (m, 2H).

MS calculated: 294.04; MS found: 295.0 [M+H]⁺.

### Example 54

### Synthesis of 5-chloro-4-(4-(3-methoxypropoxy)piperidin-1-yl)-7-nitroquinolin-8-ol dihydrochloride (54)

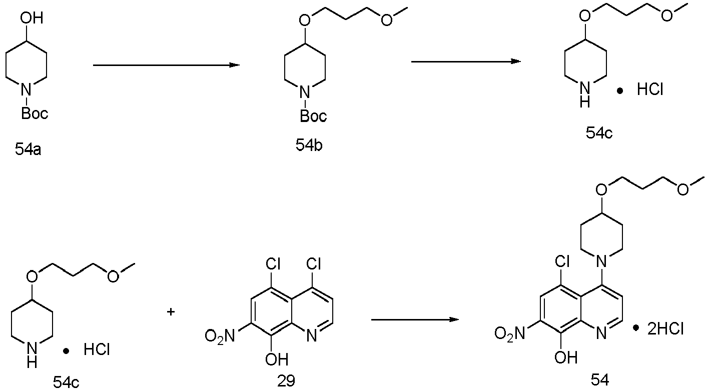

At room temperature, tert-butyl 4-hydroxypiperidine-1-carboxylate (1.0 g, 5.0 mmol) (54a) and sodium hydride (60 weight%, a mixture in mineral oil) (0.50 g, 12.5 mmol) were added successively to N,N-dimethylformamide (5.0 mL). The reaction mixture was stirred at room temperature for 1.5 hours, followed by the addition of 1-bromo-3-methoxypropane (2.3 g, 15.0 mmol). The reaction mixture was warmed up to 50°C, stirred for 4 hours, cooled to room temperature, and diluted with water (20.0 mL) and dichloromethane (20.0 mL). The organic phase was separated, and the aqueous phase was extracted with dichloromethane (20.0 mL × 3). The organic phases were combined, dried over anhydrous sodium sulfate and filtered, and the filtrate was concentrated under reduced pressure. The resulting residue was purified by silica gel column chromatography (gradient elution, eluent: petroleum ether/ethyl acetate = 30/1-20/1) to obtain *tert*-butyl 4-(3-methoxypropoxy)piperidine-1-carboxylate (54b) (0.34 g, yield 25%).

At room temperature, a solution of hydrogen chloride in 1,4-dioxane (4.0 M) (3.1 mL, 12.4 mmol) was added to *tert*-butyl 4-(3-methoxypropoxy)piperidine-1-carboxylate (54b) (340.0 mg, 1.24 mmol). The reaction mixture was stirred at room temperature for 2 hours, and concentrated to dryness under reduced pressure to obtain the crude product 4-(3-methoxypropoxy)piperidine hydrochloride (54c) (310.0 mg, crude yield >100%).

At room temperature, 4,5-dichloro-7-nitroquinolin-8-ol (29) (40.0 mg, 0.15 mmol) and 4-(3-methoxypropoxy)piperidine hydrochloride (54c) (310.0 mg, 1.8 mmol) were added to N,N-dimethylformamide (3.0 mL). The reaction mixture was stirred at 100°C for 4 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile=100% - 70%/30%, gradient elution, 0.1% formic acid was added to the mobile phase by volume percentage, and the flow rate was 20.0 mL/min) to obtain 5-chloro-4-(4-(3-methoxypropoxy)piperidin-1-yl)-7-nitroquinolin-8-ol dihydrochloride (54) (14.0 mg, yield 24%).

¹H NMR (400 MHz, DMSO-*d*₆) δ: 8.35 - 8.20 (m, 1H), 7.90 - 7.82 (m, 1H), 7.42 - 7.30 (m, 1H), 3.40 - 3.28 (m, 12 H), 2.10 - 1.60 (m, 6 H).

MS calculated: 395.12; MS found: 396.1 [M+H]⁺.

### Example 55

### Synthesis of 5-chloro-4-(4,4-difluoropiperidin-1-yl)-7-nitroquinolin-8-ol (55)

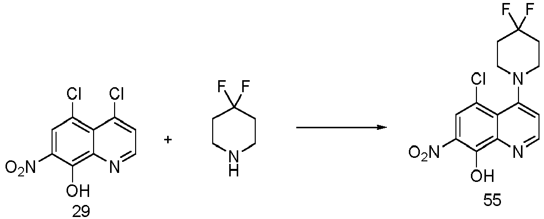

In accordance with the same synthesis method of Example 31, 5-chloro-4-(4,4-difluoropiperidin-1-yl)-7-nitroquinolin-8-ol (55) (43.0 mg, yield 54%) was obtained from 4,5-dichloro-7-nitroquinolin-8-ol (29) (60.0 mg, 0.23 mmol) and 4,4-difluoroperidine (140.0 mg, 1.16 mmol).

¹H NMR (400 MHz, DMSO-*d*₆) δ: 8.39 (d, *J* = 8.0 Hz, 1H), 7.92 (s, 1H), 7.44 (d, *J* = 8.0 Hz, 1H), 3.80 - 3.60 (m, 4 H), 2.35 - 2.05 (m, 4 H).

MS calculated: 343.05; MS found: 344.1 [M+H]⁺.

### Example 56

### Synthesis of 1-(5-chloro-8-hydroxy-7-nitroquinolin-4-yl)piperidin-4-one (56)

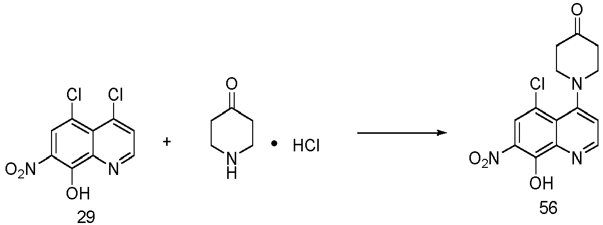

At room temperature, 4,5-dichloro-7-nitroquinolin-8-ol (29) (40.0 mg, 0.15 mmol), 4-piperidone hydrochloride (37.0 mg, 0.37 mmol) and triethylamine (45.0 mg, 0.45 mmol) were added successively to N,N-dimethylformamide (2.0 mL). The reaction mixture was stirred at 100°C for 2 hours, cooled to room temperature, and concentrated under reduced pressure to remove the organic solvent. Water (2.0 mL) was added to the resulting residue, stirred for 3 minutes, and filtered under reduced pressure. The filter cake was washed with water (0.5 mL × 2), and dried under vacuum to obtain 1-(5-chloro-8-hydroxy-7-nitroquinolin-4-yl)piperidin-4-one (56) (20.0 mg, yield 41%).

¹H NMR (400 MHz, DMSO-*d*₆) δ 8.39 - 8.34 (m, 1H), 7.93 - 7.86 (m, 1H), 7.42 - 7.35 (m, 1H), 3.95 - 3.64 (m, 8H).

MS calculated: 321.05; MS found: 322.1 [M+H]⁺.

### Example 57

### Synthesis of (1-methyl)propyl 4-(5-chloro-8-hydroxy-7-nitroquinolin-4-yl)piperazine-1-carboxylate (57)

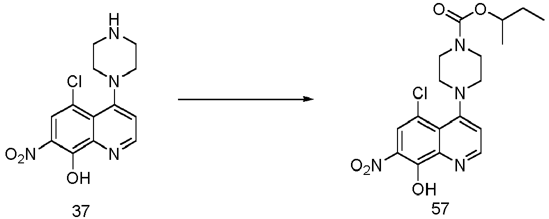

In accordance with the same synthesis method of Example 44, (1-methyl)propyl 4-(5-chloro-8-hydroxy-7-nitroquinolin-4-yl)piperazine-1-carboxylate (57) (50.0 mg, yield 68%) was obtained from 5-chloro-7-nitro-4-(piperazin-1-yl)quinolin-8-ol (37)

(56.0 mg, 0.18 mmol), *sec*-butyl chloroformate (75.0 mg, 0.55 mmol) and triethylamine (54.6 mg, 0.54 mmol).

¹H NMR (400 MHz, DMSO-*d*₆) δ 8.38 (d, *J* = 4.0 Hz, 1H), 7.92 (s, 1H), 7.39 (d, *J* = 8.0 Hz, 1H), 4.68 - 4.62 (m, 1H), 3.76 - 3.53 (m, 8H),1.56 - 1.49 (m, 2H), 1.20 - 1.14 (m, 3H), 0.90 - 0.81 (m, 3H).

MS calculated: 408.12; MS found: 409.1 [M+H]⁺.

### Example 58

### Synthesis of isopropyl 4-(5-chloro-8-hydroxy-7-nitroquinolin-4-yl)piperazine-1-carboxylate (58)

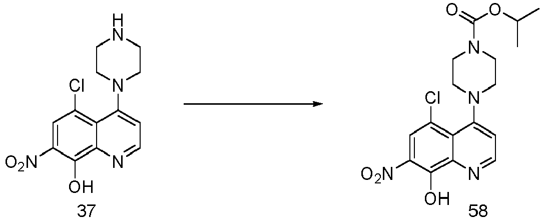

In accordance with the same synthesis method of Example 44, isopropyl 4-(5-chloro-8-hydroxy-7-nitroquinolin-4-yl)piperazine-1-carboxylate (58) (46.0 mg, yield 61%) was obtained from 5-chloro-7-nitro-4-(piperazin-1-yl)quinolin-8-ol (37) (60.0 mg, 0.19 mmol), isopropyl chloroformate (73.8 mg, 0.60 mmol) and triethylamine (67.0 mg, 0.60 mmol).

¹H NMR (400 MHz, DMSO-*d*₆) δ 8.38 (d, *J* = 4.0 Hz, 1H), 7.92 (s, 1H), 7.38 (d, *J* = 8.0 Hz, 1H), 4.86 - 4.74 (m, 1H), 3.73 - 3.45 (m, 8H), 1.25 - 1.16 (m, 6H).

MS calculated: 394.10; MS found: 395.1 [M+H]⁺.

### Example 59

### Synthesis of 5-chloro-4-(cyclopentyloxy)-7-nitroquinolin-8-ol (59)

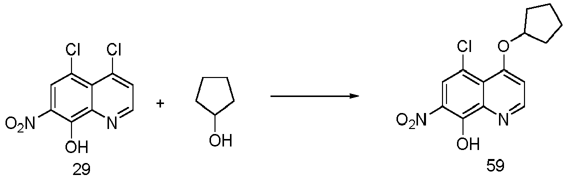

At room temperature, cyclopentanol (166.0 mg, 1.93 mmol) and sodium hydride (60 weight%, a mixture in mineral oil) (38.8 mg, 0.97 mmol) were added successively to N,N-dimethylformamide (2.0 mL). The reaction mixture was stirred at room temperature for 1 hour, followed by the addition of 4,5-dichloro-7-nitroquinolin-8-ol (29) (50.0 mg, 0.19 mmol). The resulting mixture was stirred at 100°C for 2 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile=100% - 70%/30%, gradient elution, 0.1% formic acid was added to the mobile phase by volume percentage, and the flow rate was 20.0 mL/min) to obtain 5-chloro-4-(cyclopentyloxy)-7-nitroquinolin-8-ol (59) (11.0 mg, yield 19%).

¹H NMR (400 MHz, DMSO-*d*₆) δ: 8.80-8.60 (m, 1H), 8.05-7.95 (m, 1H), 7.50-7.35 (m, 1H), 5.38-5.22 (m, 1 H), 2.10-1.60(m, 9 H).

MS calculated: 308.06; MS found: 309.0 [M+H]⁺.

### Example 60

### Synthesis of 5-chloro-4-(cyclohexyloxy)-7-nitroquinolin-8-ol (60)

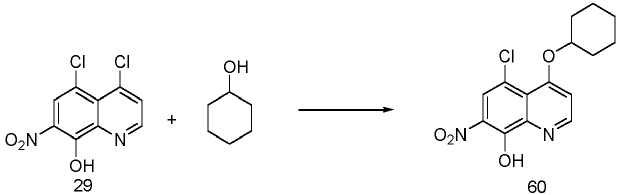

In accordance with the same synthesis method of Example 59, 5-chloro-4-(cyclohexyloxy)-7-nitroquinolin-8-ol (60) (30.0 mg, yield 16%) was obtained from 4,5-dichloro-7-nitroquinolin-8-ol (29) (150.0 mg, 0.58 mmol), cyclohexanol (117.0 mg, 1.16 mmol) and sodium hydride (60 weight%, a mixture in mineral oil) (47.0 mg, 1.16 mmol).

¹H NMR (400 MHz, DMSO-*d*₆) δ: 8.65 (d, *J* = 8.0 Hz, 1H), 7.98 (s, 1H), 7.54 (d, *J* = 8.0 Hz, 1H), 5.13 - 4.85 (m, 1H), 2.10 - 1.86 (m, 2H), 1.84 - 1.62 (m, 4H), 1.56 - 1.38 (m, 4H).

MS calculated: 322.07; MS found: 323.1 [M+H]⁺.

### Example 61

### Synthesis of 5-chloro-4-ethoxy-7-nitroquinolin-8-ol (61)

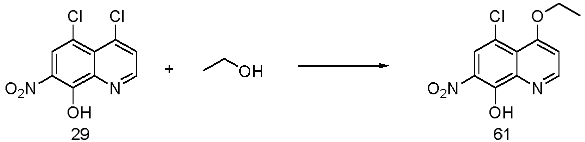

In accordance with the same synthesis method of Example 43, 5-chloro-4-ethoxy-7-nitroquinolin-8-ol (61) (23.3 mg, yield 38%) was obtained from 4,5-dichloro-7-nitroquinolin-8-ol (29) (60.0 mg, 0.23 mmol), ethanol (6.0 mL, 102.7 mmol) and sodium hydride (60 weight%, a mixture in mineral oil) (50.6 mg, 1.26 mmol).

¹H NMR (400 MHz, DMSO-*d*₆) δ: 8.75 - 8.70 (m, 1H), 8.00 (s, 1H), 7.48 - 7.41 (m, 1H), 4.50 - 4.40 (m, 2H), 1.57 - 1.45 (m, 3H).

MS calculated: 268.03; MS found: 269.0 [M+H]⁺.

### Example 62

### Synthesis of 7-nitro-4-trifluoromethylquinolin-8-ol (62)

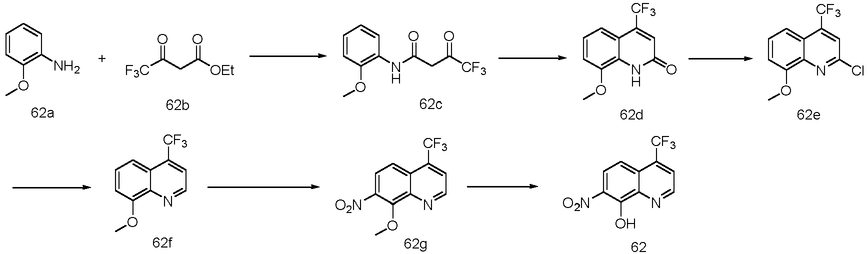

At room temperature, o-methoxyaniline (62a) (2.36 g, 19.2 mmol), ethyl 4,4,4-trifluoroacetoacetate (62b) (2.95 g, 16.0 mmol) and triethylamine (3.24 g, 32.0 mmol) were added to toluene (16.0 mL). The reaction mixture was stirred at 125°C for 4 hours, cooled to room temperature, and concentrated under reduced pressure to remove the organic solvent. The resulting residue was dissolved in dichloromethane (50.0 mL), and washed successively with hydrochloric acid (2.0 M) (15.0 mL × 2), saturated aqueous sodium bicarbonate solution (20.0 mL) and water (15.0 mL). The organic phase was dried over anhydrous sodium sulfate, filtered, and concentrated under reduced pressure to obtain the crude product 4,4,4-trifluoro-N-(2-methoxyphenyl)-3-oxobutanamide (62c) (3.48 g, crude yield 78%).

At 90°C, 4,4,4-trifluoro-N-(2-methoxyphenyl)-3-oxobutanamide (62c) (2.81 g, 10.7 mmol) was completely dissolved in polyphosphoric acid (14.0 g). The reaction mixture was stirred at 90°C for 3 hours, cooled to room temperature, and diluted with water (100.0 mL) until the polyphosphoric acid was completely dissolved. The resulting mixture was extracted with dichloromethane (40.0 mL × 3). The organic phases were combined, washed with saturated aqueous sodium bicarbonate solution (30.0 mL × 3), dried over anhydrous sodium sulfate, filtered, and concentrated under reduced pressure. The resulting residue was purified by silica gel column chromatography (eluent: petroleum ether/ethyl acetate = 40/1) to obtain 8-methoxy-4-trifluoromethyl-2(1*H*)-quinolinone (62d) (2.21 g, yield 84%).

At room temperature, 8-methoxy-4-trifluoromethyl-2(1*H*)-quinolinone (62d) (2.09 g, 8.6 mmol) was added to phosphorus oxychloride (8.6 mL). The reaction mixture was stirred at 100°C for 2 hours, cooled to room temperature, and diluted with dichloromethane (40.0 mL). The pH of the aqueous phase was adjusted to about 9 to 10 with saturated aqueous sodium carbonate solution. The organic phase was separated, and the aqueous phase was extracted with dichloromethane (15.0 mL × 2). The combined organic phase was washed successively with saturated aqueous sodium carbonate solution (25.0 mL), water (25.0 mL) and saturated brine (25.0 mL), dried over anhydrous sodium sulfate, filtered, and concentrated under reduced pressure to obtain the crude product 2-chloro-8-methoxy-4-trifluoromethylquinoline (62e) (2.26 g, crude yield 100%).

At room temperature, 2-chloro-8-methoxy-4-trifluoromethylquinoline (62e) (520.0 mg, 2.0 mmol) was added to a mixed solvent of tetrahydrofuran (10.0 mL) and methanol (10.0 mL), followed by the addition of palladium/carbon (palladium loading: 10 weight%) (210.0 mg, 0.2 mmol). The reaction mixture was stirred under a hydrogen atmosphere for 1 hour, and filtered through diatomite. The filter cake was washed with a mixed solvent of dichloromethane and methanol (volume ratio, dichloromethane/methanol = 20/1) (30.0 mL). The filtrate was concentrated under reduced pressure. The resulting residue was dissolved in dichloromethane (20.0 mL), washed with saturated aqueous sodium bicarbonate solution (10.0 mL × 2), dried over anhydrous sodium sulfate, filtered, and concentrated under reduced pressure. The resulting residue was purified by silica gel column chromatography (eluent: petroleum ether/ethyl acetate = 3/1) to obtain 8-methoxy-4-trifluoromethylquinoline (62f) (380.0 mg, yield 84%).

At 0°C, nitric acid (65 weight%) (0.69 mL, 10.0 mmol) was slowly added dropwise to a solution of 8-methoxy-4-trifluoromethylquinoline (62f) (380.0 mg, 1.7 mmol) in acetic anhydride (5.0 mL), and stirred for 10 minutes. Sulfuric acid (98 weight%) (0.12 mL, 2.2 mmol) was slowly added dropwise. The reaction mixture was stirred at 0°C for 30 minutes, and the pH of the aqueous phase was adjusted to about 9 to 10 with aqueous sodium hydroxide solution (1.0 M). The resulting mixture was extracted with dichloromethane (10.0 mL × 3). The organic phases were combined, dried over anhydrous sodium sulfate, filtered, and concentrated under reduced pressure. The resulting residue was purified by silica gel column chromatography (eluent: petroleum ether/ethyl acetate = 2/1) to obtain 8-methoxy-7-nitro-4-trifluoromethylquinoline (62g) (93.5 mg, yield 20%).

At room temperature, 8-methoxy-7-nitro-4-trifluoromethylquinoline (62g) (93.5 mg, 0.34 mmol) and lithium chloride (144.0 mg, 3.4 mmol) were added to N,N-dimethylformamide (0.4 mL). The reaction mixture was stirred at 170°C for 1 hour, cooled to room temperature, and concentrated under reduced pressure to remove the organic solvent. Water (1.0 mL) was added to the resulting residue, stirred for 3 minutes, and filtered under reduced pressure. The filter cake was washed with water (0.2 mL × 2), and dried under vacuum to obtain 7-nitro-4-trifluoromethylquinolin-8-ol (62) (70.0 mg, yield 79%).

¹H NMR (400 MHz, DMSO-*d*₆) δ: 8.79 (d, *J* = 3.6 Hz, 1H), 8.10 (d, *J* = 9.6 Hz, 1H), 7.88 (d, *J* = 4.4 Hz, 1H), 6.52 (dd, *J* = 9.6, 2.4 Hz, 1H).

MS calculated: 258.03; MS found: 259.1 [M+H]⁺.

### Example 63

### Synthesis of 5-chloro-7-nitro-4-(6-azaspiro[2.5]octan-6-yl)quinolin-8-ol (63)

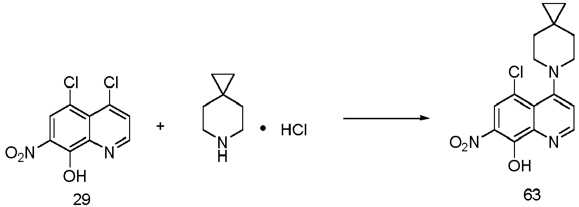

At room temperature, 6-azaspiro[2.5]octane hydrochloride (85.4 mg, 0.58 mmol) and N,N-diisopropylethylamine (125.0 mg, 0.96 mmol) were added to N,N-dimethylformamide (2.0 mL). The resulting mixture was stirred at room temperature for 1 hour, followed by the addition of 4,5-dichloro-7-nitroquinolin-8-ol (29) (50.0 mg, 0.19 mmol). The reaction mixture was stirred at 80°C for 4 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile=100% - 70%/30%, gradient elution, 0.1% formic acid was added to the mobile phase by volume percentage, and the flow rate was 20.0 mL/min) to obtain 5-chloro-7-nitro-4-(6-azaspiro[2.5]octan-6-yl)quinolin-8-ol (63) (3.5 mg, yield 6%).

¹H NMR (400 MHz, DMSO-*d*₆) δ: 8.32 - 8.20 (m, 1H), 7.90 - 7.85 (m, 1H), 7.44 - 7.33 (m, 1H), 3.75 - 3.60 (m, 4H), 2.05 - 1.95 (m, 1H), 1.65 - 1.35 (m, 4H), 0.50 - 0.28 (m, 4H).

MS calculated: 333.09; MS found: 334.1 [M+H]⁺.

### Example 64

### Synthesis of 5-chloro-4-cyclohexyl-7-nitroquinolin-8-ol (64)

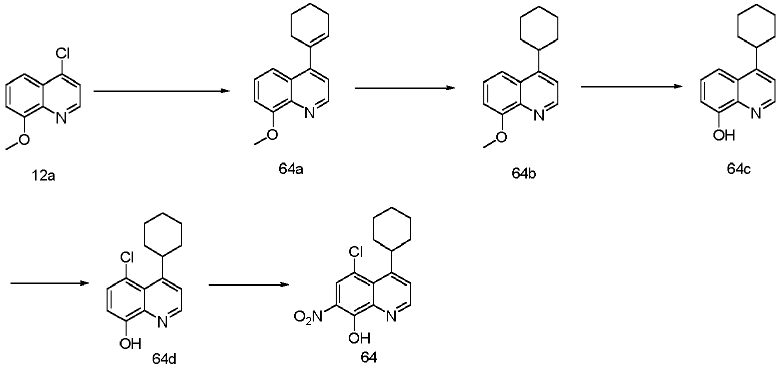

At room temperature, 4-chloro-8-methoxyquinoline (12a) (1.0 g, 5.2 mmol), cyclohexenylboronic acid (0.975 g, 7.7 mmol), tetrakis(triphenylphosphine)palladium (0.600 g, 0.50 mmol) and potassium carbonate (1.5 g, 11.0 mmol) were added successively to a mixed solvent of toluene (10.0 mL), ethanol (1.0 mL) and water (2.0 mL). The reaction mixture was stirred at 100°C for 12 hours, cooled to room temperature, and diluted with water (20.0 mL). The resulting mixture was extracted with ethyl acetate (30.0 mL × 2). The organic phases were combined, dried over anhydrous sodium sulfate and filtered, and the filtrate was concentrated under reduced pressure. The resulting residue was purified by silica gel column chromatography (eluent: petroleum ether/ethyl acetate = 1/1) to obtain 4-cyclohexenyl-8-methoxyquinoline (64a) (1.1 g, yield 89%).

At room temperature, 4-cyclohexenyl-8-methoxyquinoline (64a) (850.0 mg, 3.5 mmol) and palladium/carbon (palladium loading: 10 weight%) (85.0 mg, 0.080 mmol) were successively added to methanol (20.0 mL). The resulting mixture was stirred under a hydrogen atmosphere for 3 hours, and filtered through diatomite, and the filter cake was washed with ethyl acetate (10.0 mL × 3). The filtrate was concentrated to dryness under reduced pressure to obtain 4-cyclohexyl-8-methoxyquinoline (64b) (850.0 mg, yield 99%).

At 0°C, a solution of boron tribromide in dichloromethane (1.0 M) (7.0 mL, 7.0 mmol) was slowly added dropwise to a solution of 4-cyclohexyl-8-methoxyquinoline (64b) (420.0 mg, 1.7 mmol) in dichloromethane (2.0 ml). The reaction mixture was warmed up to room temperature, stirred at 50°C for 5 hours, and cooled to room temperature. The pH of the aqueous phase was adjusted to about 8 to 9 with saturated aqueous sodium bicarbonate solution. The resulting mixture was extracted with ethyl acetate (10.0 mL × 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 2/1) to obtain 4-cyclohexylquinolin-8-ol (64c) (250.0 mg, yield 65%).

At 0°C, N-chlorosuccinimide (147.0 mg, 1.1 mmol) was added to a solution of 4-cyclohexylquinolin-8-ol (64c) (250.0 mg, 1.1 mmol) in sulfuric acid (98 weight%) (7.0 mL). The reaction mixture was stirred at 0°C for 1 hour, and the pH of the aqueous phase was adjusted to about 8 to 9 with saturated aqueous sodium bicarbonate solution. The resulting mixture was extracted with ethyl acetate (10.0 mL × 3). The organic phases were combined, dried over anhydrous sodium sulfate and filtered, and the filtrate was concentrated under reduced pressure. The resulting residue was purified by silica gel column chromatography (eluent: petroleum ether/ethyl acetate = 3/1) to obtain 5-chloro-4-cyclohexylquinolin-8-ol (64d) (230.0 mg, yield 80%).

At room temperature, sodium nitrite (600.0 mg, 7.6 mmol) was added in batches to a suspension of 5-chloro-4-cyclohexylquinolin-8-ol (64d) (200.0 mg, 0.76 mmol) in hydrochloric acid (2.0 M) (10.0 mL). The reaction mixture was stirred at room temperature for 5 hours, and the pH of the aqueous phase was adjusted to about 8 to 9 with ammonia (25 to 28 weight%, NH₃ content). The resulting mixture was filtered under reduced pressure, and the filter cake was purified by reversed-phase high performance liquid chromatography (the chromatographic column was Eclipse XDB-C18 (21.2 mm×250 mm, 7µm), the mobile phase was water/acetonitrile=100% - 70%/30%, gradient elution, 0.1% formic acid was added to the mobile phase by volume percentage, and the flow rate was 20.0 mL/min) to obtain 5-chloro-4-cyclohexyl-7-nitroquinolin-8-ol (64) (60.0 mg, yield 26%).

¹H NMR (400 MHz, DMSO-*d*₆) δ: 8.85 (d, *J* = 4.0 Hz, 1H), 7.72 (d, *J* = 4.0 Hz, 1H), 7.68 - 7.63 (m, 1H), 7.17 - 7.11 (m, 1H), 4.36 - 4.26 (m, 1H), 1.97 - 1.73 (m, 4H), 1.61 - 1.22 (m, 6H).

MS calculated: 261.09; MS found: 262.1 [M+H]⁺.

### Example 65

### Synthesis of 5-chloro-4-fluoro-7-nitroquinolin-8-ol (65)

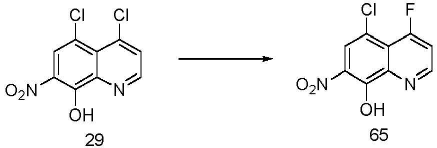

At room temperature, 4,5-dichloro-7-nitroquinolin-8-ol (29) (60.0 mg, 0.23 mmol) and cesium fluoride (350.0 mg, 2.3 mmol) were added to dimethyl sulfoxide (2.0 mL). The resulting mixture was stirred at 180°C for 2 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile=100% - 70%/30%, gradient elution, 0.1% formic acid was added to the mobile phase by volume percentage, and the flow rate was 20.0 mL/min) to obtain 5-chloro-4-fluoro-7-nitroquinolin-8-ol (65) (12.0 mg, yield 22%).

¹H NMR (400 MHz, DMSO-*d*₆) δ: 9.13 - 9.01 (m, 1H), 8.18 (s, 1H), 7.88 - 7.73 (m, 1H).

MS calculated: 241.99; MS found: 243.0 [M+H]⁺.

### Example 66

### Synthesis of 5-chloro-4-(3-methylpiperidin-1-yl)-7-nitroquinolin-8-ol (66)

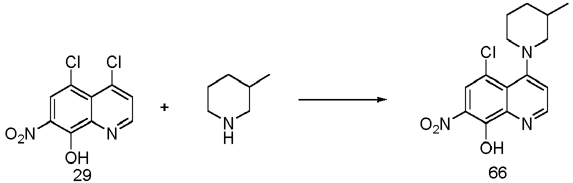

In accordance with the same synthesis method of Example 46, 5-chloro-4-(3-methylpiperidin-1-yl)-7-nitroquinolin-8-ol (66) (62.0 mg, yield 99.8%) was obtained from 4,5-dichloro-7-nitroquinolin-8-ol (29) (50.0 mg, 0.19 mmol) and 3-methylpiperidine (57.0 mg, 0.57 mmol).

¹H NMR (400 MHz, DMSO-*d*₆) δ 7.82 (dd, *J* = 16.2, 1.6 Hz, 1H), 7.27 - 7.04 (m, 2H), 3.28 - 3.13 (m, 3H), 2.76 (td, *J* = 12.7, 3.0 Hz, 1H), 2.26 - 2.12 (m, 1H), 2.07 - 1.95 (m, 1H), 1.81 - 1.71 (m, 3H), 0.91 (d, *J* = 6.5 Hz, 3H).

MS calculated: 321.1; MS found: 322.1[M+H]⁺.

### Example 67

### Synthesis of 4-(benzyloxy)-5-chloro-7-nitroquinolin-8-ol hydrochloride (67)

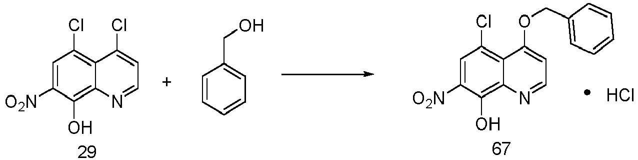

At room temperature, sodium hydride (60 weight%, a mixture in mineral oil) (55.0 mg, 1.38 mmol) was added to a solution of benzyl alcohol (100.0 mg, 0.93 mmol) in dimethyl sulfoxide (6.0 mL). The resulting mixture was stirred at room temperature for 1 hour, followed by the addition of 4,5-dichloro-7-nitroquinolin-8-ol (29) (60.0 mg, 0.23 mmol). The reaction mixture was stirred at room temperature for 3 hours, followed by the addition of hydrochloric acid (36 weight%) (1.5 mL), and filtered under reduced pressure. The filter cake was washed successively with water (0.5 mL × 2) and ethanol (0.5 mL × 2), and dried under vacuum to obtain 4-(benzyloxy)-5-chloro-7-nitroquinolin-8-ol hydrochloride (67) (29.5 mg, yield 35%).

¹H NMR (400 MHz, DMSO-*d*₆) δ: 8.83 - 8.76 (m, 1H), 8.05 - 8.00 (m, 1H), 7.62 - 7.55 (m, 3H), 7.49 - 7.35 (m, 3 H), 5.58 - 5.51 (m, 2 H).

MS calculated: 330.04; MS found: 331.0 [M+H]⁺.

### Example 68

### Synthesis of 4-chloro-5-fluoro-7-nitroquinolin-8-ol (68)

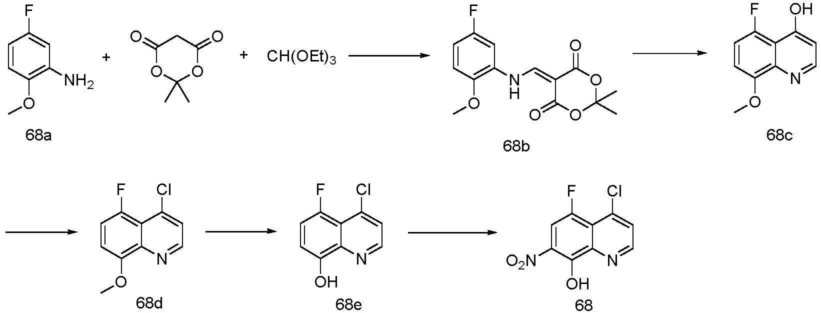

At room temperature, 5-fluoro-2-methoxyaniline (68a) (1.41 g, 10.0 mmol), 2,2-dimethyl-1,3-dioxane-4,6-dione (1.73 g, 12.0 mmol) and triethyl orthoformate (3.56 g, 24.0 mmol) were added to ethanol (10.0 mL). The resulting mixture was stirred at 95°C for 3 hours, cooled to room temperature, and filtered under reduced pressure. The filter cake was washed with ethanol (5.0 mL × 3), and dried under vacuum to obtain 5-(((5-fluoro-2-methoxyphenyl)amino)methylene)-2,2-dimethyl-1,3-dioxane-4,6-dione (68b) (3.21 g, yield 99%).

At room temperature, 5-(((5-fluoro-2-methoxyphenyl)amino)methylene)-2,2-dimethyl-1,3-dioxane-4,6-dione (68b) (2.70 g, 9.2 mmol) was added to diphenyl ether (46.0 mL). The reaction mixture was stirred at 210°C for 4 hours, cooled to room temperature, diluted with petroleum ether (boiling range: 60 to 90°C) (250.0 mL), and filtered under reduced pressure. The filter cake was washed with petroleum ether (boiling range: 60 to 90°C) (8.0 mL × 4), and dried under vacuum to obtain 5-fluoro-8-methoxyquinolin-4-ol (68c) (1.49 g, yield 84%).

At room temperature, phosphorus oxychloride (7.7 mL) was added to 5-fluoro-8-methoxyquinolin-4-ol (68c) (1.49 g, 7.7 mmol). The reaction mixture was stirred at 100°C for 3 hours, cooled to room temperature, and diluted with dichloromethane (40.0 mL). The pH of the aqueous phase was adjusted to about 9 to 10 with aqueous sodium hydroxide solution (1.0 M). The organic phase was separated, and the aqueous phase was extracted with dichloromethane (20.0 mL × 2). The combined organic phase was washed successively with saturated aqueous sodium carbonate solution (20.0 mL), water (20.0 mL) and saturated brine (20.0 mL), dried over anhydrous sodium sulfate and filtered, and the filtrate was concentrated under reduced pressure. The resulting residue was purified by silica gel column chromatography (eluent: dichloromethane/petroleum ether = 2/1) to obtain 4-chloro-5-fluoro-8-methoxyquinoline (68d) (1.33 g, yield 81%).

At 0°C, a solution of boron tribromide in dichloromethane (1.0 M) (31.4 mL, 31.4 mmol) was slowly added dropwise to 4-chloro-5-fluoro-8-methoxyquinoline (68d) (1.33 g, 6.3 mmol). The reaction mixture was warmed up to room temperature, stirred for 5 hours, followed by the addition of ice water (150.0 mL). The pH of the aqueous phase was adjusted to about 9 to 10 with solid sodium carbonate. The organic phase was separated, and the aqueous phase was extracted with dichloromethane (30.0 mL × 4). The combined organic phase was washed with saturated aqueous sodium carbonate solution (40.0 mL), dried over anhydrous sodium sulfate and filtered, and the filtrate was concentrated under reduced pressure. The resulting residue was purified by silica gel column chromatography (eluent: dichloromethane/ethyl acetate = 100/1) to obtain 4-chloro-5-fluoroquinolin-8-ol (68e) (0.65 g, yield 52%).

At 0°C, a solution of sodium nitrite (2.27 g, 32.9 mmol) in water (2.7 mL) was added dropwise to a suspension of 4-chloro-5-fluoroquinolin-8-ol (68e) (0.65 g, 3.3 mmol) in hydrochloric acid (2.4 M) (13.7 mL). The reaction mixture was warmed up to room temperature, and stirred for 10 hours. The pH of the aqueous phase was adjusted to about 9 to 10 with solid sodium carbonate, and the mixture was filtered under reduced pressure. The filter cake was washed with water (3.0 mL × 3), and dried under vacuum to obtain 4-chloro-5-fluoro-7-nitroquinolin-8-ol (68) (0.354 g, yield 44%).
¹H NMR (400 MHz, DMSO-*d*₆) δ: 8.62 (s, 1H), 7.79 (overlapping s, 2H).
MS calculated: 241.99; MS found: 243.1, 245.1 [M+H]⁺.

### Example 69

### Synthesis of 4-(4,4-difluoropiperidin-1-yl)-5-fluoro-7-nitroquinolin-8-ol (69)

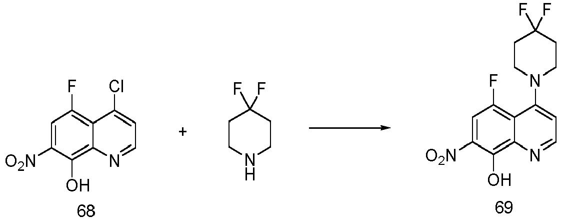

At room temperature, 4-chloro-5-fluoro-7-nitroquinolin-8-ol (68) (42.0 mg, 0.17 mmol) and 4,4-difluoroperidine (63.0 mg, 0.52 mmol) were added to N,N-dimethylformamide (3.0 mL). The reaction mixture was stirred at 90°C for 4 hours, cooled to room temperature, and concentrated under reduced pressure to remove the organic solvent. Water (2.0 mL) was added to the resulting residue, stirred for 3 minutes, and filtered under reduced pressure. The filter cake was washed with water (0.5 mL × 2), and dried under vacuum to obtain 4-(4,4-difluoropiperidin-1-yl)-5-fluoro-7-nitroquinolin-8-ol (69) (30.0 mg, yield 54%).

¹H NMR (400 MHz, DMSO-*d*₆) δ: 8.63 - 8.50 (m, 1 H), 7.78 - 7.70 (m, 1 H), 7.35 - 7.28 (m, 1 H), 3.50 - 3.40 (m, 4 H), 2.30 - 2.15 (m, 4 H).

MS calculated: 327.08; MS found: 328.1 [M+H]⁺.

### Example 70

### Synthesis of 5-fluoro-7-nitro-4-(4-(trifluoromethyl)piperidin-1-yl)quinolin-8-ol (70)

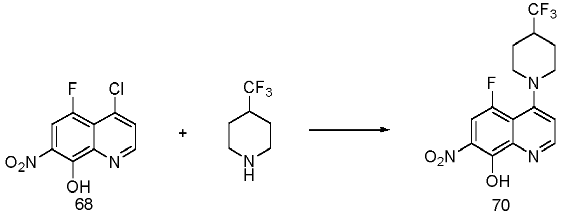

In accordance with the same synthesis method of Example 69, 5-fluoro-7-nitro-4-(4-(trifluoromethyl)piperidin-1-yl)quinolin-8-ol (70) (40.0 mg, yield 56%) was obtained from 4-chloro-5-fluoro-7-nitroquinolin-8-ol (68) (48.0 mg, 0.20 mmol) and 4-(trifluoromethyl)piperidine (153.0 mg, 1.0 mmol).

¹H NMR (400 MHz, DMSO-*d*₆) δ: 8.58 - 8.40 (m, 1 H), 7.68 - 7.55 (m, 1 H), 7.22 - 7.11 (m, 1 H), 3.74 - 3.61 (m, 2 H), 3.46 - 3.39 (m, 2 H), 3.01 - 2.92 (m, 1 H), 2.00 - 1.95 (m, 2 H), 1.72 - 1.65 (m, 2 H).

MS calculated: 359.09; MS found: 360.1 [M+H]⁺.

### Example 71

### Synthesis of ethyl 1-(5-fluoro-8-hydroxy-7-nitroquinolin-4-yl)piperidine-4-carboxylate (71)

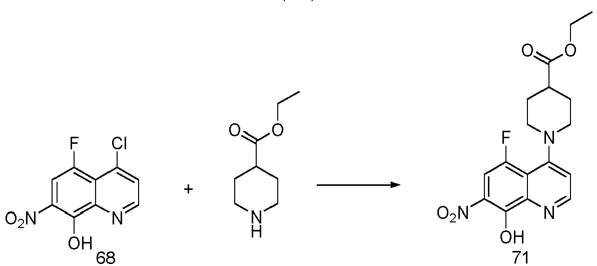

In accordance with the same synthesis method of Example 69, ethyl 1-(5-fluoro-8-hydroxy-7-nitroquinolin-4-yl)piperidine-4-carboxylate (71) (33.0 mg, yield 48%) was obtained from 4-chloro-5-fluoro-7-nitroquinolin-8-ol (68) (45.0 mg, 0.19 mmol) and ethyl 4-piperidinecarboxylate (210 mg, 1.33 mmol).

¹H NMR (400 MHz, DMSO-*d*₆) δ: 8.45 - 8.35 (m, 1H), 7.52 - 7.44 (m, 1H), 7.05 - 6.98 (m, 1H), 4.14 - 4.04 (m, 2H), 3.30 - 3.15 (m, 4H), 2.85 - 2.70 (m, 1H), 2.00 - 1.90 (m, 2H), 1.80 - 1.60 (m, 2H), 1.25 - 1.15 (m, 3H).

MS calculated: 363.12; MS found: 364.1 [M+H]⁺.

### Example 72

### Synthesis of 4-(cyclopropylmethoxy)-5-fluoro-7-nitroquinolin-8-ol (72)

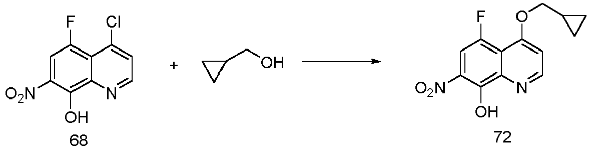

In accordance with the same synthesis method of Example 69, 4-(cyclopropylmethoxy)-5-fluoro-7-nitroquinolin-8-ol (72) (38.0 mg, yield 65%) was obtained from 4-chloro-5-fluoro-7-nitroquinolin-8-ol (68) (50.0 mg, 0.21 mmol) and cyclopropylmethanol (149.0 mg, 2.1 mmol).

¹H NMR (400 MHz, DMSO-*d*₆) δ: 8.82 - 8.78 (m, 1H), 7.80 - 7.73 (m, 1H), 7.39 - 7.33 (m, 1H), 4.23 - 4.18 (m, 2H), 0.88 - 0.80 (m, 1H), 0.62 - 0.60 (m, 2H), 0.46 - 0.40 (m, 2H).

MS calculated: 278.07; MS found: 279.1 [M+H]⁺.

### Example 73

### Synthesis of 5-chloro-7-nitro-4-(trifluoromethyl)-quinolin-8-ol hydrochloride (73)

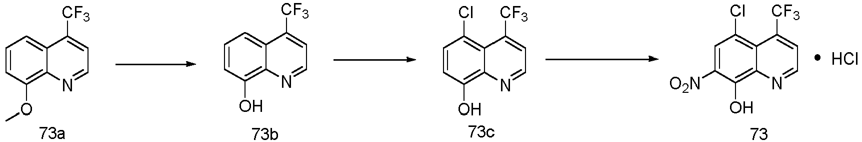

At room temperature, 8-methoxy-4-(trifluoromethyl)quinoline (73a) (230.0 mg, 1.0 mmol) was added to a solution of boron tribromide in dichloromethane (1.0 M) (5.0 mL, 5.0 mmol). The reaction mixture was stirred at room temperature for 7 hours, and diluted with dichloromethane (20.0 mL). The pH of the aqueous phase was adjusted to about 9 to 10 with saturated aqueous sodium carbonate solution. The organic phase was separated, and the aqueous phase was extracted with dichloromethane (10.0 mL × 4). The combined organic phase was dried over anhydrous sodium sulfate, filtered, and concentrated under reduced pressure. The resulting residue was purified by silica gel column chromatography (eluent: dichloromethane/methanol = 50/1) to obtain 4-trifluoromethyl-8-hydroxyquinoline (73b) (190.0 mg, yield 88%).

At room temperature, N-chlorosuccinimide (64.0 mg, 0.48 mmol) was added to a solution of 4-trifluoromethyl-8-hydroxyquinoline (73b) (85.0 mg, 0.4 mmol) in sulfuric acid (98 weight%) (2.0 mL). The reaction mixture was stirred at room temperature for 24 hours, and the pH of the aqueous phase was adjusted to about 9 to 10 with saturated aqueous sodium carbonate solution. The resulting mixture was extracted with dichloromethane (5.0 mL × 4), the combined organic phase was dried over anhydrous sodium sulfate, filtered, and concentrated under reduced pressure. The resulting residue was purified by silica gel column chromatography (eluent: dichloromethane/methanol = 50/1) to obtain 5-chloro-4-(trifluoromethyl)quinolin-8-ol (73c) (87.0 mg, yield 88%).

At 0°C, sodium nitrite (240.0 mg, 3.5 mmol) was added in batches to a suspension of 5-chloro-4-(trifluoromethyl)quinolin-8-ol (73c) (87.0 mg, 0.35 mmol) in hydrochloric acid (2.0 M) (1.75 mL). The reaction mixture was warmed up to room temperature, stirred for 54 hours, and filtered under reduced pressure. The filter cake was washed with a mixed solvent of ethanol and water (volume ratio, ethanol/water = 1/1) (1.0 mL × 2), and dried under vacuum to obtain 5-chloro-7-nitro-4-(trifluoromethyl)-quinolin-8-ol hydrochloride (73) (69.0 mg, yield 60%).

¹H NMR (400 MHz, DMSO-*d*₆) δ: 9.28 (d, *J* = 4.4 Hz, 1H), 8.41 (overlapping s, 2H).

MS calculated: 291.99; MS found: 293.0, 295.0 [M+H]⁺.

### Example 74

### Synthesis of 4-chloro-3-cyclopropyl-7-nitroquinolin-8-ol

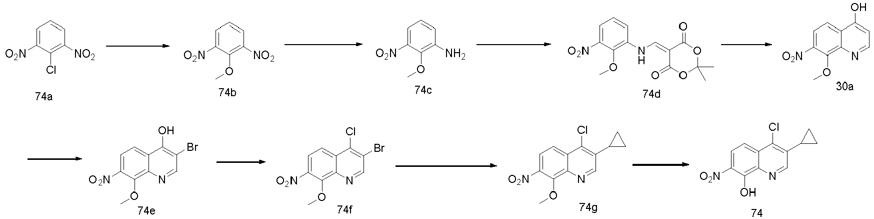

At room temperature, sodium methoxide (10.7 g, 198.0 mmol) was slowly added to a solution of 2,6-dinitrochlorobenzene (74a) (10.0 g, 49.5 mmol) in methanol (100.0 mL). The reaction mixture was stirred at room temperature for 12 hours, and concentrated under reduced pressure to remove the organic solvent. The resulting residue was dissolved in water (30.0 mL) and dichloromethane (30.0 mL). The organic phase was separated, and the aqueous phase was extracted with dichloromethane (30.0 mL × 2). The combined organic phase was dried over anhydrous sodium sulfate, filtered, and concentrated to dryness under reduced pressure to obtain 2-methoxy-1,3-dinitrobenzene (74b) (9.8 g, yield 99%).

At room temperature, reduced iron powder (3.38 g, 60.6 mmol) was added to a solution of 2-methoxy-1,3-dinitrobenzene (74b) (4.0 g, 20.2 mmol) in acetic acid (50.0 mL). The reaction mixture was stirred at 65°C for 1.5 hours, cooled to room temperature, and concentrated under reduced pressure to remove the organic solvent. Dichloromethane (30.0 mL) was added to the resulting residue, and the pH of the aqueous phase was adjusted to about 8 to 9 with saturated aqueous sodium bicarbonate solution. The resulting mixture was filtered under reduced pressure. The organic phase was separated from the filtrate, washed with saturated brine (15.0 mL), dried over anhydrous sodium sulfate, filtered, and concentrated under reduced pressure. The resulting residue was purified by silica gel column chromatography (eluent: petroleum ether/dichloromethane/ethyl acetate = 8/1/1) to obtain 2-methoxy-3-nitroaniline (74c) (2.06 g, yield 61%).

At room temperature, 2-methoxy-3-nitroaniline (74c) (2.06 g, 12.3 mmol), 2,2-dimethyl-1,3-dioxane-4,6-dione (2.12 g, 14.7 mmol) and triethyl orthoformate (4.36 g, 29.4 mmol) were added to ethanol (15.0 mL). The reaction mixture was stirred at 98°C for 4 hours, cooled to room temperature, and filtered under reduced pressure. The filter cake was washed with petroleum ether (boiling range: 60 to 90°C) (10.0 mL × 3), and dried under vacuum to obtain 5-(((2-methoxy-3-nitrophenyl)amino)methylene)-2,2-dimethyl-1,3-dioxane-4,6-dione (74d) (3.85 g, yield 97%).

At room temperature, 5-(((2-methoxy-3-nitrophenyl)amino)methylene)-2,2-dimethyl-1,3-dioxane-4,6-dione (74d) (3.85 g, 11.9 mmol) was added to diphenyl ether (50.0 mL). The reaction mixture was stirred at 265°C for 2 hours, cooled to room temperature, diluted with petroleum ether (boiling range: 60 to 90°C) (250.0 mL), and filtered under reduced pressure. The filter cake was washed with petroleum ether (boiling range: 60 to 90°C) (10.0 mL × 4), and dried under vacuum to obtain 8-methoxy-7-nitroquinolin-4-ol (30a) (2.33 g, yield 89%).

At room temperature, N-bromosuccinimide (404.0 mg, 2.3 mmol) was added to a suspension of 8-methoxy-7-nitroquinolin-4-ol (30a) (500.0 mg, 2.3 mmol) in acetonitrile (6.0 mL). The reaction mixture was stirred at room temperature for 2 hours, and concentrated under reduced pressure to remove the organic solvent. Water (30.0 mL) was added to the resulting residue, stirred for 15 minutes, and filtered under reduced pressure. The filter cake was washed with water (5.0 mL × 2), and dried under vacuum to obtain the crude product 3-bromo-8-methoxy-7-nitroquinolin-4-ol (74e) (628.0 mg, crude yield 93%).

At room temperature, 3-bromo-8-methoxy-7-nitroquinolin-4-ol (74e) (628.0 mg, 0.45 mmol) was added to phosphorus oxychloride (8.0 mL). The reaction mixture was stirred at 100°C for 2 hours, cooled to room temperature, and concentrated under reduced pressure to remove phosphorus oxychloride. The resulting residue was dissolved in dichloromethane (15.0 mL), and the pH of the aqueous phase was adjusted to about 9 to 10 with saturated aqueous sodium carbonate solution. The organic phase was separated, washed with saturated brine (10.0 mL), dried over anhydrous sodium sulfate, filtered, and concentrated under reduced pressure to obtain the crude product 3-bromo-4-chloro-8-methoxy-7-nitroquinoline (74f) (670.0 mg, crude yield 100%).

At room temperature, 3-bromo-4-chloro-8-methoxy-7-nitroquinoline (74f) (350.0 mg, 1.1 mmol), cyclopropylboronic acid (123.0 mg, 1.43 mmol), palladium acetate (25.0 mg, 0.11 mmol), tricyclohexylphosphine (62.0 mg, 0.22 mmol) and potassium phosphate (817.0 mg, 3.85 mmol) were added successively to a mixed solvent of toluene (7.0 mL) and water (0.70 mL). The reaction solution was stirred at 95°C for 3 hours, cooled to room temperature, and diluted with ethyl acetate (10.0 mL) and water (10.0 mL). The organic phase was separated, and the aqueous phase was extracted with ethyl acetate (10.0 mL × 2). The organic phases were combined, washed with saturated brine (20.0 mL), dried over anhydrous sodium sulfate, filtered, and concentrated under reduced pressure. The resulting residue was purified by silica gel column chromatography (eluent: petroleum ether/dichloromethane/ethyl acetate = 7/2/1) to obtain 4-chloro-3-cyclopropyl-8-methoxy-7-nitroquinoline (74g) (117.0 mg, yield 38%).

At room temperature, 4-chloro-3-cyclopropyl-8-methoxy-7-nitroquinoline (74g) (45.0 mg, 0.16 mmol) and lithium chloride (68.0 mg, 1.6 mmol) were added to 1-methyl-2-pyrrolidone (2.0 mL). The reaction mixture was stirred at 180°C for 30 minutes, cooled to room temperature, and concentrated under reduced pressure to remove the organic solvent. Water (2.0 mL) was added to the resulting residue, stirred for 3 minutes, and filtered under reduced pressure. The filter cake was washed with a mixed solvent of ethanol and water (volume ratio, ethanol/water = 1/1) (2.0 mL × 2), and dried under vacuum to obtain 4-chloro-3-cyclopropyl-7-nitroquinolin-8-ol (74) (50.7 mg, yield 91%).

¹H NMR (400 MHz, DMSO-*d*₆) δ: 8.22 (s, 1H), 8.01 (d, *J* = 10.0 Hz, 1H), 6.63 (d, *J* = 9.6 Hz, 1H), 2.30-2.24 (m, 1H), 1.17 - 1.12 (m, 2H), 0.97 - 0.91(m, 2H).

MS calculated: 264.03; MS found: 265.1, 267.0 [M+H]⁺.

### Example 75

### Synthesis of 3-cyclopropyl-4-methoxy-7-nitroquinolin-8-ol (75)

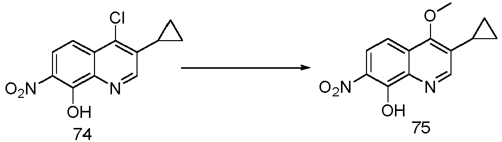

At room temperature, 4-chloro-3-cyclopropyl-7-nitroquinolin-8-ol (74) (42.0 mg, 0.16 mmol) and sodium methoxide (43.0 mg, 0.80 mmol) were added to a mixed solvent of 1-methyl-2-pyrrolidone (1.0 mL) and dimethyl sulfoxide (1.0 mL). The resulting mixture was stirred at 120°C for 2 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/methanol = 100%/0 - 10%/90%, gradient elution, 0.1% formic acid was added to the mobile phase by volume percentage, and the flow rate was 20.0 mL/min) to obtain 3-cyclopropyl-4-methoxy-7-nitroquinolin-8-ol (75) (10.7 mg, yield 26%).

¹H NMR (400 MHz, DMSO-*d*₆) δ: 7.97 (d, *J* = 9.6 Hz, 1H), 7.86 (s, 1H), 6.80 (d, *J* = 10.0 Hz, 1H), 3.98 (s, 3H), 2.29 - 2.27 (m, 1H), 1.13 - 1.07 (m, 2H), 1.06 - 1.01 (m, 2H).

MS calculated: 260.08; MS found: 261.1 [M+H]⁺.

### Example 76

### Synthesis of 8-hydroxy-7-nitroquinoline-4-carbonitrile (76)

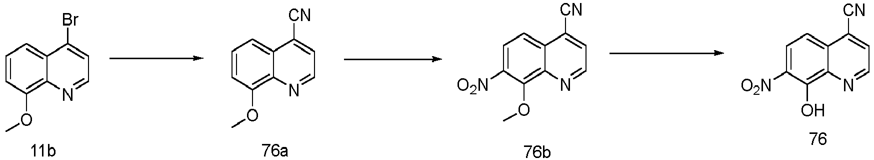

At room temperature, zinc cyanide (788.0 mg, 6.71 mmol) and tetrakis(triphenylphosphine)palladium (728.0 mg, 0.63 mmol) were added to a solution of 4-bromo-8-methoxyquinoline (11b) (1.0 g, 4.2 mmol) in N,N-dimethylformamide (15.0 mL). The reaction mixture was stirred at 100°C for 2.5 hours, cooled to room temperature, and filtered under reduced pressure. The filtrate was concentrated under reduced pressure to remove the organic solvent, and the resulting residue was dissolved in dichloromethane (15.0 mL) and water (15.0 mL). The organic phase was separated, washed with saturated brine (10.0 mL), dried over anhydrous sodium sulfate, filtered, and concentrated under reduced pressure. Ethyl acetate (5.0 mL) was added to the resulting residue, stirred for 3 minutes, and filtered under reduced pressure. The filter cake was washed with ethyl acetate (2.5 mL × 2), and dried under vacuum to obtain 8-methoxyquinoline-4-carbonitrile (76a) (650.0 mg, yield 84%).

At 0°C, nitric acid (65 weight%) (0.45 mL, 6.52 mmol) was slowly added to a solution of 8-methoxyquinoline-4-carbonitrile (76a) (200.0 mg, 1.08 mmol) in acetic anhydride (5.0 mL). The reaction mixture was stirred at 0°C for 10 minutes, followed by the slowly dropwise addition of sulfuric acid (98 weight%) (0.1 mL, 1.87 mmol), and continued to stir for 30 minutes. The pH of the aqueous phase was adjusted to about 9 to 10 with aqueous sodium hydroxide solution (1.0 M). The resulting mixture was extracted with dichloromethane (10.0 mL × 3). The organic phases were combined, washed with saturated brine (10.0 mL), dried over anhydrous sodium sulfate, filtered, and concentrated under reduced pressure. The resulting residue was purified by silica gel column chromatography (eluent: petroleum ether/ethyl acetate = 4/1) to obtain 8-methoxy-7-nitroquinoline-4-carbonitrile (76b) (65.0 mg, yield 26%).

At room temperature, 8-methoxy-7-nitroquinoline-4-carbonitrile (76b) (65.0 mg, 0.28 mmol) and lithium chloride (119.0 mg, 2.8 mmol) were added to N,N-dimethylformamide (2.0 mL). The reaction mixture was stirred at 160°C for 30 minutes, cooled to room temperature, and concentrated under reduced pressure to remove the organic solvent. Water (2.0 mL) was added to the resulting residue, stirred for 3 minutes, and filtered under reduced pressure. The filter cake was washed successively with ethanol (2.0 mL) and water (2.0 mL), and dried under vacuum to obtain 8-hydroxy-7-nitroquinoline-4-carbonitrile (76) (44.0 mg, yield 72%).

¹H NMR (400 MHz, DMSO-*d₆*) δ: 8.75 (d, *J* = 4.4 Hz, 1H), 8.15 (d, *J* = 9.2 Hz, 1H), 8.02 (d, *J* = 4.4 Hz, 1H), 6.54 (d, *J* = 9.6 Hz, 1H).

MS calculated: 215.03; MS found: 216.1 [M+H]⁺.

### Example 77

### Synthesis of 4-(difluoromethoxy)-7-nitroquinolin-8-ol (77)

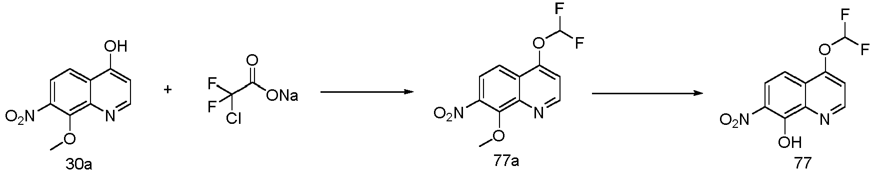

At room temperature, 8-methoxy-7-nitroquinolin-4-ol (30a) (66.1 mg, 0.30 mmol) and sodium difluorochloroacetate (137.0 mg, 0.90 mmol) were added successively to a suspension of cesium carbonate (293.0 mg, 0.30 mmol) in N,N-dimethylformamide (1.5 mL). The reaction mixture was stirred at room temperature for 15 minutes, and then at 80°C for 30 minutes, cooled to room temperature, and diluted with water (10.0 mL) and dichloromethane (10.0 mL). The organic phase was separated, and the aqueous phase was extracted with dichloromethane (5.0 mL × 3). The combined organic phase was dried over anhydrous sodium sulfate, filtered, and concentrated under reduced pressure. The resulting residue was purified by silica gel column chromatography (eluent: dichloromethane/petroleum ether = 3/1) to obtain 4-(difluoromethoxy)-8-methoxy-7-nitroquinoline (77a) (52.6 mg, yield 65%).

At room temperature, 4-(difluoromethoxy)-8-methoxy-7-nitroquinoline (77a) (52.6 mg, 0.195 mmol) and lithium chloride (82.5 mg, 1.95 mmol) were added to N,N-dimethylformamide (0.24 mL). The reaction mixture was stirred at 160°C for 30 minutes, cooled to room temperature, and concentrated under reduced pressure to remove the organic solvent. Water (1.0 mL) was added to the resulting residue, stirred for 3 minutes, and filtered under reduced pressure. The filter cake was washed with a mixed solvent of ethanol and water (volume ratio, ethanol/water = 1/1) (0.5 mL × 3), and dried under vacuum to obtain 4-(difluoromethoxy)-7-nitroquinolin-8-ol (77) (37.2 mg, yield 74%).

¹H NMR (400 MHz, DMSO-*d₆*) δ: 8.59 (d, *J* = 5.2 Hz, 1H), 7.98 (d, *J* = 9.6 Hz, 1H), 7.62 (t, *J* = 72.8 Hz, 1H), 7.32 (d, *J* = 5.2 Hz, 1H), 6.59 (d, *J* = 9.6 Hz, 1H).

MS calculated: 256.03; MS found: 257.1 [M+H]⁺.

### Example 78

### Synthesis of 3-(8-hydroxy-7-nitroquinolin-4-yl)-N-acryloylmorpholine (78)

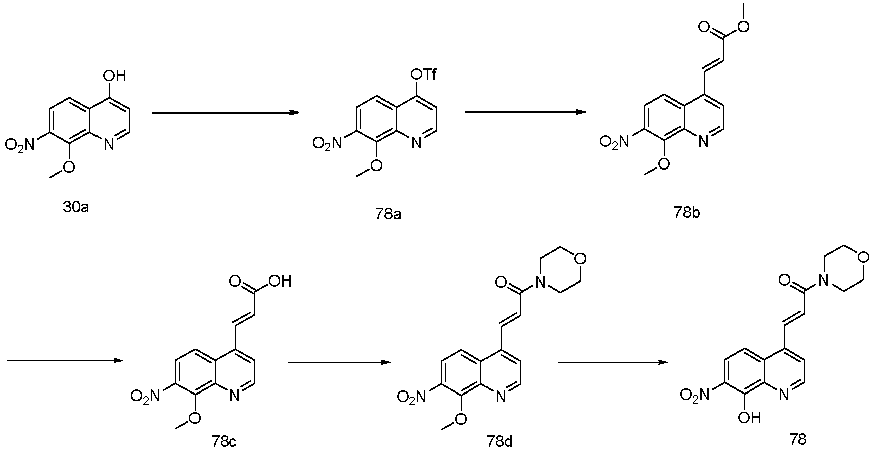

At room temperature, 8-methoxy-7-nitroquinolin-4-ol (30a) (5.07 g, 23.0 mmol) and N,N-diisopropylethylamine (12.3 mL, 70.3 mmol) were added to N,N-dimethylformamide (75.0 mL), followed by the slow addition of N-phenylbis(trifluoromethanesulfonyl)imide (11.1 g, 31.1 mmol). The reaction mixture was stirred at room temperature for 12 hours, and diluted with ethyl acetate (100.0 mL) and water (100.0 mL). The organic phase was separated, and the aqueous phase was extracted with ethyl acetate (50.0 mL × 3). The combined organic phase was dried over anhydrous sodium sulfate, filtered, and concentrated under reduced pressure. The resulting residue was purified by silica gel column chromatography (eluent: petroleum ether/ethyl acetate = 8/1) to obtain 8-methoxy-7-nitroquinolin-4-yl trifluoromethanesulfonate (78a) (3.99 g, yield 50%).

At room temperature, 8-methoxy-7-nitroquinolin-4-yl trifluoromethanesulfonate (78a) (3.99 g, 11.3 mmol), methyl acrylate (2.2 mL, 24.3 mmol), palladium acetate (254.0 mg, 1.13 mmol), tris(o-methylphenyl)phosphine (516.0 mg, 1.7 mmol) and triethylamine (4.3 mL, 31.5 mmol) were added successively to N,N-dimethylformamide (75.0 mL). The reaction mixture was stirred at 100°C for 12 hours, cooled to room temperature, and concentrated under reduced pressure to remove the organic solvent. Ethyl acetate (40.0 mL) and water (40.0 mL) were added to the resulting residue. The organic phase was separated, and the aqueous phase was extracted with ethyl acetate (20.0 mL × 2). The combined organic phase was dried over anhydrous sodium sulfate, filtered, and concentrated under reduced pressure. The resulting residue was purified by silica gel column chromatography (eluent: petroleum ether/dichloromethane/ethyl acetate = 8/1/1) to obtain methyl 3-(8-methoxy-7-nitroquinolin-4-yl)acrylate (78b) (618.9 mg, yield 19%).

At room temperature, methyl 3-(8-methoxy-7-nitroquinolin-4-yl)acrylate (78b) (618.9 mg, 2.15 mmol) was added to a mixed solvent of tetrahydrofuran (8.0 mL), methanol (8.0 mL) and water (2.7 mL), followed by the addition of lithium hydroxide (77.4 mg, 3.23 mmol). The reaction mixture was stirred at room temperature for 12 hours, and concentrated under reduced pressure to remove the organic solvent. Ethyl acetate (15.0 mL) and water (15.0 mL) were added to the resulting residue, and the aqueous phase was separated. The pH of the aqueous phase was adjusted to about 3 to 4 with hydrochloric acid (1.0 M) solution. The resulting mixture was filtered under reduced pressure, the filter cake was washed with water (2.0 mL × 2), and dried under vacuum to obtain 3-(8-methoxy-7-nitroquinolin-4-yl)acrylic acid (78c) (560.1 mg, yield 95%).

At room temperature, 3-(8-methoxy-7-nitroquinolin-4-yl)acrylic acid (78c) (143.0 mg, 0.52 mmol), morpholine (68.0 mg, 0.78 mmol), O-(7-azabenzotriazol-1-yl)-N,N,N',N'-tetramethyluronium hexafluorophosphate (297.0 mg, 0.78 mmol) and N,N-diisopropylethylamine (0.28 mL, 1.56 mmol) were added successively to N,N-dimethylformamide (2.0 mL). The reaction mixture was stirred at room temperature for 12 hours, and concentrated under reduced pressure to remove the organic solvent. Ethyl acetate (10.0 mL) and water (10.0 mL) were added to the resulting residue. The organic phase was separated, and the aqueous phase was extracted with ethyl acetate (10.0 mL × 2). The combined organic phase was dried over anhydrous sodium sulfate, filtered, and concentrated under reduced pressure. The resulting residue was purified by silica gel column chromatography (eluent: dichloromethane/ethyl acetate = 7/3) to obtain 3-(8-methoxy-7-nitroquinolin-4-yl)-N-acryloylmorpholine (78d) (88.0 mg, yield 49%).

At room temperature, 3-(8-methoxy-7-nitroquinolin-4-yl)-N-acryloylmorpholine (78d) (44.0 mg, 0.13 mmol) and lithium chloride (53.8 mg, 1.3 mmol) were added to N,N-dimethylformamide (1.0 mL). The reaction mixture was stirred at 160°C for 1 hour, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/methanol = 100%/0 - 80%/20%, gradient elution, 0.1% formic acid was added to the mobile phase by volume percentage, and the flow rate was 20.0 mL/min) to obtain 3-(8-hydroxy-7-nitroquinolin-4-yl)-N-acryloylmorpholine (78) (20.2 mg, yield 24%).

¹H NMR (400 MHz, DMSO-*d₆*) δ: 8.70 (d, *J* = 4.6 Hz, 1H), 8.09 (d, *J* = 15.6 Hz, 1H), 8.02 (d, *J* = 9.6 Hz, 1H), 7.99 (d, *J* = 4.6 Hz, 1H), 7.51 (d, *J* = 15.2 Hz, 1H), 6.87 (d, *J* = 10.0 Hz, 1H), 3.81 - 3.74 (m, 2H), 3.68 - 3.60 (m, 4H), 3.44 - 3.37 (m, 2H).

MS calculated: 329.10; MS found: 330.2 [M+H]⁺.

### Example 79

### Synthesis of 3-(8-hydroxy-7-nitroquinolin-4-yl)-N-methacrylamide (79)

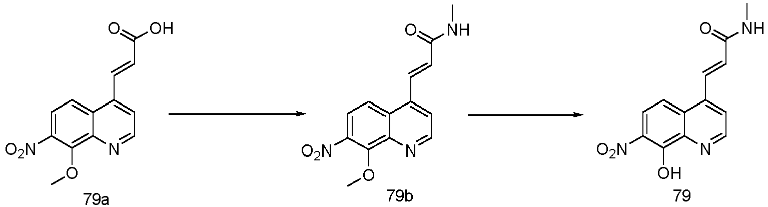

At room temperature, 3-(8-methoxy-7-nitroquinolin-4-yl)acrylic acid (79a) (150.0 mg, 0.55 mmol), methylamine hydrochloride (55.0 mg, 0.82 mmol), O-(7-azabenzotriazol-1-yl)-N,N,N',N'-tetramethyluronium hexafluorophosphate (312.0 mg, 0.82 mmol) and N,N-diisopropylethylamine (0.30 mL, 1.64 mmol) were added successively to N,N-dimethylformamide (2.0 mL). The reaction mixture was stirred at room temperature for 12 hours, and concentrated under reduced pressure to remove the organic solvent. Ethyl acetate (10.0 mL) and water (10.0 mL) were added to the resulting residue. The organic phase was separated, and the aqueous phase was extracted with ethyl acetate (10.0 mL × 2). The combined organic phase was dried over anhydrous sodium sulfate, filtered, and concentrated under reduced pressure. The resulting residue was purified by silica gel column chromatography (eluent: dichloromethane/ethyl acetate = 7/3) to obtain 3-(8-methoxy-7-nitroquinolin-4-yl)-N-methylacrylamide (79b) (105.0 mg, yield 67%).

At room temperature, 3-(8-methoxy-7-nitroquinolin-4-yl)-N-methylacrylamide (79b) (105.0 mg, 0.37 mmol) and lithium chloride (157.0 mg, 3.7 mmol) were added to N,N-dimethylformamide (3.0 mL). The reaction mixture was stirred at 160°C for 1 hour, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/methanol = 100%/0 - 80%/20%, gradient elution, 0.1% formic acid was added to the mobile phase by volume percentage, and the flow rate was 20.0 mL/min) to obtain 3-(8-hydroxy-7-nitroquinolin-4-yl)-N-methacrylamide (79) (18.7 mg, yield 19%).

¹H NMR (400 MHz, DMSO-*d₆*) δ: 9.03 (d, *J* = 4.8 Hz, 1H), 8.44 (d, *J* = 4.4 Hz, 1H), 8.12 (d, *J* = 9.6 Hz, 1H), 8.08 (d, *J* = 16.0 Hz, 1H), 7.97 (d, *J* = 4.4 Hz, 1H), 7.70 (d, *J* = 9.2 Hz, 1H), 6.92 (d, *J* = 15.6 Hz, 1H), 2.79 (d, *J* = 4.8 Hz, 3H).

MS calculated: 273.07; MS found: 274.1 [M+H]⁺.

### Example 80

### Synthesis of 4-ethynyl-7-nitroquinolin-8-ol (80)

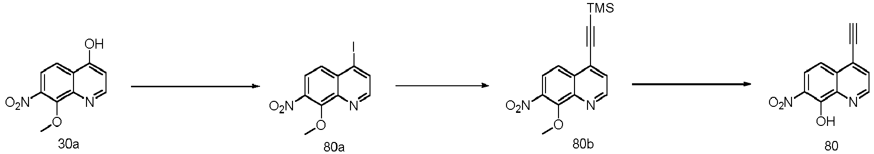

At 0°C, 8-methoxy-7-nitroquinolin-4-ol (30a) (300.0 mg, 1.36 mmol) and pyridine (129.0 mg, 1.63 mmol) were added to dichloromethane (6.0 mL), followed by the slowly dropwise addition of trifluoromethanesulfonic anhydride (751.0 mg, 2.66 mmol). The reaction mixture was warmed up to room temperature, and stirred for 3.5 hours, followed by the addition of sodium iodide (1.02 g, 6.8 mmol). Trifluoromethanesulfonic acid (0.2 mL, 2.26 mmol) was slowly added dropwise at 0°C. The reaction mixture was stirred at room temperature for 16 hours, and the pH of the aqueous phase was adjusted to about 9 to 10 with saturated aqueous sodium carbonate solution. The resulting mixture was extracted with dichloromethane (10.0 mL × 3). The organic phases were combined, dried over anhydrous sodium sulfate, filtered, and concentrated under reduced pressure. The resulting residue was purified by silica gel column chromatography (eluent: petroleum ether/ethyl acetate = 5/1) to obtain 4-iodo-8-methoxy-7-nitroquinoline (80a) (210.0 mg, yield 47%).

At room temperature, (trimethylsilyl)acetylene (188.0 mg, 1.9 mmol), bis(triphenylphosphine)palladium(II) chloride (45.0 mg, 0.06 mmol), copper iodide (6.0 mg, 0.03 mmol) and triethylamine (0.27 mL, 1.9 mmol) were added successively to a solution of 4-iodo-8-methoxy-7-nitroquinoline (80a) (210.0 mg, 0.64 mmol) in N,N-dimethylformamide (5.0 mL). The reaction mixture was stirred at 60°C for 16 hours, cooled to room temperature, and concentrated under reduced pressure to remove the organic solvent. The resulting residue was purified by silica gel column chromatography (eluent: petroleum ether/ethyl acetate = 4/1) to obtain 8-methoxy-7-nitro-4-((trimethylsilyl)ethynyl)quinoline (80b) (91.0 mg, yield 48%).

At room temperature, 8-methoxy-7-nitro-4-((trimethylsilyl)ethynyl)quinoline (80b) (81.0 mg, 0.27 mmol) and lithium chloride (113.0 mg, 2.7 mmol) were added to N,N-dimethylformamide (2.0 mL). The reaction mixture was stirred at 150°C for 1 hour, cooled to room temperature, and concentrated under reduced pressure to remove the organic solvent. Potassium carbonate (372.0 mg, 2.7 mmol) and methanol (5.0 mL) were added to the resulting residue, stirred at room temperature for 2 hours, and filtered under reduced pressure. The filtrate was concentrated under reduced pressure. Water (2.0 mL) was added to the resulting residue, stirred for 3 minutes, and filtered under reduced pressure. A mixed solvent of ethanol (2.0 mL) and water (2.0 mL) was added to the resulting filter cake, stirred for 10 minutes, and filtered under reduced pressure. The filter cake was dried under vacuum to obtain 4-ethynyl-7-nitroquinolin-8-ol (80) (57.0 mg, yield 99%).

¹H NMR (400 MHz, DMSO-*d₆*) δ: 8.58 (s, 1H), 8.01 (d, *J* = 9.5 Hz, 1H), 7.62 (s, 1H), 6.67 (d, *J* = 9.5 Hz, 1H), 4.96 (s, 1H).

MS calculated: 214.04; MS found: 215.1 [M+H]⁺.

### Example 81

### Synthesis of 4-(difluoromethyl)-7-nitroquinolin-8-ol (81)

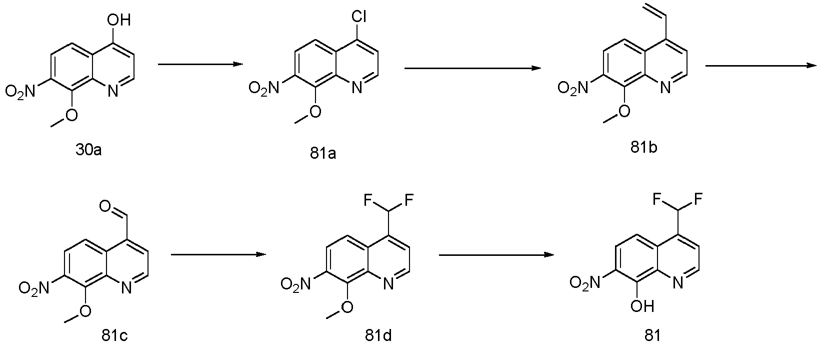

At room temperature, 8-methoxy-7-nitroquinolin-4-ol (30a) (1.5 g, 6.81 mmol) was added to phosphorus oxychloride (15.0 mL). The reaction mixture was stirred at 100°C for 2 hours, cooled to room temperature, and concentrated under reduced pressure. Dichloromethane (20.0 mL) was added to the resulting residue, and the pH of the aqueous phase was adjusted to about 8 to 9 with saturated aqueous sodium bicarbonate solution. The organic phase was separated, and the aqueous phase was extracted with dichloromethane (20.0 mL × 2). The combined organic phase was dried over anhydrous sodium sulfate, filtered, and concentrated under reduced pressure. The resulting residue was purified by silica gel column chromatography (eluent: petroleum ether/ethyl acetate = 4/1) to obtain 4-chloro-8-methoxy-7-nitroquinoline (81a) (510.0 mg, yield 32%).

At room temperature, 4-chloro-8-methoxy-7-nitroquinoline (81a) (510.0 mg, 2.13 mmol), potassium vinyl trifluoroborate (458.0 mg, 3.42 mmol), [1,1'-bis(diphenylphosphino)ferrocene]dichloropalladium(II) (234.0 mg, 0.32 mmol) and potassium carbonate (885.0 mg, 6.41 mmol) were added successively to a mixed solvent of 1,4-dioxane (8.0 mL) and water (2.0 mL). The reaction mixture was stirred at 95°C for 16 hours, cooled to room temperature, and concentrated under reduced pressure. Dichloromethane (10.0 mL) and water (10.0 mL) were added to the resulting residue. The organic phase was separated, and the aqueous phase was extracted with dichloromethane (10.0 mL × 2). The combined organic phase was dried over anhydrous sodium sulfate, filtered, and concentrated under reduced pressure. The resulting residue was purified by silica gel column chromatography (eluent: petroleum ether/ethyl acetate = 4/1) to obtain 8-methoxy-7-nitro-4-vinylquinoline (81b) (350.0 mg, yield 71%).

At 0°C, 8-methoxy-7-nitro-4-vinylquinoline (81b) (350.0 mg, 1.52 mmol) and potassium osmate dihydrate (56.0 mg, 0.15 mmol) were added successively to a mixed solvent of tetrahydrofuran (18.0 mL) and water (4.5 mL). The reaction mixture was stirred at 0°C for 10 minutes, followed by the addition of sodium periodate (975.0 mg, 4.56 mmol). The resulting mixture was warmed up to room temperature, stirred for 16 hours, and diluted with ethyl acetate (10.0 mL) and water (10.0 mL). The organic phase was separated, and the aqueous phase was extracted with ethyl acetate (10.0 mL × 2). The combined organic phase was dried over anhydrous sodium sulfate, filtered, and concentrated under reduced pressure to obtain the crude product 8-methoxy-7-nitroquinoline-4-carbaldehyde (81c) (355.0 mg, crude yield 100%).

At 0°C, diethylaminosulfur trifluoride (180 mg, 1.11 mmol) was slowly added dropwise to a solution of 8-methoxy-7-nitroquinoline-4-carbaldehyde (81c) (86.0 mg, 0.37 mmol) in dichloromethane (18.0 mL). The reaction mixture was stirred at 0°C for 10 minutes, and at room temperature for 3 hours. The pH of the aqueous phase was adjusted to about 8 to 9 with saturated aqueous sodium bicarbonate solution. The organic phase was separated, and the aqueous phase was extracted with dichloromethane (10.0 mL × 2). The combined organic phase was dried over anhydrous sodium sulfate, filtered, and concentrated under reduced pressure. The resulting residue was purified by silica gel column chromatography (eluent: petroleum ether/ethyl acetate = 4/1) to obtain 4-(difluoromethyl)-8-methoxy-7-nitroquinoline (81d) (65.0 mg, yield 69%).

At room temperature, 4-(difluoromethyl)-8-methoxy-7-nitroquinoline (81d) (65.0 mg, 0.256 mmol) and lithium chloride (107.0 mg, 2.56 mmol) were added to N,N-dimethylformamide (2.0 mL). The reaction mixture was stirred at 160°C for 40 minutes, cooled to room temperature, and concentrated under reduced pressure to remove the organic solvent. Water (2.0 mL) was added to the resulting residue, stirred for 3 minutes, and filtered under reduced pressure. A mixed solvent of ethanol (2.0 mL) and water (2.0 mL) was added to the resulting filter cake, stirred for 10 minutes, and filtered under reduced pressure. The filter cake was dried under vacuum to obtain 4-(difluoromethyl)-7-nitroquinolin-8-ol (81) (47.3 mg, yield 77%).

¹H NMR (400 MHz, DMSO-*d₆*) δ: 8.74 (d, *J* = 4.3 Hz, 1H), 8.03 (d, *J* = 9.6 Hz, 1H), 7.72 (d, *J* = 4.3 Hz, 1H), 7.55 (t, *J* = 54.0 Hz, 1H), 6.62 (d, *J* = 9.6 Hz, 1H).

MS calculated: 240.03; MS found: 241.1 [M+H]⁺.

### Example 82

### Synthesis of 4-chloro-7-nitro-3-phenylquinolin-8-ol (82)

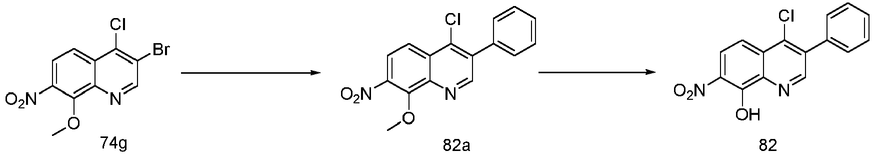

At room temperature, 3-bromo-4-chloro-8-methoxy-7-nitroquinoline (74g) (120.0 mg, 0.38 mmol), phenylboronic acid (48.0 mg, 0.40 mmol), palladium acetate (8.5 mg, 0.038 mmol), tricyclohexylphosphine (21.2 mg, 0.076 mmol) and potassium phosphate (281.0 mg, 1.32 mmol) were added successively to a mixed solvent of toluene (3.0 mL) and water (0.5 mL). The reaction mixture was stirred at 92°C for 3 hours, cooled to room temperature, and diluted with ethyl acetate (10.0 mL) and water (10.0 mL). The organic phase was separated, and the aqueous phase was extracted with ethyl acetate (10.0 mL × 2). The combined organic phase was dried over anhydrous sodium sulfate, filtered, and concentrated under reduced pressure. The resulting residue was purified by silica gel column chromatography (eluent: petroleum ether/dichloromethane/ethyl acetate = 8/1/1) to obtain 4-chloro-8-methoxy-7-nitro-3-phenylquinoline (82a) (61.0 mg, yield 51%).

At room temperature, 4-chloro-8-methoxy-7-nitro-3-phenylquinoline (82a) (61.0 mg, 0.194 mmol) and lithium chloride (81.0 mg, 1.93 mmol) were added to N,N-dimethylformamide (2.0 mL). The reaction mixture was stirred at 160°C for 50 minutes, cooled to room temperature, and concentrated under reduced pressure to remove the organic solvent. Water (2.0 mL) was added to the resulting residue, stirred for 3 minutes, and filtered under reduced pressure. A mixed solvent of ethanol (2.0 mL) and water (2.0 mL) was added to the resulting filter cake, stirred for 10 minutes, and filtered under reduced pressure. The filter cake was dried under vacuum to obtain 4-chloro-7-nitro-3-phenylquinolin-8-ol (82) (57.0 mg, yield 98%).

¹H NMR (400 MHz, DMSO-*d₆*) δ: 8.54 (s, 1H), 8.09 (d, *J* = 9.7 Hz, 1H), 7.61 - 7.50 (m, 5H), 6.73 (d, *J* = 9.7 Hz, 1H).

MS calculated: 300.03; MS found: 301.1/303.0 [M+H]⁺.

### Example 83

### Synthesis of 5-chloro-3-methyl-7-nitroquinolin-8-ol (83)

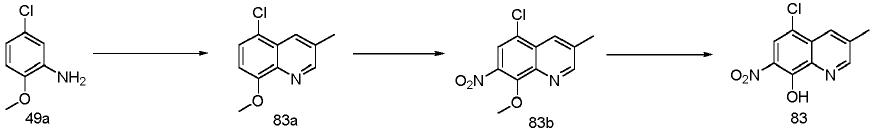

At room temperature, 5-chloro-2-methoxyaniline (49a) (3.0 g, 19.0 mmol) was added to hydrochloric acid solution (6.0 M) (30.0 mL). The reaction mixture was warmed up to 100°C, followed by the slowly dropwise addition of 2-methacrolein (3.34 g, 47.7 mmol). The reaction mixture was stirred at 100°C for 2 hours, and cooled to room temperature. The pH of the aqueous phase was adjusted to about 7 to 8 with aqueous sodium hydroxide solution (1.0 M). The resulting mixture was extracted with dichloromethane (50.0 mL × 3). The organic phases were combined, dried over anhydrous sodium sulfate and filtered, and the filtrate was concentrated under reduced pressure. The resulting residue was purified by silica gel column chromatography (gradient elution, eluent: petroleum ether/ethyl acetate = 7/1 to 3/1) to obtain 3-methyl-5-chloro-8-methoxyquinoline (83a) (1.35 g, yield 34%).

At 0°C, nitric acid (65 weight%) (1.35 mL, 19.6 mmol) was slowly added to a solution of 3-methyl-5-chloro-8-methoxyquinoline (83a) (1.35 g, 6.5 mmol) in acetic anhydride (27.0 mL). The reaction mixture was stirred at 0°C for 5 minutes, followed by the slowly dropwise addition of sulfuric acid (98 weight%) (0.35 mL, 6.5 mmol), and continued to stir for 30 minutes. The pH of the aqueous phase was adjusted to about 9 to 10 with aqueous sodium hydroxide solution (1.0 M). The resulting mixture was extracted with dichloromethane (10.0 mL × 3). The organic phases were combined, washed with saturated brine (10.0 mL), dried over anhydrous sodium sulfate, filtered, and concentrated under reduced pressure. The resulting residue was purified by silica gel column chromatography (eluent: petroleum ether/ethyl acetate = 10/1) to obtain 3-methyl-5-chloro-7-nitro-8-methoxyquinoline (83b) (0.64 g, yield 39%).

At room temperature, 3-methyl-5-chloro-7-nitro-8-methoxyquinoline (83b) (30.0 mg, 0.12 mmol) and lithium chloride (50.0 mg, 1.2 mmol) were added to N,N-dimethylformamide (1.0 mL). The reaction mixture was stirred at 140°C for 2 hours, cooled to room temperature, and concentrated under reduced pressure to remove the organic solvent. Water (1.0 mL) was added to the resulting residue, stirred for 3 minutes, and filtered under reduced pressure. The filter cake was washed with a mixed solvent of water and ethanol (volume ratio, water/ethanol = 1/1) (0.2 mL × 2), and dried under vacuum to obtain 3-methyl-5-chloro-7-nitro-8-hydroxyquinoline (83) (19.0 mg, yield 67%).

¹H NMR (400 MHz, DMSO-*d₆*) δ: 8.61 (s, 1H), 8.03 (d, *J* = 12.3 Hz, 2H), 2.50 (s, 3H).

MS calculated: 238.01; MS found: 239.0 [M+H]⁺.

### Example 84

### Synthesis of 5-cyclopropyl-3-methyl-7-nitroquinolin-8-ol (84)

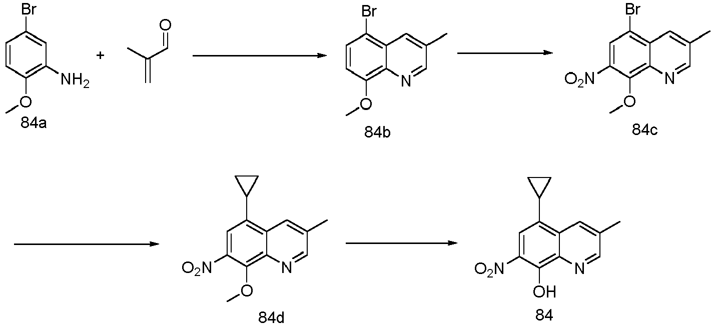

At room temperature, 5-bromo-2-methoxyaniline (84a) (1.5 g, 7.43 mmol) was added to hydrochloric acid solution (6.0 M) (40.0 mL). The reaction mixture was warmed up to 110°C, followed by the slowly dropwise addition of 2-methacrolein (1.8 mL, 22.2 mmol), and stirred for 3 hours. The reaction mixture was cooled to room temperature, and the pH of the aqueous phase was adjusted to about 7 to 8 with aqueous sodium hydroxide solution (6.0 M). The resulting mixture was extracted with ethyl acetate (50.0 mL × 3). The organic phases were combined, dried over anhydrous sodium sulfate, filtered, and concentrated under reduced pressure. The resulting residue was purified by silica gel column chromatography (eluent: petroleum ether/ethyl acetate = 7/3) to obtain 5-bromo-8-methoxy-3-methylquinoline (84b) (1.0 g, yield 53%).

At room temperature, nitric acid (65 weight%) (0.51 mL, 12.0 mmol) was slowly added dropwise to a solution of 5-bromo-8-methoxy-3-methylquinoline (84b) (251.0 mg, 1.0 mmol) in acetic anhydride (5.5 mL). The reaction mixture was stirred at room temperature for 10 minutes, followed by the addition of sulfuric acid (98 weight%) (0.12 mL, 2.4 mmol), and continued to stir for 2 hours. The reaction mixture was diluted with water (30.0 mL), and the pH of the aqueous phase was adjusted to about 8 to 9 with aqueous sodium hydroxide solution (1.0 M). The resulting mixture was extracted with dichloromethane (10.0 mL × 3). The organic phases were combined, dried over anhydrous sodium sulfate, filtered, and concentrated under reduced pressure. The resulting residue was purified by silica gel column chromatography (eluent: petroleum ether/ethyl acetate = 85%/15%) to obtain 5-bromo-8-methoxy-3-methyl-7-nitroquinoline (84c) (50.0 mg, yield 17%).

At room temperature, 5-bromo-8-methoxy-3-methyl-7-nitroquinoline (84c) (50.0 mg, 0.17 mmol), cyclopropylboronic acid (29.0 mg, 0.34 mmol), tetrakis(triphenylphosphine) palladium (39.0 mg, 0.034 mmol) and potassium carbonate (70.4 mg, 0.51 mmol) were added successively to a mixed solvent of toluene (3.0 mL) and water (0.3 mL). The reaction mixture was stirred at 105 °C for 3 hours, cooled to room temperature, and extracted with ethyl acetate (10.0 mL × 3). The combined organic phase was dried over anhydrous sodium sulfate, filtered, and concentrated under reduced pressure. The resulting residue was purified by silica gel column chromatography (eluent: petroleum ether/ethyl acetate = 4/1) to obtain 5-cyclopropyl-8-methoxy-3-methyl-7-nitroquinoline (84d) (28.0 mg, yield 64%).

At room temperature, 5-cyclopropyl-8-methoxy-3-methyl-7-nitroquinoline (84d) (28.0 mg, 0.11 mmol) and lithium chloride (45.6 mg, 1.1 mmol) were added to N,N-dimethylformamide (1.0 mL). The reaction mixture was stirred at 150°C for 1 hour, cooled to room temperature, and concentrated under reduced pressure to remove the organic solvent. Water (2.0 mL) was added to the resulting residue, stirred for 3 minutes, and filtered under reduced pressure. The filter cake was washed successively with water (0.5 mL × 2) and ethanol (0.5 mL × 2), and dried under vacuum to obtain 5-cyclopropyl-3-methyl-7-nitroquinolin-8-ol (84) (16.0 mg, yield 60%).

¹H NMR (400 MHz, DMSO-*d*₆) δ: 8.55 - 8.48 (m, 1H), 8.36 - 8.30 (m, 1H), 7.71 - 7.64 (m, 1H), 2.52 - 2.50 (m, 3H), 2.05 - 1.97 (m, 1H), 0.98 - 0.90 (m, 2H), 0.58 - 0.52 (m, 2H).

MS calculated: 244.08; MS found: 245.1 [M+H]⁺.

### Example 85

### Synthesis of 7-nitro-4-(piperidin-1-yl-methyl)quinolin-8-ol (85)

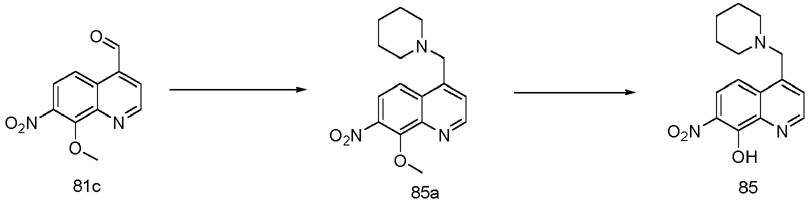

At room temperature, 8-methoxy-7-nitroquinoline-4-carbaldehyde (81c) (110.0 mg, 0.47 mmol) and piperidine (52.0 mg, 0.61 mmol) were added successively to a mixed solvent of tetrahydrofuran (3.0 mL) and dichloromethane (3.0 mL). The reaction mixture was stirred at room temperature for 90 minutes, followed by the addition of sodium triacetoxyborohydride (301.0 mg, 1.42 mmol), and continued to stir for 3 hours. The pH of the aqueous phase was adjusted to about 7 to 8 with saturated aqueous sodium bicarbonate solution. The resulting mixture was extracted with dichloromethane (10.0 mL × 3). The organic phases were combined, washed with saturated brine (10.0 mL), dried over anhydrous sodium sulfate, filtered, and concentrated under reduced pressure. The resulting residue was purified by silica gel thin layer chromatography (developing solvent: dichloromethane/methanol=16/1) to obtain 8-methoxy-7-nitro-4-(piperidin-1-ylmethyl)quinoline (85a) (130.0 mg, yield 72%).

At room temperature, 8-methoxy-7-nitro-4-(piperidin-1-yl-methyl)quinoline (85a) (130.0 mg, 0.43 mmol) and lithium chloride (180 mg, 4.3 mmol) were added to N,N-dimethylformamide (2.0 mL). The reaction mixture was stirred at 160°C for 45 minutes, cooled to room temperature, and concentrated under reduced pressure to remove the organic solvent. Water (2.0 mL) was added to the resulting residue, stirred for 3 minutes, and filtered under reduced pressure. A mixed solvent of ethanol (2.0 mL) and water (2.0 mL) was added to the resulting filter cake, stirred for 10 minutes, and filtered under reduced pressure. The filter cake was dried under vacuum to obtain 7-nitro-4-(piperidin-1-yl-methyl)quinolin-8-ol (85) (112.0 mg, yield 91%).

¹H NMR (400 MHz, DMSO-*d₆*) δ 8.56 (d, *J* = 4.3 Hz, 1H), 7.92 (d, *J* = 9.7 Hz, 1H), 7.54 (d, *J* = 4.3 Hz, 1H), 6.76 (d, *J* = 9.7 Hz, 1H), 3.76 (s, 2H), 2.43 (s, 4H), 1.53 (d, *J* = 4.8 Hz, 4H), 1.43 (d, *J* = 3.6 Hz, 2H).

MS calculated: 287.13; MS found: 288.1[M+H]⁺.

### Example 86

### Synthesis of 5-fluoro-3-methyl-7-nitroquinolin-8-ol (86)

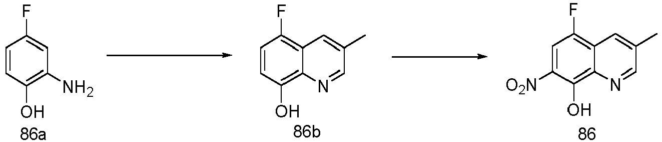

At room temperature, 2-amino-4-fluorophenol (86a) (2.00 g, 15.7 mmol) was added to hydrochloric acid solution (6.0 M) (20.0 mL). The reaction mixture was warmed up to 100°C, followed by the slowly dropwise addition of 2-methacrolein (3.3 mL, 39.4 mmol), and stirred for 2 hours. The reaction mixture was cooled to room temperature, and the pH of the aqueous phase was adjusted to about 7 to 8 with aqueous sodium hydroxide solution (6.0 M). The resulting mixture was extracted with dichloromethane (50.0 mL × 3). The organic phases were combined, dried over anhydrous sodium sulfate, filtered, and concentrated under reduced pressure. The resulting residue was purified by silica gel column chromatography (gradient elution, eluent: petroleum ether/ethyl acetate=10/1 to 5/1) to obtain 5-fluoro-3-methylquinolin-8-ol (86b) (400 mg, yield 14.3%).

At room temperature, sodium nitrite (350.0 mg, 4.1 mmol) was added to a solution of 5-fluoro-3-methylquinolin-8-ol (86b) (90.0 mg, 0.51 mmol) in acetic acid (2.0 mL). The reaction mixture was stirred at room temperature for 1 hour, and the pH of the aqueous phase was adjusted to about 8 to 9 with saturated aqueous sodium bicarbonate solution. The resulting mixture was filtered under reduced pressure, the filter cake was washed with a mixed solvent of ethanol and water (volume ratio, ethanol/water = 1/1) (1.0 mL × 2), and dried under vacuum to obtain 5-fluoro-3-methyl-7-nitroquinolin-8-ol (86) (65.0 mg, yield 58%).

¹H NMR (400 MHz, DMSO-*d₆*) δ 8.77 (s, 1H), 8.03 (s, 1H), 7.69 (d, *J* = 12.5 Hz, 1H), 2.49 (s, 3H).

MS calculated: 222.04; MS found: 223.0 [M+H]⁺.

### Example 87

### Synthesis of 5-(morpholinomethyl)-7-nitroquinolin-8-ol acetate (87)

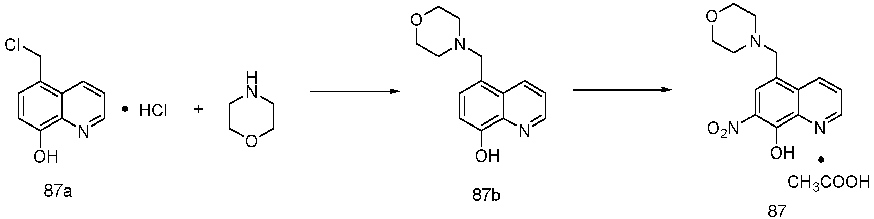

At 0°C, 5-(chloromethyl)quinolin-8-ol hydrochloride (87a) (120.0 mg, 0.62 mmol) and morpholine (270.0 mg, 2.5 mmol) were added successively to dichloromethane (3.0 mL). The reaction mixture was stirred at room temperature for 2 hours, and concentrated under reduced pressure. The resulting residue was purified by silica gel column chromatography (eluent: dichloromethane/methanol = 49/1, and adding 0.5% ammonia (25 to 28weight%, NH₃ content) by the total volume of the eluent) to obtain 5-(morpholinomethyl)quinolin-8-ol (87b) (80.0 mg, yield 53%).

At room temperature, sodium nitrite (34.0 mg, 0.49 mmol) was added to a solution of 5-(morpholinomethyl)quinolin-8-ol (87a) (80.0 mg, 0.33 mmol) in acetic acid (1.5 mL). The reaction mixture was stirred at room temperature for 2 hours, and concentrated under reduced pressure to remove the organic solvent. Water (1.0 mL) was added to the resulting residue, stirred for 10 minutes, and filtered under reduced pressure. The filter cake was washed with water (0.5 mL × 2), and dried under vacuum to obtain 5-(morpholinomethyl)-7-nitroquinolin-8-ol acetate (87) (76.0 mg, yield 45%).

¹H NMR (400 MHz, DMSO-*d*₆) δ: 9.08 - 9.035 (m, 1H), 9.02 - 8.97 (m, 1H), 8.42 - 8.36 (s, 1H), 8.01 - 7.95 (m, 1H), 4.80 - 4.70 (m, 2H), 3.96 - 3.64 (m, 4H), 3.35 - 3.22 (m, 4H).

MS calculated: 289.11; MS found: 290.1 [M+H]⁺.

### Example 88

### Synthesis of ethyl 8-hydroxy-7-nitroquinoline-3-carboxylate (88)

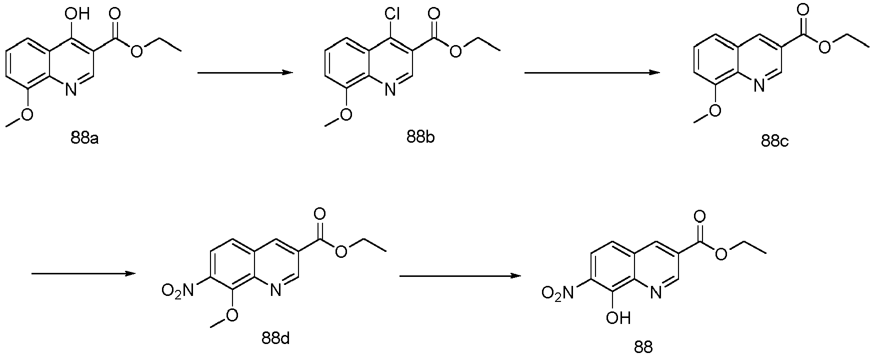

At room temperature, phosphorus oxychloride (2.3 mL, 24.3 mmol) was added to a solution of ethyl 4-hydroxy-8-methoxyquinoline-3-carboxylate (88a) (2.0 g, 8.1 mmol) in acetonitrile (20.0 mL). The reaction mixture was stirred at 90°C for 2.5 hours, cooled to room temperature, and concentrated under reduced pressure. The resulting residue was diluted with dichloromethane (30.0 mL), followed by the addition of triethylamine (4.91 g, 48.5 mmol), stirred for 30 minutes and filtered under reduced pressure. The filter cake was washed with dichloromethane (5.0 mL × 3), and the filtrate was concentrated under reduced pressure. The resulting residue was purified by silica gel column chromatography (eluent: petroleum ether/ethyl acetate = 3/1) to obtain ethyl 4-chloro-8-methoxyquinoline-3-carboxylate (88b) (2.1 g, yield 98%).

At room temperature, ethyl 4-chloro-8-methoxyquinoline-3-carboxylate (88b) (2.1 g, 7.9 mmol), triethylamine (1.2 g, 11.9 mmol), palladium/carbon (palladium loading: 10 weight%) (0.42 g, 0.39 mmol) were added successively to a mixed solvent of tetrahydrofuran (10.0 mL) and methanol (10.0 mL). The reaction mixture was stirred under a hydrogen atmosphere for 2 hours, and filtered through diatomite. The filter cake was washed with tetrahydrofuran (10.0 mL × 3), and the filtrate was concentrated under reduced pressure. The resulting residue was diluted with dichloromethane (20.0 mL), washed with saturated brine (10.0 mL), dried over anhydrous sodium sulfate, filtered, and concentrated under reduced pressure to obtain the crude product ethyl 8-methoxyquinoline-3-carboxylate (88c) (1.8 g, yield 99%).

At 0°C, nitric acid (65 weight%) (0.45 mL, 6.5 mmol) was slowly added to a solution of ethyl 8-methoxyquinoline-3-carboxylate (88c) (500.0 mg, 2.2 mmol) in acetic anhydride (10.0 mL), and stirred for 10 minutes. Concentrated sulfuric acid (98 weight%) (0.12 mL, 2.2 mmol) was slowly added dropwise. The reaction mixture was stirred at 0°C for 2 hours, and diluted with water (20.0 mL). The pH of the aqueous phase was adjusted to about 8 to 9 with saturated aqueous potassium carbonate solution. The resulting mixture was extracted with dichloromethane (30.0 mL × 3). The organic phases were combined, dried over anhydrous sodium sulfate and filtered, and the filtrate was concentrated under reduced pressure. The resulting residue was purified by silica gel column chromatography (gradient elution, eluent: petroleum ether/ethyl acetate = 10/1 to 2/1) to obtain ethyl 8-methoxy-7-nitroquinoline-3-carboxylate (88d) (88.0 mg, yield 15%).

At room temperature, ethyl 8-methoxy-7-nitroquinoline-3-carboxylate (88d) (88.0 mg, 0.32 mmol) and lithium chloride (135.0 mg, 3.2 mmol) were added to N,N-dimethylformamide (2.0 mL). The reaction mixture was stirred at 130°C for 45 minutes, cooled to room temperature, and concentrated under reduced pressure to remove the organic solvent. Water (2.0 mL) was added to the resulting residue, stirred for 3 minutes, and filtered under reduced pressure. The filter cake was washed with water (0.5 mL × 2), and dried under vacuum to obtain ethyl 8-hydroxy-7-nitroquinoline-3-carboxylate (88) (71.0 mg, yield 85%).

¹H NMR (400 MHz, DMSO-*d₆*) δ 9.01 (s, 1H), 8.63 (d, *J* = 1.5 Hz, 1H), 7.96 (d, *J* = 9.3 Hz, 1H), 6.60 (d, *J* = 9.1 Hz, 1H), 4.39 (q, *J* = 7.1 Hz, 2H), 1.37 (t, *J* = 7.1 Hz, 3H).

MS calculated: 262.06; MS found: 263.1 [M+H]⁺.

### Example 89

### Synthesis of 4-(morpholinomethyl)-7-nitroquinolin-8-ol (89)

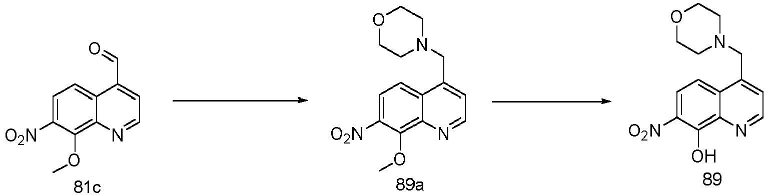

At room temperature, 8-methoxy-7-nitroquinoline-4-carbaldehyde (81c) (77.0 mg, 0.33 mmol) and morpholine (58.0 mg, 0.61 mmol) were added successively to a mixed solvent of tetrahydrofuran (2.0 mL) and dichloromethane (2.0 mL). The reaction mixture was stirred at room temperature for 2 hours, followed by the addition of sodium triacetoxyborohydride (210.0 mg, 0.99 mmol), and continued to stir for 4.5 hours. The pH of the aqueous phase was adjusted to about 7 to 8 with saturated aqueous sodium bicarbonate solution. The resulting mixture was extracted with dichloromethane (10.0 mL × 3). The organic phases were combined, washed with saturated brine (10.0 mL), dried over anhydrous sodium sulfate, filtered, and concentrated under reduced pressure. The resulting residue was purified by silica gel column chromatography (gradient elution, eluent: dichloromethane/methanol=80/1 to 70/1) to obtain 4-((8-methoxy-7-nitroquinolin-4-yl)methyl)morpholine (89a) (61.0 mg, yield 61%).

At room temperature, 4-((8-methoxy-7-nitroquinolin-4-yl)methyl)morpholine (89a) (61.0 mg, 0.2 mmol) and lithium chloride (85.0 mg, 2.0 mmol) were added to N,N-dimethylformamide (2.0 mL). The reaction mixture was stirred at 160°C for 40 minutes, cooled to room temperature, and concentrated under reduced pressure to remove the organic solvent. Water (2.0 mL) was added to the resulting residue, stirred for 3 minutes, and filtered under reduced pressure. A mixed solvent of ethanol (2.0 mL) and water (2.0 mL) was added to the resulting filter cake, stirred for 10 minutes, and filtered under reduced pressure. The filter cake was dried under vacuum to obtain 4-(morpholinomethyl)-7-nitroquinolin-8-ol (89) (38.9 mg, yield 67%).

¹H NMR (400 MHz, DMSO-*d₆*) δ 8.57 (d, *J* = 4.4 Hz, 1H), 7.94 (d, *J* = 9.7 Hz, 1H), 7.56 (d, *J =* 4.5 Hz, 1H), 6.78 (d, *J* = 9.7 Hz, 1H), 3.82 (s, 2H), 3.66 - 3.58 (m, 4H), 2.47 (br s, 4H).

MS calculated: 289.11; MS found: 290.1[M+H]⁺.

### Example 90

### Synthesis of 5-chloro-3-((diethylamino)methyl)-7-nitroquinolin-8-ol ditrifluoroacetate (90)

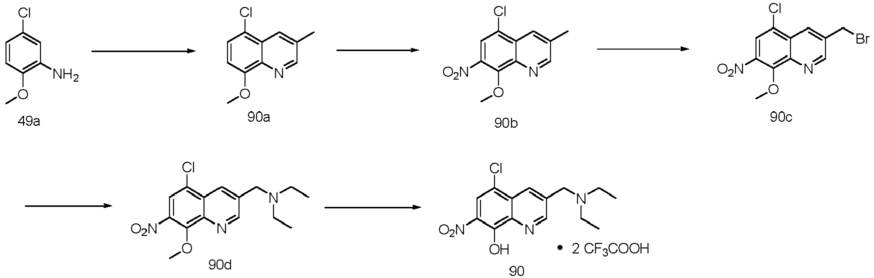

At room temperature, 5-chloro-2-methoxyaniline (49a) (6.0 g, 38.0 mmol) was added to hydrochloric acid solution (6.0 M) (60.0 mL). The reaction mixture was warmed up to 100°C, followed by the slowly dropwise addition of 2-methacrolein (7.8 mL, 95.4 mmol), and stirred for 2 hours. The reaction mixture was cooled to room temperature, and the pH of the aqueous phase was adjusted to about 7 to 8 with aqueous sodium hydroxide solution (6.0 M). The resulting mixture was extracted with dichloromethane (100.0 mL × 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 7/1 to 3/1) to obtain 5-chloro-8-methoxy-3-methylquinoline (90a) (2.7 g, yield 34%).

At room temperature, nitric acid (65 weight%) (1.50 mL, 21.9 mmol) was slowly added dropwise to a solution of 5-chloro-8-methoxy-3-methylquinoline (90a) (1.5 g, 7.2 mmol) in acetic anhydride (30.0 mL). The reaction mixture was stirred at room temperature for 10 minutes, followed by the slowly dropwise addition of sulfuric acid (98 weight%) (0.39 mL, 7.2 mmol), and continued to stir for 2 hours. The reaction mixture was diluted with water (50.0 mL), and the pH of the aqueous phase was adjusted to about 8 to 9 with saturated aqueous potassium carbonate solution. The resulting mixture was extracted with dichloromethane (50.0 mL × 3). The organic phases were combined, dried over anhydrous sodium sulfate, filtered, and concentrated under reduced pressure. The resulting residue was purified by silica gel column chromatography (eluent: petroleum ether/ethyl acetate = 10/1) to obtain 5-chloro-8-methoxy-3-methyl-7-nitroquinoline (90b) (660.0 mg, yield 36%).

At room temperature, 5-chloro-8-methoxy-3-methyl-7-nitroquinoline (90b) (660.0 mg, 2.5 mmol), N-bromosuccinimide (473.0 mg, 2.6 mmol) and 2,2'-azobisisobutyronitrile (250.0 mg, 1.5 mmol) were added successively to carbon tetrachloride (20.0 mL). The reaction mixture was warmed up to 80°C, stirred for 12 hours, cooled to room temperature, and filtered under reduced pressure. The filter cake was washed with carbon tetrachloride (10.0 mL × 3), and the filtrate was concentrated under reduced pressure to obtain the crude product 3-(bromomethyl)-5-chloro-8-methoxy-7-nitroquinoline (90c) (838.0 mg, crude yield > 100%).

At room temperature, the crude 3-(bromomethyl)-5-chloro-8-methoxy-7-nitroquinoline (90c) (102.0 mg, 0.31 mmol) and diethylamine (225.0 mg, 3.1 mmol) were added successively to tetrahydrofuran (4.0 mL). The reaction mixture was stirred at room temperature for 2 hours, and concentrated under reduced pressure. The resulting residue was purified by silica gel thin layer chromatography (developing solvent: petroleum ether/ethyl acetate=3/1) to obtain 5-chloro-3-(diethylamino)methyl-8-methoxy-7-nitroquinoline (90d) (65.0 mg, yield 65%).

At room temperature, 5-chloro-3-(diethylamino)methyl-8-methoxy-7-nitroquinoline (90d) (65.0 mg, 0.2 mmol) and lithium chloride (85.0 mg, 2.0 mmol) were added to N,N-dimethylformamide (1.5 mL). The reaction mixture was stirred at 130°C for 1.5 hours, cooled to room temperature, and concentrated under reduced pressure to remove the organic solvent. The resulting residue was completely dissolved in a mixed solvent of trifluoroacetic acid (0.5 mL) and water (2.0 mL), and purified by reversed-phase high performance liquid chromatography (the chromatographic column was Eclipse XDB-C18 (21.2 mm×250 mm, 7µm), the mobile phase was water/acetonitrile=100%/0 - 75%/25%, gradient elution, and the flow rate was 20.0 mL/min) to obtain 5-chloro-3-((diethylamino)methyl)-7-nitroquinolin-8-ol ditrifluoroacetate (90) (17.0 mg, yield 16%).

¹H NMR (400 MHz, DMSO-*d₆*) δ: 8.87 (s, 1H), 8.49 (s, 1H), 8.11 (s, 1H), 4.51 (s, 2H), 3.13 (dd, *J* = 13.9, 6.8 Hz, 4H), 1.25 (t, *J* = 7.1 Hz, 6H).

MS calculated: 309.09; MS found: 310.1[M+H]⁺.

### Example 91

### Synthesis of 7-nitro-5-(piperidin-1-ylmethyl)quinolin-8-ol acetate (91)

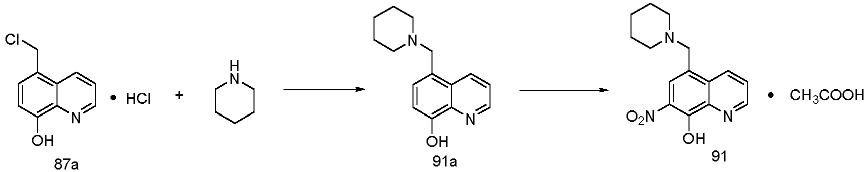

At 0°C, 5-(chloromethyl)quinolin-8-ol hydrochloride (87a) (120.0 mg, 0.62 mmol) and piperidine (264.0 mg, 3.1 mmol) were added successively to dichloromethane (3.0 mL). The reaction mixture was stirred at room temperature for 2 hours, and concentrated under reduced pressure. The resulting residue was purified by silica gel column chromatography (eluent: dichloromethane/methanol = 49/1, and adding 0.5% ammonia (25 to 28weight%, NH₃ content) by the total volume of the eluent) to obtain 5-(piperidin-1-ylmethyl)quinolin-8-ol (91a) (100.0 mg, yield 67%).

At room temperature, sodium nitrite (68.0 mg, 0.99 mmol) was added to a solution of 5-(piperidin-1-ylmethyl)quinolin-8-ol (91a) (100.0 mg, 0.41 mmol) in acetic acid (2.0 mL). The reaction mixture was stirred at room temperature for 2 hours, and filtered under reduced pressure. The filter cake was washed with water (0.5 mL × 2), and dried under vacuum to obtain 7-nitro-5-(piperidin-1-ylmethyl)quinolin-8-ol acetate (91) (129.0 mg, yield 88%).

¹H NMR (400 MHz, DMSO-*d₆*) δ: 9.01-8.97 (m, 1H), 8.92-8.87 (m, 1H), 8.38-8.35 (s, 1H), 7.95-7.89 (m, 1H), 4.71 - 4.65 (m, 2H), 3.35 - 3.10 (m, 4H), 1.93 (s, 3.57 H, the methyl signal of acetic acid)), 1.80 - 1.65 (m, 4H), 1.60 - 1.45 (m, 2H).

MS calculated: 287.11; MS found: 288.1 [M+H]⁺.

### Example 92

### Synthesis of 5-chloro-7-nitro-3-(piperidin-1-ylmethyl)quinolin-8-ol (92)

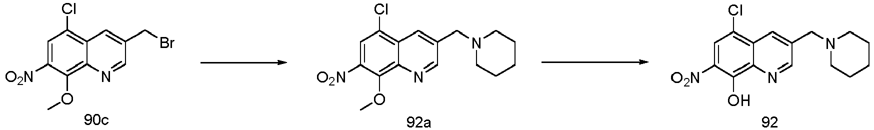

At room temperature, the crude 3-(bromomethyl)-5-chloro-8-methoxy-7-nitroquinoline (90c) (88.0 mg, 0.27 mmol) and piperidine (113.0 mg, 1.33 mmol) were added successively to tetrahydrofuran (2.0 mL). The reaction mixture was stirred at room temperature for 2 hours, and concentrated under reduced pressure. The resulting residue was purified by silica gel thin layer chromatography (developing solvent: dichloromethane/methanol=25/1) to obtain 5-chloro-8-methoxy-7-nitro-3-(piperidin-1-ylmethyl)quinoline (92a) (35.0 mg, yield 39%).

At room temperature, 5-chloro-8-methoxy-7-nitro-3-(piperidin-1-ylmethyl)quinoline (92a) (35.0 mg, 0.10 mmol) and lithium chloride (44.0 mg, 1.0 mmol) were added to N,N-dimethylformamide (1.0 mL). The reaction mixture was stirred at 130°C for 45 minutes, cooled to room temperature, and concentrated under reduced pressure to remove the organic solvent. Water (1.0 mL) was added to the resulting residue, stirred for 3 minutes, and filtered under reduced pressure. The filter cake was washed with water (0.5 mL × 3), and dried under vacuum to obtain 5-chloro-7-nitro-3-(piperidin-1-ylmethyl)quinolin-8-ol (92) (30.7 mg, yield 92%).

¹H NMR (400 MHz, DMSO-*d₆*) δ: 8.62 (s, 1H), 8.09 (s, 1H), 8.02 (s, 1H), 3.65 (s, 2H), 2.37 (s, 4H), 1.54 - 1.47 (m, 4H), 1.39 (d, *J* = 4.4 Hz, 2H).

MS calculated: 321.09; MS found: 322.1 [M+H]⁺.

### Example 93

### Synthesis of 7-nitro-5-(pyrrolidin-1-ylmethyl)quinolin-8-ol acetate (93)

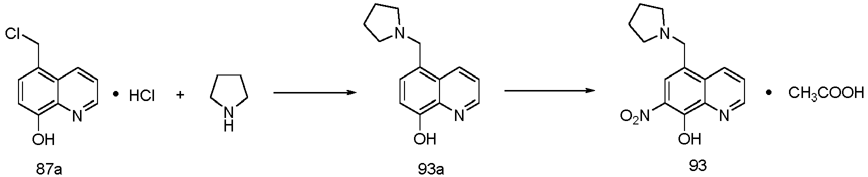

At 0°C, 5-(chloromethyl)quinolin-8-ol hydrochloride (87a) (100.0 mg, 0.43 mmol) and tetrahydropyrrole (147.0 mg, 2.1 mmol) were added successively to dichloromethane (2.0 mL). The reaction mixture was stirred at room temperature for 2 hours, and concentrated under reduced pressure. The resulting residue was purified by silica gel column chromatography (eluent: dichloromethane/methanol = 49/1, and adding 0.5% ammonia (25 to 28weight%, NH₃ content) by the total volume of the eluent) to obtain 5-(pyrrolidin-1-ylmethyl)quinolin-8-ol (93a) (55.0 mg, yield 47%).

At room temperature, sodium nitrite (50.0 mg, 0.72 mmol) was added to a solution of 5-(pyrrolidin-1-ylmethyl)quinolin-8-ol (93a) (55.0 mg, 0.24 mmol) in acetic acid (2.0 mL). The reaction mixture was stirred at room temperature for 2 hours, and filtered under reduced pressure. The filter cake was washed with water (0.5 mL × 2), and dried under vacuum to obtain 7-nitro-5-(pyrrolidin-1-ylmethyl)quinolin-8-ol acetate (93) (22.7 mg, yield 31%).

¹H NMR (400 MHz, DMSO-*d*₆) δ: 9.08-9.04 (m, 1H), 8.96-8.92 (m, 1H), 8.42(s, 1 H), 8.01-7.95 (m, 1H), 4.82 (s, 2H), 3.75-3.40 (m, 5H), 2.04-1.93 (m, 3H), 1.94 (s, 1.34 H, overlapped with CH₃COOH in 1: 0.45 mol. ratio).

MS calculated: 273.11; MS found: 274.1 [M+H]⁺.

### Example 94

### Synthesis of 5-((diethylamino)methyl)-7-nitroquinolin-8-ol (94)

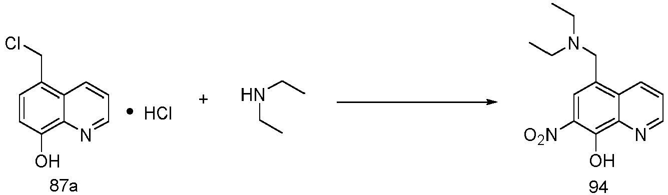

At 0°C, 5-(chloromethyl)quinolin-8-ol hydrochloride (87a) (110.0 mg, 0.48 mmol) and diethylamine (140.0 mg, 1.91 mmol) were added successively to dichloromethane (2.5 mL). The reaction mixture was stirred at room temperature for 2 hours, and concentrated under reduced pressure. Acetic acid (2.0 mL) was added to the resulting residue, followed by the addition of sodium nitrite (99.0 mg, 1.43 mmol). The resulting mixture was stirred at room temperature for 2 hours, and concentrated under reduced pressure. The resulting residue was purified by reversed-phase high performance liquid chromatography (the chromatographic column was Eclipse XDB-C18 (21.2 mm×250 mm, 7µm), the mobile phase was water/methanol=100%/0 - 70%/30%, gradient elution, and the flow rate was 20.0 mL/min) to obtain 5-((diethylamino)methyl)-7-nitroquinolin-8-ol (94) (27.0 mg, yield 31%).

¹H NMR (400 MHz, DMSO-*d*₆) δ: 8.82-8.75 (m, 1H), 8.56-8.49 (m, 1H), 8.07 (s, 1H), 7.67-7.62 (m, 1 H), 4.21 (s, 2 H), 2.95-2.88 (m, 4 H), 1.20-1.15 (m, 4 H).

MS calculated: 275.13; MS found: 276.1 [M+H]⁺.

### Example 95

### Synthesis of 7-nitro-3-(piperidin-1-ylmethyl)quinolin-8-ol (95)

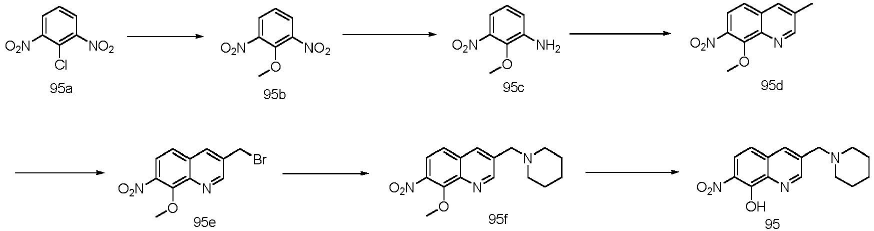

At room temperature, sodium methoxide (5.35 g, 99.0 mmol) was slowly added to a solution of 2,6-dinitrochlorobenzene (corrected in the reaction formula) (95a) (4.7 g, 23.2 mmol) in methanol (50.0 mL). The reaction mixture was stirred at room temperature for 16 hours, and concentrated under reduced pressure to remove the organic solvent. The resulting residue was dissolved in dichloromethane (20.0 mL) and water (20.0 mL). The organic phase was separated, and the aqueous phase was extracted with dichloromethane (20.0 mL × 2). The combined organic phase was washed with saturated brine (20.0 mL), dried over anhydrous sodium sulfate, filtered, and concentrated to dryness under reduced pressure to obtain the crude product 2-methoxy-1,3-dinitrobenzene (95b) (4.56 g, yield 99%).

At room temperature, reduced iron powder (3.86 g, 69.1 mmol) was added to a solution of 2-methoxy-1,3-dinitrobenzene (95b) (4.56 g, 23.0 mmol) in acetic acid (60.0 mL). The reaction mixture was stirred at 65°C for 1.5 hours. The reaction solution was concentrated under reduced pressure to remove the organic solvent. Dichloromethane (20.0 mL) was added to the resulting residue, and the pH of the aqueous phase was adjusted to about 8 to 9 with saturated aqueous sodium bicarbonate solution. The resulting mixture was filtered under reduced pressure. The organic phase was separated from the filtrate, washed with saturated brine (10.0 mL), dried over anhydrous sodium sulfate, filtered, and concentrated under reduced pressure. The resulting residue was purified by silica gel column chromatography (eluent: petroleum ether/dichloromethane/ethyl acetate = 8/1/1) to obtain 2-methoxy-3-nitroaniline (95c) (3.23 g, yield 84%).

At room temperature, 2-methoxy-3-nitroaniline (95c) (3.23 g, 19.2 mmol) was added to hydrochloric acid solution (6.0 M) (60.0 mL). The reaction mixture was warmed up to 100°C, followed by the slowly dropwise addition of 2-methacrolein (3.96 mL, 48.0 mmol), and stirred for 2 hours. The reaction mixture was cooled to room temperature, and the pH of the aqueous phase was adjusted to about 7 to 8 with aqueous sodium hydroxide solution (6.0 M). The resulting mixture was extracted with dichloromethane (50.0 mL × 3). The organic phases were combined, dried over anhydrous sodium sulfate, filtered, and concentrated under reduced pressure. The resulting residue was purified by silica gel column chromatography (eluent: petroleum ether/ethyl acetate = 85%/15%) to obtain 8-methoxy-3-methyl-7-nitroquinoline (95d) (1.67 g, yield 40%).

At room temperature, 8-methoxy-3-methyl-7-nitroquinoline (95d) (106.0 mg, 0.48 mmol), N-bromosuccinimide (95.0 mg, 0.53 mmol), and 2,2'-azobisisobutyronitrile (16.0 mg, 0.1 mmol) were added successively to carbon tetrachloride (6.0 mL). The reaction mixture was warmed up to 90°C, stirred for 16 hours, cooled to room temperature, and filtered under reduced pressure. The filter cake was washed with carbon tetrachloride (5.0 mL × 3), and the filtrate was concentrated under reduced pressure to obtain the crude product 3-(bromomethyl)-8-methoxy-7-nitroquinoline (95e) (143.0 mg, crude yield >100%).

At room temperature, a solution of the crude 3-(bromomethyl)-8-methoxy-7-nitroquinoline (95e) (143.0 mg, 0.48 mmol) in dichloromethane (3.0 mL) was slowly added dropwise to a solution of piperidine (123.0 mg, 1.44 mmol) in dichloromethane (2.0 mL). The reaction mixture was stirred at room temperature for 2 hours, and diluted with dichloromethane (20.0 mL) and water (20.0 mL). The organic phase was separated, and the aqueous phase was extracted with dichloromethane (10.0 mL × 2). The combined organic phase was washed with saturated brine (10.0 mL), dried over anhydrous sodium sulfate, filtered, and concentrated under reduced pressure. The resulting residue was purified by silica gel column chromatography (gradient elution, eluent: dichloromethane/methanol=80/1 to 60/1) to obtain 8-methoxy-7-nitro-3-(piperidin-1-ylmethyl)quinoline (95f) (37.0 mg, yield 25%).

At room temperature, 8-methoxy-7-nitro-3-(piperidin-1-ylmethyl)quinoline (95f) (37.0 mg, 0.12 mmol) and lithium chloride (51.6 mg, 1.22 mmol) were added to N,N-dimethylformamide (2.0 mL). The reaction mixture was stirred at 150°C for 50 minutes, cooled to room temperature, and concentrated under reduced pressure to remove the organic solvent. Water (2.0 mL) was added to the resulting residue, stirred for 3 minutes, and filtered under reduced pressure. Water (1.0 mL) was added to the resulting filter cake, stirred for 10 minutes, and filtered under reduced pressure. The filter cake was dried under vacuum to obtain 7-nitro-3-(piperidin-1-ylmethyl)quinolin-8-ol (95) (21.3 mg, yield 60%).

¹H NMR (400 MHz, DMSO-*d₆*) δ: 8.53 (s, 1H), 7.97 (s, 1H), 7.90 (d, *J* = 9.4 Hz, 1H), 6.50 (d, *J* = 9.4 Hz, 1H), 3.60 (s, 2H), 2.38 (s, 4H), 1.59 - 1.48 (m, 4H), 1.42 (d, *J* = 4.4 Hz, 2H).

MS calculated: 287.13; MS found: 288.1 [M+H]⁺.

### Example 96

### Synthesis of 5-chloro-3-(morpholinomethyl)-7-nitroquinolin-8-ol (96)

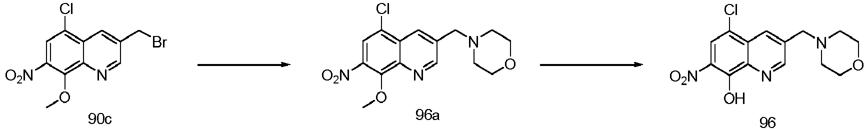

At room temperature, the crude 3-(bromomethyl)-5-chloro-8-methoxy-7-nitroquinoline (90c) (113.0 mg, 0.34 mmol) and morpholine (148.0 mg, 1.70 mmol) were added successively to tetrahydrofuran (2.5 mL). The reaction mixture was stirred at room temperature for 2 hours, and concentrated under reduced pressure. The resulting residue was purified by silica gel thin layer chromatography (developing solvent: dichloromethane/methanol=24/1) to obtain 5-chloro-8-methoxy-7-nitro-3-(morpholinmethyl)quinoline (96a) (52.0 mg, yield 45%).

At room temperature, 5-chloro-8-methoxy-7-nitro-3-(morpholinmethyl)quinoline (96a) (52.0 mg, 0.15 mmol) and lithium chloride (65.0 mg, 1.5 mmol) were added to N,N-dimethylformamide (1.5 mL). The reaction mixture was stirred at 130°C for 1 hour, cooled to room temperature, and concentrated under reduced pressure to remove the organic solvent. Water (1.0 mL) was added to the resulting residue, stirred for 3 minutes, and filtered under reduced pressure. The filter cake was washed with a mixed solvent of ethanol and water (volume ratio, ethanol/water = 1/1) (0.5 mL × 2), and dried under vacuum to obtain 5-chloro-3-(morpholinomethyl)-7-nitroquinolin-8-ol (96) (38.7 mg, yield 78%).

¹H NMR (400 MHz, DMSO-*d₆*) δ: 8.76 (s, 1H), 8.13 (s, 1H), 8.03 (s, 1H), 3.69 (s, 2H), 3.58 (br s, 4H), 2.40 (br s, 4H).

MS calculated: 323.07; MS found: 324.1 [M+H]⁺.

### Example 97

### Synthesis of 3-(morpholinomethyl)-7-nitroquinolin-8-ol (97)

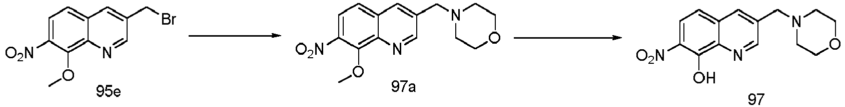

At room temperature, a solution of the crude 3-(bromomethyl)-8-methoxy-7-nitroquinoline (95e) (238.0 mg, 0.8 mmol) in dichloromethane (3.0 mL) was slowly added dropwise to a solution of morpholine (209.0 mg, 2.4 mmol) in dichloromethane (2.0 mL). The reaction mixture was stirred at room temperature for 2 hours, and diluted with dichloromethane (20.0 mL) and water (20.0 mL). The organic phase was separated, and the aqueous phase was extracted with dichloromethane (10.0 mL × 2). The combined organic phase was washed with saturated brine (10.0 mL), dried over anhydrous sodium sulfate, filtered, and concentrated under reduced pressure. The resulting residue was purified by silica gel column chromatography (eluent: dichloromethane/methanol = 61.5/1) to obtain 8-methoxy-7-nitro-3-(morpholinmethyl)quinoline (97a) (61.0 mg, yield 25%).

At room temperature, 8-methoxy-7-nitro-3-(morpholinmethyl)quinoline (97a) (61.0 mg, 0.2 mmol) and lithium chloride (84.8 mg, 2.0 mmol) were added to N,N-dimethylformamide (2.0 mL). The reaction mixture was stirred at 150°C for 1 hour, cooled to room temperature, and concentrated under reduced pressure to remove the organic solvent. Water (2.0 mL) was added to the resulting residue, stirred for 3 minutes, and filtered under reduced pressure. Water (2.0 mL) was added to the resulting filter cake, stirred for 10 minutes, and filtered under reduced pressure. The filter cake was dried under vacuum to obtain 3-(morpholinomethyl)-7-nitroquinolin-8-ol (97) (48.5 mg, yield 83%).

¹H NMR (400 MHz, DMSO-*d₆*) δ: 8.55 (s, 1H), 8.01 (s, 1H), 7.91 (d, *J* = 9.4 Hz, 1H), 6.51 (d, *J* = 9.4 Hz, 1H), 3.64 (s, 2H), 3.62 - 3.58 (m, 4H), 2.41 (s, 4H).

MS calculated: 289.11; MS found: 290.2 [M+H]⁺.

### Example 98

### Synthesis of 3-((diethylamino)methyl)-7-nitroquinolin-8-ol (98)

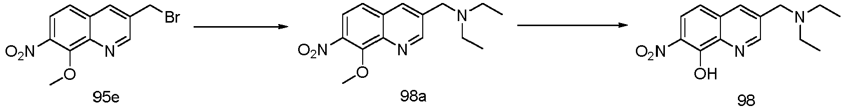

At room temperature, a solution of the crude 3-(bromomethyl)-8-methoxy-7-nitroquinoline (95e) (238.0 mg, 0.80 mmol) in dichloromethane (3.0 mL) was slowly added dropwise to a solution of diethylamine (176.8 mg, 2.4 mmol) in dichloromethane (2.0 mL). The reaction mixture was stirred at room temperature for 2 hours, and diluted with dichloromethane (20.0 mL) and water (20.0 mL). The organic phase was separated, and the aqueous phase was extracted with dichloromethane (10.0 mL × 2). The combined organic phase was washed with saturated brine (10.0 mL), dried over anhydrous sodium sulfate, filtered, and concentrated under reduced pressure. The resulting residue was purified by silica gel column chromatography (eluent: petroleum ether/ethyl acetate = 5.7/1) to obtain 8-methoxy-7-nitro-3-((diethylamino)methyl)quinoline (98a) (47.0 mg, yield 20%).

At room temperature, 8-methoxy-7-nitro-3-((diethylamino)methyl)quinoline (98a) (47.0 mg, 0.16 mmol) and lithium chloride (67.8 mg, 1.6 mmol) were added to N,N-dimethylformamide (2.0 mL). The reaction mixture was stirred at 150°C for 1 hour, cooled to room temperature, and concentrated under reduced pressure to remove the organic solvent. Water (2.0 mL) was added to the resulting residue, stirred for 3 minutes, and filtered under reduced pressure. Water (2.0 mL) was added to the resulting filter cake, stirred for 10 minutes, and filtered under reduced pressure. The filter cake was dried under vacuum to obtain 3-((diethylamino)methyl)-7-nitroquinolin-8-ol (98) (25.8 mg, yield 58%).

¹H NMR (400 MHz, DMSO-*d₆*) δ: 8.55 (s, 1H), 7.99 (s, 1H), 7.90 (d, *J* = 9.4 Hz, 1H), 6.50 (d, *J* = 9.4 Hz, 1H), 3.69 (s, 2H), 2.52 - 2.47 (m, 4H), 1.02 (t, *J* = 7.1 Hz, 6H).

MS calculated: 275.13; MS found: 276.2[M+H]⁺.

### Example 99

### Synthesis of 5-((methylamino)methyl)-7-nitroquinolin-8-ol dihydrochloride (99)

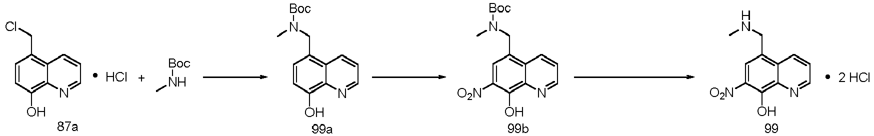

At 0°C, 5-(chloromethyl)quinolin-8-ol hydrochloride (87a) (135.0 mg, 0.59 mmol) and *tert*-butyl N-methylcarbamate (384.0 mg, 2.93 mmol) were added successively to acetonitrile (4.0 mL). The reaction mixture was stirred at 85°C for 2 hours, cooled to room temperature, and concentrated to dryness under reduced pressure to obtain the crude product *tert*-butyl N-((8-hydroxyquinolin-5-yl)methyl)-N-methylcarbamate (99a) (436.6 mg, crude yield >100%).

At room temperature, sodium nitrite (108.0 mg, 1.56 mmol) was added to a solution of the crude *tert*-butyl N-((8-hydroxyquinolin-5-yl)methyl)-N-methylcarbamate (99a) (436.6 mg, 0.59 mmol) in acetic acid (2.0 mL). The reaction mixture was stirred at room temperature for 2 hours, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile = 100%/0 - 30%/70%, gradient elution, and the flow rate was 20.0 mL/min) to obtain *tert*-butyl N-(8-hydroxy-7-nitroquinolin-5-yl)methyl-N-methylcarbamate (99b) (30.0 mg, yield 17%).

At room temperature, a solution of hydrogen chloride in 1,4-dioxane (4.0 M) (1.5 mL, 6.0 mmol) was added dropwise to a solution of *tert*-butyl N-(8-hydroxy-7-nitroquinolin-5-yl)methyl-N-methylcarbamate (99b) (30.0 mg, 0.090 mmol) in dichloromethane (1.0 mL). The reaction mixture was stirred at room temperature for 2 hours, and concentrated to dryness under reduced pressure to obtain 5-((methylamino)methyl)-7-nitroquinolin-8-ol dihydrochloride (99) (27.0 mg, yield 98%).

¹H NMR (400 MHz, DMSO-*d₆*) δ 9.22 (br s, 2H), 9.07 (d, *J* = 3.2 Hz, 1H), 8.96 (d, *J* = 8.4 Hz, 1H), 8.40 (s, 1H), 7.97 (dd, *J* = 8.8, 4.4 Hz, 1H), 4.59 (t, *J* = 4.8 Hz, 2H), 2.65 (t, *J* = 4.8 Hz, 3H).

MS calculated: 233.08; MS found: 234.1 [M+H]⁺.

### Example 100

### Synthesis of 4-((8-hydroxy-7-nitroquinolin-3-yl)methyl)piperazin-2-one (100)

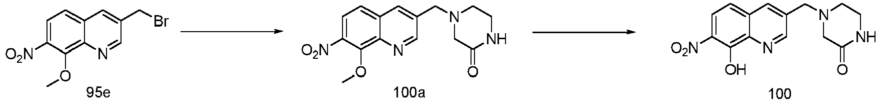

At room temperature, 2-piperazinone (275.3 mg, 2.8 mmol) was added to a mixed solvent of dichloromethane (3.0 mL) and N,N-dimethylformamide (2.0 mL). A solution of the crude 3-(bromomethyl)-8-methoxy-7-nitroquinoline (95e) (272.0 mg, 0.92 mmol) in dichloromethane (3.0 mL) was slowly added dropwise. The reaction mixture was stirred at room temperature for 2 hours, and diluted with dichloromethane (20.0 mL) and water (20.0 mL). The organic phase was separated, and the aqueous phase was extracted with dichloromethane (10.0 mL × 2). The combined organic phase was washed with saturated brine (10.0 mL), dried over anhydrous sodium sulfate, filtered, and concentrated under reduced pressure. The resulting residue was purified by silica gel column chromatography (eluent: dichloromethane/methanol=15/1) to obtain 4-((8-methoxy-7-nitroquinolin-3-yl)methyl)piperazin-2-one (100a) (75.0 mg, yield 26%).

At room temperature, 4-((8-methoxy-7-nitroquinolin-3-yl)methyl)piperazin-2-one (100a) (75.0 mg, 0.24 mmol) and lithium chloride (99.6 mg, 2.4 mmol) were added to N,N-dimethylformamide (2.0 mL). The reaction mixture was stirred at 150°C for 1 hour, cooled to room temperature, and concentrated under reduced pressure to remove the organic solvent. The resulting residue was dissolved in water (5.0 mL), and purified by reversed-phase high performance liquid chromatography (the chromatographic column was Eclipse XDB-C18 (21.2 mm×250 mm, 7µm), the mobile phase was water/acetonitrile = 100%/0 - 70%/30%, gradient elution, and the flow rate was 20.0 mL/min) to obtain 4-((8-hydroxy-7-nitroquinolin-3-yl)methyl)piperazin-2-one (100) (41.3 mg, yield 58%).

¹H NMR (400 MHz, DMSO-*d₆*) δ 8.57 (s, 1H), 8.05 (s, 1H), 7.92 (d, *J* = 9.4 Hz, 1H), 7.83 (s, 1H), 6.53 (d, *J* = 9.4 Hz, 1H), 3.73 (s, 2H), 3.18 (s, 2H), 3.00 (s, 2H), 2.60 (t, *J* = 5.2 Hz, 2H).

MS calculated: 302.10; MS found: 303.1[M+H]⁺.

### Example 101

### Synthesis of 7-nitro-5-(piperazin-1-ylmethyl)quinolin-8-ol trihydrochloride (101)

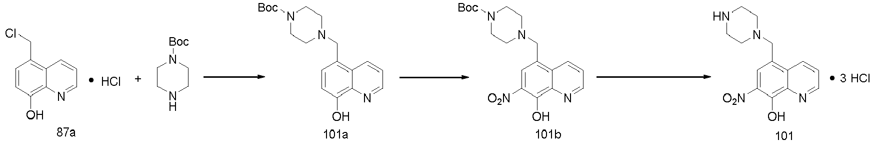

At 0°C, 5-(chloromethyl)quinolin-8-ol hydrochloride (87a) (69.0 mg, 0.3 mmol) and tert-butyl 1-piperazinecarboxylate (56.0 mg, 0.3 mmol) were added successively to acetonitrile (4.0 mL). The reaction mixture was stirred at 85°C for 2 hours, cooled to room temperature, and concentrated under reduced pressure. The resulting residue was purified by silica gel column chromatography (eluent: dichloromethane/methanol = 49/1, and adding 0.5% ammonia (25 to 28weight%, NH₃ content) by the total volume of the eluent) to obtain *tert*-butyl 4-((8-hydroxyquinolin-5-yl)methyl)piperazine-1-carboxylate (101a) (100.0 mg, yield 97%).

At room temperature, sodium nitrite (56.0 mg, 0.81 mmol) was added to a solution of *tert*-butyl 4-((8-hydroxyquinolin-5-yl)methyl)piperazine-1-carboxylate (101a) (100.0 mg, 0.27 mmol) in acetic acid (2.0 mL). The reaction mixture was stirred at room temperature for 2 hours, and filtered under reduced pressure. The filter cake was washed with ethanol (0.5 mL × 2), and dried under vacuum to obtain *tert*-butyl 4-((8-hydroxy-7-nitroquinolin-5-yl)methyl)piperazine-1-carboxylate (101b) (30.0 mg, yield 29%).

At room temperature, a solution of hydrogen chloride in 1,4-dioxane (4.0 M) (1.5 mL, 6.0 mmol) was added dropwise to a solution of *tert*-butyl 4-((8-hydroxy-7-nitroquinolin-5-yl)methyl)piperazine-1-carboxylate (101b) (30.0 mg, 0.077 mmol) in dichloromethane (0.5 mL). The reaction mixture was stirred at room temperature for 2 hours, and concentrated to dryness under reduced pressure to obtain 7-nitro-5-(piperazin-1-ylmethyl)quinolin-8-ol trihydrochloride (101) (24.0 mg, yield 78%).

¹H NMR (400 MHz, DMSO-*d₆*) δ: 10.09 (br s, 1H), 9.15 (s, 1H), 9.04 (d, *J* = 4.0 Hz, 1H), 8.49 (s, 1H), 7.95 (dd, *J* = 8.4, 4.2 Hz, 1H), 4.95 - 4.72 (m, 2H), 3.46 - 3.38 (m, 2H).

MS calculated: 288.12; MS found: 289.1 [M+H]⁺.

### Example 102

### Synthesis of 7-nitro-3-(piperazin-1-ylmethyl)quinolin-8-ol trihydrochloride (102)

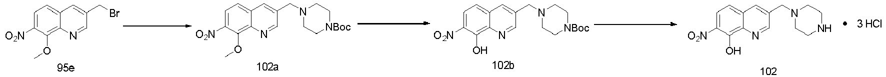

At room temperature, a solution of the crude 3-(bromomethyl)-8-methoxy-7-nitroquinoline (95e) (272.0 mg, 0.92 mmol) in dichloromethane (3.0 mL) was slowly added dropwise to a solution of *tert*-butyl 1-piperazinecarboxylate (853.0 mg, 4.6 mmol) in dichloromethane (3.0 mL). The reaction mixture was stirred at room temperature for 2 hours, and diluted with dichloromethane (20.0 mL) and water (20.0 mL). The organic phase was separated, and the aqueous phase was extracted with dichloromethane (10.0 mL × 2). The combined organic phase was washed with saturated brine (10.0 mL), dried over anhydrous sodium sulfate, filtered, and concentrated under reduced pressure. The resulting residue was purified by silica gel column chromatography (eluent: dichloromethane/methanol = 70/1) to obtain *tert*-butyl 4-((8-methoxy-7-nitroquinolin-3-yl)methyl)piperazine-1-carboxylate (102a) (83.0 mg, yield 23%).

At room temperature, *tert*-butyl 4-((8-methoxy-7-nitroquinolin-3-yl)methyl)piperazine-1-carboxylate (102a) (83.0 mg, 0.21 mmol) and lithium chloride (89.0 mg, 2.1 mmol) were added to N,N-dimethylformamide (2.0 mL). The reaction mixture was stirred at 150°C for 1 hour, cooled to room temperature, and concentrated under reduced pressure to remove the organic solvent. Water (2.0 mL) was added to the resulting residue, stirred for 3 minutes, and filtered under reduced pressure. Water (2.0 mL) was added to the resulting filter cake, stirred for 10 minutes, and filtered under reduced pressure. The filter cake was dried under vacuum to obtain *tert*-butyl 4-((8-hydroxy-7-nitroquinolin-3-yl)methyl)piperazine-1-carboxylate (102b) (80.0 mg, yield 99%).

At room temperature, tert-butyl 4-((8-hydroxy-7-nitroquinolin-3-yl)methyl)piperazine-1-carboxylate (102b) (80.0 mg, 0.21 mmol) was added to a solution of hydrogen chloride in 1,4-dioxane (4.0 M) (6.0 mL, 24.0 mmol). The reaction mixture was stirred at room temperature for 2 hours, and concentrated under reduced pressure to remove the organic solvent. Ethyl acetate (5.0 mL) was added to the resulting residue, stirred for 3 minutes, and filtered under reduced pressure. The filter cake was washed with ethyl acetate (0.5 mL × 2), and dried under vacuum to obtain 7-nitro-3-(piperazin-1-ylmethyl)quinolin-8-ol trihydrochloride (102) (62.7 mg, yield 77%).

¹H NMR (400 MHz, D₂O*-d₆*) δ: 10.03 (s, 2H), 9.31 (s, 1H), 8.76 (s, 1H), 8.07 (d, *J* = 9.2 Hz, 1H), 7.49 (d, *J* = 9.2 Hz, 1H), 4.66 (s, 2H), 3.45 (m, 8H).

MS calculated: 288.12; MS found: 289.1[M+H]⁺.

### Example 103

### Synthesis of 5-((ethylamino)methyl)-7-nitroquinolin-8-ol dihydrochloride (103)

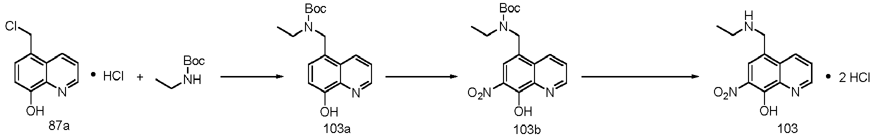

At 0°C, 5-(chloromethyl)quinolin-8-ol hydrochloride (87a) (115.0 mg, 0.5 mmol) and *tert*-butyl N-ethylcarbamate (290.0 mg, 2.0 mmol) were added successively to acetonitrile (4.0 mL). The reaction mixture was stirred at 85°C for 2 hours, cooled to room temperature, and filtered under reduced pressure. The filter cake was washed with acetonitrile (1.0 mL × 2), and the filtrate was concentrated to dryness under reduced pressure to obtain *tert*-butyl N-(8-hydroxyquinolin-5-yl)methyl-N-ethylcarbamate (103a) (94.0 mg, yield 60%).

At room temperature, sodium nitrite (37.0 mg, 0.54 mmol) was added to a solution of *tert*-butyl N-(8-hydroxyquinolin-5-yl)methyl-N-ethylcarbamate (103a) (54.0 mg, 0.18 mmol) in acetic acid (1.5 mL). The reaction mixture was stirred at room temperature for 2 hours, and filtered under reduced pressure. The resulting filtrate was purified by reversed-phase high performance liquid chromatography (the chromatographic column was Eclipse XDB-C18 (21.2 mm×250 mm, 7µm), the mobile phase was water/acetonitrile = 100%/0 - 30%/70%, gradient elution, and the flow rate was 20.0 mL/min) to obtain *tert*-butyl N-(8-hydroxy-7-nitroquinolin-5-yl)methyl-N-ethylcarbamate (103b) (28.0 mg, yield 45%).

At room temperature, a solution of hydrogen chloride in 1,4-dioxane (4.0 M) (1.5 mL, 6.0 mmol) was added dropwise to a solution of *tert*-butyl N-(8-hydroxy-7-nitroquinolin-5-yl)methyl-N-ethylcarbamate (103b) (28.0 mg, 0.081 mmol) in dichloromethane (1.0 mL). The reaction mixture was stirred at room temperature for 2 hours, and concentrated to dryness under reduced pressure to obtain 5-((ethylamino)methyl)-7-nitroquinolin-8-ol dihydrochloride (103) (26.0 mg, yield 88%).

¹H NMR (400 MHz, DMSO-*d₆*) δ: 9.30 (br s, 2H), 9.08 (d, *J* = 4.0 Hz, 1H), 8.97 (d, *J* = 8.6 Hz, 1H), 8.43 (s, 1H), 7.97 (dd, *J* = 8.4, 4.4 Hz, 1H), 4.59 (t, *J* = 5.6 Hz, 2H), 3.11 (dd, *J* = 12.4, 6.8 Hz, 2H), 1.29 (t, *J* = 7.2 Hz, 3H).

MS calculated: 247.25; MS found: 248.1 [M+H]⁺.

### Example 104

### Synthesis of 5-((dimethylamino)methyl)-7-nitro-4-(trifluoromethyl)quinolin-8-ol (104)

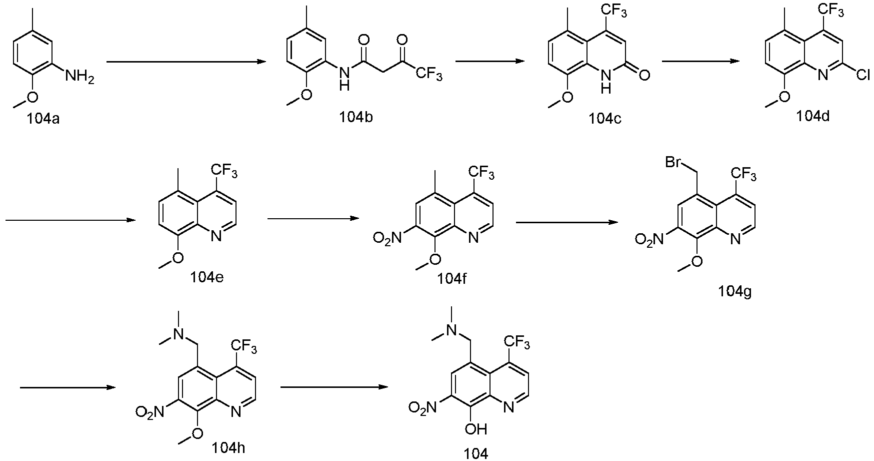

At room temperature, 2-methoxy-5-methylaniline (104a) (27.4 g, 199.73 mmol) was added to ethyl 4,4,4-trifluoroacetoacetate (75.0 g, 399.47 mmol). The reaction mixture was warmed up to 135°C, stirred for 1 hour, cooled to 0°C, followed by the addition of water (4.0 mL), and stirred for 30 minutes. The resulting mixture was filtered under reduced pressure, the filter cake was washed with petroleum ether (boiling range: 60 to 90°C) (25.0 mL × 3), and dried under vacuum to obtain the crude product 4,4,4-trifluoro-N-(2-methoxy-5-methylphenyl)-3-oxobutanamide (104b) (28.0 g, crude yield 51%).

At room temperature, the crude 4,4,4-trifluoro-N-(2-methoxy-5-methylphenyl)-3-oxobutanamide (104b) (6.0 g, 21.80 mmol) was added to polyphosphoric acid (30.0 mL). The reaction mixture was warmed up to 150°C, stirred for 1 hour, cooled to about 60°C to 70°C, followed by the addition of ice water (100.0 mL), stirred vigorously for 30 minutes, and filtered under reduced pressure. The filter cake was washed with water (20.0 mL × 4), and dried under vacuum to obtain the crude product 8-methoxy-5-methyl-4-trifluoromethyl-2(*1H*)-quinolinone (104c) (5.0 g, crude yield 89%).

At room temperature, the crude 8-methoxy-5-methyl-4-trifluoromethyl-2(*1H*)-quinolinone (104c) (5.0 g, 19.4 mmol) was added to phosphorus oxychloride (20.0 mL). The reaction mixture was stirred at 100°C for 3 hours, cooled to room temperature, followed by the addition of ice water (100.0 mL), stirred vigorously for 30 minutes, and filtered under reduced pressure. The filter cake was washed with water (20.0 mL × 3), and dried under vacuum to obtain the crude product 2-chloro-8-methoxy-5-methyl-4-(trifluoromethyl)quinoline (104d) (4.5 g, crude yield 84%).

At room temperature, the crude 2-chloro-8-methoxy-5-methyl-4-(trifluoromethyl)quinoline (104d) (1.7 g, 6.17 mmol) and aqueous hydrazine hydrate solution (80 weight%) (2.2 g, 43.17 mmol) were added successively to ethanol (20.0 mL), followed by the addition of palladium/carbon (palladium loading: 10 weight%) (0.17 g, 0.16 mmol). The reaction mixture was stirred at 90°C for 5 hours, cooled to room temperature, and filtered through diatomite. The filter cake was washed with ethyl acetate (10.0 mL × 4), and the filtrate was concentrated under reduced pressure. Ethyl acetate (20.0 mL) and water (20.0 mL) were added to the resulting residue. The organic phase was separated, and the aqueous phase was extracted with ethyl acetate (20.0 mL × 2). The combined organic phase was washed with saturated brine (20.0 mL), dried over anhydrous sodium sulfate, filtered, and concentrated under reduced pressure. The resulting residue was purified by silica gel column chromatography (eluent: petroleum ether/ethyl acetate = 1/1) to obtain 8-methoxy-5-methyl-4-(trifluoromethyl)quinoline (104e) (1.0 g, yield 67%).

At 0°C, nitric acid (65 weight%) (2.6 mL, 37.3 mmol) was slowly added dropwise to a solution of 8-methoxy-5-methyl-4-(trifluoromethyl)quinoline (104e) (1.5 g, 6.22 mmol) in acetic anhydride (15.0 mL), and stirred for 10 minutes. Sulfuric acid (98 weight%) (0.41 mL, 7.50 mmol) was slowly added dropwise. The reaction mixture was stirred at 0°C for 10 minutes, and the pH of the aqueous phase was adjusted to about 9 to 10 with aqueous sodium hydroxide solution (1.0 M). The resulting mixture was extracted with ethyl acetate (50.0 mL × 2). The organic phases were combined, dried over anhydrous sodium sulfate, filtered, and concentrated under reduced pressure. The resulting residue was purified by silica gel column chromatography (eluent: petroleum ether/ethyl acetate = 20/1) to obtain 8-methoxy-5-methyl-7-nitro-4-(trifluoromethyl)quinoline (104f) (0.15 g, yield 8%).

At room temperature, N-bromosuccinimide (87.3 mg, 0.44 mmol) and 2,2'-azobisisobutyronitrile (120.4 mg, 0.74 mmol) were added successively to a solution of 8-methoxy-5-methyl-7-nitro-4-(trifluoromethyl)quinoline (104f) (105.0 mg, 0.37 mmol) in 1,2-dichloroethane (4.0 mL). The reaction mixture was warmed up to 90°C, stirred for 16 hours, cooled to room temperature, and concentrated under reduced pressure. The resulting residue was purified by silica gel column chromatography (eluent: petroleum ether/ethyl acetate = 3/1) to obtain 5-(bromomethyl)-8-methoxy-7-nitro-4-(trifluoromethyl)quinoline (104g) (100.0 mg, yield 74%).

At room temperature, dimethylamine hydrochloride (44.6 mg, 0.55 mmol) and triethylamine (110.9 mg, 1.10 mmol) were added successively to a solution of 5-(bromomethyl)-8-methoxy-7-nitro-4-(trifluoromethyl)quinoline (104g) (100.0 mg, 0.27 mmol) in dichloromethane (2.0 mL). The reaction mixture was stirred at room temperature for 2 hours, and concentrated under reduced pressure. The resulting residue was purified by silica gel column chromatography (eluent: petroleum ether/ethyl acetate = 3/1) to obtain 5-((dimethylamino)methyl)-8-methoxy-7-nitro-4-(trifluoromethyl)quinoline (104h) (70.0 mg, yield 79%).

At room temperature, 5-((dimethylamino)methyl)-8-methoxy-7-nitro-4-(trifluoromethyl)quinoline (104h) (70.0 mg, 0.21 mmol) and lithium chloride (26.5 mg, 0.63 mmol) were added to N,N-dimethylformamide (2.0 mL). The reaction mixture was stirred at 150°C for 1 hour, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/acetonitrile = 100%/0 - 70%/30%, gradient elution, and the flow rate was 20.0 mL/min) to obtain 5-((dimethylamino)methyl)-7-nitro-4-(trifluoromethyl)quinolin-8-ol (108) (15.1 mg, yield 23%).

¹H NMR (400 MHz, MeOH-*d₄*) δ 9.18 (s, 1H), 8.55 (s, 1H), 8.26 (s, 1H), 4.77 (s, 2H), 2.82 (s, 6H).

MS calculated: 315.08; MS found: 316.1 [M+H]⁺.

### Example 105

### Synthesis of 7-nitro-4-(piperidin-4-yl)quinolin-8-ol (105)

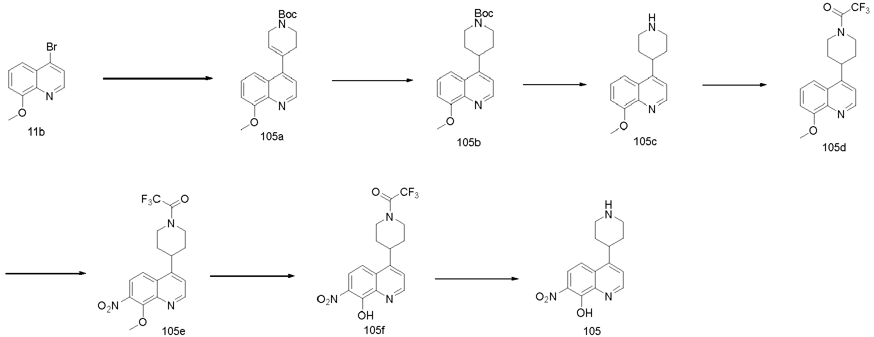

At room temperature, 4-bromo-8-methoxyquinoline (11b) (3.0 g, 12.68 mmol) and N-*tert*-butoxycarbonyl-1,2,5,6-tetrahydropyridine-4-boronic acid pinacol ester (4.7 g, 15.22 mmol) were added to a mixture of 1,4-dioxane (60.0 mL) and water (15.0 mL). Sodium carbonate (4.03 g, 38.04 mmol) and [1,1'-bis(diphenylphosphino)ferrocene]dichloropalladium(II) (1.03 g, 1.27 mmol) were added successively. The resulting reaction mixture was stirred at 80°C for 16 hours, cooled to room temperature, followed by the addition of ethyl acetate (100.0 mL) and water (100.0 mL). The organic phase was separated, and the aqueous phase was extracted with ethyl acetate (50.0 mL). The organic phases were combined, washed with saturated brine (100.0 mL), dried over anhydrous sodium sulfate, filtered, and concentrated under reduced pressure. The resulting residue was purified by silica gel column chromatography (eluent: petroleum ether/ethyl acetate = 3/1) to obtain tert-butyl 4-(8-methoxyquinolin-4-yl)-3,6-dihydropyridine-1(*2H*)-carboxylate (105a) (4.0 g, yield 93%).

At room temperature, *tert*-butyl 4-(8-methoxyquinolin-4-yl)-3,6-dihydropyridine-1(2*H*)-carboxylate (105a) (4.0 g, 11.76 mmol) and palladium/carbon (palladium loading: 10 weight%) (0.40 g, 0.376 mmol) were added successively to anhydrous methanol (40.0 mL). The resulting reaction mixture was stirred under a hydrogen atmosphere for 16 hours, and filtered through diatomite. The filter cake was washed with ethyl acetate (10.0 mL × 3), and the filtrate was concentrated under reduced pressure. The resulting residue was purified by silica gel column chromatography (eluent: petroleum ether/ethyl acetate = 4/1) to obtain *tert*-butyl 4-(8-methoxyquinolin-4-yl)piperidine-1-carboxylate (105b) (3.2 g, yield 80%).

At room temperature, *tert*-butyl 4-(8-methoxyquinolin-4-yl)piperidine-1-carboxylate (105b) (1.5 g, 4.37 mmol) was added to a solution of hydrogen chloride in ethyl acetate (3.0 M) (20.0 mL). The resulting reaction mixture was stirred at room temperature for 1 hour, and concentrated to dryness under reduced pressure to obtain the crude product 8-methoxy-4-(piperidin-4-yl)quinoline (105c) (1.1 g, crude yield 90%).

At room temperature, triethylamine (0.94 g, 9.26 mmol) and trifluoroacetic anhydride (0.778 g, 3.71 mmol) were added successively to a solution of 8-methoxy-4-(piperidin-4-yl)quinoline (105c) (0.86 g, 3.09 mmol) in dichloromethane (15.0 mL). The resulting reaction mixture was stirred at room temperature for 2 hours, followed by the addition of ethyl acetate (30.0 mL) and water (30.0 mL). The organic phase was separated, and the aqueous phase was extracted with ethyl acetate (20.0 mL). The organic phases were combined, washed with saturated brine (50.0 mL), dried over anhydrous sodium sulfate, filtered, and concentrated under reduced pressure. The resulting residue was purified by silica gel column chromatography (eluent: petroleum ether/ethyl acetate = 1/1) to obtain N-trifluoroacetyl-8-methoxy-4-(piperidin-4-yl)quinoline (105d) (0.67 g, yield 64%).

At 0°C, nitric acid (65 weight%) (0.79 mL, 11.89 mmol) was slowly added dropwise to a solution of N-trifluoroacetyl-8-methoxy-4-(piperidin-4-yl)quinoline (105d) (0.67 g, 1.98 mmol) in acetic anhydride (6.0 mL), and stirred for 10 minutes. Sulfuric acid (98 weight%) (0.13 mL, 2.38 mmol) was slowly added dropwise. The reaction mixture was stirred at 0°C for 10 minutes, and the pH of the aqueous phase was adjusted to about 9 to 10 with aqueous sodium hydroxide solution (1.0 M). The resulting mixture was extracted with ethyl acetate (50.0 mL × 2). The organic phases were combined, washed with saturated brine (30.0 mL), dried over anhydrous sodium sulfate, filtered, and concentrated under reduced pressure. The resulting residue was purified by silica gel column chromatography (eluent: petroleum ether/ethyl acetate = 2/1) to obtain N-trifluoroacetyl-7-nitro-8-methoxy-4-(piperidin-4-yl)quinoline (105e) (0.15 g, yield 20%).

At room temperature, N-trifluoroacetyl-7-nitro-8-methoxy-4-(piperidin-4-yl)quinoline (105e) (0.15 g, 0.39 mmol) and lithium chloride (0.050 g, 2.38 mmol) were added to N,N-dimethylformamide (10.0 mL). The reaction mixture was stirred at 150°C for 1 hour, cooled to room temperature, and concentrated under reduced pressure to remove the organic solvent to obtain the crude product N-trifluoroacetyl-7-nitro-4-(piperidin-4-yl)quinolin-8-ol (105f) (0.20 g, crude yield >100%).

At room temperature, the crude N-trifluoroacetyl-7-nitro-4-(piperidin-4-yl)quinolin-8-ol (105f) (0.20 g, 0.39 mmol (theoretical amount)) was added to a mixture of methanol (8.0 mL) and water (2.0 mL), followed by the addition of potassium hydroxide (0.11 g, 1.95 mmol). The resulting reaction mixture was stirred at room temperature for 2 hours, and concentrated to dryness under reduced pressure. The resulting residue was purified by reversed-phase high performance liquid chromatography (the chromatographic column was Eclipse XDB-C18 (21.2 mm×250 mm, 7µm), the mobile phase was water/acetonitrile=100%/0 - 30%/70%, gradient elution, and the flow rate was 20.0 mL/min) to obtain 7-nitro-4-(piperidin-4-yl)-quinolin-8-ol (105) (0.042 g, yield 29%).

¹H NMR (400 MHz, MeOH-*d₄*) δ 8.91 (d, *J* = 4.6 Hz, 1H), 8.18 (d, *J* = 9.6 Hz, 1H), 7.73 (d, *J* = 9.6 Hz, 1H), 7.65 (d, *J* = 4.6 Hz, 1H), 3.80 (s, 1H), 3.57 (d, *J* = 13.1 Hz, 2H), 3.33 (d, *J* = 13.2 Hz, 2H), 2.22 (d, *J* = 13.3 Hz, 2H), 2.07 - 1.96 (m, 3H).

MS calculated: 273.11; MS found: 274.35 [M+H]⁺.

### Example 106

### Synthesis of 5-fluoro-7-nitroquinolin-8-ol (106)

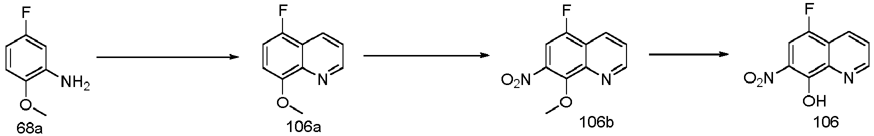

At room temperature, 5-fluoro-2-methoxyaniline (68a) (1.0 g, 7.08 mmol) was added to hydrochloric acid (6.0 M) (10.0 mL). The reaction mixture was warmed up to 110°C, followed by the slowly dropwise addition of acrolein diethyl acetate (2.31 g, 17.7 mmol), and stirred for 2 hours. The reaction mixture was cooled to room temperature, and the pH of the aqueous phase was adjusted to about 7 to 8 with saturated aqueous potassium carbonate solution. The resulting mixture was extracted with ethyl acetate (50.0 mL × 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 7/1 to 4/1) to obtain 5-fluoro-8-methoxyquinoline (106a) (0.20 g, yield 16%).

At 0°C, nitric acid (65 weight%) (0.23 mL, 3.56 mmol) was slowly added dropwise to a solution of 5-fluoro-8-methoxyquinoline (106a) (0.20 g, 1.13 mmol) in acetic anhydride (4.0 mL), and stirred for 10 minutes. Sulfuric acid (98 weight%) (0.061 mL, 1.13 mmol) was slowly added dropwise. The reaction mixture was stirred at 0°C for 2 hours, and the pH of the aqueous phase was adjusted to about 9 to 10 with aqueous sodium hydroxide solution (1.0 M). The resulting mixture was extracted with ethyl acetate (10.0 mL × 2). The organic phases were combined, dried over anhydrous sodium sulfate, filtered, and concentrated under reduced pressure. The resulting residue was purified by silica gel column chromatography (eluent: petroleum ether/ethyl acetate = 8/1) to obtain 5-fluoro-8-methoxy-7-nitroquinoline (106b) (0.165 g, yield 66%).

At room temperature, 5-fluoro-8-methoxy-7-nitroquinoline (106b) (65.0 mg, 0.29 mmol) and lithium chloride (124.0 mg, 2.90 mmol) were added to N,N-dimethylformamide (2.0 mL). The reaction mixture was stirred at 130°C for 1 hour, cooled to room temperature, and concentrated under reduced pressure to remove the organic solvent. Water (2.0 mL) was added to the resulting residue, stirred for 3 minutes, and filtered under reduced pressure. The resulting filter cake was washed with a mixed solvent of ethanol and water (volume ratio, ethanol/water = 1/1) (1.0 mL × 2), and dried under vacuum to obtain 5-fluoro-7-nitroquinolin-8-ol (106) (56.0 mg, yield 92%).

¹H NMR (400 MHz, DMSO-*d₆*) δ 8.76 (s, 1H), 8.21 (dd, *J* = 8.1, 0.9 Hz, 1H), 7.68 (d, *J* = 12.8 Hz, 1H), 7.65 (dd, *J* = 8.4, 4.5 Hz, 1H).

MS calculated: 208.03; MS found: 209.15 [M+H]⁺.

### Example 107

### Synthesis of 5-chloro-6-methyl-7-nitroquinolin-8-ol (107)

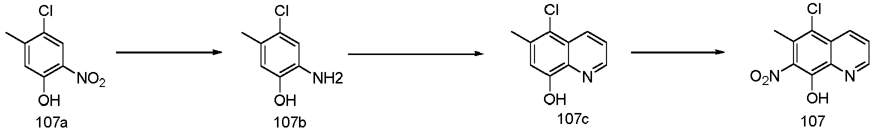

At room temperature, 4-chloro-5-methyl-2-nitrophenol (107a) (1.0 g, 5.35 mmol), iron powder (1.5 g, 26.74 mmol) and ammonium chloride (2.8 g, 53.78 mmol) were added successively to a mixture of ethanol (15.0 mL) and water (8.0 mL). The reaction mixture was stirred at 95°C for 2 hours, cooled to room temperature, and filtered through diatomite. The filter cake was washed with ethyl acetate (10.0 mL × 4), and the filtrates were combined. The organic phase was separated, and the aqueous phase was extracted with ethyl acetate (5.0 mL × 3). The organic phases were combined, dried over anhydrous sodium sulfate, filtered, and concentrated under reduced pressure to obtain the crude product 2-amino-4-chloro-5-methylphenol (107b) (0.57 g, crude yield 68%).

At room temperature, the crude 2-amino-4-chloro-5-methylphenol (107b) (0.435 g, 2.77 mmol) was added to hydrochloric acid (6.0 M) (10.0 mL). The reaction mixture was warmed up to 110°C, followed by the slowly dropwise addition of acrolein diethyl acetate (1.2 mL, 8.31 mmol), and stirred for 5 hours. The reaction mixture was cooled to room temperature, and the pH of the aqueous phase was adjusted to about 7 to 8 with saturated aqueous potassium carbonate solution. The resulting mixture was extracted with ethyl acetate (50.0 mL × 3). The organic phases were combined, dried over anhydrous sodium sulfate, filtered, and concentrated under reduced pressure. The resulting residue was purified by silica gel column chromatography (eluent: petroleum ether/ethyl acetate = 3/1) to obtain 5-chloro-6-methylquinolin-8-ol (107c) (0.41 g, yield 77%).

At room temperature, sodium nitrite (128.6 mg, 1.86 mmol) was added to a solution of 5-chloro-6-methylquinolin-8-ol (107c) (60.0 mg, 0.31 mmol) in acetic acid (3.0 mL). The resulting reaction mixture was stirred at room temperature for 3 hours, and filtered under reduced pressure. The filter cake was washed with a mixed solvent of ethanol and water (volume ratio, ethanol/water = 1/1) (1.0 mL × 2), and dried under vacuum to obtain 5-chloro-6-methyl-7-nitroquinolin-8-ol (107) (73.0 mg, yield 99%).

¹H-NMR (400 MHz, DMSO-*d₆*) δ: 10.22-10.00 (bs, 1 H), 8.93-8.86 (m, 1 H), 8.56-8.49 (m, 1 H), 7.75-7.67 (m, 1 H), 7.13 (s, 1 H), 2.56-2.51 (m, 3 H).

MS calculated: 238.63; MS found: 239.1 [M+H]⁺.

### Example 108

### Synthesis of 5-(difluoromethyl)-7-nitroquinolin-8-ol trifluoroacetate (108)

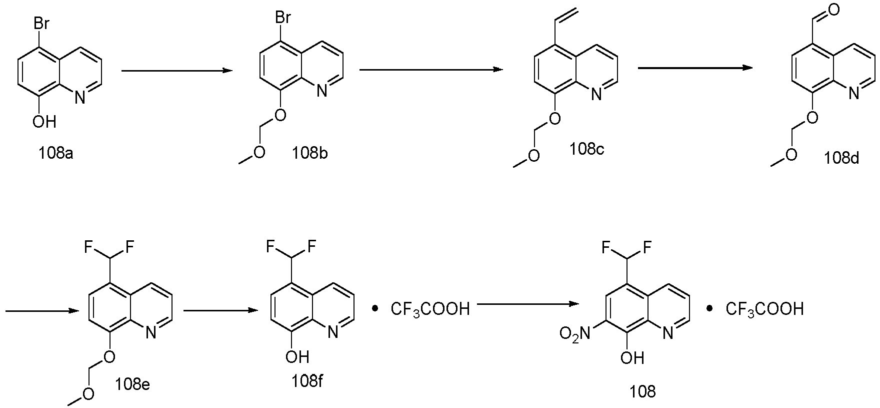

At 0°C, bromomethyl methyl ether (1.0 mL, 12.25 mmol) was slowly added dropwise to a solution of 5-bromo-8-hydroxyquinoline (108a) (2.04 g, 9.10 mmol) and diisopropylethylamine (4.4 mL, 24.90 mmol) in dichloromethane (40.0 mL). The resulting mixture was warmed up to room temperature, stirred for 5 hours, followed by the addition of water (20.0 mL). The organic phase was separated, and the aqueous phase was extracted with dichloromethane (20.0 mL × 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 100%/0 to 2/1) to obtain 5-bromo-8-(methoxymethoxy)quinoline (108b) (2.33 g, yield 96%).

At room temperature, 5-bromo-8-(methoxymethoxy)quinoline (108b) (616.0 mg, 2.30 mmol), potassium vinyltrifluoroborate (493.0 mg, 3.70 mmol), [1,1'-bis(diphenylphosphino)ferrocene]dichloropalladium(II) (168.0 mg, 0.23 mmol) and potassium carbonate (952.0 mg, 6.90 mmol) were added successively to a mixture of 1,4-dioxane (10.0 mL) and water (2.5 mL). The resulting mixture was stirred at 90°C for 18 hours, cooled to room temperature, and concentrated to dryness under reduced pressure. Dichloromethane (10.0 mL) and water (10.0 mL) were added to the resulting residue. The organic phase was separated, and the aqueous phase was extracted with dichloromethane (10.0 mL × 2). The organic phases were combined, dried over anhydrous sodium sulfate, filtered, and concentrated under reduced pressure. The resulting residue was purified by silica gel column chromatography (gradient elution, eluent: petroleum ether/ethyl acetate = 100%/0 to 4/1) to obtain 5-vinyl-8-(methoxymethoxy)quinoline (108c) (441.0 mg, yield 89%).

At 0°C, potassium osmate dihydrate (75.0 mg, 0.20 mmol) was added to a solution of 5-vinyl-8-(methoxymethoxy)quinoline (108c) (441.0 mg, 2.05 mmol) in tetrahydrofuran (36 mL), followed by the addition of water (9.0 mL). The reaction mixture was stirred at 0°C for 10 minutes, followed by the addition of sodium periodate (1.32 g, 6.15 mmol), and then stirred at room temperature for 16 hours. Ethyl acetate (15.0 mL) and water (10.0 mL) were added, the organic phase was separated, and the aqueous phase was extracted with ethyl acetate (10.0 mL × 3). The organic phases were combined, dried over anhydrous sodium sulfate, filtered, and concentrated to dryness under reduced pressure to obtain the crude product 8-(methoxymethoxy)quinoline-5-carbaldehyde (108d) (475.0 mg, crude yield 100%).

At 0°C, diethylaminosulfur trifluoride (610.0 mg, 3.77 mmol) was slowly added dropwise to a solution of 8-(methoxymethoxy)quinoline-5-carbaldehyde (108d) (230.0 mg, 1.06 mmol) in dichloromethane (10.0 mL). The reaction mixture was stirred at 0°C for 10 minutes, at room temperature for 16 hours, and then cooled to 0°C. The pH of the aqueous phase was adjusted to about 7 to 8 by the slowly dropwise addition of saturated aqueous sodium bicarbonate solution. The resulting mixture was extracted with dichloromethane (10.0 mL × 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 100%/0 to 1/1) to obtain 5-(difluoromethyl)-8-(methoxymethoxy)quinoline (108e) (132.0 mg, yield 52%).

At room temperature, 5-(difluoromethyl)-8-(methoxymethoxy)quinoline (108e) (55.0 mg, 0.23 mmol) was added to trifluoroacetic acid (3.0 mL). The resulting reaction solution was stirred at room temperature for 3 hours, and concentrated to dryness under reduced pressure to obtain the crude product 5-(difluoromethyl)-8-hydroxyquinoline trifluoroacetate (108t) (45.0 mg, crude yield >100%).

At room temperature, sodium nitrite (49.0 mg, 0.71 mmol) was added to a solution of 5-(difluoromethyl)-8-hydroxyquinoline trifluoroacetate (108f) (45.0 mg, 0.23 mmol) in acetic acid (3.0 mL). The reaction mixture was stirred at room temperature for 3 hours, and filtered under reduced pressure. The filter cake was washed with a mixed solvent of ethanol and water (volume ratio, ethanol/water = 1/1) (1.0 mL × 2), and dried under vacuum to obtain 5-(difluoromethyl)-7-nitroquinolin-8-ol trifluoroacetate (108) (75.6 mg, the total yield of the above two steps was 93%).

¹H NMR (400 MHz, DMSO-*d₆*) δ 9.07 (d, *J* = 4.6 Hz, 1H), 8.82 (t, *J* = 8.6 Hz, 1H), 8.45 (s, 1H), 8.03 (dd, *J* = 8.6, 4.6 Hz, 1H), 7.48 (t, *J* = 54.2 Hz, 1H).

MS calculated: 240.03; MS found: 241.1 [M+H]⁺.

### Example 109

### Synthesis of 5-(hydroxymethyl)-7-nitroquinolin-8-ol (109)

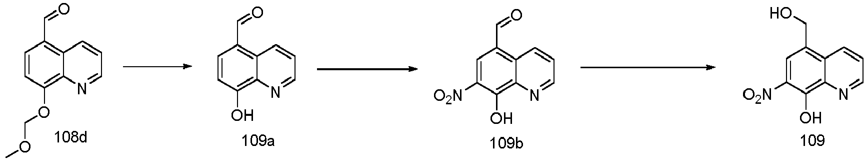

At room temperature, 8-(methoxymethoxy)quinoline-5-carbaldehyde (108d) (760.0 mg, 3.5 mmol) was added to trifluoroacetic acid (20.0 mL). The resulting reaction solution was stirred at room temperature for 3 hours, and concentrated to dryness under reduced pressure to obtain the crude product 8-hydroxyquinoline-5-carbaldehyde (109a) (606.0 mg, crude yield 100%).

At room temperature, the crude 8-hydroxyquinoline-5-carbaldehyde (109a) (606.0 mg, 3.5 mmol) was added to nitric acid (9 weight%) (10.0 mL). The reaction mixture was stirred at 90°C for 1 hour, cooled to room temperature, and filtered under reduced pressure. The filter cake was washed with water (3.0 mL × 3), and dried under vacuum to obtain 8-hydroxy-7-nitroquinoline-5-carbaldehyde (109b) (459.0 mg, the total yield of the above two steps was 60%).

At room temperature, 8-hydroxy-7-nitroquinoline-5-carbaldehyde (109b) (178.0 mg, 0.80 mmol) was added to a mixture of tetrahydrofuran (3.0 mL) and methanol (1.0 mL). The resulting reaction solution was cooled to 0°C, followed by the addition of sodium borohydride (34.0 mg, 0.90 mmol). The reaction mixture was stirred at 0°C for 1.5 hours, and concentrated to dryness under reduced pressure. The resulting residue was purified by reversed-phase high performance liquid chromatography (the chromatographic column was Eclipse XDB-C18 (21.2 mm×250 mm, 7µm), the mobile phase was water/acetonitrile=100%/0 - 99%/1%, gradient elution, and the flow rate was 20.0 mL/min) to obtain 5-(hydroxymethyl)-7-nitroquinolin-8-ol (109) (30.6 mg, yield 17%).

¹H NMR (400 MHz, DMSO-*d₆*) δ 8.62 (d, *J* = 4.2 Hz, 1H), 8.27 (d, *J* = 8.1 Hz, 1H), 7.92 (s, 1H), 7.54 (dd, *J* = 8.1, 4.2 Hz, 1H), 5.00 (s, 1H), 4.64 (s, 2H).

MS calculated: 220.05; MS found: 221.1 [M+H]⁺.

### Example 110

### Synthesis of 5-chloro-6-fluoro-7-nitroquinolin-8-ol (110)

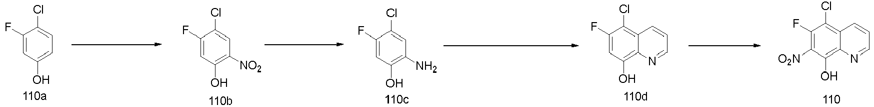

At room temperature, 3-fluoro-4-chlorophenol (110a) (1.5 g, 10.20 mmol) and tetrabutylammonium bromide (0.33 g, 0.102 mmol) were added successively to 1,2-dichloroethane (15.0 mL), followed by the slow addition of nitric acid (7 weight%) (18.0 mL). The reaction mixture was stirred at room temperature for 4 hours, and diluted with water (20.0 mL). The resulting mixture was extracted with dichloromethane (20.0 mL × 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 100%/0 to 10/1) to obtain 4-chloro-5-fluoro-2-nitrophenol (110b) (1.45 g, yield 74%).

At room temperature, 4-chloro-5-fluoro-2-nitrophenol (110b) (1.45 g, 7.57 mmol) was added to a mixture of acetic acid (14.5 mL) and water (4.5 mL), followed by the addition of reduced iron powder (2.11 g, 37.85 mmol). The resulting mixture was stirred at 100°C for 0.5 hour, cooled to room temperature, and filtered through diatomite, and the filter cake was washed with dichloromethane (10.0 mL × 4). The filtrates were combined, washed with saturated aqueous sodium bicarbonate solution (30.0 mL × 4) and saturated brine (30.0 mL) successively, dried over anhydrous sodium sulfate, filtered, and concentrated under reduced pressure. The resulting residue was purified by silica gel column chromatography (gradient elution, eluent: petroleum ether/ethyl acetate = 7/1 to 5/1) to obtain 2-amino-4-chloro-5-fluorophenol (110c) (0.247 g, yield 20%).

At room temperature, 2-amino-4-chloro-5-fluorophenol (110c) (0.247 g, 1.53 mmol), sodium 3-nitrobenzene sulfonate (0.413 g, 1.84 mmol) and glycerol (0.352 g, 3.83 mmol) were added successively to sulfuric acid (70 weight%) (2.0 mL). The resulting mixture was stirred at 140°C for 3 hours, and cooled to room temperature. The pH of the aqueous phase was adjusted to about 8 to 9 by the slowly dropwise addition of saturated aqueous sodium carbonate solution. The resulting mixture was extracted with dichloromethane (20.0 mL × 3). The organic phases were combined, dried over anhydrous sodium sulfate, filtered, and concentrated under reduced pressure. The resulting residue was purified by silica gel column chromatography (eluent: petroleum ether/ethyl acetate = 4/1) to obtain 5-chloro-6-fluoroquinolin-8-ol (110d) (0.12 g, yield 40%).

At room temperature, sodium nitrite (175.0 mg, 2.5 mmol) was added in batches to a suspension of 5-chloro-6-fluoroquinolin-8-ol (110d) (50.0 mg, 0.25 mmol) in hydrochloric acid (3.0 M) (1.67 mL). The reaction mixture was stirred at room temperature for 16 hours, and the pH of the aqueous phase was adjusted to about 8 to 9 by the slowly dropwise addition of saturated aqueous sodium carbonate solution. The resulting mixture was filtered under reduced pressure. The filter cake was washed with water (1.0 mL × 2) and a mixed solvent of ethanol and water (volume ratio, ethanol/water = 1/1) (0.5 mL × 2) successively, and dried under vacuum to obtain 5-chloro-6-fluoro-7-nitroquinolin-8-ol (110) (31.0 mg, yield 50%).

¹H NMR (400 MHz, DMSO-*d₆*) δ 9.06 (d, *J* = 3.7 Hz, 1H), 8.66 (d, *J* = 8.5 Hz, 1H), 7.96 (dd, *J* = 8.5, 4.2 Hz, 1H).

MS calculated: 241.99; MS found: 243.0, 245.0 [M+H]⁺.

### Example 111

### Synthesis of 5-chloro-7-nitro-4-(3-phenylpiperidin-1-yl)quinolin-8-ol (111)

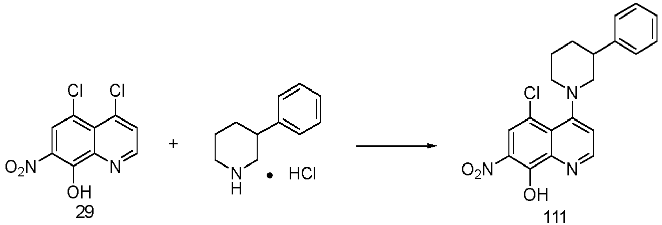

At room temperature, 4,5-dichloro-7-nitroquinolin-8-ol (29) (200.0 mg, 0.77 mmol) and 3-phenylpiperidine hydrochloride (161.0 mg, 1.0 mmol) were added to N,N-dimethylformamide (4.0 mL). The resulting mixture was stirred at 130°C for 5 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/methanol=100%/0 - 33%/67%, gradient elution, and the flow rate was 20.0 mL/min) to obtain 5-chloro-7-nitro-4-(3-phenylpiperidin-1-yl)quinolin-8-ol (111) (26.4 mg, yield 9%).

¹H NMR (400 MHz, DMSO-*d₆*) δ: 8.10-8.04 (m, 1H), 7.96-7.92 (m, 1H), 7.82-7.78 (m, 1H), 7.40-7.25 (m, 5H), 3.68-3.30 (m, 2 H), 3.08-2.92 (m, 1 H), 2.06-1.56 (m, 6 H).

MS calculated: 383.10; MS found: 384.1, 386.1 [M+H]⁺.

### Example 112

### Synthesis of 3-cyclopropyl-7-nitroquinolin-8-ol (112)

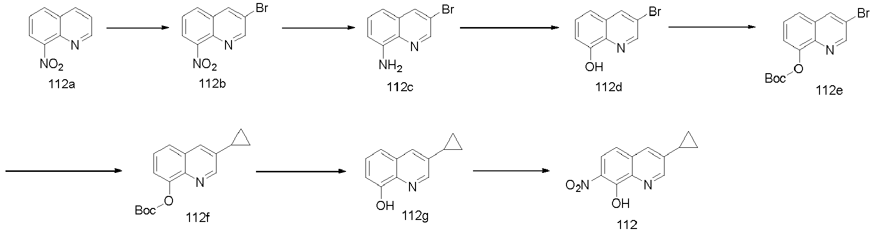

At 100°C, N-bromosuccinimide (5.34 g, 30.0 mmol) was added in three batches to a solution of 8-nitroquinoline (112a) (5.23 g, 30.0 mmol) in acetic acid (30.0 mL). The reaction mixture was stirred for 2 hours, cooled to room temperature, and concentrated under reduced pressure to remove the organic solvent. Dichloromethane (50.0 mL) was added to the resulting residue, and the pH of the aqueous phase was adjusted to about 7 to 8 with saturated aqueous sodium carbonate solution. The organic phase was separated, and the aqueous phase was extracted with dichloromethane (30.0 mL × 2). The organic phases were combined, dried over anhydrous sodium sulfate, filtered, and concentrated under reduced pressure. The resulting residue was purified by silica gel column chromatography (gradient elution, eluent: petroleum ether/ethyl acetate = 3/1 to 1.5/1) to obtain 3-bromo-8-nitroquinoline (112b) (4.61 g, yield 61%).

At room temperature, 3-bromo-8-nitroquinoline (112b) (4.23 g, 16.7 mmol), reduced iron powder (6.99 g, 125.3 mmol) and ammonium chloride (1.79 g, 33.4 mmol) were added successively to a mixture of ethanol (33.4 mL) and water (16.7 mL). The resulting reaction mixture was stirred at 100°C for 4 hours, cooled to room temperature, and filtered through diatomite. The filter cake was washed with ethyl acetate (10.0 mL × 4), and the filtrates were combined. The organic phase was separated, and the aqueous phase was extracted with ethyl acetate (20.0 mL × 3). The organic phases were combined, dried over anhydrous sodium sulfate, filtered, and concentrated under reduced pressure. The resulting residue was purified by silica gel column chromatography (gradient elution, eluent: petroleum ether/ethyl acetate= 1.5/1 to 1/1) to obtain 3-bromoquinolin-8-amine (112c) (3.16 g, yield 85%).

At 0°C, aqueous sodium nitrite (75.9 mg, 1.1 mmol) solution (1.0 mL) was added to a suspension of 3-bromoquinolin-8-amine (112c) (223.0 mg, 1.0 mmol) in sulfuric acid (26 weight%) (19.2 mL). The resulting mixture was stirred at 0°C for 20 minutes, added in batches to aqueous copper sulfate solution (11 weight%) (145.0 mL) at room temperature, followed by the addition of cuprous oxide (143.0 mg, 1.0 mmol). The reaction mixture was stirred at room temperature for 30 minutes, and extracted with dichloromethane (30.0 mL × 4). The organic phases were combined, dried over anhydrous sodium sulfate, filtered, and concentrated under reduced pressure. The resulting residue was purified by silica gel column chromatography (gradient elution, eluent: dichloromethane/methanol=200/1) to obtain 3-bromoquinolin-8-ol (112d) (143.0 mg, yield 64%).

At room temperature, 3-bromoquinolin-8-ol (112d) (143.0 mg, 0.64 mmol), triethylamine (0.13 mL, 0.96 mmol) and di-tert-butyl dicarbonate (168.0 mg, 0.768 mmol) were added successively to dichloromethane (3.2 mL). 4-(Dimethylamino)pyridine (7.8 mg, 0.064 mmol) was added. The reaction mixture was stirred for 2 hours and concentrated to dryness under reduced pressure. The resulting residue was purified by silica gel column chromatography (gradient elution, eluent: petroleum ether/ethyl acetate = 30/1) to obtain 3-bromoquinolin-8-yl tert-butyl carbonate (112e) (188.0 mg, yield 91%).

At room temperature, 3-bromoquinolin-8-yl tert-butyl carbonate (112e) (188.0 mg, 0.58 mmol), cyclopropylboronic acid (124.5 mg, 1.45 mmol), palladium acetate (26.0 mg, 0.116 mmol), tricyclohexylphosphine (65.0 mg, 0.232 mmol) and potassium phosphate (369.0 mg, 1.74 mmol) were added successively to a mixture of toluene (2.9 mL) and water (0.72 mL). The reaction mixture was stirred at 90°C for 26 hours, cooled to room temperature, and diluted with dichloromethane (10.0 mL) and water (10.0 mL). The organic phase was separated, and the aqueous phase was extracted with dichloromethane (10.0 mL × 2). The organic phases were combined, dried over anhydrous sodium sulfate, filtered, and concentrated under reduced pressure. The resulting residue was purified by silica gel column chromatography (gradient elution, eluent: petroleum ether/dichloromethane=1.5/1 to 1/1) to obtain *tert*-butyl (3-cyclopropylquinolin-8-yl) carbonate (112f) (134.0 mg, yield 81%).

At room temperature, a solution of tert-butyl (3-cyclopropylquinolin-8-yl) carbonate (112f) (134.0 mg, 0.47 mmol) in 1,4-dioxane (0.59 mL) was added to a solution of hydrogen chloride in 1,4-dioxane (4.0 M) (1.76 mL). The resulting mixture was stirred for 24 hours and concentrated to dryness under reduced pressure. Dichloromethane (10.0 mL) and saturated aqueous sodium bicarbonate solution (10.0 mL) were added to the resulting residue. The organic phase was separated, and the aqueous phase was extracted with dichloromethane (10.0 mL × 2). The organic phases were combined, dried over anhydrous sodium sulfate, filtered, and concentrated to dryness under reduced pressure to obtain the crude product 3-cyclopropylquinolin-8-ol (112g) (95.4 mg, crude yield >100%).

At room temperature, sodium nitrite (50.0 mg, 0.725 mmol) was added to a solution of the crude 3-cyclopropylquinolin-8-ol (112g) (54.6 mg, 0.29 mmol (theoretical amount)) in acetic acid (1.16 mL). The reaction mixture was stirred for 3 hours and concentrated to dryness under reduced pressure. A mixed solvent of ethanol and water (volume ratio, ethanol/water = 1/1) (1.0 mL) was added to the resulting residue, stirred for 5 minutes, and filtered under reduced pressure. The filter cake was washed with a mixed solvent of ethanol and water (volume ratio, ethanol/water = 2/1) (0.5 mL × 3), and dried under vacuum to obtain 3-cyclopropyl-7-nitroquinolin-8-ol (112) (42.1 mg, yield 63%).

¹H NMR (400 MHz, DMSO-*d₆*) δ: 9.24 (s, 1H), 8.82 (d, *J* = 1.2 Hz, 1H), 8.74 (d, *J* = 1.6 Hz, 1H), 8.51 (d, *J* = 8.8 Hz, 1H), 7.11 (d, *J* = 8.8 Hz, 1H), 2.34 - 2.26 (m, 1H), 1.21 (dd, *J* = 8.1, 1.8 Hz, 2H), 0.99 - 0.97 (m, 2H).

MS calculated: 230.07; MS found: 231.1 [M+H]⁺.

### Example 113

### Synthesis of 5-chloro-7-nitroquinolin-2-d-8-ol (113)

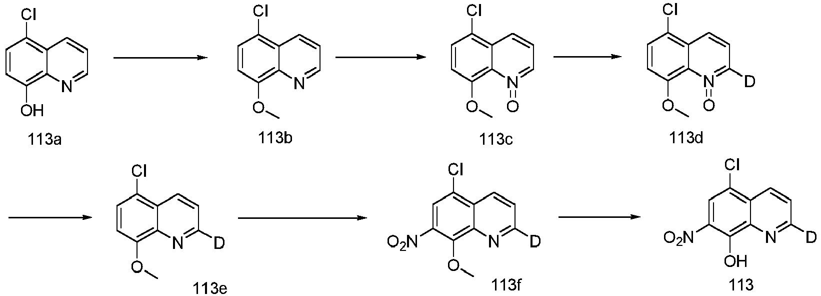

At 0°C, methyl iodide (0.68 mL, 10.8 mmol) was slowly added dropwise to a solution of 5-chloro-8-hydroxyquinoline (113a) (1.5 g, 8.35 mmol) and potassium carbonate (2.3 g, 16.7 mmol) in N,N-dimethylformamide (15.0 mL). The resulting mixture was warmed up to room temperature, stirred for 16 hours, diluted with water (50.0 mL), and extracted with dichloromethane (20.0 mL × 2). The organic phases were combined, washed with saturated brine (10.0 mL × 2), dried over anhydrous sodium sulfate and filtered, and the filtrate was concentrated under reduced pressure. The resulting residue was purified by silica gel column chromatography (gradient elution, eluent: petroleum ether/ethyl acetate = 8/1 to 2/1) to obtain 5-chloro-8-methoxyquinoline (113b) (1.48 g, yield 92%).

At room temperature, 3-chloroperoxybenzoic acid (85 weight%) (2.02 g, 9.95 mmol) was added in batches to a solution of 5-chloro-8-methoxyquinoline (113b) (1.288 g, 6.65 mmol) in dichloromethane (20.0 mL). The resulting mixture was stirred at room temperature for 16 hours, and cooled to 0°C. The pH of the aqueous phase was adjusted to about 8 to 9 with aqueous sodium hydroxide solution (1.0 M). The organic phase was separated, and the aqueous phase was extracted with dichloromethane (20.0 mL × 2). The organic phases were combined, washed with saturated brine (20.0 mL), dried over anhydrous sodium sulfate, filtered, and concentrated under reduced pressure. The resulting residue was purified by silica gel column chromatography (gradient elution, eluent: dichloromethane/methanol = 100%/0 to 91%/9%) to obtain 5-chloro-8-methoxyquinoline-1-oxide (113c) (1.0 g, yield 72%).

At room temperature, 5-chloro-8-methoxyquinoline-1-oxide (113c) (1.0 g, 4.77 mmol) was added in batches to a solution of sodium hydroxide (0.458 g, 11.45 mmol) in heavy water (5.0 mL). The reaction solution was stirred at 100°C for 6 hours, cooled to room temperature, and diluted with water (15.0 mL). The resulting mixture was extracted with dichloromethane (20.0 mL × 3). The organic phases were combined, washed with saturated brine (20.0 mL), dried over anhydrous sodium sulfate and filtered. The filtrate was concentrated to dryness under reduced pressure to obtain 5-chloro-8-methoxyquinoline-1-oxide-2-d (113d) (0.969 g, yield 96%).

At 0°C, phosphorus tribromide (0.43 mL, 4.6 mmol) was slowly added dropwise to a solution of 5-chloro-8-methoxyquinoline-1-oxide-2-d (113d) (0.486 g, 2.3 mmol) in N,N-dimethylformamide (6.0 mL). The reaction solution was stirred at room temperature for 2 hours, and cooled to 0°C. The pH of the aqueous phase was adjusted to about 8 to 9 with saturated aqueous sodium carbonate solution. The resulting mixture was extracted with dichloromethane (10.0 mL × 2). The organic phases were combined, washed with saturated brine (20.0 mL), dried over anhydrous sodium sulfate, filtered, and concentrated under reduced pressure. The resulting residue was purified by silica gel column chromatography (gradient elution, eluent: petroleum ether/ethyl acetate = 20/1 to 2/1) to obtain 5-chloro-8-methoxyquinoline-2-d (113e) (0.268 g, yield 60%).

At 0°C, nitric acid (65 weight%) (0.57 mL, 8.3 mmol) was slowly added dropwise to a solution of 5-chloro-8-methoxyquinoline-2-d (113e) (0.268 g, 1.37 mmol) in acetic anhydride (6.0 mL). The reaction mixture was stirred at 0°C for 10 minutes, followed by the slowly dropwise addition of sulfuric acid (98 weight%) (0.1 mL, 1.84 mmol). The reaction solution was warmed up to room temperature, stirred for 16 hours, and cooled to 0°C. The pH of the aqueous phase was adjusted to about 8 to 9 with aqueous sodium hydroxide solution (1.0 M). The resulting mixture was extracted with dichloromethane (10.0 mL × 3). The organic phases were combined, washed with saturated brine (10.0 mL), dried over anhydrous sodium sulfate, filtered, and concentrated under reduced pressure. The resulting residue was purified by silica gel column chromatography (gradient elution, eluent: petroleum ether/ethyl acetate = 20/1 to 10/1) to obtain 5-chloro-7-nitro-8-methoxyquinoline-2-d (113f) (0.094 g, yield 29%).

At room temperature, 5-chloro-7-nitro-8-methoxyquinoline-2-*d* (113f) (94.0 mg, 0.39 mmol) and lithium chloride (165.0 mg, 3.9 mmol) were added to N,N-dimethylformamide (3.0 mL). The reaction mixture was stirred at 140°C for 45 minutes, cooled to room temperature, and concentrated under reduced pressure to remove the organic solvent. Water (2.0 mL) was added to the resulting residue, stirred for 3 minutes, and filtered under reduced pressure. The resulting filter cake was washed with water (1.0 mL × 2), and dried under vacuum to obtain 5-chloro-7-nitroquinolin-2-*d*-8-ol (113) (84.0 mg, yield 95%).

¹H NMR (400 MHz, DMSO-*d₆*) δ: 8.30 (d, *J* = 8.3 Hz, 1H), 8.06 (s, 1H), 7.71 (d, *J* = 8.3 Hz, 1H).

MS calculated: 225.01; MS found: 226.0 [M+H]⁺.

### Example 114

### Synthesis of 6-bromo-5-chloro-7-nitroquinolin-8-ol (114)

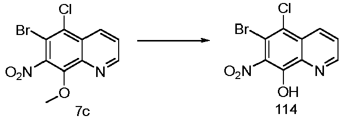

At room temperature, 6-bromo-5-chloro-7-nitro-8-methoxyquinoline (7c) (1.65 g, 5.21 mmol) and lithium chloride (2.2 g, 52.4 mmol) were added to N,N-dimethylformamide (15.0 mL). The reaction mixture was stirred at 120°C for 1 hour, cooled to room temperature, and concentrated under reduced pressure to remove the organic solvent. Water (25.0 mL) was added to the resulting residue, stirred for 5 minutes, and filtered under reduced pressure. The filter cake was washed with water (5.0 mL × 2), and dried under vacuum to obtain 6-bromo-5-chloro-7-nitroquinolin-8-ol (114) (1.41 g, yield 89%).

¹H NMR (400 MHz, DMSO-*d₆*) δ: 8.70 (dd, *J* = 4.4, 1.6 Hz, 1 H), 8.41 (dd, *J* = 8.6, 1.4 Hz, 1 H), 7.68 (dd, *J* = 8.4, 4.0 Hz, 1 H).

MS calculated: 301.91, 303.91; MS found: 303.0, 305.0 [M+H]⁺.

### Example 115

### Synthesis of 5,6-dichloro-7-nitroquinolin-8-ol (115)

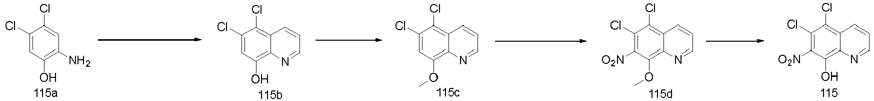

At room temperature, 2-amino-4,5-dichlorophenol (115a) (5.0 g, 28.1 mmol) was added to hydrochloric acid (6.0 M) (140.0 mL). The reaction mixture was warmed up to 110°C, followed by the slowly dropwise addition of acrolein diethyl acetate (12.0 mL, 73.8 mmol), and stirred for 1.5 hours. The reaction mixture was cooled to room temperature, and the pH of the aqueous phase was adjusted to about 9 to 10 by the slow addition of saturated aqueous sodium carbonate solution. The resulting mixture was extracted with ethyl acetate (200.0 mL × 3). The organic phases were combined, dried over anhydrous sodium sulfate, filtered, and concentrated under reduced pressure. The resulting residue was purified by silica gel column chromatography (eluent: petroleum ether/ethyl acetate = 3/1) to obtain 5,6-dichloro-8-hydroxyquinoline (115b) (3.9 g, yield 65%).

At room temperature, methyl iodide (1.35 mL, 21.7 mmol) was slowly added dropwise to a suspension of 5,6-dichloro-8-hydroxyquinoline (115b) (3.9 g, 18.2 mmol) and potassium carbonate (5.03 g, 36.4 mmol) in N,N-dimethylformamide (25.0 mL). The resulting mixture was stirred at room temperature for 18 hours, and concentrated under reduced pressure to remove the organic solvent. Water (30.0 mL) was added to the residue, and extracted with ethyl acetate (30.0 mL × 2). The organic phases were combined, dried over anhydrous sodium sulfate and filtered, and the filtrate was concentrated under reduced pressure. The resulting residue was purified by silica gel column chromatography (eluent: petroleum ether/ethyl acetate = 5/1) to obtain 5,6-dichloro-8-methoxyquinoline (115c) (3.5 g, yield 71%).

At 0°C, nitric acid (65 weight%) (13.0 mL, 197.8 mmol) was slowly added dropwise to a solution of 5,6-dichloro-8-methoxyquinoline (115c) (3.5 g, 15.4 mmol) in acetic anhydride (75.0 mL), and stirred for 10 minutes. Sulfuric acid (98 weight%) (2.5 mL, 47.0 mmol) was slowly added dropwise. The reaction mixture was stirred at 0°C for 30 minutes, then warmed up to room temperature and stirred for 72 hours, and then cooled to 0°C. The pH of the aqueous phase was adjusted to about 9 to 10 with aqueous sodium hydroxide solution (1.0 M). The resulting mixture was extracted with dichloromethane (50.0 mL × 3). The organic phases were combined, dried over anhydrous sodium sulfate, filtered, and concentrated under reduced pressure. The resulting residue was purified by silica gel column chromatography (eluent: petroleum ether/ethyl acetate = 2/1) to obtain 5,6-dichloro-8-methoxy-7-nitroquinoline (115d) (0.718 g, yield 17%).

At room temperature, 5,6-dichloro-8-methoxy-7-nitroquinoline (115d) (0.718 g, 2.64 mmol) and lithium chloride (1.38 g, 32.9 mmol) were added to N,N-dimethylformamide (15.0 mL). The reaction mixture was stirred at 120°C for 1 hour, cooled to room temperature, and concentrated under reduced pressure to remove the organic solvent. Water (25.0 mL) was added to the resulting residue, stirred for 5 minutes, and filtered under reduced pressure. The filter cake was washed with water (5.0 mL × 2), and dried under vacuum to obtain 5,6-dichloro-7-nitroquinolin-8-ol (115) (0.555 g, yield 81%).

¹H NMR (400 MHz, DMSO-*d₆*) δ: 8.69 (dd, *J* = 4.2, 1.2 Hz, 1H), 8.40 (dd, *J* = 8.4, 1.6 Hz, 1H), 7.70 (dd, *J* = 8.4, 4.4 Hz, 1H).

MS calculated: 257.96; MS found: 259.0, 261.0 [M+H]⁺.

### Example 116

### Synthesis of 7-nitroquinolin-2-<7-8-ol (116)

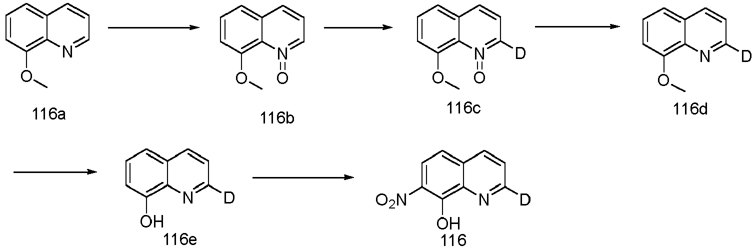

At room temperature, 3-chloroperoxybenzoic acid (85 weight%) (3.6 g, 17.9 mmol) was added in batches to a solution of 8-methoxyquinoline (116a) (1.9 g, 11.9 mmol) in dichloromethane (40.0 mL). The resulting mixture was stirred at room temperature for 16 hours, and cooled to 0°C. The pH of the aqueous phase was adjusted to about 8 to 9 with aqueous sodium hydroxide solution (1.0 M). The organic phase was separated, and the aqueous phase was extracted with dichloromethane (20.0 mL × 2). The organic phases were combined, washed with saturated brine (20.0 mL), dried over anhydrous sodium sulfate, filtered, and concentrated under reduced pressure. The resulting residue was purified by silica gel column chromatography (eluent: dichloromethane/methanol = 100%/0 to 8/1) to obtain 8-methoxyquinoline-1-oxide (116b) (0.836 g, yield 40%).

At room temperature, 8-methoxyquinoline-1-oxide (116b) (0.836 g, 4.77 mmol) was added in batches to a solution of sodium hydroxide (0.458 mg, 11.45 mmol) in heavy water (5.0 mL). The resulting mixture was stirred at 100°C for 6 hours, cooled to room temperature, diluted with water (15.0 mL), and extracted with dichloromethane (20.0 mL × 2). The organic phases were combined, washed with saturated brine (20.0 mL), dried over anhydrous sodium sulfate, filtered, and concentrated to dryness under reduced pressure to obtain 8-methoxyquinoline-1-oxide-2-*d* (116c) (0.80 g, yield 95%).

At 0°C, phosphorus tribromide (0.85 mL, 9 mmol) was slowly added dropwise to a solution of 8-methoxyquinoline-1-oxide-2-*d* (116c) (0.797 g, 4.52 mmol) in N,N-dimethylformamide (10.0 mL). The reaction solution was stirred at room temperature for 2 hours, and cooled to 0°C. The pH of the aqueous phase was adjusted to about 8 to 9 by the slow addition of saturated aqueous sodium carbonate solution. The resulting mixture was extracted with dichloromethane (20.0 mL × 2). The organic phases were combined, washed with saturated brine (20.0 mL), dried over anhydrous sodium sulfate, filtered, and concentrated under reduced pressure. The resulting residue was purified by silica gel column chromatography (gradient elution, eluent: petroleum ether/ethyl acetate = 100%/0 to 1/1.5) to obtain 8-methoxyquinoline-2-d (116d) (0.418 g, yield 58%).

At 0°C, a solution of boron tribromide in dichloromethane (1.0 M) (13.0 mL, 13.0 mmol) was slowly added dropwise to a solution of 8-methoxyquinoline-2-*d* (116d) (0.418 g, 2.6 mmol) in dichloromethane (3.0 ml). The reaction mixture was warmed up to room temperature, and stirred for 16 hours. The pH of the aqueous phase was adjusted to about 8 to 9 by the slow addition of saturated aqueous sodium carbonate solution. The resulting mixture was extracted with dichloromethane (10.0 mL × 3). The organic phases were combined, dried over anhydrous sodium sulfate, filtered, and concentrated under reduced pressure. The resulting residue was purified by silica gel column chromatography (eluent: dichloromethane/methanol = 30/1) to obtain 8-hydroxyquinoline-2-d (116e) (0.203 g, yield 53%).

At room temperature, sodium nitrite (141.0 mg, 2.0 mmol) was added to a solution of 8-hydroxyquinoline-2-d (116e) (100.0 mg, 0.68 mmol) in acetic acid (3.0 mL). The reaction mixture was stirred for 2 hours, and filtered under reduced pressure. The pH of the aqueous phase of the filtrate was adjusted to about 9 to 10 with ammonia (25 to 28 weight%, NH₃ content), and the mixture was filtered under reduced pressure. The resulting filter cake was dissolved in methanol (2.0 mL), and purified by reversed-phase high performance liquid chromatography (the chromatographic column was Eclipse XDB-C18 (21.2 mm×250 mm, 7µm), the mobile phase was water/acetonitrile=100%/0 - 92%/8%, gradient elution, 0.1% formic acid was added to the mobile phase by volume percentage, and the flow rate was 20.0 mL/min) to obtain 7-nitroquinolin-2-d-8-ol (116) (30.0 mg, yield 23%).

¹H NMR (400 MHz, DMSO-*d₆*) δ:9.20 (d, *J* = 8.8 Hz, 1H), 8.57 (d, *J* = 8.9 Hz, 1H), 7.90 (d, *J* = 8.9 Hz, 1H), 7.18 (d, *J* = 8.8 Hz, 1H).

MS calculated: 191.04; MS found: 192.2 [M+H]⁺.

### Example 117

### Synthesis of 7-nitroquinolin-6-d-8-ol (117)

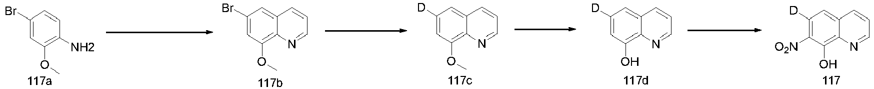

At room temperature, 4-bromo-2-methoxyaniline (117a) (1.0 g, 5.0 mmol) was added to hydrochloric acid (6.0 M) (30.0 mL). The reaction mixture was warmed up to 110°C, followed by the slowly dropwise addition of acrolein diethyl acetate (2.44 mL, 15.0 mmol), and stirred for 10 hours. The reaction mixture was cooled to room temperature, and the pH of the aqueous phase was adjusted to about 9 to 10 by the slow addition of saturated aqueous sodium carbonate solution. The resulting mixture was extracted with ethyl acetate (30.0 mL × 2). The organic phases were combined, dried over anhydrous sodium sulfate, filtered, and concentrated under reduced pressure. The resulting residue was purified by silica gel column chromatography (eluent: petroleum ether/ethyl acetate = 1/1) to obtain 6-bromo-8-methoxyquinoline (117b) (0.5 g, yield 42%).

At room temperature, 6-bromo-8-methoxyquinoline (117b) (119.0 mg, 0.5 mmol), potassium methoxide (70.0 mg, 1.0 mmol) and hexamethyldisilane (146.0 mg, 1.0 mmol) were added successively to deuterated acetonitrile (10.0 mL). The reaction mixture was stirred at room temperature for 15 hours, concentrated to dryness under reduced pressure, followed by the addition of water (30.0 mL). The resulting mixture was extracted with ethyl acetate (30.0 mL × 2). The organic phases were combined, dried over anhydrous sodium sulfate, filtered, and concentrated under reduced pressure. The resulting residue was purified by silica gel column chromatography (eluent: petroleum ether/ethyl acetate = 1/1) to obtain 8-methoxyquinoline-6-*d* (117c) (60.0 mg, yield 75%).

At 0°C, a solution of boron tribromide in dichloromethane (1.0 M) (3.0 mL, 3.0 mmol) was slowly added dropwise to a solution of 8-methoxyquinoline-6-*d* (117c) (60.0 mg, 0.38 mmol) in dichloromethane (1.0 ml). The reaction solution was warmed up to room temperature and stirred for 30 minutes. The pH of the aqueous phase was adjusted to about 8 to 9 with saturated aqueous sodium bicarbonate solution. The organic phase was separated, and the aqueous phase was extracted with dichloromethane (10.0 mL × 2). The combined organic phase was dried over anhydrous sodium sulfate, filtered, and concentrated to dryness under reduced pressure to obtain the crude product quinolin-6-*d*-8-ol (117d) (51.0 mg, crude yield 93%).

At 0°C, sodium nitrite (75.0 mg, 1.1 mmol) was added to a suspension of the crude quinolin-6-*d*-8-ol (117d) (51.0 mg, 0.35 mmol) in acetic acid (1.0 mL). The reaction mixture was stirred at 0°C for 1 hour, at room temperature for 18 hours, and filtered under reduced pressure. The resulting filter cake was added to methanol (1.0 mL), stirred for 10 minutes, and filtered under reduced pressure. The resulting filter cake was washed with methanol (0.5 mL × 2), and dried under vacuum to obtain 7-nitroquinolin-6-d-8-ol (117) (34.0 mg, yield 51%).

¹H NMR (400 MHz, DMSO-*d₆*) δ: 9.44 (dd, *J*= 8.8, 1.6 Hz, 1 H), 8.62 (dd, *J*= 4.0, 1.6 Hz, 1 H), 7.59 (dd, *J*= 8.6, 4.0 Hz, 1 H), 6.29 (s, 1 H).

MS calculated: 191.04; MS found: 192.0 [M+H]⁺.

### Example 118

### Synthesis of 5-chloro-4-(3,3-difluoropiperidin-1-yl)-7-nitroquinolin-8-ol (118)

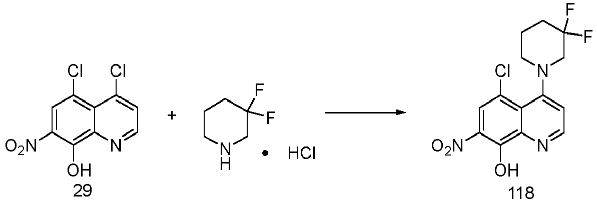

At room temperature, 4,5-dichloro-7-nitroquinolin-8-ol (29) (150.0 mg, 0.51 mmol) and 3,3-difluoroperidine hydrochloride (150.0 mg, 0.95 mmol) were added to N,N-dimethylformamide (4.0 mL). The resulting mixture was stirred at 130°C for 5 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/methanol=100%/0 - 40%/60%, gradient elution, and the flow rate was 20.0 mL/min) to obtain 5-chloro-4-(3,3-difluoropiperidin-1-yl)-7-nitroquinolin-8-ol (118) (30.0 mg, yield 17%).

¹H NMR (400 MHz, DMSO-*d₆*) δ: 8.44 (d, *J* = 8 Hz, 1 H), 7.99 (s, 1 H), 7.50 (d, *J* = 8 Hz, 1 H), 4.13-4.00 (m, 1 H), 3.94-3.83 (m, 1 H), 3.70-3.63 (m, 2 H), 2.30-2.10 (m, 2 H), 2.06-1.96 (m, 1 H), 1.88-1.78 (m, 1 H).

MS calculated: 343.05; MS found: 344.0, 346.0 [M+H]⁺.

### Example 119

### Synthesis of 5-chloro-4-(3-fluoropiperidin-1-yl)-7-nitroquinolin-8-ol (119)

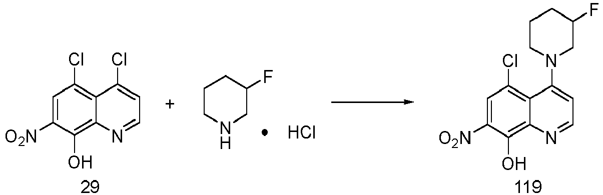

At room temperature, 4,5-dichloro-7-nitroquinolin-8-ol (29) (104.0 mg, 0.4 mmol) and 3-fluoroperidine hydrochloride (112.0 mg, 0.8 mmol) were added to N,N-dimethylformamide (4.0 mL). The resulting mixture was stirred at 130°C for 5 hours, cooled to room temperature, and concentrated under reduced pressure to remove the organic solvent. The resulting residue was purified by reversed-phase high performance liquid chromatography (the chromatographic column was Eclipse XDB-C18 (21.2 mm×250 mm, 7µm), the mobile phase was water/methanol=100%/0 - 40%/60%, gradient elution, and the flow rate was 20.0 mL/min) to obtain 5-chloro-4-(3-fluoropiperidin-1-yl)-7-nitroquinolin-8-ol (119) (26.4 mg, yield 20%).

¹H NMR (400 MHz, DMSO-*d₆*) δ: 8.44 (d, *J* = 8.0 Hz, 1 H), 7.99 (s, 1 H), 7.50 (d, *J* = 8.0 Hz, 1 H), 4.13-4.00 (m, 1 H), 3.94-3.83 (m, 1 H), 3.70-3.63 (m, 3 H), 2.30-2.10 (m, 2 H), 2.06-1.96 (m, 1 H), 1.88-1.78 (m, 1 H).

MS calculated: 325.06; MS found: 326.0, 328.0 [M+H]⁺.

### Example 120

### Synthesis of 5-chloro-7-nitroquinolin-4-d-8-ol (120)

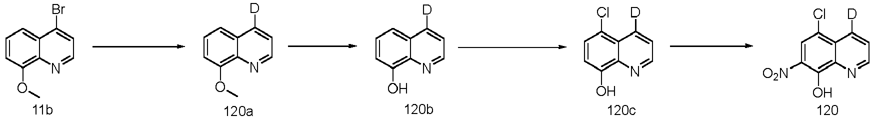

At room temperature, potassium methoxide (177.0 mg, 2.52 mmol) and hexamethyldisilane (369.0 mg, 2.52 mmol) were added successively to a solution of 4-bromo-8-methoxyquinoline (11b) (300.0 mg, 1.26 mmol) in deuterated acetonitrile (2.5 mL). The reaction mixture was stirred at room temperature for 16 hours, diluted with water (10.0 mL), and extracted with ethyl acetate (10.0 mL × 2). The organic phases were combined, washed with saturated brine (10.0 mL), dried over anhydrous sodium sulfate, filtered, and concentrated under reduced pressure. The resulting residue was purified by silica gel column chromatography (gradient elution, eluent: petroleum ether/ethyl acetate = 100%/0 to 4/1) to obtain 8-methoxyquinoline-4-*d* (120a) (125.0 mg, yield 62%).

At 0°C, a solution of boron tribromide in dichloromethane (1.0 M) (4.0 mL, 4.0 mmol) was slowly added dropwise to a solution of 8-methoxyquinoline-4-*d* (120a) (125.0 mg, 0.78 mmol) in dichloromethane (2.0 ml). The reaction solution was warmed up to room temperature, and stirred for 16 hours. The pH of the aqueous phase was adjusted to about 8 to 9 by the slow addition of saturated aqueous sodium carbonate solution. The organic phase was separated, and the aqueous phase was extracted with dichloromethane (10.0 mL × 2). The organic phases were combined, dried over anhydrous sodium sulfate, filtered, and concentrated under reduced pressure. The resulting residue was purified by silica gel column chromatography (gradient elution, eluent: dichloromethane/methanol = 100%/0 to 30/1) to obtain quinolin-4-*d*-8-ol (120b) (100.0 mg, yield 88%).

At 0°C, N-chlorosuccinimide (86.8 mg, 0.65 mmol) was added in batches to a solution of quinolin-4-*d*-8-ol (120b) (100.0 mg, 0.68 mmol) in sulfuric acid (98 weight%) (2.0 mL). The reaction mixture was stirred at 0°C for 0.5 hour, at room temperature for 5 hours, and then cooled to 0°C. The pH of the aqueous phase was adjusted to about 8 to 9 by the slow addition of saturated aqueous sodium carbonate solution. The resulting mixture was extracted with dichloromethane (10.0 mL × 3). The organic phases were combined, washed with saturated brine (10.0 mL), dried over anhydrous sodium sulfate, filtered, and concentrated under reduced pressure. The resulting residue was purified by silica gel column chromatography (gradient elution, eluent: dichloromethane/methanol = 100%/0 to 30/1) to obtain 5-chloroquinolin-4-*d*-8-ol (120c) (86.0 mg, yield 70%).

At room temperature, sodium nitrite (99.0 mg, 1.43 mmol) was added to a solution of 5-chloroquinolin-4-*d*-8-ol (120c) (86.0 mg, 0.48 mmol) in acetic acid (2.0 mL). The reaction mixture was stirred for 3 hours and filtered under reduced pressure, and the filter cake was washed with ethyl acetate (1.0 mL × 2). The resulting filtrate was left to stand at room temperature for 16 hours, and a solid precipitated. The resulting mixture was filtered under reduced pressure, the filter cake was washed with water (0.5 mL × 2), and dried under vacuum to obtain 5-chloro-7-nitroquinolin-4-*d*-8-ol (120) (24.0 mg, yield 22%).

¹H NMR (400 MHz, DMSO-*d₆*) δ: 9.13 (d, J = 4.2 Hz, 1H), 8.25 (s, 1H), 7.98 (d, J = 4.2 Hz, 1H).

MS calculated: 225.01; MS found: 226.0 [M+H]⁺.

### Example 121

### Synthesis of 5-bromo-7-nitroquinolin-8-ol (121)

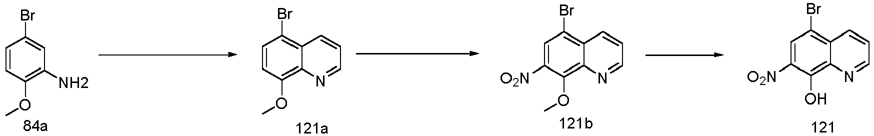

At room temperature, 5-bromo-2-methoxyaniline (84a) (2.0 g, 9.9 mmol) was added to hydrochloric acid (6.0 M) (40.0 mL). The reaction mixture was warmed up to 110°C, followed by the slowly dropwise addition of acrolein diethyl acetate (4.88 mL, 30 mmol), and stirred for 6 hours. The reaction mixture was cooled to room temperature, and the pH of the aqueous phase was adjusted to about 9 to 10 by the slow addition of saturated aqueous sodium carbonate solution. The resulting mixture was extracted with ethyl acetate (30.0 mL × 2). The organic phases were combined, dried over anhydrous sodium sulfate, filtered, and concentrated under reduced pressure. The resulting residue was purified by silica gel column chromatography (eluent: petroleum ether/ethyl acetate = 3/1) to obtain 5-bromo-8-methoxyquinoline (121a) (0.84 g, yield 36%).

At 0°C, nitric acid (65 weight%) (1.0 mL, 23.6 mmol) was slowly added dropwise to a solution of 5-bromo-8-methoxyquinoline (121a) (200.0 mg, 0.84 mmol) in acetic anhydride (2.0 mL), and stirred for 10 minutes. Sulfuric acid (98 weight%) (0.2 mL, 3.7 mmol) was slowly added dropwise. The reaction mixture was stirred at 0°C for 30 minutes, at room temperature for 18 hours, and then cooled to 0°C. The pH of the aqueous phase was adjusted to about 9 to 10 by the addition of aqueous sodium hydroxide solution (1.0 M). The resulting mixture was extracted with dichloromethane (10.0 mL × 3). The organic phases were combined, dried over anhydrous sodium sulfate, filtered, and concentrated under reduced pressure. The resulting residue was purified by silica gel column chromatography (eluent: petroleum ether/ethyl acetate = 4/1) to obtain 5-bromo-8-methoxy-7-nitroquinoline (121b) (60.0 mg, yield 25%).

At room temperature, 5-bromo-8-methoxy-7-nitroquinoline (121b) (60.0 mg, 0.21 mmol) and lithium chloride (89.0 mg, 2.1 mmol) were added to N,N-dimethylformamide (2.0 mL). The reaction mixture was stirred at 120°C for 1 hour, cooled to room temperature, and concentrated under reduced pressure to remove the organic solvent. Water (3.0 mL) was added to the resulting residue, stirred for 3 minutes, and filtered under reduced pressure. The filter cake was washed with water (0.5 mL × 2), and dried under vacuum to obtain 5-bromo-7-nitroquinolin-8-ol (121) (40.0 mg, yield 71%).

¹H NMR (400 MHz, DMSO-*d₆*) δ: 8.69 (d, *J* = 4.4 Hz, 1H), 8.23 (s, 1 H), 8.21-8.19 (m,1H), 7.68 (dd, *J* = 8.4, 4.4 Hz, 1 H).

MS calculated: 267.95; MS found: 269.0, 271.0 [M+H]⁺.

### Example 122

### Synthesis of 6-fluoro-7-nitroquinolin-8-ol (122)

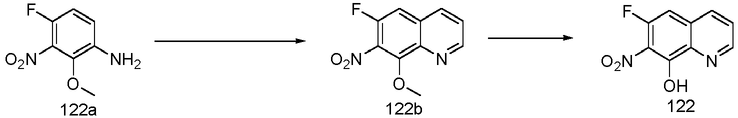

At room temperature, 2-methoxy-3-nitro-4-fluoroaniline (122a) (100.0 mg, 0.537 mmol) was added to hydrochloric acid (6.0 M) (10.0 mL). The reaction mixture was warmed up to 110°C, followed by the slowly dropwise addition of acrolein diethyl acetate (0.25 mL, 1.61 mmol), and stirred for 2 hours. The reaction mixture was cooled to room temperature, and the pH of the aqueous phase was adjusted to about 7 to 8 with aqueous sodium hydroxide solution (6.0 M). The resulting mixture was extracted with dichloromethane (15.0 mL × 3). The organic phases were combined, dried over anhydrous sodium sulfate and filtered, and the filtrate was concentrated under reduced pressure. The resulting residue was purified by silica gel column chromatography (gradient elution, eluent: petroleum ether/ethyl acetate = 100%/0 to 7/1) to obtain 6-fluoro-7-nitro-8-methoxyquinoline (122b) (53.0 mg, yield 45%).

At room temperature, 6-fluoro-7-nitro-8-methoxyquinoline (122b) (53.0 mg, 0.24 mmol) and lithium chloride (100.0 mg, 2.4 mmol) were added to N,N-dimethylformamide (2.0 mL). The reaction mixture was stirred at 130°C for 1 hour, cooled to room temperature, and concentrated under reduced pressure to remove the organic solvent. Water (2.0 mL) was added to the resulting residue, stirred for 3 minutes, and filtered under reduced pressure. The filter cake was washed with water (0.5 mL × 2), and dried under vacuum to obtain 6-fluoro-7-nitroquinolin-8-ol (122) (46.0 mg, yield 93%).

¹H NMR (400 MHz, DMSO-*d₆*) δ: 9.13 (d, *J* = 8.7 Hz, 1H), 8.58 (d, *J* = 3.6 Hz, 1H), 7.63 (dt, *J* = 17.0, 8.5 Hz, 1H), 6.14 (d, *J* = 18.0 Hz, 1H).

MS calculated: 208.03; MS found: 209.1 [M+H]⁺.

### Example 123

### Synthesis of 6-chloro-5-fluoro-7-nitroquinolin-8-ol (123)

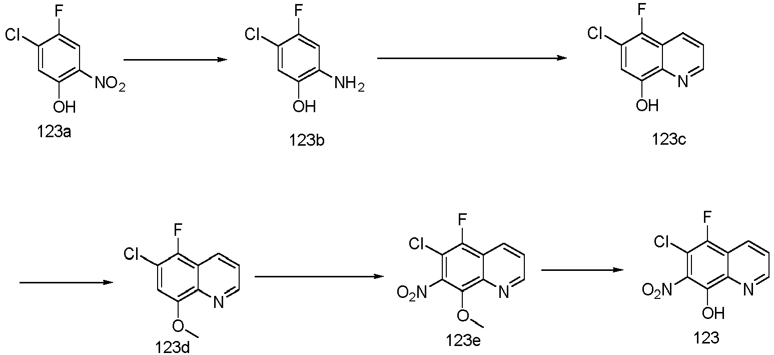

At room temperature, 2-nitro-4-fluoro-5-chlorophenol (123a) (1.92 g, 10.0 mmol) was added to a mixture of tetrahydrofuran (60.0 mL) and water (60.0 mL). Sodium dithionite (85 weight%) (12.3 g, 60.0 mmol) was added in batches. The reaction mixture was stirred at room temperature for 1 hour, and extracted with ethyl acetate (30.0 mL x 3). The organic phases were combined, washed with saturated brine (20.0 mL), dried over anhydrous sodium sulfate, filtered, and concentrated under reduced pressure. The resulting residue was purified by silica gel column chromatography (gradient elution, eluent: petroleum ether/ethyl acetate = 100%/0 to 3/1) to obtain 2-amino-4-fluoro-5-chlorophenol (123b) (0.892 g, yield 55%).

At room temperature, 2-amino-4-fluoro-5-chlorophenol (123b) (0.892 g, 5.5 mmol), sodium 3-nitrobenzene sulfonate (1.48 g, 6.57 mmol) and glycerol (1.27 g, 13.8 mmol) were added successively to sulfuric acid (70 weight%) (8.0 mL). The resulting mixture was stirred at 140°C for 4 hours, and cooled to room temperature. The pH of the aqueous phase was adjusted to about 8 to 9 with aqueous sodium hydroxide solution (6.0 M). The resulting mixture was extracted with dichloromethane (20.0 mL x 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 100%/0 to 7/1) to obtain 5-fluoro-6-chloroquinolin-8-ol (123c) (0.411 g, yield 38%).

At room temperature, methyl iodide (0.17 mL, 2.73 mmol) was slowly added dropwise to a suspension of 5-fluoro-6-chloroquinolin-8-ol (123c) (0.411 g, 2.1 mmol) and potassium carbonate (0.58 g, 4.2 mmol) in N,N-dimethylformamide (6.0 mL). The resulting mixture was stirred for 16 hours, and concentrated under reduced pressure to remove the organic solvent. Water (30.0 mL) was added to the resulting residue, and extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel column chromatography (gradient elution, eluent: petroleum ether/ethyl acetate = 100%/0 to 3/1) to obtain 5-fluoro-6-chloro-8-methoxyquinoline (123d) (0.412 g, yield 94%).

At 0°C, nitric acid (65 weight%) (0.8 mL, 11.7 mmol) was slowly added dropwise to a solution of 5-fluoro-6-chloro-8-methoxyquinoline (123d) (0.412 g, 1.95 mmol) in acetic anhydride (10.0 mL), and stirred for 10 minutes. Sulfuric acid (98 weight%) (0.12 mL, 2.25 mmol) was slowly added dropwise. The reaction mixture was warmed up to room temperature, stirred for 16 hours, and cooled to 0°C. The pH of the aqueous phase was adjusted to about 8 to 9 with aqueous sodium hydroxide solution (6.0 M). The resulting mixture was extracted with dichloromethane (20.0 mL x 3). The organic phases were combined, washed with saturated brine (20.0 mL), dried over anhydrous sodium sulfate, filtered, and concentrated under reduced pressure. The resulting residue was purified by silica gel column chromatography (gradient elution, eluent: petroleum ether/ethyl acetate = 100%/0 to 5/1) to obtain 5-fluoro-6-chloro-7-nitro-8-methoxyquinoline (123e) (84.0 mg, yield 17%).

At room temperature, 5-fluoro-6-chloro-7-nitro-8-methoxyquinoline (123e) (84.0 mg, 0.39 mmol) and lithium chloride (162.0 mg, 3.9 mmol) were added to N,N-dimethylformamide (2.0 mL). The reaction mixture was stirred at 130°C for 1 hour, cooled to room temperature, and concentrated under reduced pressure to remove the organic solvent. Water (2.0 mL) was added to the resulting residue, stirred for 3 minutes, and filtered under reduced pressure. The resulting filter cake was washed with water (0.5 mL x 2), and dried under vacuum to obtain 5-fluoro-6-chloro-7-nitroquinolin-8-ol (123) (77.0 mg, yield 82%).

¹H NMR (400 MHz, DMSO-*d₆*) δ: 8.69 (d, *J* = 3.2 Hz, 1H), 8.25 (d, *J* = 8.3 Hz, 1H), 7.63 (dt, *J* = 12.8, 6.4 Hz, 1H).

MS calculated: 241.99; MS found: 243.1 [M+H]⁺.

### Example 124

### Synthesis of 6-bromo-5-fluoro-7-nitroquinolin-8-ol (124)

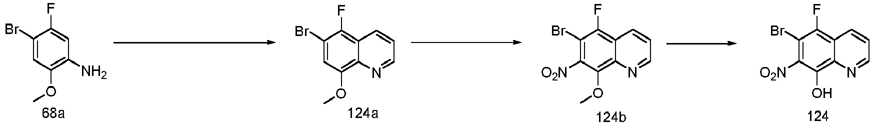

At room temperature, 4-bromo-5-fluoro-2-methoxyaniline (68a) (0.396 g, 1.8 mmol), sodium 3-nitrobenzene sulfonate (0.486 g, 2.16 mmol) and glycerol (0.33 mL, 4.5 mmol) were added successively to sulfuric acid (70 weight%) (2.7 mL). The resulting mixture was stirred at 140°C for 3 hours, and cooled to room temperature. The pH of the aqueous phase was adjusted to about 8 to 9 with aqueous sodium hydroxide solution (1.0 M). The resulting mixture was extracted with dichloromethane (10.0 mL x 3). The organic phases were combined, dried over anhydrous sodium sulfate, filtered, and concentrated under reduced pressure. The resulting residue was purified by silica gel column chromatography (gradient elution, eluent: petroleum ether/ethyl acetate = 4/1 to 2/1) to obtain 6-bromo-5-fluoro-8-methoxyquinoline (124a) (0.418 g, yield 91%).

At 0°C, nitric acid (65 weight%) (0.58 mL, 9.6 mmol) was slowly added dropwise to a solution of 6-bromo-5-fluoro-8-methoxyquinoline (124a) (0.246 g, 0.96 mmol) in acetic anhydride (4.8 mL), and stirred for 10 minutes. Sulfuric acid (98 weight%) (0.104 mL, 1.92 mmol) was slowly added dropwise. The reaction mixture was stirred at 0°C for 3 hours, at room temperature for 3 hours, and then cooled to 0°C. The pH of the aqueous phase was adjusted to about 8 to 9 with aqueous sodium hydroxide solution (1.0 M). The resulting mixture was extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel column chromatography (eluent: petroleum ether/ethyl acetate = 6/1) to obtain 6-bromo-5-fluoro-8-methoxy-7-nitroquinoline (124b) (78.0 mg, yield 27%).

At room temperature, 6-bromo-5-fluoro-8-methoxy-7-nitroquinoline (124b) (68.3 mg, 0.23 mmol) and lithium chloride (97.5 mg, 2.3 mmol) were added to N,N-dimethylformamide (0.46 mL). The reaction mixture was stirred at 130°C for 45 minutes, cooled to room temperature, and concentrated under reduced pressure to remove the organic solvent. Water (2.0 mL) was added to the resulting residue, stirred for 3 minutes, and filtered under reduced pressure. The resulting filter cake was washed with water (1.0 mL x 3), and dried under vacuum to obtain 6-bromo-5-fluoro-7-nitroquinolin-8-ol (124) (56.7 mg, yield 86%).

¹H NMR (400 MHz, DMSO-*d₆*) δ: 8.71 (d, *J* = 3.2 Hz, 1H), 8.26 (d, *J* = 8.4 Hz, 1H), 7.64 (dd, *J* = 8.4, 4.0 Hz, 1H).

MS calculated: 285.94; MS found: 287.0, 289.0 [M+H]⁺.

### Example 125

### Synthesis of 5,6-difluoro-7-nitroquinolin-8-ol (125)

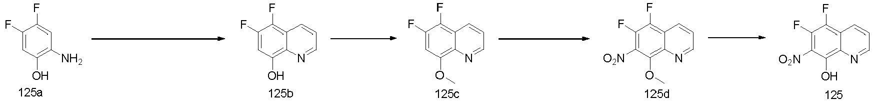

At room temperature, 2-amino-4,5-difluorophenol (125a) (0.615 g, 4.24 mmol), sodium 3-nitrobenzene sulfonate (1.432 g, 6.36 mmol) and glycerol (1.25 mL, 16.96 mmol) were added successively to sulfuric acid (70 weight%) (5.42 mL). The resulting mixture was stirred at 140°C for 8 hours, and cooled to room temperature. The pH of the aqueous phase was adjusted to about 8 to 9 with aqueous sodium hydroxide solution (1.0 M). The resulting mixture was extracted with dichloromethane (10.0 mL x 3). The organic phases were combined, dried over anhydrous sodium sulfate, filtered, and concentrated under reduced pressure. The resulting residue was purified by silica gel column chromatography (eluent: petroleum ether/ethyl acetate = 1.5/1) to obtain 5,6-difluoroquinolin-8-ol (125b) (0.603 g, yield 78%).

At room temperature, methyl iodide (0.41 mL, 6.66 mmol) was slowly added dropwise to a suspension of 5,6-difluoroquinolin-8-ol (125b) (0.603 g, 3.33 mmol) and potassium carbonate (1.381 g, 9.99 mmol) in N,N-dimethylformamide (13.3 mL). The resulting mixture was stirred for 16 hours, and concentrated under reduced pressure to remove the organic solvent. Water (20.0 mL) was added to the resulting residue, and extracted with dichloromethane (20.0 mL x 3). The organic phases were combined, washed with saturated brine (10.0 mL), dried over anhydrous sodium sulfate, filtered, and concentrated under reduced pressure. The resulting residue was purified by silica gel column chromatography (eluent: dichloromethane/ethyl acetate = 40/1) to obtain 5,6-difluoro-8-methoxyquinoline (125c) (0.533 g, yield 82%).

At 0°C, nitric acid (65 weight%) (1.79 mL, 27.3 mmol) was slowly added dropwise to a solution of 5,6-difluoro-8-methoxyquinoline (125c) (0.533 g, 2.73 mmol) in acetic anhydride (13.6 mL), and stirred for 10 minutes. Sulfuric acid (98 weight%) (0.30 mL, 5.46 mmol) was slowly added dropwise. The reaction mixture was stirred at 0°C for 5 minutes, at room temperature for 7 hours, and then cooled to 0°C. The pH of the aqueous phase was adjusted to about 8 to 9 with aqueous sodium hydroxide solution (1.0 M). The resulting mixture was extracted with dichloromethane (10.0 mL x 3). The organic phases were combined, washed with saturated brine (10.0 mL), dried over anhydrous sodium sulfate, filtered, and concentrated under reduced pressure. The resulting residue was purified by silica gel column chromatography (eluent: petroleum ether/dichloromethane = 1.2/1) to obtain 5,6-difluoro-8-methoxy-7-nitroquinoline (125d) (0.0485 g, yield 7%).

At room temperature, 5,6-difluoro-8-methoxy-7-nitroquinoline (125d) (48.5 mg, 0.20 mmol) and lithium chloride (84.8 mg, 2.0 mmol) were added to N,N-dimethylformamide (0.40 mL). The reaction mixture was stirred at 120°C for 45 minutes, cooled to room temperature, and concentrated under reduced pressure to remove the organic solvent. A mixed solvent of ethanol and water (volume ratio, ethanol/water = 1/2) (1.5 mL) was added to the resulting residue, stirred for 5 minutes, and filtered under reduced pressure. The filter cake was washed with a mixed solvent of ethanol and water (volume ratio, ethanol/water = 1/2) (0.5 mL x 3), and dried under vacuum to obtain 5,6-difluoro-7-nitroquinolin-8-ol (125) (41.5 mg, yield 92%).

¹H NMR (400 MHz, DMSO-*d₆*) δ: 8.66 (d, *J* = 2.4 Hz, 1H), 8.24 (dd, *J* = 8.4, 1.2 Hz, 1H), 7.64 (dd, *J* = 8.4, 4.4 Hz, 1H).

MS calculated: 226.02 ; MS found: 227.1 [M+H]⁺.

### Biological Assay

### Test Example 1 Determination of the inhibitory activity of the compounds of the present invention on cancer cells

### Experimental materials, reagents and test methods

### 1. Cell Lines

HUVEC (purchased from Allcells Biological Technology (Shanghai) Co., Ltd.); UM-UC-3 (ATCC); LNCaP (ATCC); RM-1 (purchased from Nanjing Cobioer Biosciences Co., Ltd.)

### 2. Reagents

Endothelial cell complete medium (Allcells, H-004)
Medium RPMI1640 (ATCC, 30-2001)
Medium EMEM (ATCC, 30-2003)
Medium DMEM (Gibco, 12430-054)
Fetal bovine serum (Gibco, 10091148)
Penicillin-streptomycin double anti-body (Gibco, S110JV)
DPBS (Gibco, 14190-144)
CellTiter-Glo^{®} assay kit (Promega, G7573)
Trypsin (0.25%), phenol red (Gibco, 25200-056)
DMSO (Sigma, D2650)

### 3. Instruments

Biological safety cabinet (Suzhou Antai Airtech Co., Ltd., BSC-1300A II)
Inverted microscope (Nikon, CKX53)
Automated cell counter (Life Technologies, countess II)
Multifunctional microplate reader (Biotek, H1FM)
Shaker (Hangzhou Allsheng Instruments Co., Ltd., OS-100)
XI,FIT 5.3 (Inforstack Shanghai Co., Ltd.)

### 4. Test Method

In this test method, the following experiments were carried out in a biological safety cabinet, and the specific steps are as follows:
(1) On the first day, the cells were seeded in a 96-well plate, and the previous medium was poured off. 5 mL of DPBS was added to wash the cells, and then removed by a pipette and discarded. Then, 1 mL of trypsin (0.25%) was added, and the plate was placed in a cell culture incubator at 37°C, 5% CO₂ for digestion, and taken out after about 2 to 5 minutes. New corresponding medium was added, and pipetted to resuspend the cells evenly, and the cells were then counted with an automated cell counter.

The digestion was observed using an inverted microscope.

Regarding HUVEC, the medium used was endothelial cell complete medium; regarding UM-UC-3, the medium used was 89 v% EMEM+10 v% fetal bovine serum+1 v% penicillin-streptomycin double anti-body; regarding LNcaP, the medium used was 89 v% RPM11640+10 v% fetal bovine serum+1 v% penicillin-streptomycin double anti-body; and regarding RM1, the medium used was 89 v% DMEM+10 v% fetal bovine serum+1 v% penicillin-streptomycin double anti-body.

(2) HUVEC cells were seeded in columns 1 to 11 of a 96-well plate (Coming, Cat. No. 3610) at a density of 2000 cells per well in 100 µL of medium, and the medium free of cell was added to the wells of column 12. The other three types of cells were also seeded, wherein UM-UC-3 and RM-1 cells were seeded at a density of 3000 cells per well, and LNCap cells were seeded at a density of 5000 cells per well. Then, the plate was placed in an incubator at 37°C, 5% CO₂ for 24 hours.

(3) On the second day, the compounds of the present invention and the reference drug nitroxoline (obtained according to the preparation method disclosed in the prior art) were diluted in gradient (2.5 folds, started at 100 µmol) to obtain 8 dose points (there were a total of 8 columns of wells; the first column of wells had the highest concentration, the concentration decreased in turn, and the last column of wells had the lowest concentration). 10 µL of the compound diluted in gradient was added to the 100 µL of cells in column 1 to column 10 with an 8-channel pipette. 10 µL of the corresponding medium containing 0.33 v% DMSO was added to column 11 and column 12. The plate was incubated in an incubator at 37°C, 5% CO₂ for 72 hours (note: column 11 was used as MAX well with cells and no compounds; and column 12 was used as MIN well with no cells and no compounds).

(4) After treating with the compounds of the present invention for 72 hours, the cell viability detection reagents in the above CellTiter-Glo^{®} assay kit were added to the 96-well plate at 50 µL per well according to the instructions. The plate was shaken on a shaker in the dark for 5 to 10 minutes, and then the cell activity was determined by a multifunctional microplate reader. Finally, the growth inhibition of the compounds of the present invention on the cells was plotted by XI,FIT 5.3 software, and the IC₅₀ values of the compounds of the present invention were calculated.

The IC₅₀ values of the compounds of the present invention for inhibition against various cancer cells are shown in Table 1 below.

**Table 1 IC₅₀ values of the compounds of the present invention for inhibition of cancer cells**

| Example | **IC₅₀ (µM)** | | | |
|---|---|---|---|---|
| | Endothelial cell line | Bladder cancer cell line | Prostate cancer cell line | Mouse prostate cancer cell line |
| | HUVEC | UM-UC-3 | LNcaP | RM1 |
| Reference Nitroxoline | 4.5 | 9.2 | 5.1 | 4.3 |
| 1 | 14.9 | 9.33 | 10.92 | 8.13 |
| 2 | 7.06 | 3.88 | 5.97 | / |
| 8 | 5.7 | 3.77 | 9.30 | 3.18 |
| 11 | 2.97 | 2.71 | 1.67 | 3.42 |
| 12 | 7.50 | 7.50 | 4.10 | / |
| 13 | >50 | 38.00 | 35.40 | / |
| 14 | 10.90 | 6.10 | 8.70 | / |
| 15 | 32.3 | 36.2 | 35.20 | / |
| 16 | 8.10 | 8.90 | 3.90 | / |
| 17 | 27.40 | 16.90 | 23.60 | / |
| 18 | 64.40 | 13.20 | 21.20 | / |
| 19 | >100 | >100 | >100 | / |
| 20 | 5.60 | 4.38 | 3.33 | 6.80 |
| 21 | 10.31 | 4.84 | 6.75 | 6.80 |
| 22 | 1.46 | 1.99 | 2.31 | 2.30 |
| 23 | >100 | >100 | >100 | >100 |
| 24 | >100 | 47.98 | 49.61 | 53.6 |
| 25 | 47.62 | 44.48 | 32.35 | 47.2 |
| 26 | 15.08 | 11.43 | 11.92 | 12.5 |
| 27 | 3.17 | 4.11 | 11.89 | 13.2 |
| 28 | 17.34 | 42.30 | 32.98 | 17.02 |
| 29 | 6.22 | 9.11 | 7.03 | 6.50 |
| 30 | >100 | >100 | >100 | >100 |
| 31 | 1.63 | 0.94 | 0.96 | 0.46 |
| 32 | 0.89 | 1.09 | 0.23 | 0.60 |
| 33 | 16.95 | 50.49 | 15.02 | 17.99 |
| 34 | 6.44 | 14.02 | 6.78 | 12.74 |
| 35 | 27.02 | 49.68 | 31.16 | >50 |
| 36 | 1.48 | 2.42 | 0.96 | 2.81 |
| 37 | >50 | >50 | 40.42 | >50 |
| 38 | 2.36 | 6.72 | 1.87 | 3.00 |
| 39 | 1.11 | 0.95 | 0.43 | 1.25 |
| 40 | 19.10 | 24.62 | 12.56 | 11.53 |
| 41 | 2.02 | 1.03 | 0.48 | 2.02 |
| 42 | 32.98 | 41.97 | 17.40 | 25.36 |
| 43 | 7.54 | 12.32 | 6.26 | 10.60 |
| 44 | >50 | >50 | >50 | >50 |
| 45 | 10.11 | 23.62 | 5.43 | 12.06 |
| 46 | 0.56 | 0.96 | 0.88 | 1.86 |
| 47 | 1.59 | 2.38 | 0.97 | 6.07 |
| 48 | 6.58 | 10.59 | 10.61 | 14.37 |
| 49 | 1.59 | 4.07 | 1.04 | 5.32 |
| 50 | 2.94 | 3.40 | 2.86 | 4.29 |
| 51 | 1.71 | 2.17 | 1.51 | 4.25 |
| 52 | 0.53 | 4.71 | 0.58 | 1.42 |
| 53 | 0.59 | 0.86 | 1.08 | 3.06 |
| 54 | 0.50 | 1.42 | 0.25 | 1.62 |
| 55 | 0.80 | 2.23 | 0.25 | 2.88 |
| 56 | 32.12 | 52.76 | 47.59 | 68.50 |
| 57 | 0.47 | 0.49 | 0.55 | 2.35 |
| 58 | 0.68 | 2.55 | 0.89 | 0.91 |
| 59 | 0.78 | 1.49 | 1.50 | 1.46 |
| 60 | 0.70 | 5.82 | 0.64 | 1.13 |
| 61 | 2.58 | 5.29 | 5.22 | 4.26 |
| 62 | 3.54 | 11.16 | 2.49 | 4.82 |
| 63 | 1.82 | 7.07 | 2.01 | 7.47 |
| 64 | 0.44 | 1.24 | 0.22 | 0.86 |
| 65 | 9.03 | 11.51 | 13.18 | 21.65 |
| 66 | 4.54 | 5.43 | 1.36 | 4.29 |
| 67 | 3.23 | 9.28 | 8.68 | 13.25 |
| 68 | 10.01 | 17.48 | 5.38 | 5.19 |
| 69 | 2.67 | 4.81 | 1.33 | 2.23 |
| 70 | 2.78 | 5.28 | 0.96 | 2.62 |
| 71 | 2.70 | 6.80 | 1.53 | 2.51 |
| 72 | 2.65 | 6.28 | 1.89 | 3.03 |
| 73 | 3.45 | 5.04 | 2.31 | 2.63 |
| 74 | 1.28 | 1.57 | 0.53 | 7.64 |
| 75 | 3.41 | 9.27 | 1.77 | 2.05 |
| 76 | 18.04 | 38.76 | 15.19 | 13.28 |

From the data in the above table, it can be seen that the compounds of the present invention have excellent anti-cancer activity, especially against endothelial cell line, bladder cancer cell line, prostate cancer cell line and mouse prostate cancer cell line. The inhibitory activity of the compounds of Examples 11, 22, 31, 32, 36, 38, 39, 41, 46, 52-55, 57 to 60, 64, and 69 to 73 of the present invention against four types of cancer cells is higher than that of the reference drug nitroxoline.

### Test Example 2 Determination of the inhibitory activity of the compounds of the present invention on bacteria

### 1. Test Drugs

The compounds of the Examples of the present invention

### 2. Test Bacteria

The test bacteria were one strain of clinically isolated carbapenem-resistant *Escherichia coli,* two strains of carbapenem-resistant *Acinetobacter baumannii,* two strains of methicillin-resistant *Staphylococcus aureus,* two strains of sensitive *Staphylococcus aureus,* and 7 strains of *Klebsiella pneumoniae.* Duplicate strains isolated from the same patient were eliminated. There were also ten quality control strains *Escherichia coli* ATCC25922, *Acinetobacter baumannii* ATCC19606, *Staphylococcus aureus* ATCC29213, *Klebsiella pneumoniae* ATCC BAA-1705, ATCC 43816, ATCC BAA-2472, ATCC-BAA-1898, ATCC-BAA-1899, NCTC 13440 and ATCC-BAA-2470. There were 24 strains in total.

### 3. Drug Susceptibility Test Method

According to the recommendations of relevant documents of the American Clinical and Laboratory Standards Institute (CLSI), the micro broth dilution method was used to determine the MIC of the compounds of the present invention against clinically isolated strains.

3.1 Formulation of antibacterial drug: All the compounds of the present invention were dissolved in DMSO. The drug concentration range was 128 mg/L to 0.06 mg/L.

3.2 Culture medium: The drug susceptibility test used cation adjusted Mueller-Hinton broth (CAMHB).

3.3 Amount of inoculum: The test bacteria cultured overnight were adjusted to 0.5 McFarland turbidity by the direct colony suspension method, and diluted 100 folds, the final inoculum amount was 10⁵ CFU/mL.

3.4 Culture conditions: The test bacteria were cultured at 35±2°C for 20 hours in air.

### 4. Interpretation and Judgment of Results

The drug susceptibility test results were interpretated by reference to CLSI 2019 M100 29^{th} edition criteria.

### 5. Quality Control Strains

The quality control strains of the drug susceptibility test were *Escherichia coli* ATCC 25922, *Acinetobacter baumannii* ATCC 19606, *Staphylococcus aureus* ATCC 29213, *Klebsiella pneumoniae* ATCC BAA-1705, ATCC 43816, ATCC BAA-2472, ATCC-BAA-1898, ATCC-BAA-1899, NCTC 13440 and ATCC-BAA-2470.

### 6. Statistics

The results of drug susceptibility test were statistically analyzed by WHONET software (version 5.6).

The MIC values of the compounds of the present invention for inhibition against various bacteria are shown in Table 2 below.

**Table 2 MIC values of the compounds of the present invention for inhibition against bacteria**

| No. | C1 | C2 | C3 | C4 | C5 | C6 | C7 | C8 | C9 | C10 | C11 | C12 |
|---|---|---|---|---|---|---|---|---|---|---|---|---|
| | *Escherichia coli* | | | *Acinetobacter baumannii* | | | *Klebsiella pneumoniae* | | | *Staphylococcus aureus* | | |
| | Standard strain ATCC 25922 | Clinical strain | | Standard strain ATCC 19606 | Clinical strain | | Standard strain ATCC 43816 | Standard strain | | Standard strain ATCC 29213 | Clinical strain | |
| | MIC | MIC50 | MIC90 | MIC | MIC50 | MIC90 | MIC | MIC5 0 | MIC9 0 | MIC | MIC50 | MIC90 |
| Nitroxoline | 4 | 8 | 16 | 2 | 4 | 4 | 4 | 8 | 16 | 4 | 4 | 4 |
| 1 | 8 | 16 | 16 | 16 | 4 | 8 | 8 | 16 | 64 | / | 4 | 4 |
| 2 | 8 | 16 | 64 | / | 8 | 8 | / | 32 | 64 | / | 4 | 4 |
| 3 | / | / | / | / | >64 | >64 | / | >64 | >64 | / | / | / |
| 4 | / | / | / | / | 32 | 32 | / | / | / | / | / | / |
| 5 | / | 32 | 32 | / | >64 | >64 | / | >64 | >64 | / | 64 | 64 |
| 6 | / | 64 | 64 | / | >64 | >64 | / | >64 | >64 | / | 64 | 64 |
| 7 | 64 | 64 | 128 | 64 | 32 | 64 | / | / | / | 16 | 8 | 16 |
| 8 | / | 4 | 4 | / | 8 | 16 | / | 32 | 32 | / | 8 | 8 |
| 9 | / | >64 | >64 | / | >64 | >64 | / | 64 | >64 | / | >64 | >64 |
| 10 | / | 32 | 32 | / | 32 | 64 | / | >64 | >64 | / | 16 | 16 |
| 11 | >128 | 8 | 128 | >128 | 16 | 128 | / | 64 | 64 | 4 | 8 | 8 |
| 12 | 4 | 4 | 4 | 4 | 2 | 4 | / | / | / | 2 | 2 | 2 |
| 13 | >128 | >128 | >128 | >128 | >128 | >128 | / | / | / | >128 | >128 | >128 |
| 14 | >128 | 128 | 128 | >128 | 128 | >128 | / | / | / | 128 | 128 | 128 |
| 15 | >128 | 128 | 128 | >128 | >128 | >128 | / | / | / | 128 | 64 | 128 |
| 16 | 32 | 16 | 16 | 32 | 8 | 8 | / | / | / | 8 | 8 | 8 |
| 17 | 128 | 64 | 64 | 128 | 128 | 128 | / | / | / | 128 | 128 | 128 |
| 18 | 128 | 128 | 128 | 128 | 128 | 128 | / | / | / | 128 | 128 | 128 |
| 19 | / | / | / | / | / | / | / | / | / | / | / | / |
| 20 | / | / | / | / | / | / | / | / | / | / | / | / |
| 21 | 8 | 8 | 32 | 32 | 32 | 64 | / | / | / | 8 | 4 | 8 |
| 22 | >128 | 128 | 128 | 128 | 128 | >128 | / | / | / | 2 | 2 | 32 |
| 23 | / | / | / | / | / | / | / | / | / | / | / | / |
| 24 | 128 | 128 | 128 | 128 | 128 | >128 | / | / | / | 128 | 128 | 128 |
| 25 | 32 | 128 | >128 | >128 | 128 | >128 | / | / | / | >128 | 16 | 16 |
| 26 | 128 | >128 | >128 | >128 | 64 | >128 | / | / | / | 128 | 16 | 64 |
| 27 | 64 | 128 | >128 | >128 | >128 | >128 | / | / | / | 64 | 64 | 64 |
| 28 | >128 | >128 | >128 | >128 | 64 | >128 | / | / | / | >128 | 32 | 32 |
| 29 | 4 | 4 | 8 | 2 | 2 | 4 | / | / | / | 1 | 0.5 | 1 |
| 30 | >128 | >128 | >128 | >128 | >128 | >128 | / | / | / | >128 | >128 | >128 |
| 31 | >128 | >128 | >128 | >128 | >128 | >128 | / | / | / | >128 | >128 | >128 |
| 32 | 64 | 64 | 64 | 32 | 32 | 32 | / | / | / | 1 | 1 | 1 |
| 33 | >128 | >128 | >128 | >128 | >128 | >128 | / | / | / | 32 | 32 | 32 |
| 34 | 8 | 8 | 32 | 32 | 32 | 32 | / | / | / | 4 | 4 | 8 |
| 35 | >128 | >128 | >128 | >128 | >128 | >128 | / | / | / | >128 | >128 | >128 |
| 36 | >128 | >128 | >128 | >128 | >128 | >128 | / | / | / | 8 | 8 | 8 |
| 37 | >128 | >128 | >128 | >128 | >128 | >128 | / | / | / | 128 | 128 | >128 |
| 38 | 128 | >128 | >128 | 128 | 128 | 128 | / | / | / | 8 | 8 | 16 |
| 39 | >128 | >128 | >128 | >128 | >128 | >128 | / | / | / | 4 | 4 | 8 |
| 40 | 64 | 128 | >128 | >128 | 128 | >128 | / | / | / | 32 | 32 | 32 |
| 41 | >128 | >128 | >128 | >128 | >128 | >128 | / | / | / | 32 | 32 | 32 |
| 42 | 128 | >128 | >128 | 128 | >128 | >128 | / | / | / | 32 | 32 | 32 |
| 43 | 128 | 128 | 128 | 128 | 128 | 128 | / | / | / | 8 | 8 | 8 |
| 44 | >128 | >128 | >128 | >128 | >128 | >128 | / | / | / | 32 | 32 | 32 |
| 45 | >128 | >128 | >128 | >128 | >128 | >128 | / | / | / | >128 | >128 | >128 |
| 46 | >128 | >128 | >128 | >128 | >128 | >128 | / | / | / | 32 | 32 | 64 |
| 47 | >128 | >128 | >128 | >128 | >128 | >128 | / | / | / | 32 | 16 | 32 |
| 48 | >128 | >128 | >128 | >128 | >128 | >128 | / | / | / | 64 | 64 | 64 |
| 49 | / | / | / | / | / | / | / | / | / | / | / | / |
| 50 | >128 | >128 | >128 | >128 | >128 | >128 | / | / | / | >128 | >128 | >128 |
| 51 | >128 | >128 | >128 | >128 | >128 | >128 | / | / | / | 1 | 1 | 2 |
| 52 | >128 | >128 | >128 | >128 | >128 | >128 | / | / | / | 128 | 64 | 128 |
| 53 | 128 | 128 | 128 | 128 | 128 | 128 | / | / | / | 16 | 8 | 16 |
| 54 | 64 | 32 | 64 | 64 | 128 | >128 | / | / | / | 16 | 16 | 16 |
| 55 | >128 | >128 | >128 | >128 | >128 | >128 | / | / | / | 4 | 4 | 4 |
| 56 | >128 | >128 | >128 | >128 | >128 | >128 | / | / | / | 128 | 64 | 64 |
| 57 | / | / | / | / | / | / | / | / | / | / | / | / |
| 58 | / | / | / | / | / | / | / | / | / | / | / | / |
| 59 | 128 | 128 | 128 | 128 | >128 | >128 | / | / | / | 16 | 16 | 16 |
| 60 | / | / | / | / | / | / | / | / | / | / | / | / |
| 61 | >128 | >128 | >128 | >128 | >128 | >128 | / | / | / | 16 | 16 | 32 |
| 62 | 4 | 8 | 8 | 4 | 4 | 4 | / | / | / | 2 | 2 | 4 |
| 63 | / | / | / | / | / | / | / | / | / | / | / | / |
| 64 | >128 | >128 | >128 | >128 | >128 | >128 | / | / | / | 0.5 | 0.5 | 1 |
| 65 | / | / | / | / | / | / | / | / | / | / | / | / |
| 66 | >128 | >128 | >128 | >128 | >128 | >128 | / | / | / | 1 | 1 | 1 |
| 67 | >128 | >128 | >128 | >128 | >128 | >128 | / | / | / | 2 | 1 | 64 |
| 68 | 8 | 8 | 32 | 2 | 4 | 8 | / | / | / | 4 | 2 | 4 |
| 69 | >128 | >128 | >128 | >128 | >128 | >128 | / | / | / | 4 | 2 | 2 |
| 70 | >128 | >128 | >128 | >128 | >128 | >128 | / | / | / | 4 | 2 | 4 |
| 71 | >128 | >128 | >128 | >128 | >128 | >128 | / | / | / | 8 | 2 | 4 |
| 72 | >128 | >128 | >128 | 64 | >128 | >128 | / | / | / | 2 | 2 | 4 |
| 73 | 8 | 8 | 64 | 2 | 8 | 32 | / | / | / | 1 | 1 | 2 |
| 74 | >128 | 128 | 128 | 4 | 128 | 128 | / | / | / | 0.5 | 0.25 | 0.5 |
| 75 | / | / | / | / | / | / | / | / | / | / | / | / |
| 76 | 32 | 32 | 64 | 32 | 32 | 64 | / | / | / | 32 | 32 | 32 |
| 77 | 16 | 16 | 128 | 8 | 16 | 32 | / | / | / | 8 | 8 | 8 |
| 78 | / | / | / | / | / | / | / | / | / | / | / | / |
| 79 | / | / | / | / | / | / | / | / | / | / | / | / |
| 80 | >128 | >128 | >128 | >128 | >128 | >128 | / | / | / | 128 | 128 | 128 |
| 81 | 16 | 32 | 128 | 16 | 16 | 32 | / | / | / | 16 | 8 | 16 |
| 82 | >128 | >128 | >128 | 128 | >128 | >128 | / | / | / | 1 | 1 | 1 |
| 83 | 32 | 128 | 128 | 16 | 32 | 64 | / | / | / | 4 | 4 | 4 |
| 84 | 64 | >128 | >128 | 64 | 64 | 128 | / | / | / | 4 | 4 | 64 |
| 85 | 64 | 64 | 64 | 32 | 32 | 64 | / | / | / | 64 | 16 | 64 |
| 86 | 16 | 32 | 32 | 16 | 16 | 32 | / | / | / | 8 | 8 | >128 |
| 87 | 128 | >128 | >128 | 128 | >128 | >128 | / | / | / | 128 | 128 | >128 |
| 88 | >128 | >128 | >128 | 128 | >128 | >128 | / | / | / | 16 | 4 | 16 |
| 89 | >128 | >128 | >128 | 128 | 128 | 128 | / | / | / | 64 | 64 | 64 |
| 90 | 64 | 64 | >128 | 32 | 32 | 128 | / | / | / | 32 | 32 | 32 |
| 91 | 128 | >128 | >128 | 128 | >128 | >128 | / | / | / | >128 | >128 | >128 |
| 92 | 64 | >128 | >128 | 32 | 32 | 128 | / | / | / | 16 | 16 | 16 |
| 93 | >128 | >128 | >128 | >128 | >128 | >128 | / | / | / | >128 | >128 | >128 |
| 94 | 64 | 128 | 128 | 128 | 64 | >128 | / | / | / | >128 | >128 | >128 |
| 95 | 64 | 64 | 64 | 32 | 64 | 64 | / | / | / | 64 | 64 | 64 |
| 96 | >128 | >128 | >128 | >128 | >128 | >128 | / | / | / | 32 | 32 | 64 |
| 97 | >128 | >128 | >128 | 128 | 128 | >128 | / | / | / | 128 | 128 | 128 |
| 98 | 64 | 128 | 128 | 64 | 64 | 128 | / | / | / | 64 | 32 | 64 |
| 99 | >128 | >128 | >128 | 128 | >128 | >128 | / | / | / | >128 | >128 | >128 |
| 100 | >128 | >128 | >128 | >128 | >128 | >128 | / | / | / | >128 | >128 | >128 |
| 101 | >128 | >128 | >128 | >128 | >128 | >128 | / | / | / | >128 | >128 | >128 |
| 102 | 128 | >128 | >128 | >128 | >128 | >128 | / | / | / | >128 | >128 | >128 |
| 103 | >128 | >128 | >128 | >128 | >128 | >128 | / | / | / | >128 | >128 | >128 |
| 104 | 128 | >128 | >128 | >128 | >128 | >128 | / | / | / | >128 | >128 | >128 |
| 105 | 128 | 128 | >128 | / | / | / | / | / | / | 128 | 128 | 128 |
| 106 | 16 | 8 | 128 | / | / | / | / | / | / | 16 | 16 | 16 |
| 107 | 32 | 64 | 128 | / | / | / | / | / | / | 8 | 4 | 8 |
| 108 | >128 | >128 | >128 | / | / | / | / | / | / | 128 | >128 | >128 |
| 109 | 64 | 32 | 64 | / | / | / | / | / | / | 64 | 128 | 128 |
| 110 | 8 | 16 | 64 | 8 | 8 | 8 | / | / | / | 4 | 2 | 2 |
| 111 | >128 | >128 | >128 | >128 | >128 | >128 | / | / | / | >128 | >128 | >128 |
| 112 | 32 | 64 | 128 | 32 | 8 | 32 | / | / | / | 4 | 2 | 4 |
| 113 | 8 | 16 | 16 | 8 | 8 | 16 | / | / | / | 4 | 4 | 4 |
| 114 | 16 | 64 | 64 | 8 | 4 | 8 | / | / | / | 2 | 2 | 2 |
| 115 | 16 | 64 | 64 | 4 | 4 | 4 | / | / | / | 2 | 1 | 1 |
| 116 | 8 | 16 | 16 | 4 | 4 | 4 | / | / | / | 4 | 4 | 8 |
| 117 | 8 | 8 | 16 | 4 | 4 | 4 | / | / | / | 4 | 4 | 8 |
| 118 | >128 | >128 | >128 | >128 | 128 | >128 | / | / | / | 128 | 8 | 128 |
| 119 | >128 | >128 | >128 | >128 | 128 | >128 | / | / | / | 128 | 16 | 128 |
| 120 | 16 | 32 | 32 | 8 | 8 | 16 | / | / | / | 8 | 4 | 16 |
| 121 | 16 | 128 | 128 | 8 | 16 | 32 | / | / | / | 8 | 4 | 16 |
| 122 | 64 | 64 | 64 | 64 | 64 | 64 | / | / | / | 128 | 64 | 64 |
| 123 | 8 | 16 | 32 | 4 | 4 | 8 | / | / | / | 4 | 2 | 4 |
| 124 | 8 | 16 | 64 | 8 | 4 | 8 | / | / | / | 4 | 4 | 4 |
| 125 | 8 | 16 | 16 | 8 | 8 | 16 | / | / | / | 8 | 8 | 8 |

From the data in the above table, it can be seen that the compounds of the present invention have excellent antibacterial activity, especially against Gram-negative bacteria such as *Escherichia coli, Acinetobacter baumannii* and *Klebsiella pneumoniae,* and Gram-positive bacteria such as *Staphylococcus aureus.* Therefore, the compounds of the present invention can treat infectious diseases caused by Gram-negative bacteria or Gram-positive bacteria. The inhibitory effect of the compounds of the Examples 12, 29 and 62 of the present invention against *Escherichia coli* is comparable to that of the reference drug nitroxoline. The inhibitory effect of the compounds of the Examples 29, 68 and 73 of the present invention against *Acinetobacter baumannii* is comparable to that of the reference drug nitroxoline. The inhibitory effect of the compounds of the Examples 11, 12, 22, 29, 32, 34, 39, 51, 55, 62, 64, 66 to 70, 72 to 74, 82 to 84, 110, 112 to 117, 123 and 124 of the present invention against *Staphylococcus aureus* is comparable to or superior than that of the reference drug nitroxoline.

The inventor also investigated the solubility, permeability and pharmacokinetics of the compounds of the present invention. The results show that the compounds of the present invention have low solubility and high permeability, and the exposure and half-life in animal are significantly improved compared to nitroxoline. The compounds of the present invention have excellent application value.

Although the specific embodiments of the present invention have been described above, those skilled in the art should understand that these are only examples, and the protection scope of the present invention is defined by the appended claims. Those skilled in the art can make various changes or modifications to these embodiments without departing from the principle and essence of the present invention, but these changes and modifications all fall within the protection scope of the present invention.

## Claims

1. A compound of formula (I), or a tautomer, a stereoisomer, a mesomer, a racemate, a enantiomer, a diastereoisomer thereof, or a mixture thereof, or a pharmaceutically acceptable salt thereof: 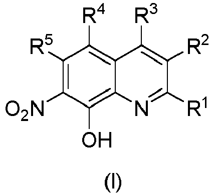 wherein:
R¹ is selected from the group consisting of H atom, D atom, alkyl, halogen, haloalkyl, hydroxyalkyl, alkoxy, haloalkoxy, hydroxy, cyano, amino, nitro, alkenyl, alkynyl, cycloalkyl, heterocyclyl, aryl and heteroaryl;
R² is selected from the group consisting of H atom, alkyl, cycloalkyl, aryl, -C(O)-OR⁶, -(CH₂)ₙ-NR⁷R⁸, -(CH₂)ₙ-NR⁹R¹⁰, D atom, haloalkyl, hydroxyalkyl, alkoxy, haloalkoxy, hydroxy, halogen, cyano, nitro, alkenyl, alkynyl, heterocyclyl, aryl and heteroaryl;
R³ is selected from the group consisting of H atom, D atom, alkyl, halogen, alkoxy, hydroxy, haloalkyl, haloalkoxy, cyano, cycloalkyl, -(CH₂)ₙ-heterocyclyl, aryl, heteroaryl, -NR⁷R⁸, -O-(CH₂)ₙ-NR⁷R⁸, -O-(CH₂)ₙ-R¹¹, -(CH=CH)ₘC(O)-NR⁷R⁸, -(CH=CH)ₘC(O)-NR⁹R¹⁰, nitro, alkenyl, alkynyl and hydroxyalkyl, wherein the cycloalkyl, heterocyclyl, aryl and heteroaryl are each optionally further substituted by one or more substituent(s) selected from the group consisting of alkyl, alkoxy, halogen, haloalkyl, oxo, -C(O)-NR⁷R⁸, -C(O)-OR⁶, -C(O)-R⁶, -S(O)ₓR₆, -O-(CH₂)ₙ-Rⁿ, aryl, cycloalkyl, heteroaryl, hydroxyalkyl, hydroxy, cyano, amino, nitro, alkenyl and alkynyl;
R⁴ is selected from the group consisting of H atom, halogen, cyano, alkyl, cycloalkyl, haloalkyl, hydroxyalkyl, -(CH₂)ₙ-NR⁷R⁸, -(CH₂)ₙ-NR⁹R¹⁰, D atom, alkoxy, haloalkoxy, hydroxy, nitro, alkenyl, alkynyl, heterocyclyl, aryl and heteroaryl;
R⁵ is selected from the group consisting of H atom, D atom, halogen, alkyl, -NR⁷R⁸, haloalkyl, hydroxyalkyl, alkoxy, haloalkoxy, hydroxy, cyano, nitro, alkenyl, alkynyl, cycloalkyl, heterocyclyl, aryl and heteroaryl;
R⁶ is selected from the group consisting of alkyl, H atom, D atom, haloalkyl, hydroxyalkyl, alkoxy, haloalkoxy, hydroxy, halogen, cyano, amino, nitro, alkenyl, alkynyl, cycloalkyl, heterocyclyl, aryl and heteroaryl;
R⁷ and R⁸ are each independently selected from the group consisting of H atom, alkyl, heterocyclyl, D atom, haloalkyl, hydroxyalkyl, alkoxy, haloalkoxy, hydroxy, halogen, cyano, amino, nitro, alkenyl, alkynyl, cycloalkyl, aryl and heteroaryl;
R⁹ and R¹⁰ together with the nitrogen atom to which they are attached form a nitrogen-containing heterocyclyl, the nitrogen-containing heterocyclyl optionally comprises one or more heteroatom(s) selected from the group consisting of N, O and S in addition to N, the nitrogen-containing heterocyclyl is optionally further substituted by one or more substituent(s) selected from the group consisting of alkyl, haloalkyl, hydroxyalkyl, alkoxy, haloalkoxy, hydroxy, halogen, oxo, cyano, amino, nitro, alkenyl, alkynyl, cycloalkyl, heterocyclyl, aryl and heteroaryl;
R¹¹ is selected from the group consisting of halogen, cycloalkyl, aryl, haloalkyl, alkyl, alkoxy, hydroxyalkyl, haloalkoxy, hydroxy, cyano, amino, nitro, alkenyl, alkynyl, heterocyclyl and heteroaryl;
m is 0, 1, 2 or 3;
n is 0, 1, 2, 3, 4 or 5; and
x is 0, 1 or 2;
provided that the compound of formula (I) is not

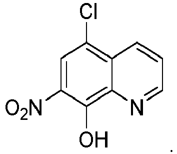

2. The compound of formula (I), or the tautomer, the stereoisomer, the mesomer, the racemate, the enantiomer, the diastereoisomer thereof, or the mixture thereof, or the pharmaceutically acceptable salt thereof according to claim 1, wherein,
R³ is selected from the group consisting of H atom, D atom, C₁₋₆ alkyl, halogen, C₁₋₆ alkoxy, hydroxy, haloC₁₋₆ alkyl, haloC₁₋₆ alkoxy, cyano, C₃₋₈ cycloalkyl, -(CH₂)ₙ-3 to 8 membered heterocyclyl, C₆₋₁₄ aryl, 5 to 14 membered heteroaryl, -NR⁷R⁸, -O-(CH₂)ₙ-NR⁷R⁸, -O-(CH₂)ₙ-Rⁿ, -(CH=CH)ₘC(O)-NR⁷R⁸ and -(CH=CH)ₘC(O)-NR⁹R¹⁰, wherein the 3 to 8 membered heterocyclyl is optionally further substituted by one or more substituent(s) selected from the group consisting of C₁₋₆ alkyl, C₁₋₆ alkoxy, halogen, haloC₁₋₆ alkyl, oxo, -C(O)-NR⁷R⁸, -C(O)-OR⁶, -C(O)-R⁶, -S(O)ₓR₆, -O-(CH₂)ₙ-Rⁿ and C₆₋₁₄ aryl;
R⁶ is C₁₋₆ alkyl;
R⁷ and R⁸ are each independently selected from the group consisting of H atom, C₁₋₆ alkyl and 3 to 8 membered heterocyclyl;
R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N;
R¹¹ is selected from the group consisting of halogen, C₃₋₈ cycloalkyl, C₆₋₁₄ aryl, haloC₁₋₆ alkyl, C₁₋₆ alkyl and C₁₋₆ alkoxy;
m is 1;
n is 0, 1, 2 or 3; and
x is 2;
particularly, the 3 to 8 membered heterocyclyl or 3 to 8 membered nitrogen-containing heterocyclyl is selected from the group consisting of pyrrolidinyl, morpholinyl, piperazinyl, oxetanyl, piperidinyl, 3-pyrrolinyl and 6-azaspiro[2.5]octyl; the 5 to 14 membered heteroaryl is imidazolyl;
preferably, R³ is selected from the group consisting of H atom, -CH₃, 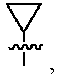 Cl 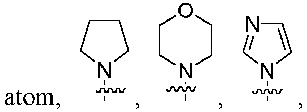 Br atom, 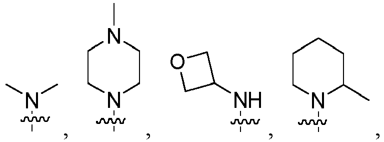 -OCH₃, 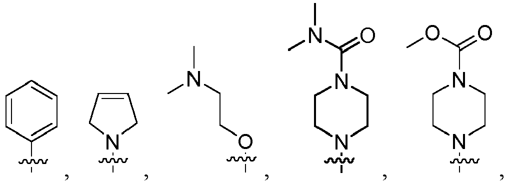 F atom, 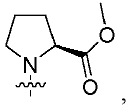 -OH, 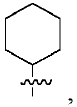 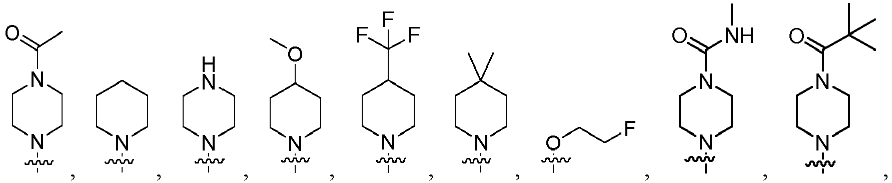 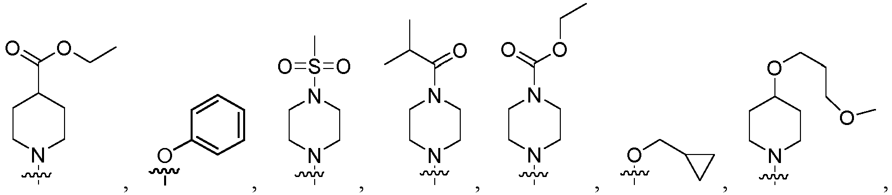 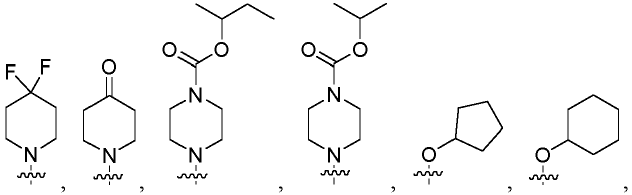 -OCH₂CH₃, -CF₃, 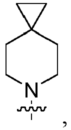 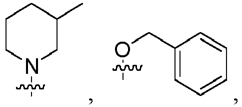 -CN, 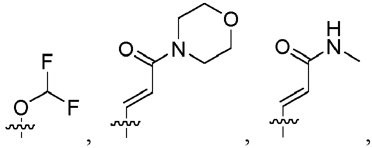 -CHF₂, 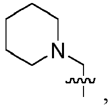 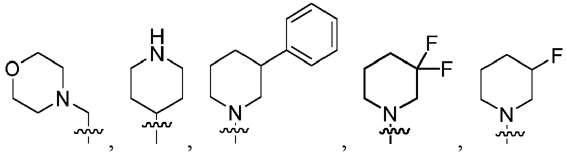 and D atom.

3. The compound of formula (I), or or the tautomer, the stereoisomer, the mesomer, the racemate, the enantiomer, the diastereoisomer thereof, or the mixture thereof, or the pharmaceutically acceptable salt thereof according to claim 1 or 2, wherein,
R⁴ is selected from the group consisting of H atom, halogen, cyano, C₁₋₆ alkyl, C₃₋₈ cycloalkyl, haloC₁₋₆ alkyl, hydroxyC₁₋₆ alkyl, -(CH₂)ₙ-NR⁷R⁸ and -(CH₂)ₙ-NR⁹R¹⁰;
R⁷ and R⁸ are each independently H atom or C₁₋₆ alkyl;
R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N; particularly, R⁹ and R¹⁰ together with the nitrogen atom to which they are attached form a morpholinyl, piperidinyl, pyrrolidinyl or piperazinyl;
preferably, R⁴ is selected from the group consisting of H atom, I atom, -CH₃, Cl atom, -CN, F atom, 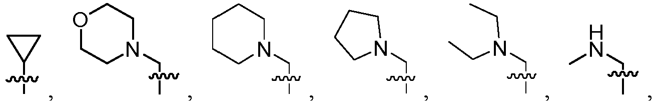 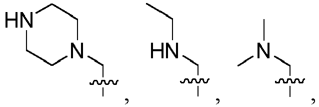 -CHF₂, -CH₂OH and Br atom.

4. The compound of formula (I), or or the tautomer, the stereoisomer, the mesomer, the racemate, the enantiomer, the diastereoisomer thereof, or the mixture thereof, or the pharmaceutically acceptable salt thereof according to any one of claims 1 to 3, wherein,
R² is selected from the group consisting of H atom, C₁₋₆ alkyl, C₃₋₈ cycloalkyl, C₆₋₁₄ aryl, -C(O)-OR⁶, -CH₂-NR⁷R⁸ and -CH₂-NR⁹R¹⁰;
R⁶ is C₁₋₆ alkyl;
R⁷ and R⁸ are each independently H atom or C₁₋₆ alkyl;
R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N, the 3 to 8 membered nitrogen-containing heterocyclyl is optionally further substituted by one or more oxo;
particularly, the 3 to 8 membered nitrogen-containing heterocyclyl is selected from the group consisting of piperidinyl, morpholinyl and piperazinyl optionally substituted by oxo;
preferably, R² is selected from the group consisting of H atom, -CH₃, 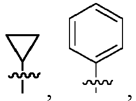 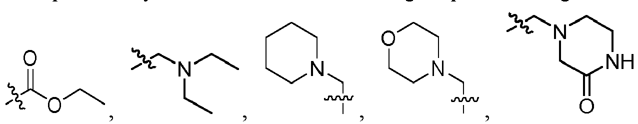 and

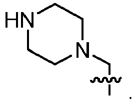

5. The compound of formula (I), or or the tautomer, the stereoisomer, the mesomer, the racemate, the enantiomer, the diastereoisomer thereof, or the mixture thereof, or the pharmaceutically acceptable salt thereof according to any one of claims 1 to 4, wherein,
R⁵ is selected from the group consisting of H atom, D atom, halogen, C₁₋₆ alkyl and -NR⁷R⁸; R⁷ is H atom, and R⁸ is C₁₋₆ alkyl;
preferably, R⁵ is selected from the group consisting of H atom, -CH₃, F atom, Br atom, Cl atom, D atom and -NHCH₃.

6. The compound of formula (I), or or the tautomer, the stereoisomer, the mesomer, the racemate, the enantiomer, the diastereoisomer thereof, or the mixture thereof, or the pharmaceutically acceptable salt thereof according to any one of claims 1 to 5, wherein,
R¹ is selected from the group consisting of H atom, halogen, D atom and C₁₋₆ alkyl;
preferably, R¹ is selected from the group consisting of H atom, F atom, -CH₃ and D atom.

7. The compound of formula (I), or or the tautomer, the stereoisomer, the mesomer, the racemate, the enantiomer, the diastereoisomer thereof, or the mixture thereof, or the pharmaceutically acceptable salt thereof according to any one of claims 1 to 6, being a compound of formula (II), or a tautomer, a stereoisomer, a mesomer, a racemate, a enantiomer, a diastereoisomer thereof, or a mixture thereof, or a pharmaceutically acceptable salt thereof: 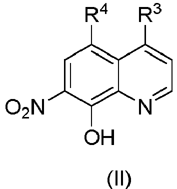 R³ is selected from the group consisting of haloC₁₋₆ alkyl, C₃₋₈ cycloalkyl, 3 to 8 membered heterocyclyl, C₆₋₁₄ aryl and -O-(CH₂)ₙ-R¹¹, wherein the 3 to 8 membered heterocyclyl is optionally further substituted by one or more substituent(s) selected from the group consisting of C₁₋₆ alkyl, C₁₋₆ alkoxy, halogen, haloC₁₋₆ alkyl, -C(O)-OR⁶ and -O-(CHz)ₙ-R¹¹; R⁶ is C₁₋₆ alkyl; R¹¹ is selected from the group consisting of C₃₋₈ cycloalkyl and C₁₋₆ alkoxy; and n is 0, 1, 2, 3 or 4; particularly, the 3 to 8 membered heterocyclyl is piperazinyl or piperidinyl; preferably, R³ is selected from the group consisting of 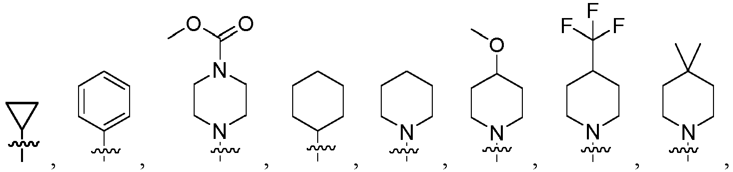 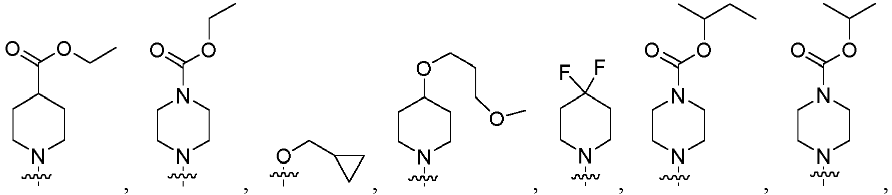 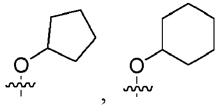 and -CF₃; and
R⁴ is H atom or halogen; preferably, R⁴ is H atom, Cl atom or F atom.

8. The compound of formula (I), or or the tautomer, the stereoisomer, the mesomer, the racemate, the enantiomer, the diastereoisomer thereof, or the mixture thereof, or the pharmaceutically acceptable salt thereof according to any one of claims 1 to 6, wherein,
R¹ is selected from the group consisting of H atom, halogen, D atom and C₁₋₆ alkyl; preferably, R¹ is selected from the group consisting of H atom, F atom, -CH₃ and D atom;
R² is selected from the group consisting of H atom, C₁₋₆ alkyl, C₃₋₈ cycloalkyl, -CH₂-NR⁷R⁸ and -CH₂-NR⁹R¹⁰; R⁷ and R⁸ are each independently H atom or C₁₋₆ alkyl; R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N; particularly, R⁹ and R¹⁰ together with the nitrogen atom to which they are attached form a piperidinyl; preferably, R² is selected from the group consisting of H atom, -CH₃, 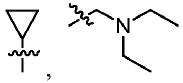 and 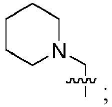
R³ is selected from the group consisting of H atom, D atom, C₁₋₆ alkyl, halogen, C₁₋₆ alkoxy, haloC₁₋₆ alkyl, haloC₁₋₆ alkoxy, cyano, C₃₋₈ cycloalkyl, -(CH₂)ₙ-3 to 8 membered heterocyclyl and -NR⁷R⁸, wherein the 3 to 8 membered heterocyclyl is optionally further substituted by one or more substituent(s) selected from the group consisting of C₁₋₆ alkyl, -C(O)-NR⁷R⁸ and -O-(CHz)ₙ-R¹¹; R⁷ and R⁸ are each independently C₁₋₆ alkyl; R¹¹ is C₁₋₆ alkoxy; n is 0, 1, 2, 3 or 4; particularly, the 3 to 8 membered heterocyclyl is piperazinyl or piperidinyl; preferably, R³ is selected from the group consisting of H atom, -CH₃, 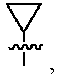 Cl atom, Br atom, 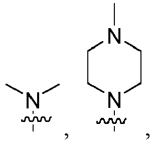 -OCH₃, 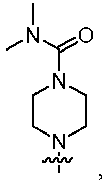 F atom, 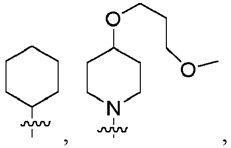 -CF₃, -CN, 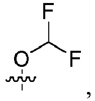 -CHF₂, 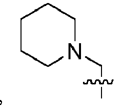 and D atom;
R⁴ is selected from the group consisting of H atom, halogen, C₃₋₈ cycloalkyl, hydroxyC₁₋₆ alkyl and -CH₂-NR⁷R⁸; R⁷ and R⁸ are each independently C₁₋₆ alkyl; preferably, R⁴ is selected from the group consisting of H atom, Cl atom, F atom, 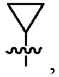 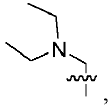 -CH₂OH and Br atom; and
R⁵ is selected from the group consisting of H atom, D atom, halogen, C₁₋₆ alkyl and -NR⁷R⁸; R⁷ is H atom, and R⁸ is C₁₋₆ alkyl; preferably, R⁵ is selected from the group consisting of H atom, -CH₃, F atom, Br atom, Cl atom, D atom and -NHCH₃;
particularly,
R¹ is H atom or D atom;
R² is selected from the group consisting of H atom, C₁₋₆ alkyl, C₃₋₈ cycloalkyl, -CH₂-NR⁷R⁸ and -CH₂-NR⁹R¹⁰; R⁷ and R⁸ are each independently H atom or C₁₋₆ alkyl; R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N; particularly, R⁹ and R¹⁰ together with the nitrogen atom to which they are attached form a piperidinyl; preferably, R² is selected from the group consisting of H atom, -CH₃, 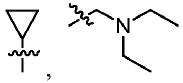 and 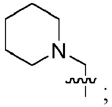
R³ is selected from the group consisting of H atom, D atom, halogen, C₁₋₆ alkoxy, haloC₁₋₆ alkyl, haloC₁₋₆ alkoxy, cyano, C₃₋₈ cycloalkyl and -(CH₂)ₙ-3 to 8 membered heterocyclyl, wherein the 3 to 8 membered heterocyclyl is optionally further substituted by one or more substituent(s) selected from the group consisting of C₁₋₆ alkyl, -C(O)-NR⁷R⁸ and -O-(CH₂)ₙ-Rⁿ; R⁷ and R⁸ are each independently C₁₋₆ alkyl; R¹¹ is C₁₋₆ alkoxy; n is 0, 1, 2, 3 or 4; particularly, the 3 to 8 membered heterocyclyl is piperazinyl or piperidinyl; preferably, R³ is selected from the group consisting of H atom, Cl atom, Br atom, 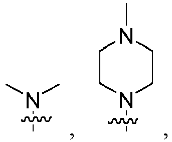 -OCH₃, 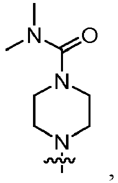 F atom, 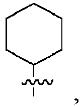 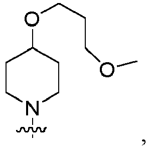 -CF₃, -CN, 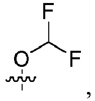 -CHF₂, 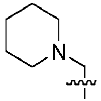 and D atom;
R⁴ is selected from the group consisting of H atom, halogen, C₃₋₈ cycloalkyl, hydroxyC₁₋₆ alkyl and -CH₂-NR⁷R⁸; R⁷ and R⁸ are each independently C₁₋₆ alkyl; preferably, R⁴ is selected from the group consisting of H atom, Cl atom, F atom, 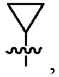 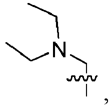 -CH₂OH and Br atom; and
R⁵ is selected from the group consisting of H atom, D atom, halogen, C₁₋₆ alkyl and -NR⁷R⁸; R⁷ is H atom, and R⁸ is C₁₋₆ alkyl; preferably, R⁵ is selected from the group consisting of H atom, -CH₃, F atom, Br atom, Cl atom, D atom and -NHCH₃;
more particularly,
R¹ is H atom;
R² is H atom;
R³ is halogen or haloC₁₋₆ alkyl; preferably, R³ is Cl atom or -CF₃;
R⁴ is H atom or halogen; preferably, R⁴ is H atom or Cl atom; and
R⁵ is H atom.

9. The compound of formula (I), or or the tautomer, the stereoisomer, the mesomer, the racemate, the enantiomer, the diastereoisomer thereof, or the mixture thereof, or the pharmaceutically acceptable salt thereof according to any one of claims 1 to 6, wherein,
R¹ is H atom or D atom;
R² is selected from the group consisting of H atom, C₁₋₆ alkyl, C₃₋₈ cycloalkyl, -CH₂-NR⁷R⁸ and -CH₂-NR⁹R¹⁰; R⁷ and R⁸ are each independently C₁₋₆ alkyl; R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N; particularly, the 3 to 8 membered nitrogen-containing heterocyclyl is piperidinyl; preferably, R² is selected from the group consisting of H atom, -CH₃, 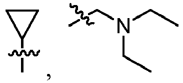 and 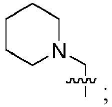
R³ is selected from the group consisting of H atom, D atom, C₁₋₆ alkyl, halogen, C₁₋₆ alkoxy, haloC₁₋₆ alkyl, haloC₁₋₆ alkoxy, cyano, C₃₋₈ cycloalkyl, -(CH₂)ₙ-3 to 8 membered heterocyclyl and -O-(CH₂)ₙ-Rⁿ, wherein the 3 to 8 membered heterocyclyl is optionally further substituted by one or more -C(O)-OR⁶ or -O-(CH₂)ₙ-Rⁿ; R⁶ is C₁₋₆ alkyl; R¹¹ is C₃₋₈ cycloalkyl or C₁₋₆ alkoxy; n is 0, 1, 2, 3 or 4; particularly, the 3 to 8 membered heterocyclyl is selected from the group consisting of pyrrolidinyl, piperazinyl and piperidinyl; preferably, R³ is selected from the group consisting of H atom, -CH₃, 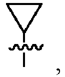 Cl atom, Br atom, -OCH₃, 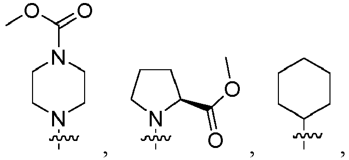 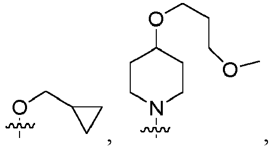 -CF₃, -CN, 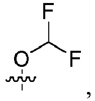 -CHF₂, 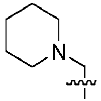 and D atom;
R⁴ is selected from the group consisting of H atom, halogen, C₁₋₆ alkyl, C₃₋₈ cycloalkyl and -CH₂-NR⁷R⁸; R⁷ and R⁸ are each independently C₁₋₆ alkyl; preferably, R⁴ is selected from the group consisting of H atom, -CH₃, Cl atom, F atom, 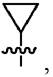 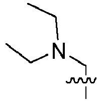 and Br atom; and
R⁵ is selected from the group consisting of H atom, D atom, halogen, C₁₋₆ alkyl and -NR⁷R⁸; R⁷ is H atom, and R⁸ is C₁₋₆ alkyl; preferably, R⁵ is selected from the group consisting of H atom, -CH₃, F atom, Br atom, Cl atom, D atom and -NHCH₃;
particularly,
R¹ is H atom or D atom;
R² is selected from the group consisting of H atom, C₁₋₆ alkyl, C₃₋₈ cycloalkyl, -CH₂-NR⁷R⁸ and -CH₂-NR⁹R¹⁰; R⁷ and R⁸ are each independently C₁₋₆ alkyl; R⁹ and R¹⁰ together with the nitrogen atom to which they are attached form a 3 to 8 membered nitrogen-containing heterocyclyl, the 3 to 8 membered nitrogen-containing heterocyclyl optionally comprises one or more heteroatom(s) selected from the group consisting of N, O and S in addition to N; particularly, the 3 to 8 membered nitrogen-containing heterocyclyl is piperidinyl; preferably, R² is selected from the group consisting of H atom, -CH₃, 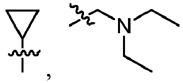 and 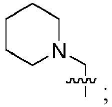
R³ is selected from the group consisting of H atom, D atom, halogen, C₁₋₆ alkoxy, haloC₁₋₆ alkyl, haloC₁₋₆ alkoxy, cyano, C₃₋₈ cycloalkyl, -(CH₂)ₙ-3 to 8 membered heterocyclyl and -O-(CH₂)n-R¹¹, wherein the 3 to 8 membered heterocyclyl is optionally further substituted by one or more -O-(CHz)ₙ-R¹¹; R¹¹ is C₃₋₈ cycloalkyl or C₁₋₆ alkoxy; n is 0, 1, 2, 3 or 4; particularly, the 3 to 8 membered heterocyclyl is piperidinyl; preferably, R³ is selected from the group consisting of H atom, Cl atom, Br atom, -OCH₃, 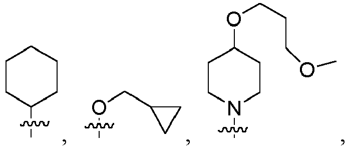 -CF₃, -CN, 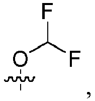 -CHF₂, 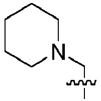 and D atom;
R⁴ is selected from the group consisting of H atom, halogen and C₃₋₈ cycloalkyl; preferably, R⁴ is selected from the group consisting of H atom, Cl atom, F atom, 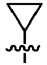 and Br atom; and
R⁵ is selected from the group consisting of H atom, D atom, halogen and -NR⁷R⁸; R⁷ is H atom, and R⁸ is C₁₋₆ alkyl; preferably, R⁵ is selected from the group consisting of H atom, F atom, Br atom, Cl atom, D atom and -NHCH₃;
more particularly,
R¹ is H atom;
R² is H atom;
R³ is halogen or haloC₁₋₆ alkyl; preferably, R³ is Cl atom or -CF₃;
R⁴ is halogen; preferably, R⁴ is Cl atom or F atom; and
R⁵ is H atom.

10. The compound of formula (I), or or the tautomer, the stereoisomer, the mesomer, the racemate, the enantiomer, the diastereoisomer thereof, or the mixture thereof, or the pharmaceutically acceptable salt thereof according to any one of claims 1 to 6, wherein,
R¹ is H atom;
R² is H atom or C₁₋₆ alkyl; preferably, R² is H atom or -CH₃;
R³ is H atom or C₃₋₈ cycloalkyl; preferably, R³ is selected from the group consisting of H atom and 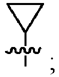
R⁴ is H atom or cyano; and
R⁵ is H atom or C₁₋₆ alkyl; preferably, R⁵ is H atom or -CH₃.

11. The compound of formula (I), or or the tautomer, the stereoisomer, the mesomer, the racemate, the enantiomer, the diastereoisomer thereof, or the mixture thereof, or the pharmaceutically acceptable salt thereof according to any one of claims 1 to 6, wherein,
R¹ is H atom or D atom;
R² is selected from the group consisting of H atom, C₁₋₆ alkyl, C₃₋₈ cycloalkyl, C₆₋₁₄ aryl and -C(O)-OR⁶; R⁶ is C₁₋₆ alkyl; preferably, R² is selected from the group consisting of H atom, -CH₃, 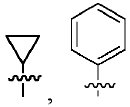 and 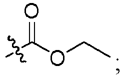
R³ is selected from the group consisting of H atom, D atom, halogen, C₁₋₆ alkoxy, haloC₁₋₆ alkyl, C₃₋₈ cycloalkyl, 3 to 8 membered heterocyclyl, C₆₋₁₄ aryl and -O-CH₂-R¹¹, wherein the 3 to 8 membered heterocyclyl is optionally further substituted by one or more substituent(s) selected from the group consisting of C₁₋₆ alkyl, halogen, haloC₁₋₆ alkyl and -C(O)-OR⁶; R⁶ is C₁₋₆ alkyl; R¹¹ is C₃₋₈ cycloalkyl or C₆₋₁₄ aryl; particularly, the 3 to 8 membered heterocyclyl is piperidinyl; preferably, R³ is selected from the group consisting of H atom, 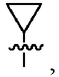 Cl atom, -OCH₃, 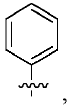 F atom, 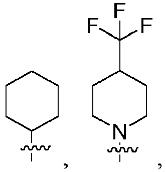 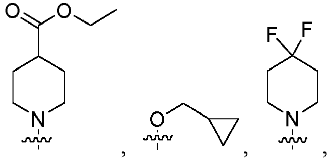 -CF₃, 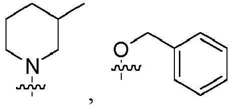 and D atom;
R⁴ is selected from the group consisting of H atom, halogen and C₃₋₈ cycloalkyl; preferably, R⁴ is selected from the group consisting of H atom, Cl atom, F atom, 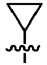 and Br atom;
R⁵ is selected from the group consisting of H atom, D atom, halogen and C₁₋₆ alkyl; preferably, R⁵ is selected from the group consisting of H atom, -CH₃, F atom, Br atom, Cl atom and D atom;
particularly,
R¹ is H atom or D atom;
R² is selected from the group consisting of H atom, C₁₋₆ alkyl, C₃₋₈ cycloalkyl and C₆₋₁₄ aryl; preferably, R² is selected from the group consisting of H atom, -CH₃, 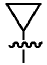 and 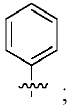
R³ is selected from the group consisting of H atom, halogen, C₁₋₆ alkoxy, haloC₁₋₆ alkyl, C₃₋₈ cycloalkyl, 3 to 8 membered heterocyclyl, C₆₋₁₄ aryl and -O-CH₂-R¹¹, wherein the 3 to 8 membered heterocyclyl is optionally further substituted by one or more substituent(s) selected from the group consisting of C₁₋₆ alkyl, halogen and haloC₁₋₆ alkyl; R¹¹ is C₃₋₈ cycloalkyl or C₆₋₁₄ aryl; particularly, the 3 to 8 membered heterocyclyl is piperidinyl; preferably, R³ is selected from the group consisting of H atom, 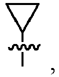 Cl atom, -OCH₃, 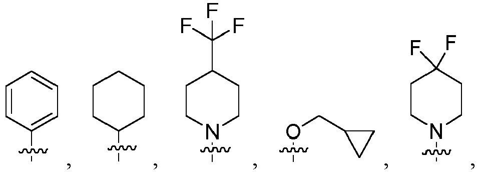 -CF₃, 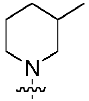 and 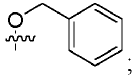
R⁴ is selected from the group consisting of H atom, halogen and C₃₋₈ cycloalkyl; preferably, R⁴ is selected from the group consisting of H atom, Cl atom, F atom and 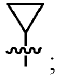
R⁵ is selected from the group consisting of H atom, D atom and halogen; preferably, R⁵ is selected from the group consisting of H atom, F atom, Br atom, Cl atom and D atom;
more particularly,
R¹ is H atom;
R² is selected from the group consisting of H atom, C₃₋₈ cycloalkyl and C₆₋₁₄ aryl; preferably, R² is selected from the group consisting of H atom, 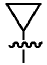 and 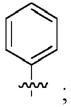
R³ is selected from the group consisting of H atom, halogen, haloC₁₋₆ alkyl, C₃₋₈ cycloalkyl, 3 to 8 membered heterocyclyl, C₆₋₁₄ aryl and -O-CH₂-R¹¹, wherein the 3 to 8 membered heterocyclyl is optionally further substituted by one or more C₁₋₆ alkyl; R¹¹ is C₃₋₈ cycloalkyl or C₆₋₁₄ aryl; particularly, the 3 to 8 membered heterocyclyl is piperidinyl; preferably, R³ is selected from the group consisting of H atom, Cl atom, 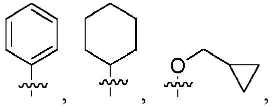 -CF₃, 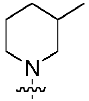 and 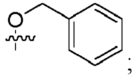
R⁴ is H atom or halogen; preferably, R⁴ is H atom, Cl atom or F atom;
R⁵ is H atom or halogen; preferably, R⁵ is H atom, Br atom or Cl atom.

12. The compound of formula (I), or or the tautomer, the stereoisomer, the mesomer, the racemate, the enantiomer, the diastereoisomer thereof, or the mixture thereof, or the pharmaceutically acceptable salt thereof according to any one of claims 1 to 6, being a compound of formula (III), or a tautomer, a stereoisomer, a mesomer, a racemate, a enantiomer, a diastereoisomer thereof, or a mixture thereof, or a pharmaceutically acceptable salt thereof: 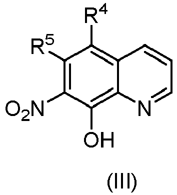
preferably, R⁴ is halogen, preferably Cl atom or F atom; and
R⁵ is halogen, preferably F atom, Cl atom or Br atom.

13. The compound of formula (I), or or the tautomer, the stereoisomer, the mesomer, the racemate, the enantiomer, the diastereoisomer thereof, or the mixture thereof, or the pharmaceutically acceptable salt thereof according to any one of claims 1 to 11, wherein, the compound of formula (I) is selected from the group consisting of: 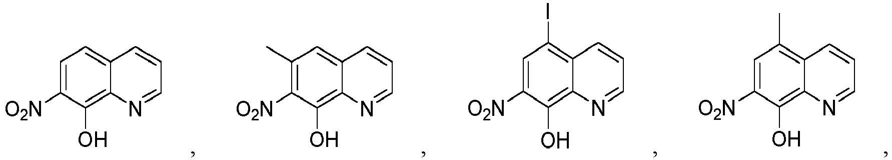 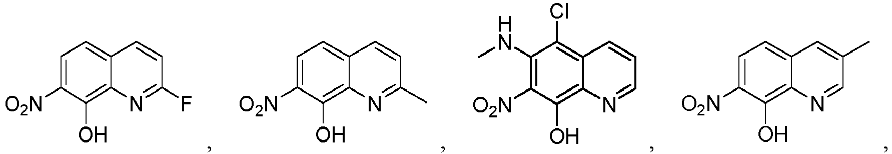 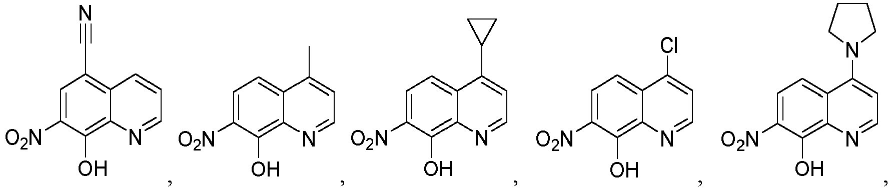 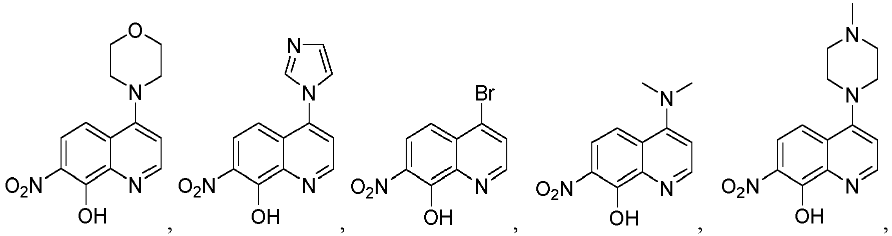 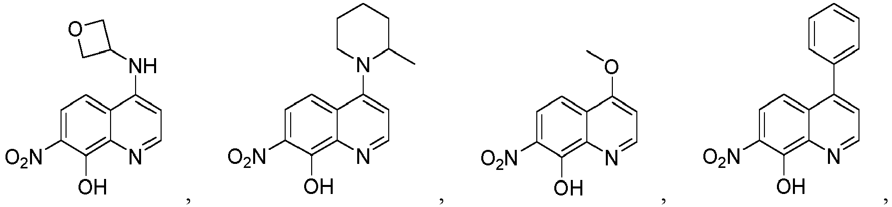 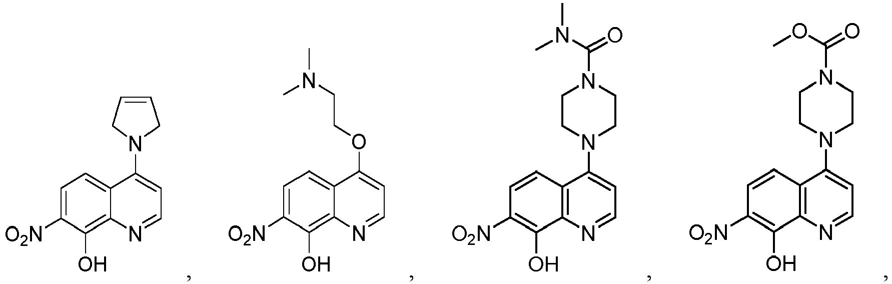 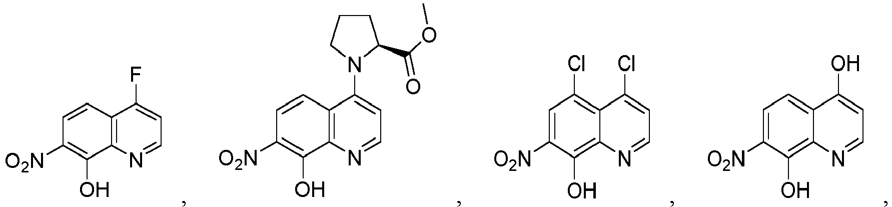 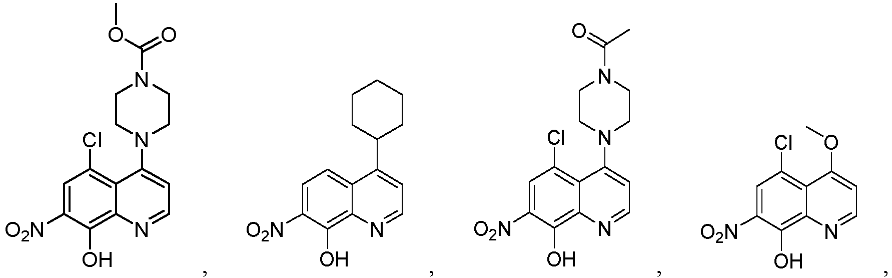 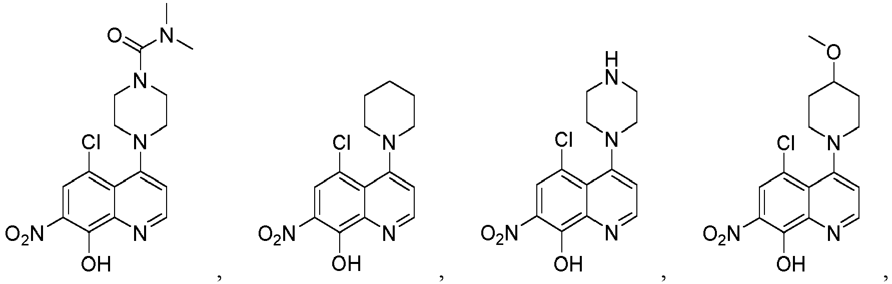 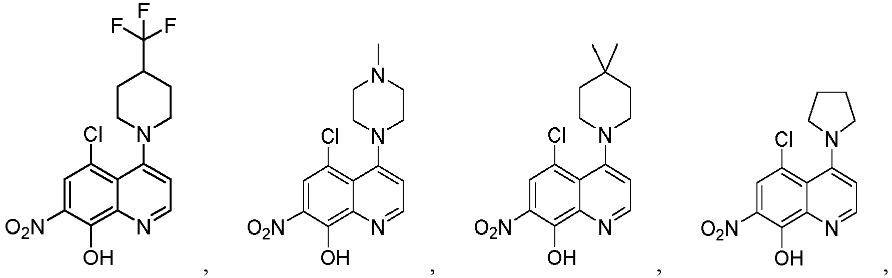 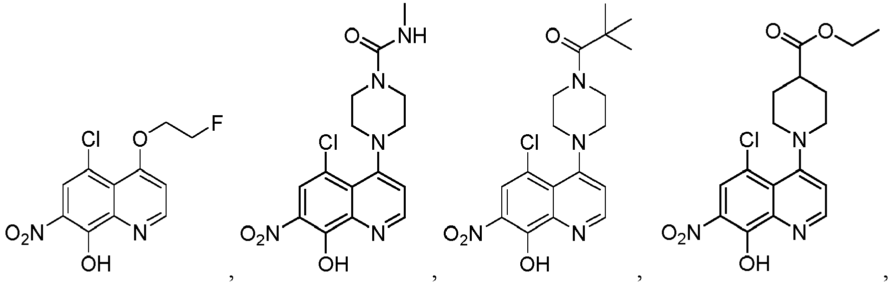 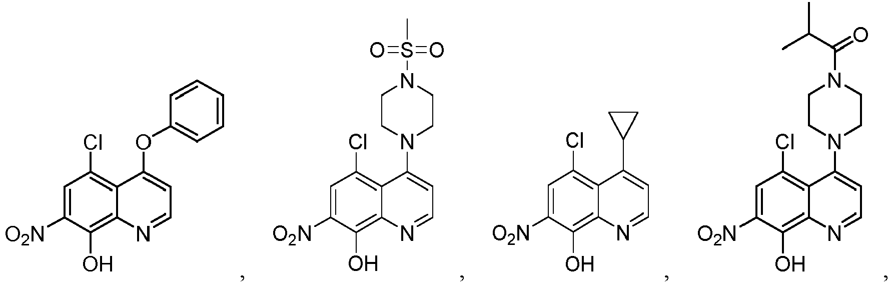 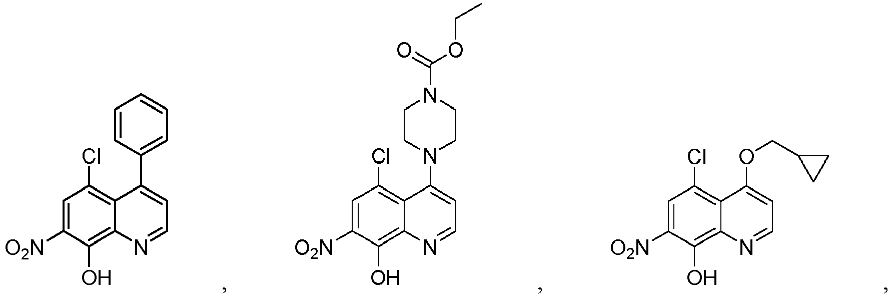 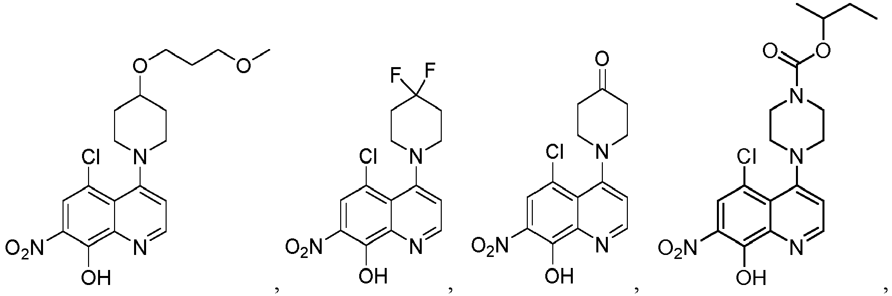 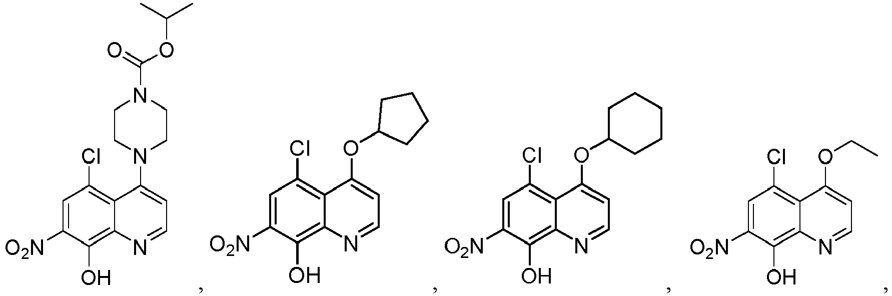 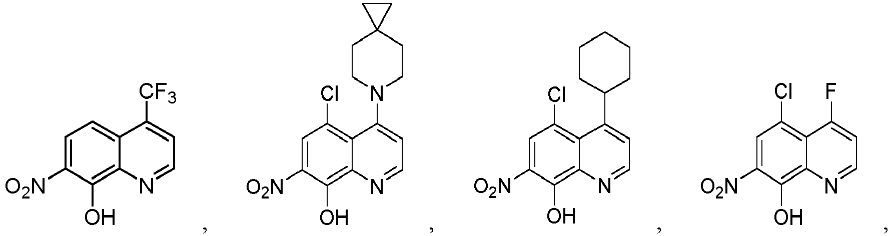 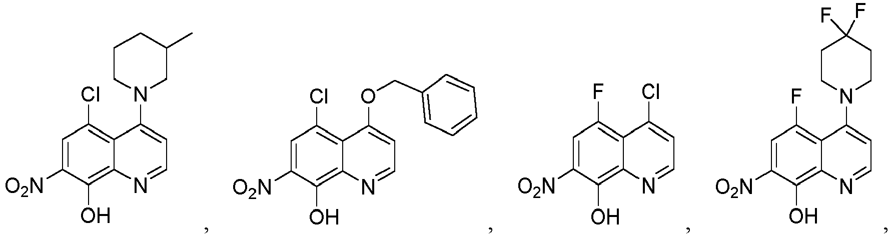 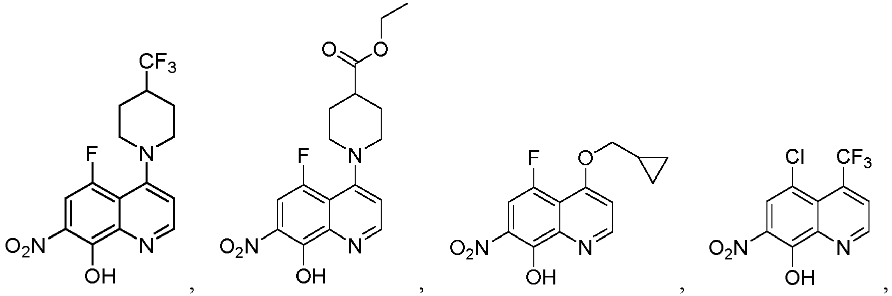 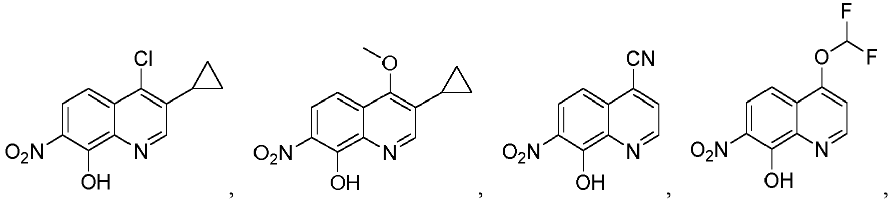 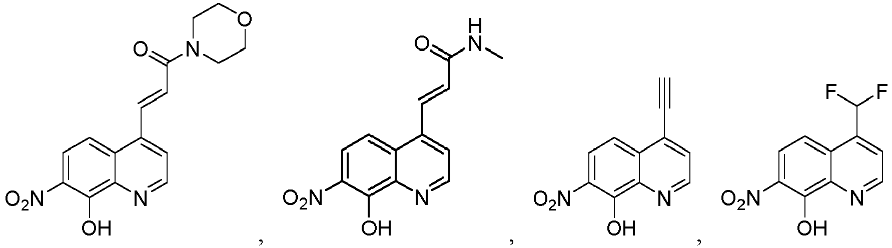 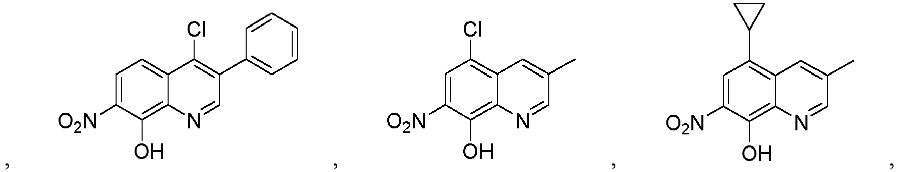 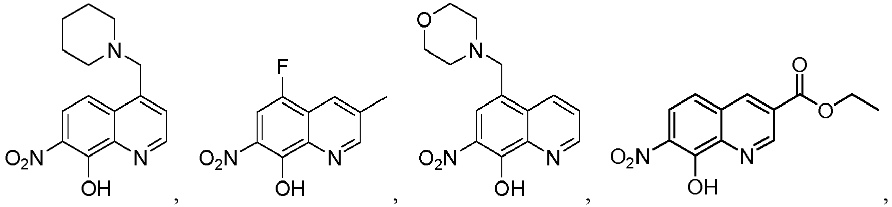 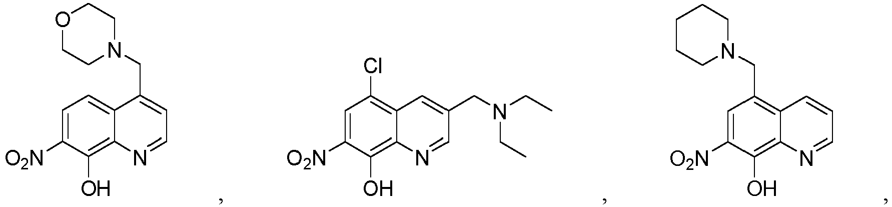 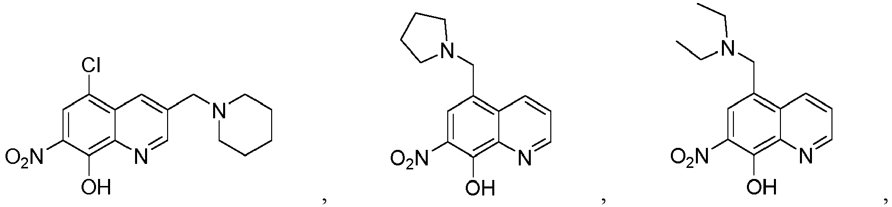 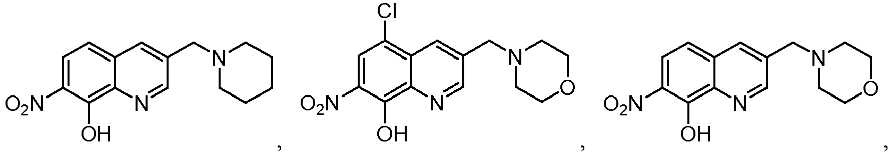 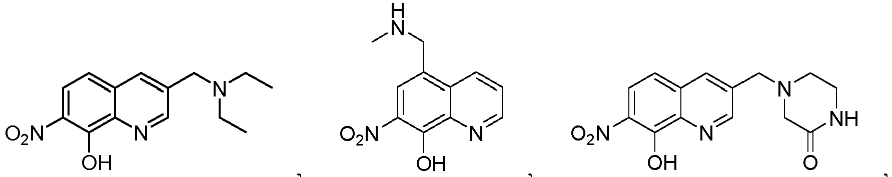 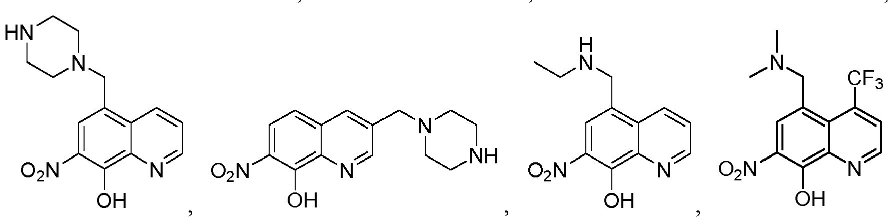 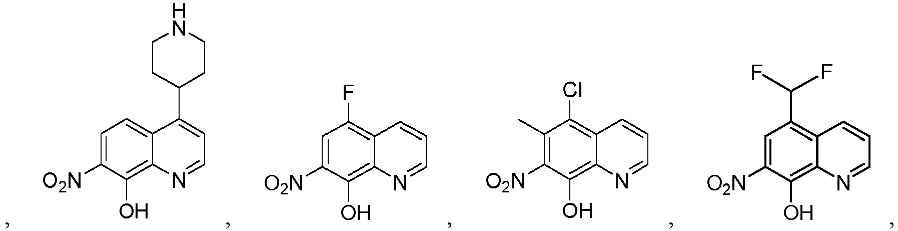 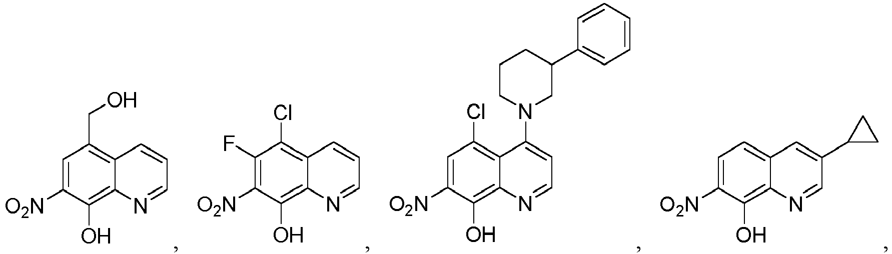 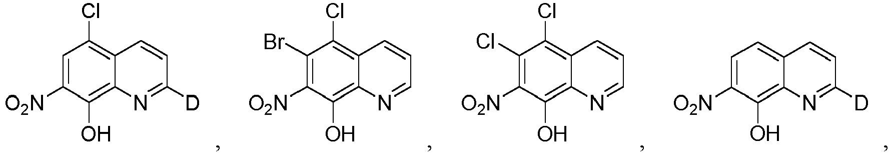 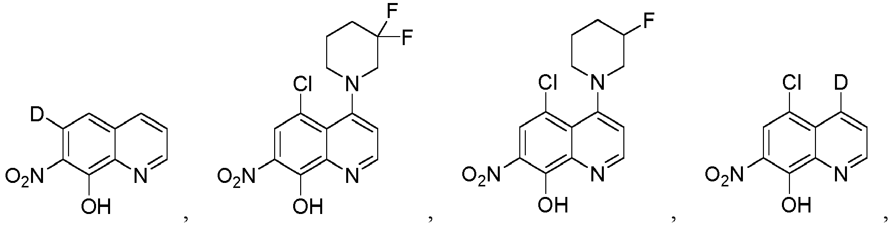 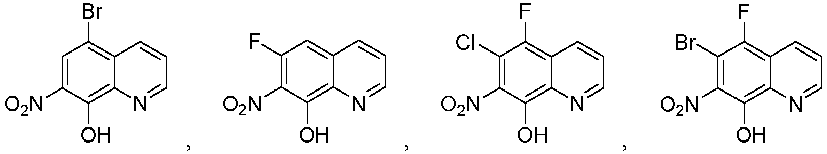 and 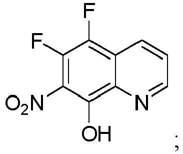
preferably, the pharmaceutically acceptable salt is the hydrochloride, acetate or trifluoroacetate of each specific compound above;
more preferably, the pharmaceutically acceptable salt is selected from the group consisting of 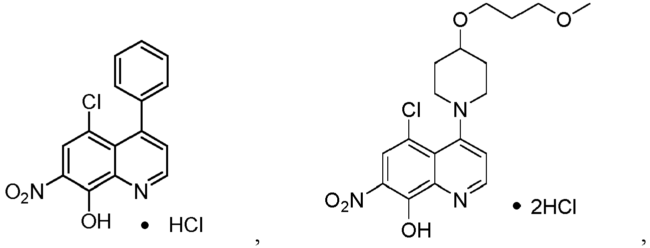 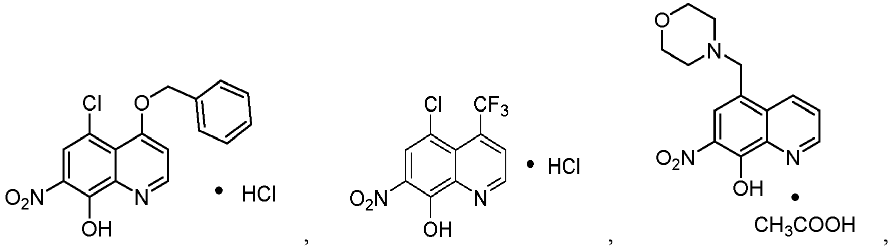 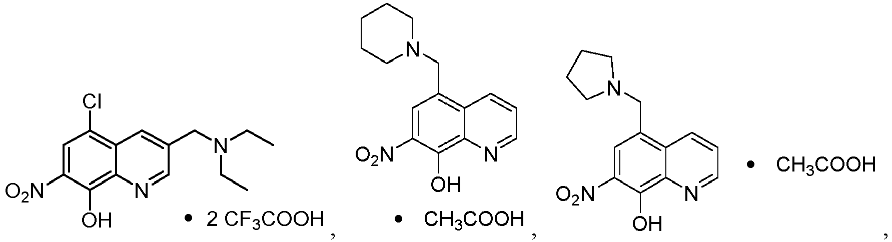 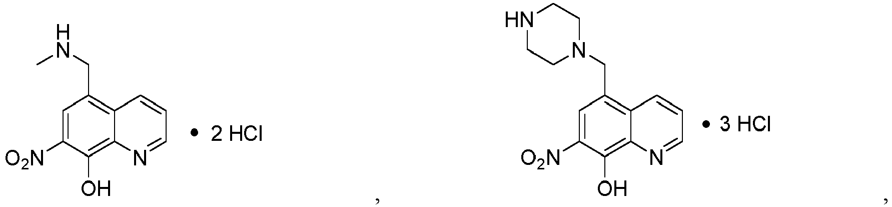 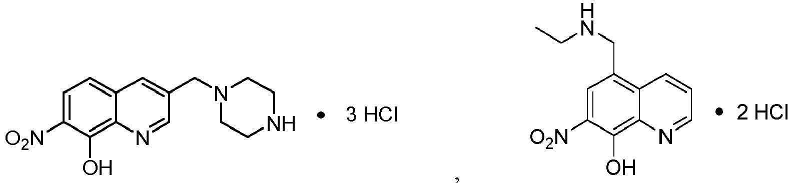 and

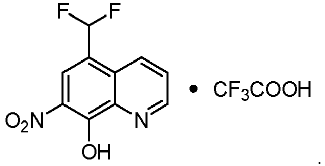

14. A compound or a pharmaceutically acceptable salt thereof as shown below: 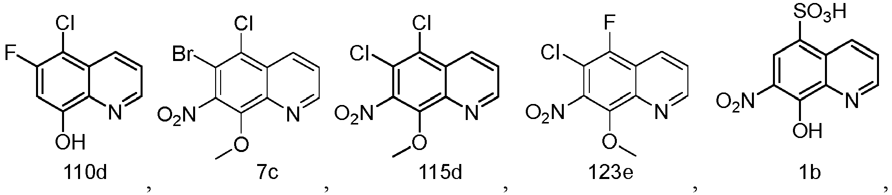 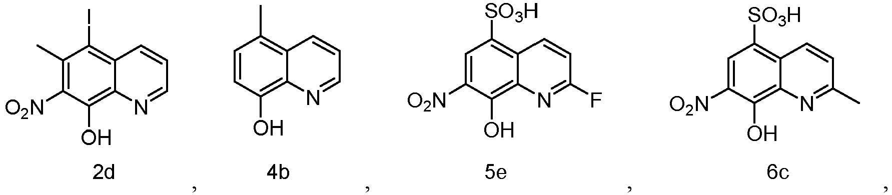 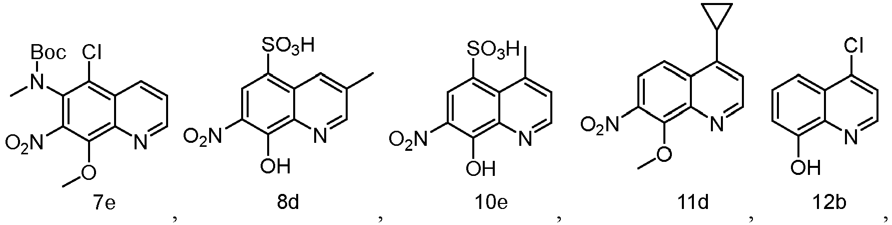 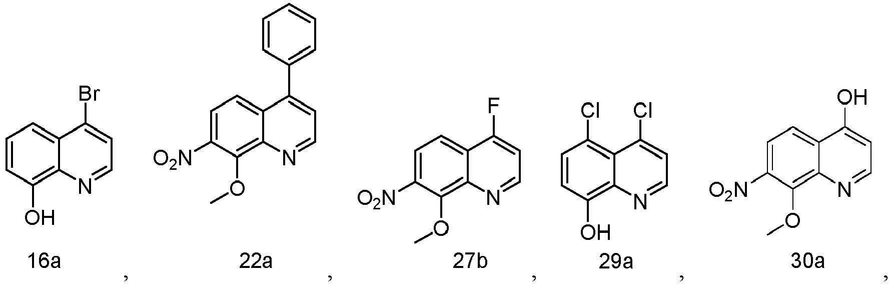 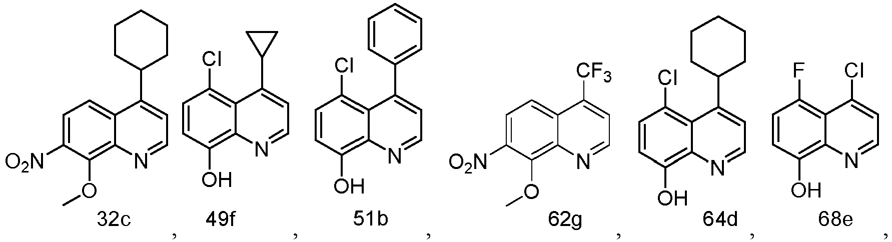 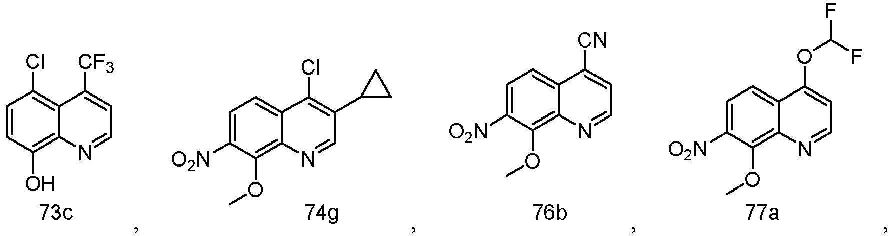 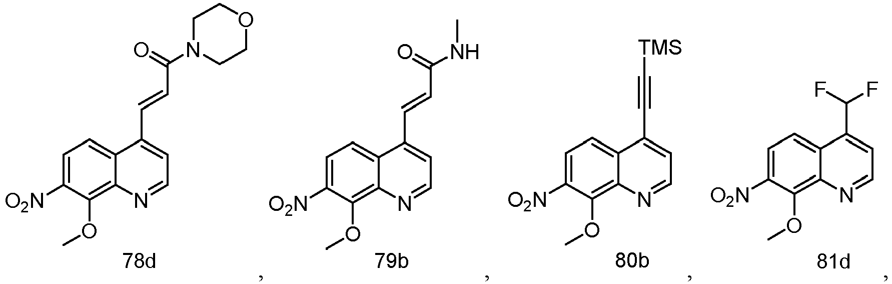 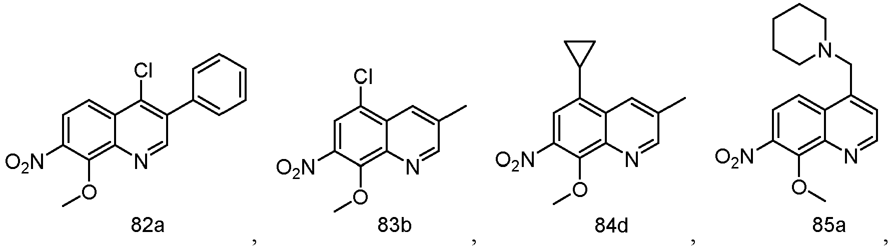 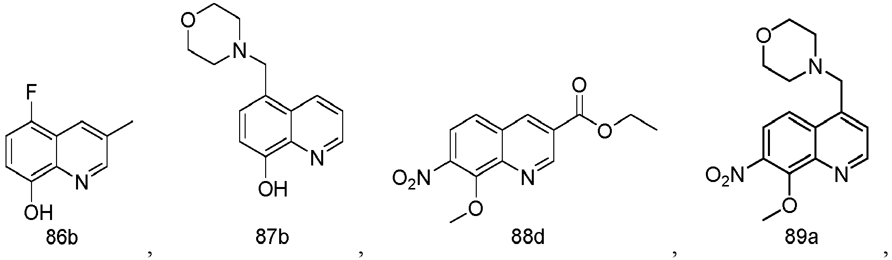 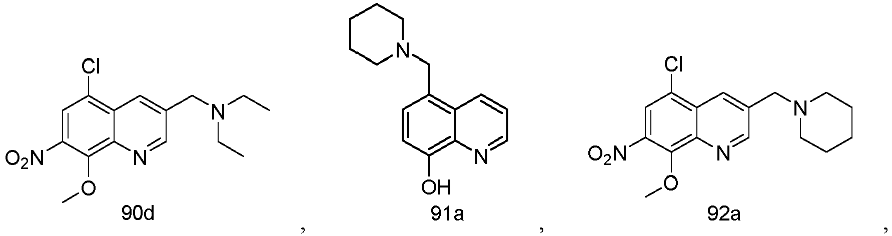 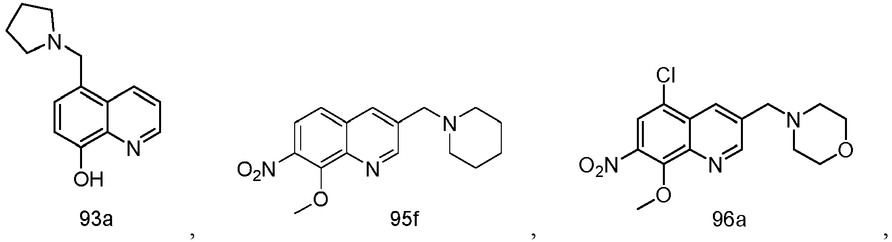 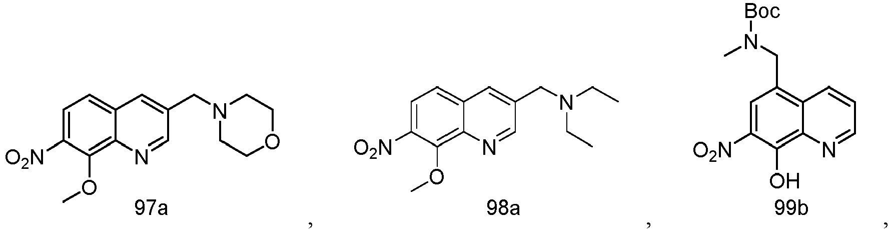 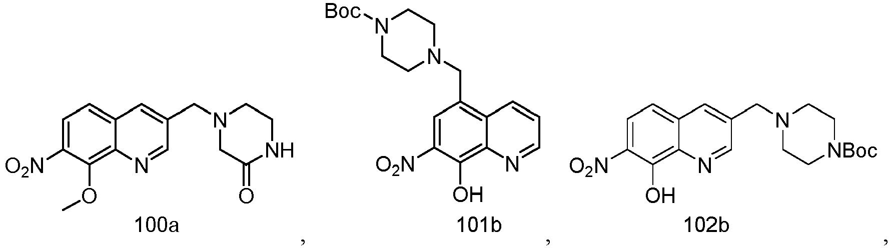 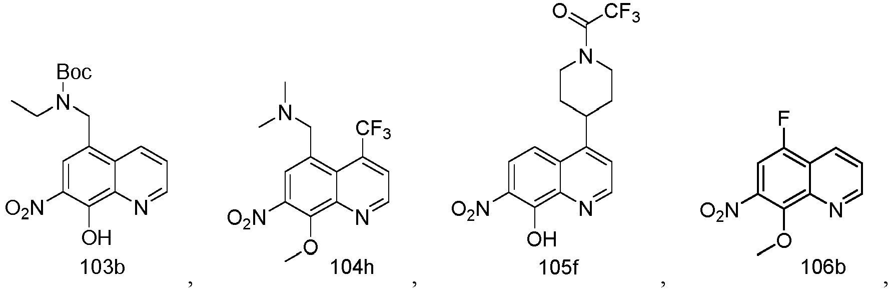 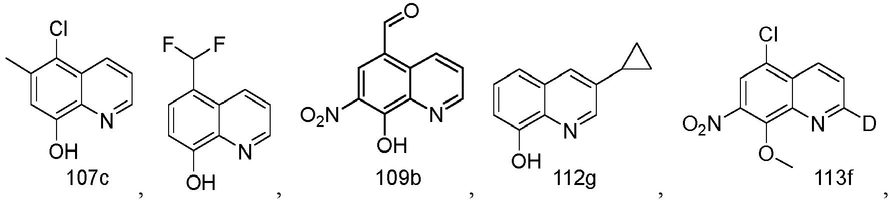 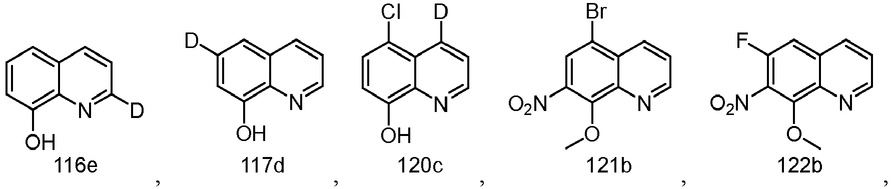 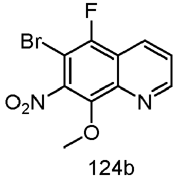 and

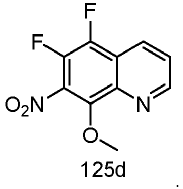

15. A method for preparing the compound of formula (III) according to claim 12, comprising the following step of:
method I: 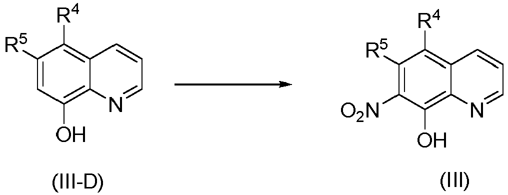
reacting a compound of formula (III-D) in the presence of a nitrite and acid to obtain the compound of formula (III); wherein, the nitrite is preferably one or more of sodium nitrite, potassium nitrite and calcium nitrite, and more preferably sodium nitrite; the acid is preferably one or more of hydrochloric acid, acetic acid and trifluoroacetic acid, and more preferably hydrochloric acid;
in method I, preferably, 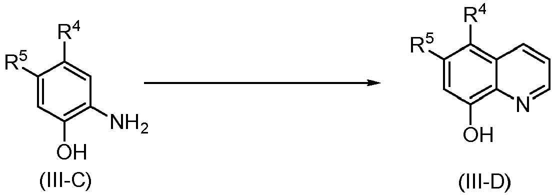
reacting a compound of formula (III-C) in the presence of glycerol or acrolein diethyl acetal and acid and oxidizing agent to obtain the compound of formula (III-D);
in method I, further preferably, 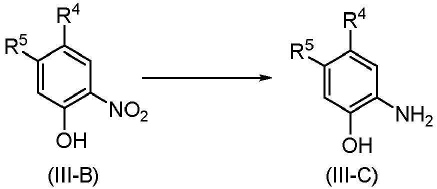
reacting a compound of formula (III-B) in the presence of a solvent and reducing agent to obtain the compound of formula (III-C); wherein, the reducing agent is preferably iron or Na₂S₂O₄;
in method I, more further preferably, 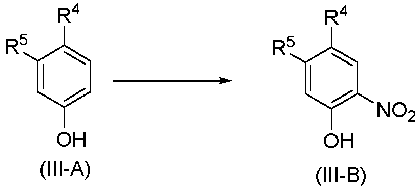
reacting a compound of formula (III-A) in the presence of a solvent and HNO₃ to obtain the compound of formula (III-B); wherein, the solvent is preferably 1,2-dichloroethane; the reaction is preferably carried out in a phase transfer catalyst, and the phase transfer catalyst is preferably tetrabutylammonium bromide;
or,
method II: 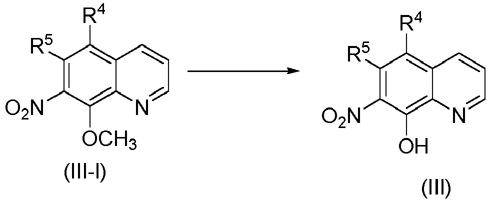 reacting a compound of formula (III-I) in the presence of a solvent and LiCl at 80 to 130°C to obtain the compound of formula (III); wherein, the solvent is preferably N,N-dimethylformamide;
in method II, preferably, 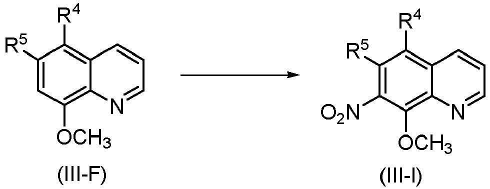 reacting a compound of formula (III-F) in the presence of a solvent, HNO₃ and sulfuric acid to obtain the compound of formula (III-I); wherein, the solvent is preferably acetic anhydride;
in method II, further preferably, 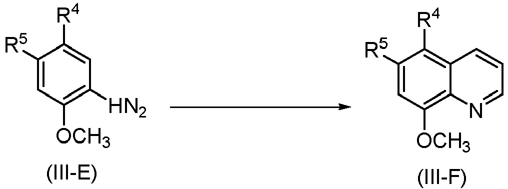 reacting a compound of formula (III-E) in the presence of glycerol or acrolein diethyl acetal and acid and oxidizing agent to obtain the compound of formula (III-F);
or,
method III: 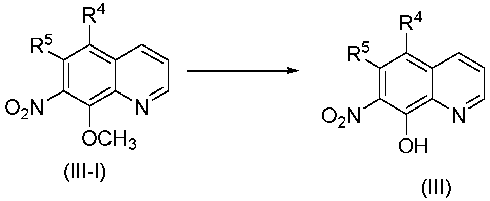 reacting a compound of formula (III-I) in the presence of a solvent and LiCl at 80 to 130°C to obtain the compound of formula (III); wherein, the solvent is preferably N,N-dimethylformamide;
in method III, preferably, 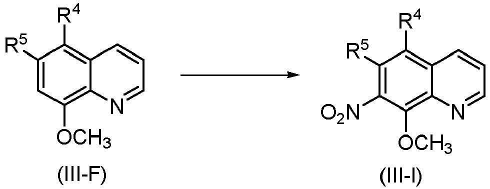 reacting a compound of formula (III-F) in the presence of a solvent, HNO₃ and sulfuric acid to obtain the compound of formula (III-I); wherein, the solvent is preferably acetic anhydride;
in method III, further preferably, 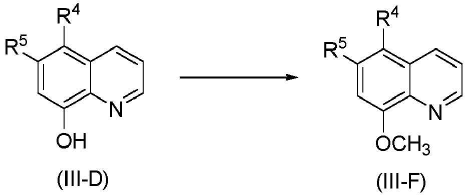 reacting a compound of formula (III-D) in the presence of a solvent, methylating reagent and base to obtain the compound of formula (III-F); wherein, the methylating reagent is preferably CH₃I or dimethyl sulfate; the base is preferably an inorganic base, and more preferably potassium carbonate, sodium carbonate;
in method III, more further preferably, 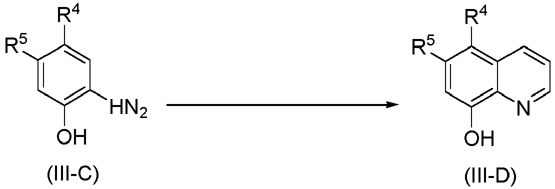 reacting a compound of formula (III-C) in the presence of glycerol or acrolein diethyl acetal and acid and oxidizing agent to obtain the compound of formula (III-D);
in method III, still more further preferably, 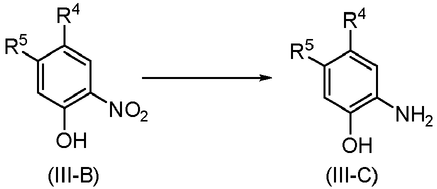 reacting a compound of formula (III-B) in the presence of a solvent and reducing agent to obtain the compound of formula (III-C); wherein, the reducing agent is preferably iron or Na₂S₂O₄.

16. A pharmaceutical composition, comprising the compound of formula (I), or or the tautomer, the stereoisomer, the mesomer, the racemate, the enantiomer, the diastereoisomer thereof, or the mixture thereof, or the pharmaceutically acceptable salt thereof according to any one of claims 1 to 13, and one or more pharmaceutically acceptable excipients.

17. Use of the compound of formula (I), or or the tautomer, the stereoisomer, the mesomer, the racemate, the enantiomer, the diastereoisomer thereof, or the mixture thereof, or the pharmaceutically acceptable salt thereof according to any one of claims 1 to 13, or the pharmaceutical composition according to claim 16 in the preparation of a medicament for treating infectious disease or cancer,
wherein, the infectious disease is preferably a systemic infection, reproductive system infection or urinary system infection; particularly, the infectious disease is induced by Gram-negative bacteria or Gram-positive bacteria; more particularly, the Gram-negative bacteria include *Escherichia coli, Acinetobacter baumannii* and *Klebsiella pneumoniae,* the *Escherichia coli* is preferably carbapenem-resistant *Escherichia coli,* the *Acinetobacter baumannii* is preferably carbapenem-resistant *Acinetobacter baumannii;* the Gram-positive bacteria include *Staphylococcus aureus,* the *Staphylococcus aureus* is preferably methicillin-resistant *Staphylococcus aureus* and/or methicillin-sensitive *Staphylococcus aureus*;
wherein, the cancer is preferably bladder cancer or prostate cancer.
